# FASCICULE DE BREVET EUROPEEN

(11) **EP 0 487 411 B1**
(45) Date de publication et mention de la délivrance du brevet: **31.01.1996**
(21) Numéro de dépôt: 91403119.0
(22) Date de dépôt: 20.11.1991
(51) Int. Cl.: C07H 17/08, A61K 31/70

(54) **Nouveaux dérivés de l'érythromycine, leur procédé de préparation, les nouveaux intermédiaires obtenus et leur application comme médicaments**
Erythromycinderivate, ihre Verfahren zur Herstellung, Zwischenprodukte und ihre Anwendung als Medikamente
Erythromycin derivatives, their preparation, intermediates obtained and their application as medicaments

(30) Priorité: 21.11.1990 FR 9014499; 27.05.1991 FR 9106333; 29.08.1991 FR 9110728
(43) Date de publication de la demande: 27.05.1992
(73) Titulaire: ROUSSEL UCLAF, F-93230 Romainville (FR)
(72) Inventeur: Agouridas, Constantin, F-94130 Nogent sur Marne (FR); Benedetti, Yannick, F-93110 Rosny sous Bois (FR); Chantot, Jean-François, F-77410 Gressy en France (FR); Denis, Alexis, F-75013 Paris (FR); Fromentin, Claude, F-75019 Paris (FR); Le Martret, Odile, F-75016 Paris (FR)
(74) Mandataire: Vieillefosse, Jean-Claude

(56) Documents cités:
- EP-A- 0 119 431
- EP-A- 0 194 833
- EP-A- 0 195 960
- EP-A- 0 216 169
- EP-A- 0 260 938
- EP-A- 0 284 203
- GB-A- 1 461 032
- US-A- 3 923 784
- JOURNAL OF MEDICINAL CHEMISTRY, vol. 17, no. 9, septembre 1974, pages 953-956, Washington, DC, US; R.A. LEMAHIEU et al.: Glycoside cleavage reactions on erythromycin A. Preparation of erythranolide A."
- ANTIMICROBIAL AGENTS AND CHEMOTHERAPY, vol. 9, no. 1, 1976, pages 128-130, Washington, DC, US; S. PESTKA et al.: Induction of erythromycin resistance in staphylococcus aureus by erythromycin derivatives"
- IDEM
- JOURNAL OF ANTIBIOTICS, vol. 43, no. 5, mai 1990, pages 570-573, Toyko, JP; S. MORIMOTO et al.: "Chemical modification of erythromycins VI. Structure and antibacterial activity of acid degradation products of 6-O-methylerythromycins A."
- IDEM
- JOURNAL OF THE AMERICAN CHEMICAL SOCIETY, vol. 103, no. 11, 3 juin 1981, pages 3215-3217, Gaston, PA, US; R.B. WOODWARD et al.: "Asymmetric total synthesis of erythromycin. 3. Total synthesis of erythromycin"
- Antimicrobial Agents and Chemotherapy, Oct. 1974, p.479-488
- Antimicrobial Agents and Chemotherapy, Oct. 1974, p. 489-491
- Antimicrobial Agents and Chemotherapy, Jan. 1976, p. 131-136

## Description

La présente invention concerne de nouveaux dérivés de l'érythromycine, leur procédé de préparation, les nouveaux intermédiaires obtenus et leur application comme médicaments.

On connaissait déjà des dérivés de l'érythromycine ne portant pas de cladinose en position 3 et portant en 6 un radical hydroxyle (cf Journal of Med. Chem., vol. 17, n° 9, 1974, pages 953, 956, USA - 3 923 784).

L'invention a pour objet les composés de formule (I) : 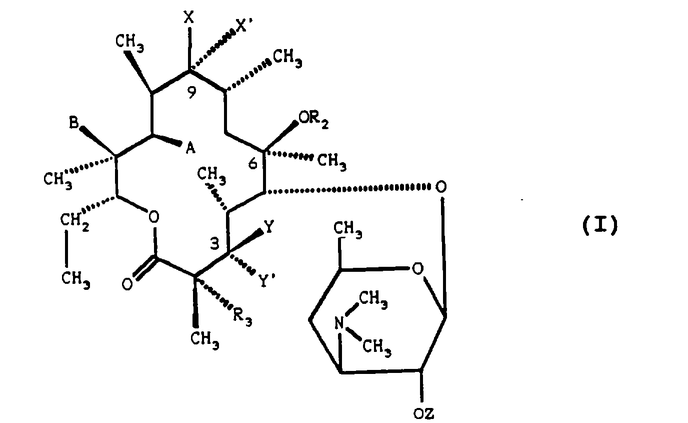
dans laquelle, **ou bien** X et X' forment ensemble avec l'atome de carbone auquel ils sont liés un groupement C=O ou C=NOR, dans lequel R représente :
- un atome d'hydrogène,
- un radical hétérocyclique renfermant au moins un atome d'azote et éventuellement un autre hétéroatome, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- un radical alkyle, alkényle ou alkynyle linéaire, ramifié ou cyclique, renfermant jusqu'à 18 atomes de carbone éventuellement substitué par un ou plusieurs groupements :
   . hydroxyle,
   . halogène,
   . cyano,
   . nitro,
   . amidinyle,
   . guanidinyle,
   . hétérocyclique, tel que défini précédemment,
   . alkyloxy, alkényloxy ou alkynyloxy ayant au plus 6 atomes de carbone,
   . alkylthio, alkénylthio ou alkynylthio ayant au plus 6 atomes de carbone, l'atome de soufre étant éventuellement oxydé en sulfoxyde ou en sulfone,
   . aryle, aralkyle,
   . aryloxy, aralkyloxy,
   . arylthio, aralkylthio, l'atome de soufre étant éventuellement oxydé en sulfoxyde ou en sulfone, (chacun de ces radicaux alkyloxy, alkényloxy, alkynyloxy, alkylthio, alkénylthio ou alkynylthio, aryle, aralkyle, aryloxy, aralkyloxy, arylthio ou aralkylthio étant éventuellement substitué par un ou plusieurs des groupements suivants : hydroxy, alkyloxy, alkylthio ayant de 1 à 6 atomes de carbone, alkénylthio, alkynylthio ayant jusqu'à 6 atomes de carbone, amino, monoalkylamino ayant jusqu'à 6 atomes de carbone, dialkylamino ayant jusqu'à 12 atomes de carbone, un radical amidinyle, guanidinyle, un radical hétérocyclique tel que défini précédemment, les radicaux aryle, aryloxy, arylthio, aralkyle, aralkyloxy et aralkylthio étant de plus éventuellement substitués par les radicaux méthyle, éthyle, propyle, carbamoyle, aminométhyle, diméthylaminométhyle, aminoéthyle, diméthylaminoéthyle, carboxyle, méthyloxycarbonyle, éthyloxycarbonyle)
   . 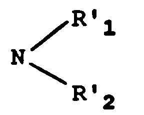 dans lequel :
      **ou bien R'₁ et R'₂** identiques ou différents représentent un atome d'hydrogène, un radical alkyle, alkényle ou alkynyle, linéaire, ramifié ou cyclique renfermant jusqu'à 18 atomes de carbone, un radical aryle ou aralkyle, chacun de ces radicaux R'**₁** et R'₂ étant éventuellement substitué par un ou plusieurs radicaux hydroxy, alkyloxy, alkényloxy, alkynyloxy, alkylthio, alkénylthio ou alkynylthio renfermant jusqu'à 8 atomes de carbone, amino, monoalkylamino renfermant jusqu'à 4 atomes de carbone, dialkylamino renfermant jusqu'à 8 atomes de carbone, cyano, carboxyle libre, estérifié ou salifié, acyle ou carbamoyle, renfermant jusqu'à 8 atomes de carbone, par un radical Si(alc)₃ ou Si(Oalc)₃ dans lequel alc représente un radical alkyle renfermant jusqu'à 4 atomes de carbone, par un radical hétérocyclique tel que défini précédemment,
      **ou bien R'₁ et R'₂** forment ensemble avec l'atome d'azote auquel ils sont liés un radical hétérocycle mono ou bicyclique, renfermant éventuellement un autre hétéroatome, saturé ou insaturé, aromatique ou non aromatique, comportant jusqu'à 12 chaînons ;
   . un groupement ammonium quaternaire,
   . 1,2-époxyéthyle ou 2,2-diméthyl 1,2-époxyéthyle ou un radical résultant de l'ouverture de ce groupement par un réactif nucléophile,
   . 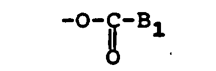 dans lequel B₁ représente soit un radical alkyle ou alkyloxy ayant au plus 6 atomes de carbone, soit un radical aryle, aralkyle, aryloxy ou aralkyloxy,
   . formyle libre ou protégé, carboxyle libre, estérifié ou salifié, thiocyanate, acyle ou carbamoyle,
   . (CH₂)ₙR', R' représentant le reste d'un acide aminé, et n représentant un nombre entier compris entre 0 et 6,

   **ou bien X** représente un radical 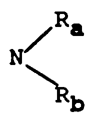
- Rₐ et R_{b} identiques ou différents l'un de l'autre, représentant un atome d'hydrogène ou un radical hydrocarboné renfermant jusqu'à 18 atomes de carbone, renfermant éventuellement un ou plusieurs hétéroatomes, éventuellement substitué par un ou plusieurs groupements fonctionnels, ou par un radical hétérocyclique renfermant au moins un atome d'azote et éventuellement un autre hétéroatome choisi parmi l'oxygène, le soufre et l'azote, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- Rₐ et R_{b} pouvant éventuellement former avec l'atome d'azote auquel ils sont liés un radical hétérocycle, renfermant au moins un atome d'azote et éventuellement un autre hétéroatome choisi parmi l'oxygène, le soufre et l'azote, mono ou bicyclique, saturé ou insaturé, aromatique ou non aromatique comportant jusqu'à 12 chaînons,
- Rₐ et R_{b} pouvant former avec le radical A un cycle 9-N, 11-O,

**et X'** représente un atome d'hydrogène,
**Y et Y'** identiques ou différents de X et X' ont la signification de X et X'
**B** représente un atome d'hydrogène ou un radical OR₄, R₄ représentant un atome d'hydrogène ou forme avec A un radical carbonate ou carbamate,
**A** forme avec le carbone qui le porte et le carbone en 10, une double liaison,
**ou A** représente un radical OR'₄, R'₄ représentant un atome d'hydrogène ou forme avec B un radical carbonate,
**ou A** représente un radical 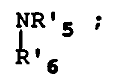
R'₅ représentant un groupement C=O formant avec B un groupement carbamate, R'₆ representant un atome d'hydrogène ou un radical alkyle, aralkyle ou alkyloxy ayant jusqu'à 12 atomes de carbone ou un groupement 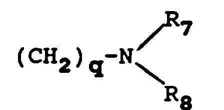
R₇ et R₈ identiques ou différents représentant un atome d'hydrogène ou un radical alkyle ou aralkyle, renfermant jusquà 18 atomes de carbone, ou formant avec l'atome d'azote un hétérocycle tel que défini précédemment, q représentant un nombre entier compris entre 1 et 6,
**ou A** représente un radical 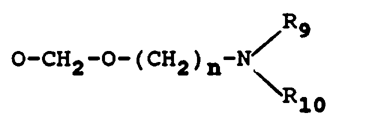
R₉ et R₁₀ représentant un atome d'hydrogène ou un radical alkyle renfermant jusqu'à 8 atomes de carbone, ou formant avec l'atome d'azote un hétérocycle tel que défini précédemment, n représentant un nombre entier compris entre 1 et 6,
R₂ représente un radical alkyle renfermant jusqu'à 8 atomes de carbone, ou un radical CONH₂ ou CONHCOR₁₁ ou CONHSO₂R₁₁ dans lesquels R₁₁ représente un radical hydrocarboné renfermant jusqu'à 18 atomes de carbone comportant éventuellement un ou plusieurs hétéroatomes,
R₃ en position alpha ou béta représente un atome d'hydrogène ou un radical alkyle, renfermant jusqu'à 8 atomes de carbone ou un radical 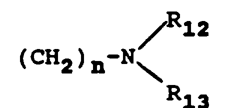 dans lequel R₁₂ et R₁₃ représente un atome d'hydrogène, un radical. alkyle renfermant jusqu'à 8 atomes de carbone ou formant avec l'atome d'azote un hétérocycle tel que défini précédemment n représentant un nombre entier compris entre 1 et 6 ou un radical 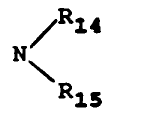
R₁₄ et R**₁₅** identiques ou différents représentant un atome d'hydrogène où un radical alkyle renfermant jusqu'à 8 atomes de carbone ou un hétéroatome ou un radical alkyle ou alkyloxy renfermant jusqu'à 8 atomes de carbone,
Z représente un atome d'hydrogène ou le reste d'un acide carboxylique renfermant jusqu'à 18 atomes de carbone les oximes que peuvent représenter X et X' ou Y et Y' peuvent être de configuration syn ou anti ainsi que les sels d'addition avec les acides des composés de formule (I).

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluèneaulfonique, chlorhydrique, bromhydrique, iodhydrique, sulfurique, phosphorique et spécialement les acides stéarique, éthylsuccinique ou laurylsulfurique.

Dans la définition des produits de l'invention :
- le radical hétérocyclique est de préférence le radical pyrrolyle, pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle, aziridinyle. On peut naturellement citer de préférence les radicaux hétérocycliques mentionnés ci-après dans la partie expérimentale,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, cyclobutyle, cyclopentyle ou cyclohexyle,
- l'halogène est de préférence le fluor ou le chlore, ou le brome,
- le radical aryle est de préférence le radical phényle,
- le radical aralkyle est de préférence un radical (C**₆**H₅)-(CH₂)ₐ, a étant un nombre entier compris entre 1 et 6, par exemple le nombre 1, 2, 3 ou 4 ou un radical naphtyle.
- le radical alkyloxy est de préférence un radical méthoxy, éthoxy, propyloxy, isopropyloxy, n-butyloxy, isobutyloxy, tert-butyloxy, n-pentyloxy, isopentyloxy, sec-pentyloxy, tertpentyloxy, néopentyloxy, n-hexyloxy, sec-hexyloxy, tert-hexyloxy.
- le radical alkylthio correspondant peut être utilisé en reprenant les mêmes valeurs et en remplaçant l'atome d'oxygène par un atome de soufre, exemple : méthylthio, éthylthio .... De plus, l'atome de soufre peut être oxydé, exemple : méthylsulfinyle, méthylsulfonyle .... le radical alkényloxy est de préférence un radical vinyloxy, 1-propényloxy, allyloxy, 1-butényloxy, 2-butényloxy, 3-butényloxy, 2-méthyl-1-butényloxy, pentényloxy, hexényloxy, 3-méthyl 2-butényloxy.
- le radical alkénylthio correspondant peut être utilisé en reprenant les mêmes valeurs et en remplaçant l'oxygène par un soufre éventuellement oxydé.
- le radical alkynyloxy est de préférence un radical éthynyloxy, propargyloxy, propynyloxy, butynyloxy, pentynyloxy, hexynyloxy.
- le radical alkynylthio correspondant peut être utilisé en reprenant les mêmes valeurs et en remplaçant l'oxygène par un soufre éventuellement oxydé.
- le radical aryloxy est de préférence un radical phényloxy, thiényloxy, furyloxy, thiazolyloxy, thiadiazolyloxy, oxazolyloxy, tétrazolyloxy, pyrrolyloxy, imidazolyloxy, pyrazolyloxy, isothiazolyloxy, isoxazolyloxy, triazolyloxy, thiatriazolyloxy, pyridyloxy, ainsi que les groupements condensés suivants :
   benzothiényloxy, benzofuryloxy, indolyloxy, benzimidazolyloxy.
- Les groupements arylthio éventuellement oxydés correspondants peuvent bien entendu être utilisés, par exemple : phénylthio, phénylsulfonyle, phénylsulfinyle ...
- le radical aralkyloxy est de préférence un radical benzyloxy, phényléthyloxy, phénylpropyloxy, thiénylméthyloxy, thiényléthyLoxy, thiénylpropyloxy, furfuryloxy, furyléthyloxy, furylpropyloxy, thiazolylméthyloxy, thiazolyléthyloxy, tétrazolylméthyloxy, thiadiazolylméthyloxy, thiadiazolyléthyloxy.
- Les groupements aralkylthio éventuellement oxydés correspondants peuvent bien entendu être utilisés.

Parmi les radicaux formyle protégés, on peut citer plus spécialement les radicaux du type acétal. On préfère les radicaux suivants : 1,3-dioxolan-2-yle, diméthoxyméthyle, diéthoxyméthyle.

Comme radicaux carboxyle estérifiés, on peut citer les radicaux alcoxycarbonyle ayant au plus 7 atomes de carbone tels que méthoxycarbonyle, éthoxycarbonyle, propyloxycarbonyle, isopropyloxycarbonyle, butyloxycarbonyle.

On peut également citer les radicaux alkyloxyalkyloxycarbonyle tels que méthoxyméthoxycarbonyle, isopropyloxyméthoxycarbonyle, les radicaux alkylthiométhoxycarbonyle tels que méthylthiométhoxycarbonyle, isopropylthiométhoxycarbonyle, les radicaux acyloxyalkyloxycarbonyle tel que pivaloyloxyméthoxycarbonyle, acétoxyéthoxycarbonyle.

Parmi les sels formés avec le groupement carboxyle, on peut citer les sels de sodium, potassium, lithium, calcium, magnésium, ammonium ou les sels formés avec les bases organiques aminées telles que la triméthylamine, la diéthylamine, la triéthylamine, le tris(hydroxyméthyl) aminométhane.

Parmi les radicaux acyle, on peut citer notamment les radicaux acétyle, propionyle, butyryle, isobutyryle, n-valéryle, isovaléryle, tert-valéryle et pivalyle.

L'invention a plus particulièrement pour objet les composés de formule (I) dans lesquels X et X' forment ensemble avec l'atome de carbone auquel ils sont liés un groupement C=NOR, R conservant la même signification que précédemment.

Parmi ces composés, l'invention a notamment pour objet, les composés de formule (I) dans lesquels R représente un radical alkyle renfermant jusqu'à 6 atomes de carbone substitué par un radical 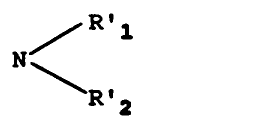 R'₁ et R'**₂** conservant la même signification que précédemment, par exemple ceux dans lesquels le radical R est un radical 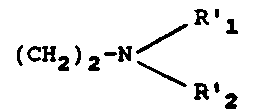 R'**₁** et R'**₂** représentant un radical alkyle, renfermant jusqu'à 4 atomes de carbone et tout spécialement un radical (CH**₂**)₂-N(CH**₃**)**₂**,
ou R'**₁** représente un atome d'hydrogène et R'**₂** représente un radical alkyle renfermant jusqu'à 4 atomes de carbone substitué par un radical hétérocycle renfermant au moins un atome d'azote, et tout spécialement un radical

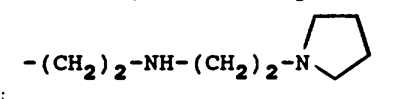

Parmi les composés préférés de l'invention, on peut également citer les composés dans lesquels R représente un radical alkyle renfermant jusqu'à 6 atomes de carbone, substitué par un radical alkyloxy renfermant jusqu'à 6 atomes de carbone éventuellement substitué par un radical méthoxy, par exemple ceux dans lesquels R représente un radical :

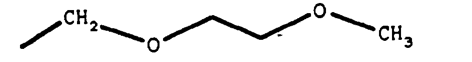

Parmi les composés préférés de l'invention, on peut encore citer les composés dans lesquels R représente un hétérocycle renfermant au moins un atome d'azote et notamment un radical 3-pipéridinyle.

L'invention a également particulièrement pour objet les composés de formule (I) dans lesquels X et X' forment ensemble avec l'atome de carbone auquel ils sont liés un groupement C=O.

Parmi les composés préférés de l'invention, on peut citer :
- les composés de formule (I) dans lesquels X et X' et Y et Y' forment ensemble un groupement C=O,
- les composés de formule (I) dans lesquels Y et Y' forment ensemble un groupement C-NOR, R conservant sa signification précédente et notamment un radical benzyle,
- les composés de formule (I) dans lesquels R**₂** représente un radical alkyle renfermant de 1 à 4 atomes de carbone, par exemple un radical méthyle,
- les composés de formule (I) dans lesquels R**₃**, représente un atome d'hydrogène, (alpha ou bêta),
- les composés de formule (I) dans lesquels A représente un radical OH,
- les composés de formule (I) dans lesquels B représente un radical OH,
- les composés de formule (I) dans lesquels A et B forment ensemble un groupement 11,12 carbonate cyclique,
- les composés de formule (I) dans lesquels A et B forment ensemble un radical 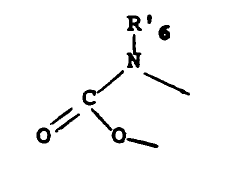 R'**₆** représentant un atome d'hydrogène ou un radical alkyle, aralkyle ou alkyloxy ayant jusqu'à 12 atomes de carbone ou un groupement 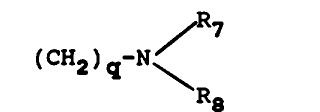 R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6.

Parmi ces composés, on peut citer plus particulièrement les composés dans lesquels R'**₆** représente un radical aralkyle comportant jusqu'à 12 atomes de carbone par exemple un radical (CH**₂**)**₄**C**₆**H**₅**.

Parmi les composés préférés de l'invention, on peut citer :
- les composés de formule (I) dans lesquels Z représente un atome d'hydrogène,

Parmi les composés préférés de l'invention, on peut citer les composés décrits dans la partie expérimentale et notamment les produits des exemples 1, 2, 3, 7, 10, 13, 36 et 37.

Les produits de formule générale (I) possèdent une très bonne activité antibiotique sur les bactéries gram^{**⊕**} telles que les staphylocoques, les streptocoques, les pneumocoques.

Les composés de l'invention peuvent donc être utilisés comme médicaments dans le traitement des infections à germes sensibles et, notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aigües primitives ou post-grippales, bronchopneumonies, suppuration pulmonaires, les streptococcies telles que les angines aigües, les otites, les sinusites, la scarlatine, les pneumococcies telles que les pneumonies, les bronchites ; la brucellose, la diphtérie, la gonococcie. Les produits de la présente invention sont également actifs contre les infections dues à des germes comme Haemophilus influenzae, Rickettsies, Mycoplasma pneumoniae, Chlamydia, Legionella, Ureaplasma, Toxoplasma.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits préférés de formule (I) définis précédemment à savoir les produits des exemples 1, 2, 3, 7, 10, 13, 36 et 37 ainsi que leurs sels pharmaceutiquement acceptables.

L'invention a également pour objet les compositions pharmaceutiques renfermant comme principe actif au moins un des médicaments défini ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'homme, avec le produit décrit à l'exemple 1.

L'invention a également pour objet un procédé de préparation des composés de formule (I) tels que définis précédemment, caractérisé en ce que l'on soumet un composé de formule (II) : 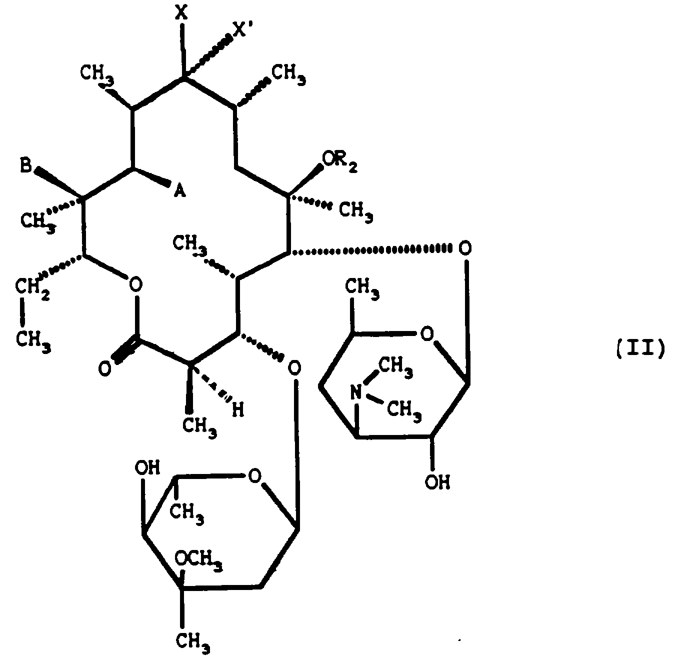 dans laquelle X, X', B et A conservent leur signification précédente, à l'action d'un acide en milieu aqueux pour obtenir le composé de formule (III) : 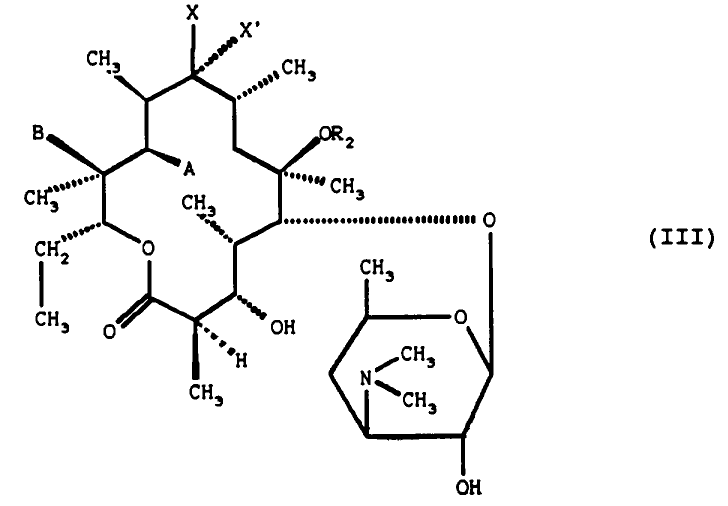 que l'on soumet à l'action d'un agent de blocage de la fonction hydroxyle en 2', pour obtenir un composé de formule (IV) : 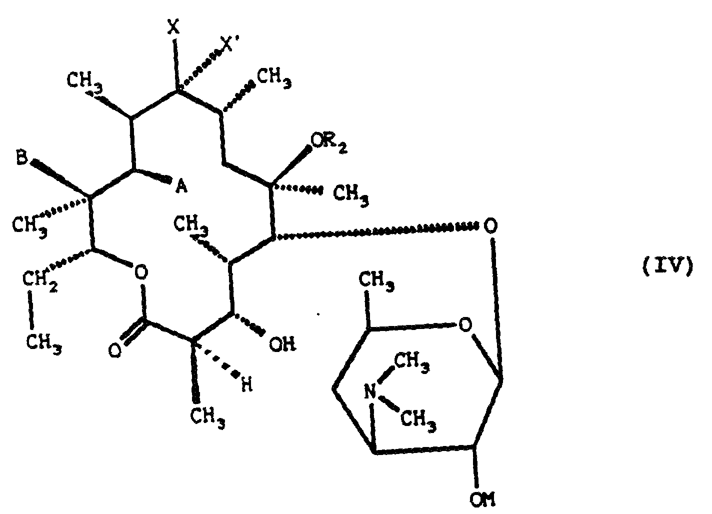 dans laquelle OM représente un groupement hydroxyle bloqué, et les autres substituants conservent leur signification précédente, que l'on soumet à l'action d'un agent d'oxydation de la fonction hydroxyle en 3, pour obtenir le composé de formule (V) : 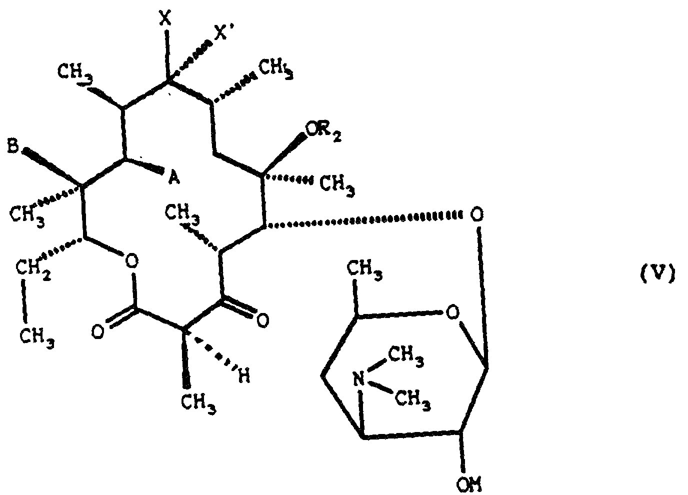 que l'on soumet, si désiré, à l'action d'un réactif susceptible d'introduire le radical R'**₃**, R'**₃** ayant la même valeur que R₃ à l'exception de l'hydrogène, puis ou bien le cas échéant à l'action d'un agent de libération de la fonction hydroxyle en 2' pour obtenir le composé de formule (I_{**A**}) : 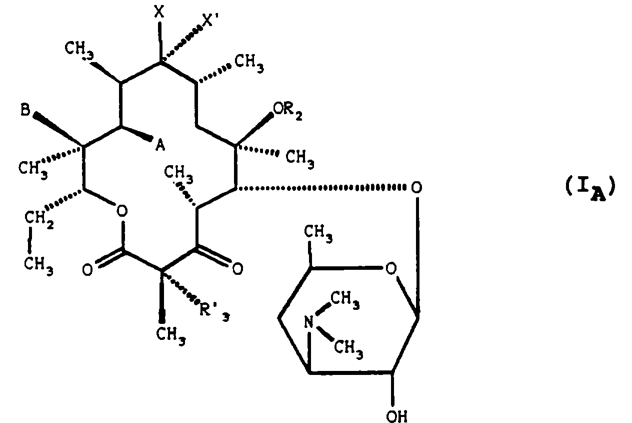 c'est-à-dire un composé de formule (I) dans laquelle Y et Y' forment ensemble avec l'atome de carbone auquel ils sont liés une fonction cétone, puis soumet si désiré, ce composé de formule (I_{**A**}) à l'action d'un agent d'oximation de la cétone ou béta-céto ester pour obtenir le composé de formule (I) recherché, puis si désiré soumet le composé obtenu à l'action d'un agent d'estérification de l'hydroxyle en 2', ou bien d'abord à l'action d'un agent d'oximation la fonction cétone ou bêtacéto ester, et ensuite le cas échéant à l'action d'un agent de libération de la fonction hydroxyle en 2' pour obtenir le composé de formule (I) recherché puis si désiré soumet le composé de formule (I) ainsi obtenu à l'action d'un acide pour en former le sel.

Dans un mode de réalisation préféré du procédé de l'invention :
- l'hydrolyse du cladinose est réalisée au moyen de l'acide chlorhydrique aqueux ou dans le méthanol,
- le blocage de l'hydroxyle en 2' est réalisé en utilisant un acide ou un dérivé fonctionnel d'acide par exemple un anhydride d'acide, un halogénure d'acide, ou des dérivés du silicium; selon l'agent de blocage utilisé, les produits de formule (V) peuvent constituer ou non des produits de formule (I).
- l'introduction du radical R'**₃** est effectuée par des méthodes connues de l'homme du métier par exemple au moyen d'un halogénure.
- la libération de la fonction hydroxyle en 2' est effectuée par méthanolyse,
- l'oxydation de l'hydroxyle en 3 est effectuée en utilisant soit l'anhydride chromique dans l'acide sulfurique dilué selon la réaction d'oxydation de Jones, soit des diimides en présence de diméthylsulfoxyde (DMSO),
- l'oximation de la fonction cétone peut être réalisée en une seule étape au moyen d'une hydroxylamine RONH**₂** portant le substituant R désiré soit au moyen d'une hydroxylamine H**₂**N-O-(CH₂)_{**n**}-Hal pour obtenir un composé de formule (I) dans laquelle 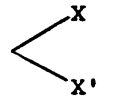 représente le groupement =N-O-(CH**₂**)_{**n**}-Hal, que l'on soumet si désiré à l'action d'une amine de formule 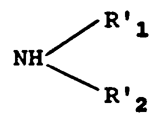 dans laquelle R'**₁** et R'**₂** ont la signification déjà indiquée, pour obtenir un composé de formule (I) dans laquelle 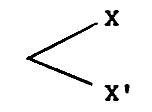 représente le groupement 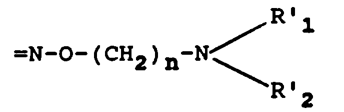 que le cas échéant l'on transforme, au moyen par exemple d'un agent d'alkylation, d'acylation, de réduction pour obtenir le composé de formule (I) désiré,
- l'estérification en 2' est réalisée selon les procédés classiques,
- la salification est réalisée au moyen d'acides selon les procédés classiques.

Les composés de formule (II) utilisés comme produits de départ sont préparés à partir des produits connus décrits dans les brevets européens 0216169, 41355 et 0180415 en utilisant les procédés décrits ci-après dans la partie expérimentale.

L'oxime de 6-O-méthyl érythromycine est décrite par exemple dans EP 0180415.

L'invention a également pour objet une variante du procédé précédent où les différentes étapes sont effectuées dans un ordre différent.

L'invention a en outre une variante du procédé décrit précédemment pour préparer les produits de formule (I) dans lesquels X et X' forment ensemble un groupement C=NOR, caractérisé en ce que le produit de formule (IV_{**A**}) utilisé dans lequel X et X' représentent le groupement C=N-OR est préparé à partir de la cétone de formule (II) correspondante par action de NH**₂**OR en milieu acide, pour obtenir selon le pH de la réaction le produit de formule (IV_{**A**}) correspondant saturé ou insaturé en 10(11) : 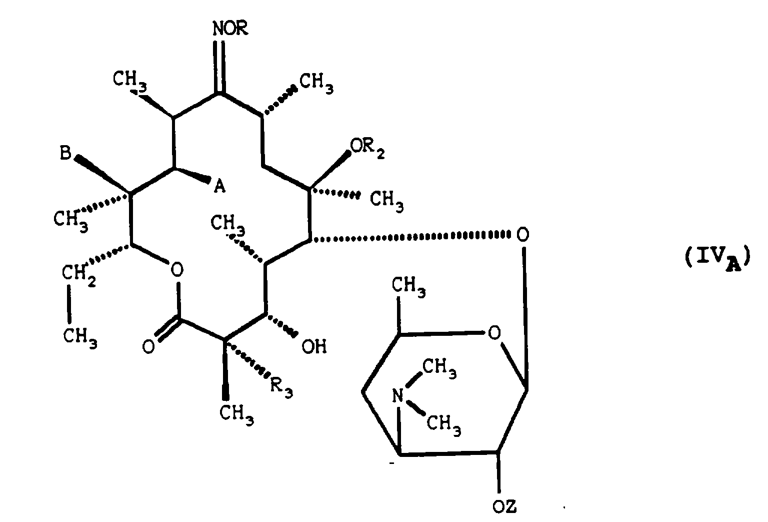 A représentant un radical OH s'il n'y a pas d'insaturation en 10(11) ou représentant un atome d'hydrogène s'il y a une insaturation en 10(11), R, R**₂**, B et Z conservant la même signification que précédemment.

L'invention a enfin pour objet une variante du procédé pour préparer les composés de formule (I) dans lesquels X et X' forment un groupement C=NOR, R étant défini comme précédemment, caractérisé en ce que l'on soumet un composé de formule (I_{**A**}) dans lequel X et X' forment ensemble un groupement céto à l'action du composé de formule NH**₂**OR pour obtenir le composé de formule (I) correspondant, dans lequel X et X' forment un groupement C=NOR et Z représente un atome d'hydrogène puis, le cas échéant, estérifie ou salifie.

Les produits intermédiaires IV et V obtenus lors de la mise en oeuvre du procédé de l'invention sont nouveaux et sont en eux-mêmes un objet de la présente invention. Les exemples suivants illustrent l'invention sans toutefois la limiter.

### Exemple 1 : 9-[O-[2-(diméthylamino) éthyl] oxime] de 3-dé[(2,6-didéoxy 3-C-méthyl 3-O-méthyl alpha-L-ribo-hexopyranosyl) ory] 6-O-méthyl 3-oxo érythromycine

### STADE A : 9-[O-[2-(diméthylamino) éthyl] oxime] de 3-O-dé(2,6-didéoxy 3-C-méthyl 3-O-méthyl alpha-L-ribo-hexopyranosyl) 6-O-méthyl érythromycine

On met en suspension dans 3 cm**³** d'eau, 100 mg de produit obtenu à la préparation 1 et 0,3 cm**³** d'une solution d'acide chlorhydrique à 22°Bé. On maintient le tout sous agitation pendant 3 heures à température ambiante.

On amène à pH basique en ajoutant quelques gouttes d'ammoniaque à 20 %, on ajoute 2 cm**³** d'une solution saturée de chlorure de sodium, extrait avec de l'acétate d'éthyle et du chloroforme. On sèche et évapore les solvants.

On chromatographie sur silice en éluant d'abord à l'acétate d'éthyle pur, puis avec le mélange acétate d'éthyle-triéthylamine (98/2).

On obtient ainsi 50 mg de produit recherché.

### ANALYSES

IR : (Nujol sur Nicolet)
CO : 1733 cm⁻¹
SM (FAB)
(M + H)**⁺** = 676⁺

### STADE B : 2′-O-acétyl 9-[O-[2-(diméthylamino) éthyl] oxime] 3-dé (2,6-didéoxy 3-C-méthyl 3-O-méthyl alpha-L-ribo-hexopyranosyl) 6-O-méthyl érythromycine

On met en suspension dans 2 cm**³** d'acétone, 150 mg du produit préparé au stade précédent, 38 mg de carbonate de potassium et 33 microlitres d'anhydride acétique. On agite la suspension ainsi obtenue pendant 20 heures.

On ajoute 1 cm**³** de glace, agite-pendant 5 minutes et sature avec du chlorure de sodium. On ajoute 1 cm**³** d'eau, extrait à l'acétate d'éthyle, sèche sur sulfate de magnésium et évapore les solvants. On obtient 110 mg de produit brut que l'on chromatographie sur silice, en éluant avec le mélange acétate d'éthyle-triéthylamine (96/4). On isola 110 mg de produit recherché.

### ANALYSES

SM (FAB)
(M + H)**⁺** = 718⁺

### STADE C : 9-[O-[2-(diméthylamino) éthyl] oxime] de 3-dé[(2,6-didéoxy 3-C-méthyl 3-O-méthyl alpha-L-ribo-hexopyranosyl) oxy] 6-O-méthyl 3-oxo érythromycine

### Stade C1 : Oxydation

On met en solution sous atmosphère d'argon, dans 2 cm**³** de chlorure de méthylène 110 mg du produit préparé au stade précédent, 0,21 cm**³** de diméthylsulfoxyde, 165 mg de chlorhydrate de 1-[3-(diméthylamino) propyl] 3-éthyl carbodiimide (EDAC). On maintient la solution sous agitation pendant 20 minutes et ajoute 165 mg de trifluoroacétate de pyridinium. On ajoute, au bout de 2 heures, 70 microlitres de diméthylsulfoxyde et 55 mg d'EDAC. On agite 20 minutes et ajoute 55 mg de trifluoroacétate de pyridinium.

On ajoute 2 cm**³** d'eau à la solution obtenue, on agite 10 minutes, reprend avec du chlorure de méthylène, lave à l'eau, sèche sur sulfate de magnésium et évapore les solvants. On obtient 240 mg de produit brut que l'on chromatographie sur silice en éluant avec le mélange acétate- d'éthyle, triéthylamine (95/5). On obtient 85 mg de produit recherché.

### Stade C2 : Libération de l'hydroxyle en 2'

On met en solution dans 3 cm**³** de méthanol 85 mg du produit obtenu précédemment. On agite pendant 24 heures. On évapore le solvant sous pression réduite. On obtient un produit que l'on purifie par chromatographie en éluant avec le mélange acétate d'éthyle-triéthylamine (95/5). On obtient 75 mg de produit recherché.

### ANALYSES

IR : (CHCl**₃** sur Nicolet)
OH : 3606, 3510, 3415 cm⁻¹
C=O : 1744, 1714 cm⁻¹
UV : max 287 nm ε=10900
SM : (FD)
M⁺ = 673⁺
RMN : (CCCl**₃**, 400 MHz, δ ppm)
3,86 (H₂), 3,12 (H**₄**), 4,31 (H₅), 1,39 (CH₃ en 6), 2,74 (OCH₃ en 6), 3,56 (H**₈**), 2,5-2,65 (H**₁₀**, CH**₂**-N), 3,89 (H**₁₁**), 1,22 (12-CH**₃**), 5,17 (H**₁₃**), 0,86 (H**₁₅**), 4,09 (O-CH**₂**-CH**₂**), 2,27 (CH**₃**-N), 0,97-1,16-1,26-1,3-1,32 (CH**₃**) , 4,31 (H'₁), 3,19 (H'₂), 2,5 (H'₃), 3,64 (H'**₅**). [alpha]_{**D**} = +4° (c - 0,5 % CHCl**₃**).

### PREPARATION 1 : 9-(O-[2-(diméthylamino) éthyl] oxima] de 6-O-méthyl érythromycine

On met en solution sous atmosphère d'azote dans 1,5 cm**³** de diméthylsulfoxyde, 160 mg de chlorhydrate de chloro 2-N,N-diméthylamine. On ajoute 60 mg d'hydrure de sodium à 50 % dans l'huile. On agite pendant 30 minutes sous atmosphère d'azote et ajoute 380 mg de 9-oxime de 6-O-méthyl érythromycine, 0,5 cm**³** de tétrahydrofuranne et 30 mg d'hydrure de sodium. On maintient la solution ainsi obtenue pendant 4 heures sous atmosphère d'azote.

On ajoute quelques gouttes d'une solution saturée de chlorure d'ammonium. On ajoute 20 cm**³** d'acétate d'éthyle, lave avec une solution aqueuse saturée en carbonate acide de sodium, puis avec une solution aqueuse saturée en chlorure de sodium. On sèche sur sulfate de magnésium. On évapore les solvants On obtient un résidu que l'on chromatographie sur silice en éluant avec un mélange de chloroforme, méthanol, ammoniaque 97/7/0,5. On obtient 200 mg de produit recherché. [alpha]_{**D**} = -99° (c = 1 %, chloroforme).
Spectre IR : (CHCl**₃**)
OH : 3600 cm⁻¹
C=O : 1728 cm⁻¹
C=N : 1626 cm⁻¹
Spectre de masse : (FAB)
(M + H)**⁺** = 834⁺

### EXEMPLE 2 : 9-(O-[(2-méthoxy éthoxy) méthyl] oxime] de 3-dé[(2,6-didéoxy 3-C-méthyl 3-O-méthyl alpha-L-ribo-hexopyranosyl) oxy] 6-O-méthyl 3-oxo érythromycine

### STADE A : 9-[O-[(2-méthoxy éthoxy) méthyl] oxime] de 3-O-dé(2,6-didéoxy 3-C-méthyl 3-O-méthyl alpha-L-ribo-hexopyranosyl) 6-O-méthyl érythromycine

En opérant comme à l'exemple 1, stade A à partir de 1,7 g du produit obtenu à la préparation 2, on a obtenu 1,25 g du produit recherché.
[alpha]_{**D**} = -28° ± 1,5° (c = 0,95 % CHCl**₃**).
Spectre IR : (CHCl₃ sur Nicolet)
OH : 3420 cm⁻¹
C=O : 1725 cm⁻¹
C=N : 1636 cm⁻¹
Spectre de masse : (FAB)
(M + H)**⁺** = 693⁺

### STADE B : 9-[O-[(2-méthoxy éthoxy) méthyl] oxime] de 2'-O-acétyL 3-dé(2,6-didéoxy 3-C-méthyl 3-O-méthyl alpha-L-ribo-hexopyranosyl) 6-O-méthyl érythromycine

En opérant comme à l'exemple 1 stade B, à partir de 346 mg du produit obtenu au stade A, on a obtenu 351 mg du produit recherché.

### ANALYSES

IR : (CHCl₃ sur Nicolet)
OH : 3620, 3600 cm⁻¹
C=O : 1730 cm⁻¹
SM : (FAB)
(M + H)**⁺** = 735⁺
[alpha]_{**D**} = -52,5° ± 1° (c = 1 % CHCl**₃**)

### STADE C : 9-[O-[(2-méthoxy éthoxy) méthyl] oxime] de 2'-O-acétyl 3-dé(2,6-didéoxy 3-C-méthyl 3-O-méthyl alpha-L-ribo-hexopyranosyl) oxy] 6-O-méthyl 3-oxo érythromycine.

On met en solution dans 100 cm**³** d'acétone, 4 g de produit préparé au stade B et 2,71 cm**³** de réactif de Jones. On agite le mélange réactionnel 1 heure à 0°C.

On ajoute 10 cm**³** de 1-propanol et poursuit l'agitation à 0°C durant 20 minutes. On évapore l'acétone sous pression réduite. On reprend le résidu avec 50 cm**³** de chlorure de méthylène et 20 cm**³** d'eau. On amène à pH 8 avec du carbonate de potassium. On extrait avec du chlorure de méthylène, lave à l'eau, sèche sur sulfate de magnésium, filtre puis évapore à sec sous pression réduite. On obtient 4,5 g de produit que l'on chromatographie sur silice (éluant : acétate d'éthyle-triéthylamine 98-2), on obtient 2,45 g du produit recherché. Spectre IR : (CHCl₃ sur Nicolet)
- C=N :: 1630 cm⁻¹
- C-O :: 1742, 1716 cm⁻¹
- OH :: 3510, 3410 cm⁻¹

### STADE D : 9-[O-[(2-méthoxy éthoxy) méthyl] oxime] de 3-dé[(2,6-didéoxy 3-C-méthyl 3-O-méthyl alpha-L-ribo-hexopyranosyl) oxy] 6-O-méthyl 3-oxo érythromycine

En opérant comme au stade C**₂** de l'exemple 1 , à partir de 300 mg du produit obtenu au stade C ci-dessus, on obtient 0,27 g du produit recherché.

### ANALYSES

IR : (CHCl**₃** sur Nicolet)
OH : 3430, 3505 cm⁻¹
C=O : 1744, 1714 cm⁻¹
RMN : (CDCl**₃**, 400 MHz**)**
0,86 ppm (CH**₃** éthyle), 1,00-1,17 ppm 1,26-1,30-1,32 ppm (CH**₃** non attribués), 1,23 ppm (CH**₃** en 12), 1,38 ppm (CH**₃** en 6), 2,37 ppm (N(CH**₃**)₂), 2,61 ppm (H**₁₀** et H₃,), 2,73 ppm (OCH**₃** en 6), 3,13 ppm (H**₄**), 3,26 ppm (H**₂**,), 3,38 ppm (OCH**₃** de MEM**),** 3,53 ppm, 3,76 ppm (OCH**₂**CH**₂**O de MEM), 3,59 ppm (H**₅**,), 3,70 ppm (H**₈**), 3,86 ppm (H**₂**), 3,91 ppm (H**₁₁**), 4,33 ppm (H**₁**, et H5), 5,14 ppm (OCH**₂**O), 5,18 ppm (H**₁₃**).
SM : pic moléculaire (M⁺) : 690⁺

### PREPARATION 2 : 9-[O-[(2-méthoxy éthoxy) méthyl] oxime] de 6-O-méthyl érythromycine

A une solution de 15,2 g de 9-oxime de 6-O-méthyl érythromycine dans 80 cm**³** de tétrahydrofuranne, on ajoute, à +5°C, 1,35 g de méthylate de sodium, on agite 15 minutes à +5°C puis ajoute en 1 heure : 2,85 cm**³** de chlorure de (2méthoxy éthoxy) méthyle en solution dans 20 cm**³** de tétrahydrofuranne ; on agite 30 minutes à +5°C puis laisse revenir à température ambiante. On évapore le tétrahydrofuranne sous pression réduite, reprend le résidu avec du chlorure de méthylène, lave à l'eau, sèche et évapore à sec. On chromatographie le résidu (16,1 g) sur silice (éluant : chlorure de méthylèneméthanol-ammoniaque 95-5-0,1) on obtient une première fraction de 8,1 g puis une seconde fraction de 3,56 g du produit recherché.

### Spectre IR (CHCl₃)

- OH :: 3600 cm⁻¹
- C-O :: 1728 cm⁻¹
- C-N :: 1630 cm⁻¹

Spectre de masse (FAB)
(M + H)**⁺** : 851⁺

### EXEMPLE 3 : 3-dé[(2,6-didéoxy 3-C-méthyl 3-O-méthyl alpha-L-ribo-hexopyrmnosyl) oxy] 6-O-méthyl 3-oxo érythromycine

### STADE A : 3-O-dé(2,6-didéoxy 3-C-méthyl 3-O-méthyl alpha-L-ribo-hexopyranosyl) 6-O-méthyl érythromycine

On met en suspension dans 3 cm**³** d'eau, 380 mg de 6-O-méthyl érythromycine. On ajoute 0,3 cm**³** d'acide chlorhydrique 22°Bé. On maintient le mélange réactionnel sous agitation pendant 2 heures.

On amère à pH basique (> 8) en ajoutant quelques gouttes d'ammoniaque à 20°C, puis dilue avec 5 cm**³** d'acétate d'éthyle. On sature la phase aqueuse avec du chlorure de sodium, décante et extrait à l'acétate d'éthyle. On sèche sur sulfate de magnésium et évapore le solvant. On obtient 350 mg de produit brut que l'on chromatographie sur silice, en éluant avec le mélange acétate d'éthyle-triéthylamine (96-4). On obtient 200 mg de produit recherché.

### Spectre IR (CHCl₃ sur NiCOlet)

OH : 3450 cm⁻¹
C-O : 1725, 1689 cm⁻¹

### Spectre de masse (FAB)

(M + H)**⁺**: 590⁺

### STADE B : 2′-O-acétyl 3-O-dé(2,6-didéoxy 3-C-méthyl 3-O-méthyl alpha-L-ribo-hexopyranosyl) 6-O-méthyl érythromycine

On met en solution sous agitation et sous atmosphère d'azote, dans 4 cm**³** d'acétone 310 mg du produit préparé au stade précédent, 80 microlitres d'anhydride acétique et 90 mg de carbonate de potassium. Au bout de 12 heures à la température ambiante, on ajoute 20 microlitres d'anhydride acétique et 10 mg de carbonate de potassium. On agite à nouveau 12 heures à température ambiante.

On ajoute de la glace, agite et extrait avec du chlorure de méthylène. On sèche sur sulfate de magnésium et évapore le solvant.

On chromatographie le produit obtenu sur silice en éluant avec le mélange acétate d'éthyle-triéthylamine (96-4). On obtient le produit recherché.

### ANALYSE :

### Spectre de masse (FAB)

(M + H)**⁺**: 631⁺

### STADE C : 3-dé[(2,6-didéoxy 3-C-méthyl 3-O-méthyl alpha-L-ribo-hexopyranosyl) oxy] 6-O-méthyl 3-oxo érythromycine Stade C1 : Oxydation

On met en solution dans 5 cm**³** de chlorure de méthylène, 420 mg de produit préparé au stade précédent, 0,84 cm**³** de diméthylsulfoxyde, 0,84 g de chlorhydrate de 1-[3-(diméthylamino) propyl] 3-éthyl carbodiimide. On agite la solution obtenue pendant 4 heures à la température ambiante.

On ajoute 4 cm**³** d'eau à la solution. On agite 10 minutes et reprend avec 20 cm**³** de chlorure de méthylène. On lave à l'eau. On sèche sur sulfate de magnésium et évapore les solvants.

On chromatographie le produit obtenu sur silice en éluant avec le mélange éther isopropylique-triéthylamine (9-1). On obtient 130 mg de produit recherché.

### Stace C2 : Hydrolyse

En opérant comme au stade C2 de l'exemple 1, à partir de 130 mg du produit préparé ci-dessus, après chromatographie sur silice (éluant : éther isopropylique-triéthylamine (9-1)), on obtient 100 mg de produit recherché.

### ANALYSES

IR : (CHCl**₃** sur Nicolet)
OH : 3475 cm⁻¹
C=O : 1745, 1714, 1689 cm⁻¹
SM : (M + H)**⁺** = 588⁺
RMN : (CDCl₃, 300 MHz, δ ppm)
3,86 (H**₂**), 2,6 (H**₄**), 1,35 (6-CH**₃**), 2,7 (6-OCH**₃**), 3,1 (H**₈**), 2,97 (H**₁₀**), 3,91 (H**₁₁**), 1,22 (H**₁₂**), 5,12 (H**₁₃**), 0,86 (H**₁₅**), 4,32 (H**₁**), 3,18 (H'**₂**), 2,46 (H'**₃**), 2,26 (N-CH**₃**), 3,57 (H'**₅**). [alpha]_{**D**} = +21° (c = 0,5 %, CHCl**₃**)

### EXEMPLE 4 : 9-[O-[(2-méthoxy éthoxy) méthyl] oxime] de 3-dé[(2,6-didéoxy 3-C-méthyl 3-O-méthyl alpha-L-ribo-hexopyra- nosyl) oxy] 6-O-méthyl 3-[(phénylméthoxy) imino] érythromycine

On met en solution dans 10 cm**³** d'éthanol, 500 mg de produit préparé à l'exemple 2. On ajoute 172 microlitres de triéthylamine et 543 mg de chlorhydrate de O-benzylhydroxylamine. On porte au reflux. On maintient le reflux pendant 4 jours. On ajoute 172 microlitres de triéthylamine et 543 mg de chlorhydrate de 0-benzyl hydroxylamine. On maintient le reflux pendant encore 3 jours. On filtre le milieu réactionnel. On évapore les solvants. On reprend le résidu au chlorure de méthylène et à l'eau. On neutralise avec de l'ammoniaque. On décante, extrait la phase aqueuse avec du chlorure de méthylène. On réunit les phases organiques, les lave à l'eau, sèche sur sulfate de magnésium, filtre et concentre à sec. On obtient 800 mg d'une huile que l'on chromatographie sur silice, en éluant d'abord à l'acétate d'éthyle seul, puis avec un mélange AcOEt/TEA (99-1). On récupère 500 mg d'un solide que l'on purifie par HPLC préparative.
Eluant : acétonitrile-acétate d'ammonium 0,2M (4-1)
On récupère : 130 mg du produit recherché.
RMN : (CDCl**₃**) 300 MHz
0,86 (t) ppm (CH**₃** éthyle), 0,90 à 1,45 (les autres méthyles), 2,27 (s) (N(Me)**₂**), 2,90 (dq) (H**₁₀**), ^{∼}2,30 (m) (H**₃**,), ^{∼}3,28 (m) (H**₄** ou H**₅**, blindé), 4,52 (q) (H₂), 2,70 (s) (6-OMe), 3,54 (m) et 3,76 (m) (OCH₂CH**₂**O de MEM), ^{**∼**}5,13 (OCH**₂**O de MEM et OCH2φ), 4,00 (s large) (H**₁₁**), 4,59 (d) (H**₁**,), 3,18 (dd) (H**₂**,), 4,01 (d) (H**₅**), 5,29 (dd) (H**₁₃**), ^{∼}7,31 (Phényle).
Spectre de masse : Pic moléculaire (M+H)⁺ = 796⁺
Spectre IR : (CHCl₃ sur Nicolet)

| | |
|---|---|
| OH : | ^{∼}3600 cm ⁻¹ + associé complexe |
| C=O : | 1730 cm ⁻¹ |
| C=N : | ^{∼}1636 cm⁻¹ |
| | 1606 cm⁻¹ |
| Aromatique : | 1494 cm⁻¹ |

### EXEMPLE 5 : 9-[O-[(2-méthoxy éthoxy) méthyl] oxime] de 3-dé[(2,6-didéoxy 3-C-méthyl 3-O-méthyl alpha-L-ribo-hexopyranosyl) oxy] 3-(hydroxyimino) 6-O-méthyl érythromycine

On met en solution dans 12,5 cm**³** d'éthanol, 110 mg du produit préparé à l'exemple 4. On ajoute 52 mg de palladium à 10 % sur charbon actif. On maintient sous pression d'hydrogène pendant 2 jours. On filtre. On évapore. On obtient un produit que l'on purifie par chromatographie sur silice en éluant avec le mélange éther isopropylique-méthanol-triéthylamine (90-5-5). On récupère 39 mg de produit recherché (1 isomère).

### RMN: (CDCl₃) 300 MHz

0,87 (t) ppm (CH**₃** éthyle), 0,99 (d) 1,18 (d) 1,26 (d) 1,40 (d) (les autres méthyles), 1,23 (s) (12-Me) , 1,36 (s) (6-Me), 2,23 (s) (N(Me)**₂**), 2,90 (dq) (H**₁₀**), ^{**∼**}2,30 (m) (H**₃**, + 1 autre H), 4,49 (q) (H**₂**), 2,86 (s) (6-OMe), 3,38 (s) (OMe de MEM), 3,54 (m) et 3,76 (m) (OCH**₂**CH**₂**O de MEM), 5,15 (OCH**₂**O de MEM), 4,56 (d) (H**₁**,), 3,27 (dd) (H**₂**,), 4,05 (s large) 4,20 (d) (H₅ et H**₁₁**), 5,31 (dd) (H**₁₃**), -3,31 ; 4,39 (s) 1,80 (H mobiles),
- SM :: Pic moléculaire M**⁺** = 705⁺
- IR :: (CHCl**₃** sur Nicolet)

| | |
|---|---|
| OH : | ^{∼}3590 cm ⁻¹ + associé complexe |
| C=O : | 1725 cm ⁻¹ |

### EXEMPLE 6 : 9-[O-(2-méthoxy éthoxy) méthyl] oxime] de 3-dé(2,6-didéoxy 3-C-méthyl 3-O-méthyl alpha-L-ribo-hexopyranosyl) oxy] 2-méthyl 6-O-méthyl 3-oxo érythromycine

### STADE A : 9-(O-[(2-méthoxy éthoxy) méthyl] oxime] de 2'-O-acétyl 3-dé[(2,6-didéoxy 3-C-méthyl 3-O-méthyl alpha-L-ribo-hexopyranosyl) oxy] 2-méthyl 6-O-méthyl 3-oxo érythromycine

On met en solution dans 1 cm³ de chlorure de méthylène, 0,1 g de produit préparé à l'exemple 2, stade C et 17 microlitres d'iodure de méthyle. On ajoute ensuite 0,046 g d'hydrogènosulfate de tétrabutylammonium, 0,20 cm³ d'eau et 0,27 cm³ d'une solution normale de soude. On agite pendant 5 heures. On extrait au chlorure de méthylène. On lave à l'eau. On réunit les phases organiques, les sèches. On filre. On évapore à sec sous pression réduite. On reprend à l'acétate d'éthyle et filtre. On évapore à sec le filtrat. On obtient 130 mg de produit que l'on chromatographie sur silice en éluant avec le mélange acétate d'éthyle-triéthylamine (98-2). On obtient 49 mg de produit recherché rf = 0,2.

### ANALYSES

### RMN : (CDCl₃, 300 MHz)

On note la disparition du proton en 2 et une modification du proton H₄.
0,85 ppm (CH₃(-CH**₂**)), 0,99 - 1,28 - 1,88 ppm (CH**₃**(-CH)), 1,25 - 1,36 - 1,50 ppm (CH**₃**(-C)), 2,03 ppm (OAc), 3,30 ppm (dq, J - 3 et 7 hz) (H**₄**), 3,4 à 3,8 ppm (OCH**₂**CH**₂**O).

### STADE B : 9-[O-[(2-méthoxy éthoxy) méthyl] oxime] de 3-dé[(2,6-didéoxy 3-C-méthyl 3-O-méthyl alpha-L-ribo-hexopyranosyl) oxy] 2-méthyl 6-O-méthyl 3-oxo érythromycine

On met en solution dans 5 cm³ de méthanol, 0,095 g de produit préparé comme au stade A. On agite 24 heures à température ambiante. On obtient 0,95 mg de produit que l'on purifie par chromatographie sur silice en éluant avec le mélange acétate d'éthyle-triéthylamine (98-2). On obtient 46 mg de produit recherché.

### ANALYSES :

### RMN : (CDCl₃, 300 MHz)

On note la disparition des protons du groupe "OAc". 0,85 ppm (CH**₃**(-CH**₂**)), 0,99 - 1,18 - 1,23 - 1,35 ppm (CH**₃**(-CH)), 1,26 - 1,32 - 1,37 - 1,52 ppm (CH**₃**(-C)), 2,82 ppm (6-OMe), 3,54 à 3,76 ppm (OCH**₂**CH**₂**O), 3,35 - 4,33 ppm (H mobiles).

### SM : (M+H)⁺: 705⁺

### EXEMPLE 7 :11,12-carbonate cyclique de 3-de[(2,6-dideoxy 3-C-méthyl 3-O-méthyl-alpha-L-ribo-hexopyranosyl) oxy] 6-O-méthyl 3-oxo érythromycine

### STADE A : 2'-acétate 11,12-carbonate cyclique de 3-0-de(2,6-dideoxy 3-C-méthyl 3-O-méthyl-alpha-L-ribo-hexopyranosyl) 6-O-méthyl érythromycine

A une suspension de 876 mg de 2'-acétate 4"-(phénylméthyl carbonate) 11,12-carbonate cyclique de 6-O-méthyl érythromycine (obtenu selon W. R. Baker, J. D. Clark, R. L. Stephens et K. H. Kim, J. Org. Chem., 1988, 53, 2340-2345) dans 25 cm**³** de méthanol, on ajoute 952 microlitres d'acide chlorhydrique 22°Be. On agite 16 heures à température ambiante, on évapore le méthanol, neutralise le milieu avec de la soude 2N. On extrait avec du chlorure de méthylène, sèche, filtre et évapore à sec. On chromatographie le résidu sur silice, éluant acétate d'éthyle-triéthylamine (95-5), on recueille 463 mg de produit recherché.

### Spectre de RMN : (CDCl₃) 300 MHz

0,87 (t) ppm (CH**₃** de l'éthyle), 1,28 (s) (6-Me), 0,94 (d) - 1,11 (d) - 1,19 (d) - 1,24 (d) - 1,25 (d) (autres Me), 1,49 (s) (12-Me), 2,06 (s) (OAc), 2,26 (s) (N(Me)**₂**), 2,5 à 2,75 (H**₂**, H**₃**,, H₈), 2,95 (q) (H**₁₀**), 2,92 (s) (6-OMe), 3,49 (m) (H**₅**, et H**₃**), 3,70 (d,J-2,5) (H**₅**), 4,73 (s) (H**₁₁**), 4,58 (d,J-7,5) (H**₁**,), 4,75 (dd) (H**₂**,), 5,13 (dd) (H**₁₃**).

### STADE B : 2'-acétate de 11,12-carbonate cyclique de 3-de[(2,6-dideoxy 3-C-méthyl 3-O-méthyl-alpha-L-ribo-hexopyranosyl) oxy] 6-O-méthyl 3-oxo érythromycine

A une solution de 368 mg du produit obtenu au stade A ci-dessus, on ajoute 962 microlitres de diméthyl sulfoxyde et 752 mg de chlorhydrate de 1-éthyl 3-(3-diméthylamino propyl) carbodiimide (EDAC), on agite 20 minutes à température ambiante et ajoute 742 mg de trifluoroacétate de pyridinium et l'on poursuit l'agitation 16 heures. On ajoute 10 cm**³** d'eau, agite et extrait avec du chlorure de méthylène, lave avec une solution de bicarbonate de sodium, sèche, filtre et évapore à sec. On chromatographie le résidu sur silice, éluant acétate d'éthyle-triéthylamine (98-2). On obtient 278 mg de produit recherché, utilisé tel quel pour le stade suivant.

### STADE C : 11,12-carbonate cyclique de 3-de[(2,6-dideoxy 3-C-méthyl 3-O-méthyl-alpha-L-ribo-hexopyranosyl] oxy) 6-O-méthyl 3-oxo érythromycine

On agite 16 heures à température ambiante 278 mg du produit obtenu au stade B, en solution dans 20 cm**³** de méthanol. On évapore le solvant et chromatographie le résidu (245 mg auxquels sont ajoutés 75 mg d'une précédente préparation) sur silice, on obtient ainsi 254 mg de produit recherché que l'on cristallise dans l'éther pour recueillir 176 mg de produit attendu.

### [alpha]_{D} + 63° (c = 0,45 %, CHCl₃)

### Spectre de RMN : (CDCl₃) 400 MHz ppm

2,65 (s) (6-OCH**₃**), 2,68 (m) (H**₈**), 2,97 (q) (H**₁₀**), 3,04 (q) (H**₄**), 3,18 (dd) (H**₂**,), 3,81 (q) (H**₂**), 4,31 (d) (H**₁**,), 4,18 (d) (H**₅**), 4,61 (H**₁₁**).

### EXEMPLE 8 : (9S) 3-de[(2,6-dideoxy 3-C-méthyl 3-O-méthyl-alpha-L-ribo-hexopyranosyl] oxy) 9-deoxo 6-O-méthyl 3-oxo 9-(1-pipéridinyl) érythromycine

### STADE A : 9-deoxo 9-imino 6-O-méthyl érythromycine

A une solution de 8,4 g de 9-oxime de 6-O-méthyl érythromycine (obtenue selon EP 0180415) avec 220 cm**³** de méthanol et 44 g d'acétate d'ammonium, on ajoute : 45,1 cm**³** de chlorure de titane à 15 %. On agite 3 heures à température ambiante, verse sur 500 cm**³** de chlorure de méthylène. On ajoute une solution à 10 % de carbonate de potassium, filtre, décante, lave à l'eau, sèche et évapore à sec. On obtient 7,08 g du produit attendu, que l'on utilise tel quel pour le stade suivant.

### STADE B : 9-amino 9-deoxo 6-O-méthyl érythromycine

7,0 g du produit obtenu au stade précédent sont mis en solution dans 140 cm**³** d'acide acétique et réduits catalytiquement en présence de 700 mg d'oxyde de platine à 80 %, sous atmosphère d'hydrogène à une pression de 1400 mbar. L'absorption terminée, on filtre, lave avec du chlorure de méthylène et évapore à sec. On reprend avec du chlorure de méthylène, lave avec une solution de bicarbonate de sodium, sèche et évapore à sec, on obtient 6,71 g du produit attendu que l'on utilise tel quel pour le stade suivant.

### STADE C : 9-amino 3-O-de(2,6-dideoxy 3-C-méthyl 3-O-méthyl-alpha-L-ribo-hexawranosyl) 9-déoxo 6-O-méthyl érythromycine

On agite pendant 5 heures à température ambiante, 2 g du produit obtenu ci-dessus, avec 40 cm**³** d'eau et 1 cm**³** d'acide chlorhydrique 22°Bé. On ajoute, ensuite, du chlorure de sodium puis amène à pH 8-9, avec de l'ammoniaque. On extrait avec du chlorure de méthylène et amène à sec sous pression réduite. Le résidu (2,2 g) est chromatographié sur silice, éluant acétate d'éthyle-méthanol-triéthylamine (92-5-3). On obtient 1,22 g du produit recherché, utilisé tel quel pour le stade suivant.

### STADE D : (9S) 3-O-de(2,6-dideoxy 3-C-méthyl 3-O-méthyl-alpha-L-ribo-hexopyranosyl) 9-deoxo 6-O-méthyl 9-(1-pipéridinyl) érythromycine

A une solution de 0,59 g du produit obtenu au stade C, dans 2,8 cm³ de méthanol, on ajoute 0,28 cm**³** d'acide acétique et 0,6 cm**³** de glutaraldéhyde à 50 % dans l'eau puis 0,125 g de cyanoborohydrure de sodium. On agite 1 heure 30 à température ambiante. On verse le milieu réactionnel sur 90 cm**³** d'une solution aqueuse de phosphate monosodique à 5 %, extrait avec du chlorure de méthylène, sèche, filtre et évapore à sec. On obtient 0,7 g de résidu que l'on chromatographie sur silice (éluant acétate d'éthyle-triéthylamine (98-2)). On obtient 328 mg de produit recherché.
Spectre IR : (CHCl₃ sur Nicolet)

| | |
|---|---|
| OH complexe | 3490 - 3390 cm⁻¹ |
| C=O | 1723 cm⁻¹ |

Spectre de RMN : (CDCl₃) 300MHz
0,85 (t) ppm (CH**₃** éthyle), 1,01(s) (12-CH**₃**), 1,28 (s) (6-CH**₃**), 2,72 (dq) (H**₂**), 3,84 (dl) (H₃), ≃ 1,54 (m) (H**₄**), ≃ 3,39 (masqué) (H**₅**), 3,10 (s) (6-OMe), 5,02 (dd) (H**₁₃**), 1,47 (m) et 1,89 (m) (CH**₂** éthyle), 3,93 (s) (H**₁₁**), 2,85 à 3,1 (m) (H**₉** et H**₁₀**), 2,65 (mélange) et 2,86 (mélange) (NCH**₂**,) 4,62 (d) (H**₁**,), 3,24 (dd) (H**₂**,), 2,50 (m) (H_{**3'**}), 1,27 (m) et 1,66 (m) (CH₂ en 4'), 3,53 (m) (H_{5'}).

### STADE E : (9S) 2'-acétate de 3-O-de(2,6-dideoxy 3-C-méthyl 3-O-méthyl-alpha-L-ribo-hexopyranosyl) 9-deoxo 6-O-méthyl 9-(1-pipéridinyl) érythromycine

A une solution de 160 mg du produit obtenu au stade D, dans 9 cm**³** d'acétone, on ajoute 242,8 mg de carbonate de potassium et 172,7 microlitres d'anhydride acétique. On agite 72 heures à température ambiante. On coule le milieu réactionnel sur de la glace, extrait avec de l'éther, lave avec une solution de bicarbonate de sodium puis à l'eau, sèche et évapore à sec, on obtient 164 mg du produit recherché.
Spectre IR : (CDCl₃ sur Nicolet)

| | |
|---|---|
| OAc | 1743 cm⁻¹ |
| lactone | 1723 cm⁻¹ |
| OH | ^{∼}3520 cm⁻¹ |

### Spectre de RMN : (CDCl₃) 250 MHz

0,84 (t) ppm (CH₃ éthyle), 1,05 à 1,30 (CH**₃** des CH**₃**CH), 2,11 (s) (OAc), 3,12 (s large) (6-OMe), ≃ 5,01 (H**₁₃**), 3,94 (s large) (H**₁₁**), 2,6 à 3,1 (CH**₂**N et H**₂**, H**₉**, H**₁₀**), 4,85 (d) (H**₁**,), 4,65 (dd) (H_{**2'**}), ≃ 3,46 (H**₅**,).

### STADE F : (9S) 2'acétate de 3-de[(2,6-dideoxy 3-C-méthyl 3-O-méthyl-alpha-L-ribopyranosyl) oxy] 9-deoxo 6-O-méthyl 3-oxo 9-(1-pipéridinyl) érythromycine

On opère comme au stade B de l'exemple 7, à partir de 207 mg de produit obtenu comme au stade E, en utilisant 489 microlitres de diméthyl sulfoxyde, 374 mg de [1-éthyl 3-(3-diméthyl aminopropyl) carbodiimide] (E.D.A.C.) et 374 mg de trifluoroacétate de pyridinium. Après chromatographie sur silice (éluant éther isopropylique-méthanol-triéthylamine (95-5-5)) on obtient 120 mg du produit attendu, utilisé tel quel pour le stade suivant.

### STADE G : (9S) 3-de[(2,6-dideoxy 3-C-méthyl 3-O-méthyl-alpha-L-ribo-hexopyranosyl) oxy] 9-deoxo 6-O-méthyl 3-oxo 9-(1-pipéridinyl) érythromycine

On opère comme au stade C de l'exemple 7, à partir de 120 mg du produit obtenu au stade E, ci-dessus. Après chromatographie sur silice (éluant éther isopropylique-méthanol-triéthylamine (95-5-5)) puis Microbondapack ® C18 éluant : acétonitrile-eau (80-20) (avec 0,075 % d'acide trifluoroacétique), on obtient 70 mg du produit recherché.

### [alpha]_{D} + 39° (c = 1 % CHCl₃)

### Spectre de RMN : (CDCl₃) 300 MHz

0,88 (t) ppm (CH**₃** éthyle), 1,08 (s) (12-Me), 1,23 (s) (6-Me), 1,01 à 1,28 ; 1,44 (d) (CH**₃** des CH**₃**CH), 2,34 (s) (N(Me)**₂**), 2,5 à 2,8 (CH**₂**N, H**₃**, et autres), 3,13 (s) (OMe), 3,51 (m) (H**₅**, et H**₂**,), 3,22 (s) (H**₁₁**,), 3,98 (q) (H**₂**), 4,35 (d) (H**₁**,), 4,78 (d) (H₅,), 5,05 (dd) (H**₁₃**,).

### EXEMPLE 9 : (9S) 9-amino 3-de[(2,6-dideoxy 3-C-méthyl 3-O-méthyl-alpha-L-ribo-hexopyranosyl) oxy] 9-deoxo 6-O-méthyl 3-oxo érythromycine

### STADE A : 2'-(phénylméthyl carbonate) de 3-O-de(2,6-dideoxy 3-C-méthyl 3-O-méthyl-alpha-L-ribo-hexopyranosyl) 9-deoxo 6-O-méthyl 9-[[(phénylméthoxy) carbonyl] amino] érythromycine

A un mélange de 1,5 g de produit obtenu au stade C de l'exemple 8 avec 11 cm**³** de dioxanne et 0,88 g de carbonate de potassium, on ajoute 0,8 cm**³** de chloroformiate de benzyle. On agite 5 heures à températue ambiante et ajoute 0,44 g de carbonate de potassium et 0,4 g de chloroformiate de benzyle. On poursuit l'agitation encore 2 heures, reprend avec du chlorure de méthylène, lave à l'eau, sèche et évapore à sec sous vide. On chromatographie le résidu (2,5 g) sur silice (éluant : chlorure de méthylène-méthanol-triéthylamine (96-3-1)). On obtient 1,71 g du produit recherché, utilisé tel quel pour le stade suivant.

### STADE B : (9S) 2'-(phénylméthyl carbonate) de 3-de[(2,6-dideoxy 3-C-méthyl 3-O-méthyl-alpha-L-ribo-hexopyranosyl) oxy] 9-deoxo 6-O-méthyl 3-oxo 9-[[(phénylméthoxy) carbonyl] amino] érythromycine

On opère comme au stade B de l'exemple 7, à partir de 2 g de produit obtenu comme au stade A ci-dessus, en utilisant 1,5 cm**³** de diméthylsulfoxyde, 1,8 *g* de E.D.A.C. et 1,8 g de trifluoroacétate de pyridinium. Après chromatographie sur silice (éluant chlorure de méthylène-méthanol (97-3), on obtient 757 mg de produit recherché, utilisé tel quel pour le stade suivant.

### STADE C : (9S) 3-de[(2,6-dideoxy 3-C-méthyl 3-O-méthyl-alpha-L-ribo-hexopyranosyl) oxy] 9-deoxo 6-O-méthyl 3-oxo 9-[[(phénylméthoxy) carbonyl] amino] érythromycine

On opère comme au stade C de l'exemple 7, à partir de 0,75 g du produit obtenu ci-dessus. Après chromatographie sur silice, on recueille 372 mg du produit recherché, utilisé tel quel pour le stade suivant.

### STADE D : (9S) 9-amino 3-de[(2,6-dideoxy 3-C-méthyl 3-O-méthyl-alpha-L-ribo-hexopyranosyl] oxy) 9-deoxo 6-O-méthyl 3-oxo érythromycine

On agite 24 heures sous pression d'hydrogène (1,5 bar) 150 mg du produit obtenu au stade E, 6 cm**³** d'acide acétique et 150 mg de palladium à 9,5 % sur charbon actif. On filtre, lave avec de l'éthanol et évapore à sec. On reprend le résidu avec du chlorure de méthylène, lave avec de la soude 2N puis à l'eau, sèche et évapore à sec. Après chromatographie sur silice (éluant chloroforme-méthanol-ammoniaque (9-1-0,1)) on obtient 50 mg du produit recherché.

### Spectre de RMN : (CDCl₃) ppm

1,88 (H**₈**), 1,94 (H**₁₀**), 2,48 (H**₃**,), 2,55 (H**₉**), 3,08 (H**₄**), 3,28 (H**₂**,), 3,65 (H**₁₁**), 3,8 (H₂), 4,3 (H₁,), 5,13 (H**₁₃**).

### EXEMPLE 10 : 11,12-dideoxy-3-de[(2,6-dideoxy-3-C-méthyl-3-O-méthyl-alpha-L-ribohexopyrannosyl) oxy]-6-O-méthyl-3-oxo-12,11-[ozycarbonyl[(4-phénylbutyl)imino]] érythromycine

### Stade A : 2'-acetate de 11,12-dideoxy-3-O-de(2,6-dideoxy 3-C-méthyl 3-O-méthyl-alpha-L-ribohexopyrannosyl)-6-O-méthyl- l2,11-[oxycarbonyl[(4-phénylbutyl) imino]] érythromycine

On introduit 649 mg de 2'acétate-4"-(phénylméthyl carbonate) de 11,12-dideoxy-6-O-méthyl-12,11-[oxycarbonyl [(4-phénylbutyl)imino]] érythromycine dont la préparation est décrite ci-après dans une solution renfermant 13 cm**³** de méthanol et 0,23 cm**³** d'acide chlorhydrique concentré. On maintient le mélange réactionnel sous agitation pendant 48 heures à la température ambiante. On évapore le méthanol sous pression réduite et ajoute 10 cm**³** d'acétate d'éthyle. On glace, neutralise et décante. On extrait la phase aqueuse de l'acétate d'éthyle, lave et sèche. On obtient 626 mg d'un produit que l'on chromatographie sur silice : éluant acétate d'éthyle, méthanol (95-5). On obtient ainsi 339 mg du produit recherché.

| Spectre IR dans CHCl₃ | |
|---|---|
| OH | 3618cm⁻¹ |
| | 3594cm⁻¹ |
| C=O | 1740cm⁻¹ |
| | 1711cm⁻¹ |
| C₆H₅C | 1492cm⁻¹ |

### Spectre de masse pic moléculaire 789,6 = Mh⁺

Spectre RMN CDCl3 300 MHz

| | |
|---|---|
| 2,45-2,8 (m) | CH₂Ph+H₂+H₈+H_{3'}ax |
| 3,42 (dd) | H₃ |
| 3,65 (m) | CH₂N-C=O |
| 4,76 (dd) | H_{2'}ax |
| 7,11-7,28 | aromatiques |

### Stade B : 2′-acétate de 11,12-dideoxy-3-de[(2,6-dideoxy 3-C-méthyl 3-O-méthyl-alpha-L-ribohexopyranosyl) oxy]-6-O-méthyl-12,11-[oxycarbonyl[(4-phénylbutyl) imino]] érythromycine

On verse à 21° C, une solution renfermant 300 mg de produit préparé au stade précédent et 2,15 cm**³** de chlorure de méthylène dans une suspension renfermant 0,4 cm**³** de DMSO, 1,6 cm**³** de chlorure de méthylène et 438 mg d'EDAC. On maintient le mélange réactionnel sous agitation à la température ambiante pendant 30 minutes. On refroidit à 15° C et introduit une solution de 438 mg de trifluoroacétate de pyridinium dans 1,5 cm**³** de chlorure de méthylène. On lave par une solution de bicarbonate de sodium puis à l'eau, sèche et évapore à sec. On obtient 348 mg d'un produit que l'on utilise tel quel dans le stade suivant. rf - 0,13.
- RMN CDCl**₃**: ppm
- 2,07 (s): 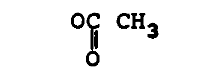
- 3,00: H₄
- 3,89 (q): H₂
- 3,66 (m): 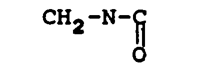
- 7,10 à 7,30: H aromatique
- 4,74 (dd): H_{**2'**}

### STADE C : 11, 12-dideoxy-3-de[ (2, 6-dideoxy-3-C-méthyl-3-O-méthyl-alpha-L-ribohexopyrannosyl) oxy]) -6-O-méthyl-3-oxo-12,11-[oxycarbonyl[(4-phénylbutyl)imino]]-érythromycine

On met en suspension 278 mg de produit préparé au stade précédent dans 3 cm**³** de méthanol. On maintient le mélange réactionnel sous agitation pendant 60 heures à la température ambiante. On chromatographie sur silice en éluant avec le mélange acétate d'éthyle-méthanol (95-5). On évapore le méthanol sous pression réduite. On obtient 280 mg d'un produit que l'on chromatographie avec un mélange chlorure de méthylène méthanol (9-1). On obtient 133mg de produit recherché.

| Spectre IR : | |
|---|---|
| OH : | 3440cm⁻¹ |
| C=O : | 1747cm⁻¹ |
| | 1711cm⁻¹ |

| RMN | |
|---|---|
| 2,49 (dd) | H_{3'} |
| 3,20 dd | H_{2'} |
| 3,10 | H**₄** |
| 3,86 | H₂ |

### Préparation du produit de départ de l'exemple 10.

On introduit sous atmoshère d'argon, 830 mg de 4-phényl-butylamine dans une suspension renfermant 1,17 g de 2'-acétate-12-(1H-imidazole-l-carboxylate)-4"-(phénylméthyl carbonate) de 10,11-didehydro-11-deoxy-6-O-méthyl-érythromycine préparé comme indiqué dans J. Org. CHem. (1988) 53,2340-2345, 2,7 cm**³** de cyanure de méthylène et 0,27 cm**³** d'eau. On maintient le mélange réactionnel sous agitation pendant 2 heures à 50°, On ajoute 150 cm**³** de chlorure de méthylène, et refroidit au bain de glace. On ajoute 30 cm**³** d'une solution de phosphate acide de sodium 0,5 M. On décante, extrait au chlorure de méthylène, lave, sèche et évapore à sec. On chromatographie sur silice le produit obtenu : éluant acétate d'éthyle-méthanol (95-5). On obtient 952 mg du produit recherché.

| Spectre IR | |
|---|---|
| C=O | 1739 cm⁻¹ |
| | 1711 cm^{₋₁} |
| C₆H**₅**C | 1495 cm⁻¹ |

### Spectre Ultra-violet

inf : 216 nm E**₁** = 103
inf : 259 nm E**₁** = 4,5
inf : 266 nm E₁ = 3

### Spectre de masse

pic moléculaire = 1081,7 = MH⁺

### Spectre RMN

Dans CDCl₃ sur 400 MHz.
- 2,5 à 2,8: N CH**₂**C**₆**H**₅**H**₈**H**₃'**
- 3,66: 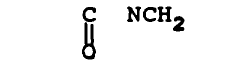
- 3,60: H**₁'**
- 710 à 7,25: C**₆**H**₅** (CH**₂**)₄
- 7,35: C₆H**₅** CH**₂**O

### EXEMPLE 11 : 11,12-dideoxy-3-de[ (2,6-dideoxy-3-C-méthyl-3-O-méthyl-alpha-L-ribohexopyrannosyl) oxy]-6-O-méthyl-3-oxo-12,11-[oxycarbonyl[[2-[méthyl(phénylméthyl)amino] éthyl] imino]] érythromycine

### Stade A : 11,12-dideoxy-3-O-de(2,6-dideoxy 3-C-méthyl 3-O-méthyl-alpha-L-ribohexopyranosyl)-6-O-méthyl-12,11-[oxycarbonyl[[2-[méthyl (phénylméthyl) amine]éthyl]imino]] érythromycine

On maintient sous agitation pendant une heure à la température ambiante 460 mg du produit préparé ci-après (préparation du produit de départ de l'exemple 11), 9,2 cm³ de méthanol et 0,23 cm**³** d'une solution d'acide chlorhydique concentré, puis 48 heures à la température ambiante. On reprend le résidu à l'eau puis au chlorure de méthylène. On amène à PH basique. On décante, extrait la phase aqueuse au chlorure de méthylène. On lave, sèche et évapore à sec. On obtient 432 mg d'un produit que l'on chromatographie sur silice : éluant acétate d'éthyle-méthanole (9-1). On obtient ainsi 312 mg du produit recherché.
Spectre IR :

| | |
|---|---|
| OH : | 3618 cm**₋₁** |
| | 3594 |
| C=O : | 1742 cm₋₁ |
| | 1709 cm**₋₁** |

RMN dans CDCl**₃** sur 300 MHz
- 1,42 (s): 6Me
- 1,24 (s): 12Me
- 2,20 (s): CH**₃**-N<
- 3,48 (d) -3,77 (d): >N-CH**₂**-C**₆**H₅
- 3,80(m)-3,95(m): 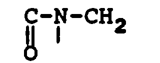
- 3,40 -: 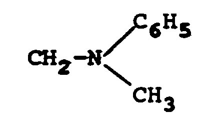
- 4,74: H_{**2'**}

### Stade B : 2'-acétate de 11,12-dideoxy-3-de[(2,6-dideoxy-3-C-méthyl 3-O-méthyl-alpha-L-ribohexopyranosyl) oxy]-6-O-méthyl-12,11-[oxycarbonyl[2-[méthyl(phényl méthyl) amino] éthyl] imino]] érythromycine

On ajoute à 20°C 200 mg du produit préparé au stade précédent en solution dans 1,4 cm**³** de chlorure de méthylène dans une suspension renfermant 286 mg d'EDAC, 0,26 mg de DMSO et 1,2 cm**³** de chlorure de méthylène. On maintient le mélange réactionnel sous agitation pendant 30 minutes à la température ambiante. On refroidit à 14°C et introduit à cette température une solution renfermant 286 mg de trifluoroacétate de pyridinium et 1,3 cm**³** de chlorure de méthylène. On agite pendant 30 minutes à 15°C et laisse revenir à la température ambiante. On traite avec du bicarbonate de sodium, dilue au chlorure de méthylène, lave, sèche et évapore à sec. On obtient 350 mg du produit recherché brut que l'on purifie par chromatographie sur silice éluant : acétate d'éthyle-méthanol (95-5). On obtient 145 mg de produit recherché :

| Spectre UV dans EtOH | |
|---|---|
| max 258 nm | ε.= 400 |
| dans EtOH-NaOH 0,1N | |
| max 292 nm | ε.= 20100 |

Présence β ceto ester

### RMN

- 2,17(s): 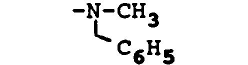
- 2,25(s): 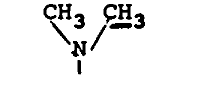
3,48(d)-3,70(d)N-CH**₂**-Φ
- 3,80: H₂
- 3,87: 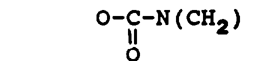
- 4,74 (dd): H_{2'}
- 7,17 à 7,30: aromatiques

### Stade C : 11,12'-dideoxy-3-de[(2,6-dideoxy-3-C-méthyl 3-O-méthyl-alpha-L-ribohexopyranosyl) oxy] 6-O-méthyl-3-oxo-12,11-[oxycarbonyl [[2-[méthyl(phénylméthyl) amino] éthyl] imino] érytromycine

On maintient sous agitation pendant une nuit à la température ambiante 106 mg du produit préparé au stade précédent et 6 cm**³** de méthanol. On maintient le mélange réactionnel pendant une nuit à la température ambiante. On évapore le méthanol. On obtient 106 mg d'un produit que l'on chromatographie sur silice éluant : acétate d'éthyle-triéthylamine (95-5) on obtient 55 mg du produit recherché.
Analyse pour C**₄₁**H**₆₅** N**₃**O**₁₀** = 759-99

### RMN:

- 1,34 (s): 12 Me
- 1,48 (s): 6 Me
- 2,18 (s): 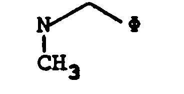
- 2,27 (s): 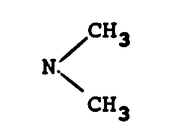
- 3,00 à 3,24: H**₂'** et H₄
- 3,57 (s): H₁₁
- 4,23 (d) et 4,28 (d): H**₁**, et H₅
2,48 H**₃'** H**₈** et CH**₂**Φ

### Préparation du produit de départ de l'exemple 11 11,12-dideoxy-6-O-méthyl-12,11-[oxycarbonyl[[2-[méthyl(phényl-méthyl) amino] éthyl] imino]] érythromycine

### Stade A : 11, 12-dideoxy-6-O-méthyl-12, 11-[oxycarbonyl[[2-[(phénylméthyl) amino] éthyl] imino]] érythromycine

On introduit 1,1 g de 2'-acétate-12-(1H-imidazole-1-carboxylate)-4"-(phénylméthyl carbonate) de 10,11-didehydro-11-deoxy-6-O-méthyl-érythromycine, dans un mélange de 2,7 cm**³** de cyanure de méthyle et 0,27 cm**³** d'eau. On introduit 1,8 cm**³** de N-benzyléthylènediamine. On maintient le mélange réactionnel sous agitation à 50°C pendant 5 heures. On dilue au chlorure de méthylène et refroidit au bain de glace. On ajoute 30 cm**³** d'une solution 0,5 M de phosphate monosodique. On décante, extrait au chlorure de méthylène, lave, sèche et évapore à sec. On obtient 1,6 g de produit que l'on purifie par chromatographie sur silice éluant acétate d'éthyle-méthanol (98-2). On obtient 800 mg de produit recherché.

Analyses physiques

| Spectre infra-rouge | |
|---|---|
| C=O | 1739 cm⁻¹ |
| | 1710 cm**⁻¹** |
| C₆H**₅** | 1494 cm⁻¹ |

Spectre de masse
Pic moléculaire = 1082 = MH⁺
Spectre RMN
Dans CDCl₃ sur 300 MHz

| | |
|---|---|
| 3,07 (q) | H₁₀ |
| 3,34 (s) | 3"OMe |
| 3,61 (s) | H₁₁ |
| 3,83 (m) | CH₂N-C=O et N-CH₂Ph |
| 7,15 à 7,35 | aromatiques N-CH**₂**Ph |

### Stade B : 11,12-dideoxy-6-O-méthyl-12, 11-[oxycarbonyl[[2-[méthyl(phénylméthyl) amino] éthyl] imino]] érythromycine

On ajoute 50 cm**³** d'une solution d'aldehyde formique à 37 % et de l'acide formique dans une solution renfermant 13 cm**³** de chlorure de méthylène et 0,60 g du produit préparé au stade précédent. On agite le mélange réactionnel sous agitation pendant 4 heures à la température ambiante puis à 70°C pendant 7 heures. On dilue au chlorure de méthylène, ajoute de l'eau et neutralise avec du bicarbonate de sodium. On décante, lave, sèche et évapore à sec. On chromatographie sur silice : éluant acétate d'éthyle-méthanol (95-5). On obtient 480 mg de produit recherché.

### Analyses

| Spectre IR : | |
|---|---|
| C=O | 1737 cm⁻¹ |
| | 1708 cm**⁻¹** |
| C₆H**₅** | 1494 cm**⁻¹** |
| SM : | |
| MH⁺ = | 1096,9**⁺** |

| Spectre RMN : | |
|---|---|
| 1,12 (s) | 12 Me |
| 1,40 (s) | 6 Me |
| 2,19 | CH³-N〈 |
| 2,96 (s) | 6.0 Me |
| 3,62 (sl) | H**₁₁** |
| 4,30 (m) | H_{5"} ax |
| 4,73 (d) | H_{2"} ax |
| 7,15 à 7,39 | aromatiques |
| 2,87 | H**₂** |

### EXEMPLE 12 : 9-[O-(2-bromoéthyl) oxime de 3-dé [(2,6-didéoxy 3-C-méthyl 3-O-méthyl alpha-L-ribo-hexopyranosyl) oxy] 6-O-méthyl 3-oxo érythromycine

A 1,18 g de produit obtenu à l'exemple 3 dans 20 cm**³** de méthanol, on ajoute 880 mg puis 440 mg d'hydrobromure de 2-bromoéthyloxyamine après 24 heures d'agitation, à température ambiante en maintenant le pH = 3. On agite 18 heures supplémentaires et ajoute 30 cm**³** d'eau, essore les cristaux, les lave à l'eau, les sèche à 60°C et obtient 1,2 g de (9R) 9-déoxo 12-déoxy 3-dé (2,6-didéoxy 3-C-méthyl 3-O-méthyl alpha L-ribo hexopyranosyl) oxy] 9,12-époxy 9-méthoxy érythromycine (F = 188-190°C) dans 10 cm**³** d'eau et ajoute de l'ammoniaque concentré jusqu'à pH - 11-12. On extrait à l'acétate d'éthyle, concentre sous pression réduite et obtient 1,0 g de produit attendu. F = 150-152°C.

### Spectre RMN (ppm) CDCl₃ :

| Spectre RMN (ppm) CDCl**₃** : | |
|---|---|
| 0,86 (t) | CH**₃** éthyle |
| 1,23 (s) | 12-Me |
| 1,40 (s) | 6-Me |
| 2,26 (s) | N(Me)**₂** |
| 2,45 (m) | H₃ |
| 2,60 (pl) | H**₁₀** |
| 2,76 (s) | 6-OMe |
| 3,13 (m) | H₄ |
| 3,18 (dd) | H_{**2'**} |
| 3,52 (m) | CH**₂**Br |
| 3,55 (m) | H**'₅** |
| 3,70 (m) | H₈ isom. E |
| 3,86 (q) | H**₂** |
| 3,90 (d) | H₁₁ |
| 4,18 à 4,35 | H**'**_{**1'**} H₅ et NOCH**₂** + 1H mobile |
| 5,17 (dd) | H₁₃ |
| 3,26 (s) 3,45 | H mobiles. |

### EXEMPLE 13 : (E) 9-O-[2-[[2-(1-pyrrolidinyl éthyl] aino] éthyl] oxime de 3-dé [(2,6-didéoxy 3C-méthyl 3-O-méthyl alpha-L-ribo hexopyranosyl) oxy] 6-O-méthyl 3-oxo érythromycine.

On agite 0,3 g du produit obtenu à l'exemple 12 et 3 cm³ de N-aminoéthyl pyrrolidine pendant 72 heures à température ambiante, on ajoute 5 cm³ d'éthanol et agite 24 heures supplémentaires, évapore sous pression réduite, chromatographie l'extrait sec (0,35 g) sur silice (éluant : acétate d'éthyle-méthanol-triéthylamine 90-8-2) et recueille 0,204 g du produit recherché.

### Spectre RMN (ppm) 300 MHz CDCl₃ :

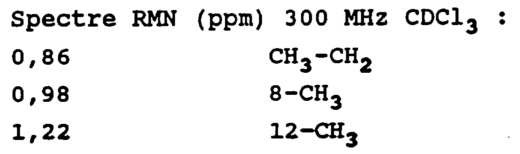

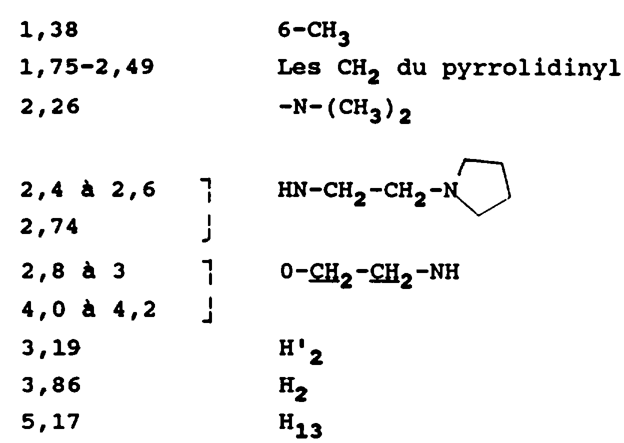

En opérant comme à l'exemple 12 à partir du produit obtenu à l'exemple 3 et du dérivé d'hydroxylamine approprié sous forme de chlorhydrate, on a préparé les produits des exemples suivants :

**EXEMPLE 14 : (E) 9-O-[(2-pyridinyl) méthyl] oxime de 3-dé [(2,6-didéoxy 3C-méthyl 3-O-méthyl alpha-L-ribo hexopyranosyl) ozy] 6-O-méthyl 3-oxo érythromycine**.
F = 167-169°C.

**EXEMPLE 15 : (E) 9-O-[(3,5-diméthyl 4-isoxazolyl) méthyl] oxime de 3-dé [(2,6-didéoxy 3C-méthyl 3-O-méthyl alpha-L-ribo hezopyranowyl) oxy] 6-O-méthyl 3-oxo érythromycine.**
F = 222-224°C.

**EXEMPLE 16 : (E) 9-O-[(4-nitrophényl) méthyl] oxime de 3-dé [(2,6-didéoxy 3C-méthyl 3-O-méthyl alpha-L-ribo hexopyranosyl) oxy] 6-o-méthyl 3-oxo érythromycine.**
Rf = 0,40 (CH**₂**Cl**₂**-MEOH-9-1)

**EXEMPLE 17 : (E) 9-oxime de 3-dé [(2,6-didéoxy 3C-méthyl 3-O-méthyl alpha-L-ribo hexopyranosyl) oxy] 6-O-méthyl 3-oxo érythromycine.**
F = 268-270°C.

En opérant comme à l'exemple 13 à partir du dérivé bromé obtenu à l'exemple 12 et des réactifs aminés appropriés, on a préparé les produits des exemples suivants :

**EXEMPLE 18 : 9-O-[2-(diéthylamino) éthyl] oxime de 3-dé [(2,6-didéoxy 3C-méthyl 3-O-méthyl alpha-L-ribo hexopyranosyl) oxy] 6-O-méthyl 3-oxo érythromycine.**
F = 158-160°C. [alpha]_{**D**} = + 2,5 (c = 0,5% CHCl**₃**)

**EXEMPLE 19 : 9-O-[2-(1-pyrrolidinyl) éthyl] oxime de 3-dé** **[(2,6-didéoxy 3C-méthyl 3-O-méthyl alpha-L-ribo hexopyranosyl) oxy] 6-O-méthyl 3-oxo érythromycine.**
F = 210-212°C. [alpha]_{**D**} = + 8,5 (c = 0,85% CHCl**₃**)

**EXEMPLE 20 : 9-O-[2-(1-azétidinyl) éthyl] oxime de 3-dé [(2,6-didéoxy 3C-méthyl 3-O-méthyl alpha-L-ribo hexopyranosyl) oxy] 6-O-méthyl 3-oxo érythromycine.**
Rf = 0,37 (AcOEt-MEOH-TEA 90-5-5)

**EXEMPLE 21 : 9-O-[2-(4-morpholinyl) éthyl] oxime de 3-dé [(2,6-didéoxy 3C-méthyl 3-O-méthyl alpha-L-ribo hexopyranosyl) oxy] 6-O-méthyl 3-oxo érythromycine.**
F = 198-200°C. [alpha]_{**D**} = + 5 à + 8 (c = 0,85% CHCl**₃**)

**EXEMPLE 22** **: 9-O-[2-(1-pipéridinyl) éthyl] oxime de 3-dé [(2,6-didéoxy 3C-méthyl 3-O-méthyl alpha-L-ribo hexopyranosyl) oxy] 6-O-méthyl 3-oxo érythromycine.**
F = 194-196°C. [alpha]_{**D**} = + 10° (c = 0,6% CHCl**₃**)

**EXEMPLE 23** **: (E) 9-O-[2-(propylamino) éthyl] oxime de 3-dé [(2,6-didéoxy 3C-méthyl 3-O-méthyl alpha-L-ribo hexopyranosyl) oxy] 6-O-méthyl 3-oxo érythromycine.**
Rf = 0,34 (AcOEt-TEA 95-5)

**EXEMPLE 24 : (E) 9-O-[2-[[2-(diméthylamino) éthyl] amino) éthyl] oxime de 3-de [(2,6-didéoxy 3C-méthyl 3-O-méthyl alpha-L-ribo hexopyranosyl) oxy] 6-O-méthyl 3-oxo érythromycine.**
Rf = 0,17 (AcOEt-MEOH-TEA 80-10-10)

**EXEMPLE 25 : (E) 9-O-[2-(4-méthyl 1-pipérazinyl) éthyl] oxime de 3-dé [(2,6-didéoxy 3C-méthyl 3-O-méthyl alpha-L-ribo hexohexopyranosyl) oxy] 6-O-méthyl 3-oxo érythromycine.**
F = 160-162°C. [alpha]_{**D**} = - 3° (c = 0,4% MEOH)

**EXEMPLE 26: (E) 9-O-[2-[[3-(diméthylamino) propyl] amino] éthyl] oxime de 3-dé [(2,6-didéoxy 3C-méthyl 3-O-méthyl alpha-L-ribo hexopyranosyl) oxy] 6-O-méthyl 3-oxo érythromycine.**
[alpha]_{**D**} = + 6° (c = 1% CHCl**₃**)

**EXEMPLE 27 : (E) 9-O-[2-[[2-(1-pipéridinyl) éthyl] amino] éthyl] oxime de 3-dé [(2,6-didéoxy 3C-méthyl 3-O-méthyl alpha-L-ribo hexopyranosyl) oxy] 6-O-méthyl 3-oxo érythromycine.**
[alpha]_{**D**} = + 4° (c = 0,7% CHCl**₃**)

**EXEMPLE 28 : (E) 9-O-[2-[(1-méthyléthyl) amino] éthyl] oxime de 3-dé [(2,6-didéoxy 3C-méthyl 3-O-méthyl alpha-L-ribo hexopyrmomyl) oxy] 6-O-méthyl 3-oxo érythromycine.**
F = 188-190°C. [alpha]_{**D**} = + 7° (c = 1% CHCl₃)

**EXEMPLE 29: (E) 9-O-[2-(hexahydro 1H-azépin-1-yl) éthyl) oxime de 3-dé [(2,6-didéozy 3C-méthyl 3-O-méthyl alpha-L-ribo hexopyranosyl) oxy] 6-O-méthyl 3-oxo érythromycine.**
F = 206-208°C. [alpha]_{D} = + 1,2° (c = 0,85% MEOH)

**EXEMPLE 30 : (E) 9-O-(2-aminoéthyl) oxime de 3-dé ((2,6-didéoxy 3C-méthyl 3-O-méthyl alpha-L-ribo hexopyranoayl) oxy] 6-O-méthyl 3-oxo érythromycine.**
F = 190-192°C.

**EXEMPLE 31 : (E) 9-O-[2-[(2-propynyl) amino] éthyl] oxime de 3-de [(2,6-dLdéoxy 3C-méthyl 3-O-méthyl alpha-L-ribo hexopyranosyl) oxy] 6-O-méthyl 3-oxo érythromycine.**
F = 152-154°C.

**EXEMPLE 32 : 9-O-(2-[(phénylméthyl) amino] éthyl) oxime de 3-de [(2,6-didéoxy 3C-méthyl 3-O-méthyl alpha-L-ribo hexopyranosyl) oxy] 6-O-méthyl 3-oxo érythromycine.**
F = 200 puis 222-224°C. [alpha]_{**D**} = + 2° (c - 1% MEOH)

**EXEMPLE 33 : (E) 9-O-(2-[méthyl (phénylméthyl) amino] éthyl] oxime de 3-dé [(2,6-didéoxy 3C-méthyl 3-O-méthyl alpha-L-ribo hexopyranosyl) oxy] 6-O-méthyl 3-oxo érythromycine.**
F = 130-135°C. [alpha]_{**D**} = + 15° (c = 0,9% MEOH)

**EXEMPLE 34 : (E) 9-O-[2-[[3-(diéthylamino) propyl] méthylamino] éthyl] oxime de 3-dé [(2,6-didéoxy 3C-méthyl 3-O-méthyl alpha-L-ribo hexopyranosyl) oxy] 6-O-méthyl 3-oxo érythromycine.**
Rf = 0,2 (CH**₂**Cl**₂**-MEOH-NH₄ OH 90-10-1)

**EXEMPLE 35 : (3S) 9-O-[2-(diméthylamino) éthyl] oxime de 3-O-dé [(2,6-didéoxy 3C-méthyl 3-O-méthyl alpha-L-ribo hexopyranosyl) oxy] 6-O-méthyl 3-oxo érythromycine.**

On chauffe au reflux pendant 4 heures une solution de 0,59 g du produit obtenu au stade A de l'exemple 3 et 0,27 g de chlorhydrate de N,N-diméthylaminoéthoxyamine dans 12 cm**³** de méthanol. On reprend avec de l'éthanol aqueux et amène à pH = 8-9 avec de l'ammoniaque, décante, lave, sèche, filtre et évapore à sec sous pression réduite. On chromatographie le résidu (0,76 g) sur silice (éluant : acétate d'éthyle-triéthylamine 95-5) et recueille 158 mg de produit attendu cristallisé dans l'hexane. F = 156°C.

| Spectre RMN 300 MHz (CDCl**₃**) : | |
|---|---|
| 0,87 (t) | CH**₃** éthyle |
| 1,33-1,41 (s) | 6 et 12 méthyle |
| 2,72 (d,q) | H**₂** |
| 3,48 (d,J=10,5Hz) | H₃ |
| ^{∼}3,72 (d,J=1,5Hz) | H₅ |
| 4,32 (d,d) | H**₁₃** |
| 1,84 (d,J=1Hz) | 10 méthyle |
| 5,77 | H₁₁ |
| 4,21 | OCH**₂** |
| 2,66 | CH₂N |
| 4,39 (d) | H**'₁** |
| 3,25 (dd) | H**'₂** |
| 2,48 (m) | H**'₃** |

**EXEMPLE 36 : (E) 9-O-[2-(diméthylamino) éthyl] oxime de 3-dé [(2,6-didéoxy 3C-méthyl 3-O-méthyl alpha-L-ribo hexopyranosyl) oxy] 11-déoxy 10,11-didéhydro 6-O-méthyl 3-oxo érythromycine.**

### Stade A : Acétate de (3S) 9-O-[2-(diméthylamino) éthyl] oxime de 3-O-dé [(2,6-didéoxy 3C-méthyl 3-O-méthyl alpha-L-ribo hexopyranosyl) oxy] 6-O-méthyl érythromycine.

On agite 24 heures à température ambiante 228 mg du produit obtenu à l'exemple 35, 96 mg de carbonate de potassium et 0,05 cm**³** d'anhydride acétique dans 4 cm**³** d'acétone. On ajoute encore 19 mg de carbonate de potassium et 0,010 cm³ d'anhydride acétique et agite 4 heures. On verse le mélange réactionnel sur de la glace et amène à pH = 8-9 avec de l'ammoniaque. On extrait avec du chlorure de méthylène, sèche, filtre et évapore à sec sous pression réduite. On obtient 250 mg de produit que l'on chromatographie sur silice (éluant : acétate d'éthyle-triéthylamine 95-5) et recueille 175 mg du produit recherché.

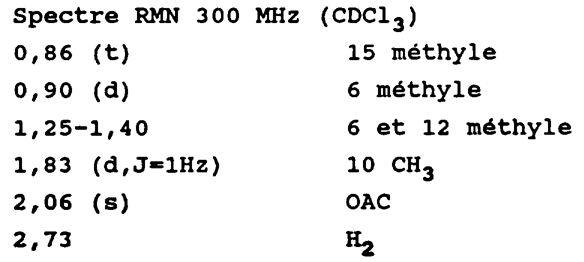

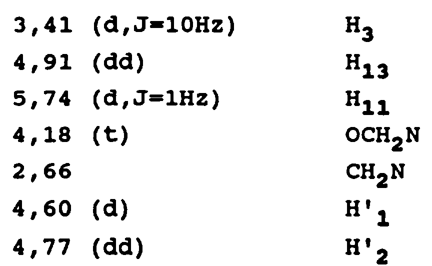

### Stade B : (E) 9-O-[2-(diméthylamino) éthyl] oxime de 3-dé [(2,6-didéoxy 3C-méthyl 3-O-méthyl alpha-L-ribo hexopyranosyl) oxy] 11-déoxy 10,11-didéhydro 6-O-méthyl 3-oxo érythromycine.

A une solution de 240 microlitres de diméthylsulfoxyde et 240 mg de 1-éthyl 3-(3-diméthylaminopropyl) carbodiimide (EDAC) dans 2 cm**³** de chlorure de méthylène, on ajoute 160 mg du produit obtenu au stade A ci-dessus en solution dans 2 cm**³** de chlorure de méthylène. On agite 30 minutes à température ambiante, refroidit à 15°C et en maintenant la température, on ajoute 260 mg de trifluoroacétate de pyridinium en solution dans 2 cm**³** de chlorure de méthylène. On agite encore 1 heure à 15°C, ajoute 5 cm**³** d'eau, amène à pH - 8-9 avec de l'ammoniaque, extrait avec du chlorure de méthylène, lave à l'eau, sèche, filtre, évapore à sec sous pression réduite, reprend avec 10 cm**³** de méthanol, agite 16 heures et évapore à sec sous pression réduite. L'extrait sec 0,3 g est chromatographié sur silice (éluant : acétate d'éthyle-triéthylamine 95-5). On recueille 62 mg de produit recherché.

| Spectre RMN 300 MHz (CDCl₃) | |
|---|---|
| 0,86 (t) | 15 CH₃ |
| 1,77 et 1,85 | 10CH₃ |
| 3,89 | H₂ |
| 4,91 (dd) | H₁₃ |
| 5,66 et 5,76 | H₁₁ |
| 4,10 à 4,30 | OCH₂ |
| 2,65 | CH₂N |
| 3,20 (dd) | H**'₂** |
| 2,26 et 2,27-2,30 | N(CH₃)₂ |

### EXEMPLE 37 : (E) 9-O-(3-pipéridinyl) oxime de 3-dé [(2,6-didéoxy 3C-méthyl 3-O-méthyl alpha-L-ribo hexopyranosyl) oxy] 6-O-méthyl 3-oxo érythromycine.

a) On chauffe à 65°C pendant 6 heures une solution de 0,6 g du produit obtenu à l'exemple 3 et 0,86 g de produit obtenu à la préparation ci-dessous dans 12 cm**³** de méthanol. On évapore le solvant, reprend le résidu avec 10 cm**³** de chlorure de méthylène, ajoute 10 cm**³** d'eau et amène à pH = 8 avec de l'ammoniaque. On décante, lave à l'eau salée, sèche et évapore à sec sous pression réduite après chromatographie sur silice (éluant acétate d'éthyle-triéthylamine 98-2), on obtient 550 mg du composé benzyloxycarbonyl intermédiaire.

### b) hydrogénolyse

On agite 12 heures à température ambiante une solution de 250 mg du produit obtenu ci-dessus dans 10 cm**³** de méthanol avec 100 mg de palladium sur charbon actif et sous pression de 1,5 bar d'hydrogène. On filtre, lave avec du méthanol et évapore le filtrat à sec sous pression réduite. On chromatographie le résidu (200 mg) sur silice (éluant : chloroforme-méthanol-ammoniaque 92-8-0,5) et obtient 160 mg du produit recherché.

| Spectre RMN 300 MHz (CDCl₃) | |
|---|---|
| 0,86 (t) | CH₃ éthyle |
| 1,39 (s,d) | 6 CH₃ |
| 2,26 (s) | N(CH₃)₂ |
| 2,58 | H₁₀ |
| 2,67-3,16 | -CH₂-NH-CH₂- |
| 3,58 | H**'₅** |
| 3,70 | H₈ |
| 3,87 | H₃ |

### Préparation de l'exemple 37 : Chlorhydrate de O-[1-(benzyloxycarbonyl) pipéridin-3-yl] hydroxylamine.

### Stade A : 1-benzyloxycarbonyl 3-hydroxypipéridine.

A une solution de 5 g de 3-hydroxypipéridine dans 50 cm**³** de dioxane, on ajoute 8,2 g de carbonate de potassium et, goutte à goutte à 0°C, 7,7 cm**³** de chloroformiate de benzyle en solution dans 10 cm**³** de dioxane. On agite 2 heures à température ambiante et rajoute 5 g de carbonate de potassium, 20 cm**³** d'eau et 3 cm³ de chloroformiate de benzyle en solution dans 10 cm³ de dioxane, agite 1 heure à température ambiante, concentre sous pression réduite, reprend avec 10 cm**³** d'eau, extrait à l'éther, sèche et concentre sous pression réduite. Après chromatographie sur silice (éluant : acétate d'éthyle-hexane 1-1), on obtient 10,8 g de produit recherché.

| Spectre IR CHCl₃ | |
|---|---|
| OH | 3612 cm⁻¹ |
| C=O | 1693 cm⁻¹ |
| aromatiques | 1498 cm⁻¹ |

### Stade B : N-([1-(benzyloxycarbonyl) pipéridin-3-yl] oxy] phtalimide.

A une solution de 10,8 g du produit obtenu au stade A, 8,24 g de N-hydroxyphtalimide et 13,25 g de triphénylphosphine dans 225 cm**³** de tétrahydrofuranne, on ajoute à 25°C en 30 minutes 10,05 g de diéthylazodicarboxylate. On agite 4 heures puis évapore Le solvant sous pression réduite. On chromatographie le résidu obtenu sur silice (éluant : acétate d'éthyle-hexane 1-1) et obtient 13 g du produit attendu sous forme de 2 diastéréoisoméres.

| Spectre IR CHCl**₃** | |
|---|---|
| CO phtalimide | 1790, 1732 cm⁻¹ |
| CO carbobenzyloxy | 1693 cm⁻¹ |

### Stade C : Chlorhydrate de O-[1-(benzyloxycarbonyl) pipéridin-3-yl] hydroxylamine.

On agite 1 heure à 60°C une solution de 11,9 g du produit obtenu au stade B et 1,46 cm**³** d'hydrate d'hydrazine dans 60 cm**³** d'éthanol. On filtre l'insoluble, lave à l'éther et évapore le filtrat à sec sous pression réduite. On chromatographie le résidu sur silice (éluant : acétate d'éthyle-hexane 4-6) et obtient 6 g du produit attendu sous forme de base. Préparation du chlorhydrate.

On dissout le produit obtenu ci-dessus dans un mélange éther-méthanol et traite la solution avec de l'éther chlorhydrique. On essore, lave à l'éther et sèche. On obtient 6,32 g du chlorhydrate attendu.

| Spectre IR CHCl₃ | |
|---|---|
| ONH₂ | 3330, 1586 cm⁻¹ |
| CO | 1688 cm⁻¹ |
| phényl | 1498 cm⁻¹ |

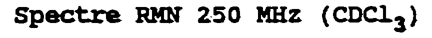

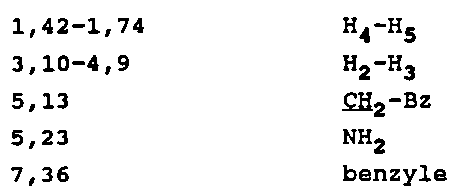

### EXEMPLE 38 : 9-O-[2-[(2-méthoxyéthyl) amino] éthyl] oxime de 3-dé [(2,6-didéoxy 3C-méthyl 3-O-méthyl alpha-L-ribo hexopyranosyl) oxy] 6-O-méthyl 3-oxo érythromycine.

### Stade A : 9-O-[2-(bromo) éthyl] oxime de 3-O-dé (2,6-didéoxy 3C-méthyl 3-O-méthyl alpha-L-ribo hexopyranosyl) 6-O-méthyl érythromycine.

On opère comme à l'exemple 12 en faisant réagir le réactif bromé sur le 3-O-dé (2,6-didéoxy 3-C-méthyl 3-O-méthyl alpha-L-ribo hexopyranosyl) 6-O-méthyl érythromycine préparé à l'exemple 3, Stade A. On obtient le produit attendu.

### Stade B : 9-O-[2-[(2-méthoxyéthyl) amino] éthyl] oxime de 3-O-dé (2,6-didéoxy 3C-méthyl 3-O-méthyl alpha-L-ribo hexopyranosyl) 6-O-méthyl érythromycine.

On agite pendant 20 heures à température ambiante 0,72 mg de produit obtenu au stade A dans 3 cm**³** de 2-méthoxyéthylamine. On évapore le solvant sous pression réduite, chromatographie le résidu sur silice- (éluant : acétate d'éthyle-triéthylamine-méthanol 9-0,5-0,5) et obtient 730 mg de produit attendu que l'on cristallise dans l'éther. Stade C : 2'-O-(phénylméthylcarbonate) de 9-O-[2-[(benzyloxycarbonyl) (2-méthoxyéthyl) amino] éthyl] oxime de 3-O-dé (2,6-didéoxy 3C-méthyl 3-O-méthyl alpha-L-ribo hexopyranosyl) 6-O-méthyl érythromycine.

On ajoute goutte à goutte 0,44 cm**³** de chloroformiate de benzyle dissous dans 5 cm**³** de dioxane au mélange comprenant 620 mg de produit obtenu au stade B et 490 mg de carbonate de potassium dans 10 cm³ de dioxane. Après 3 heures d'agitation à température ambiante, on ajoute 0,4 cm³ du chloroformiate et 500 mg de carbonate de potassium supplémentaire et agite à température ambiante pendant 20 heures. On ajoute 3 cm³ d'eau, agite 30 minutes, extrait à l'acétate d'éthyle, sèche et évapore le solvant. Après chromatographie sur silice (éluant : acétate d'éthyle), on obtient 616 mg de produit attendu.

### Rf = 0,27.

### Stade D : 2'-O-(phénylméthylcarbonate) de 9-O-[2-[(benzyloxycarbonyl) (2-méthoxyéthyl) amino] éthyl] oxime de 3-O-dé [(2,6-didéoxy 3C-méthyl 3-O-méthyl alpha-L-ribo hexopyranosyl) oxy] 6-O-méthyl 3-oxo érythromycine.

On ajoute 724 mg de 1-éthyl 3-(3-diméthylaminopropyl) carbodiimide (EDAC) à 0,670 cm³ de diméthylsulfoxyde dans 4 cm³ de chlorure de méthylène, ajoute 616 mg du produit obtenu au stade C en solution dans 4 cm³ de chlorure de méthylène puis agite 15 minutes en maintenant la température à 16°C environ. On ajoute goutte à goutte 724 mg de trifluoroacétate de pyridinium dissous dans 5 cm³ de chlorure de méthylène. Après 3 heures d'agitation, on ajoute 10 cm³ de chlorure de méthylène et 10 cm³ d'eau, alcalinise à pH = 8 à l'aide d'ammoniaque, décante, lave avec une solution aqueuse saturée en chlorure de sodium, sèche et évapore le solvant. Après chromatographie sur silice (éluant : acétate d'éthyle), on obtient 369 mg de produit attendu. Rf = 0,28.

### Stade E : 9-O-[2-[(2-méthoxyéthyl) amino] éthyl] oxime de 3-dé [(2,6-didéoxy 3C-méthyl 3-O-méthyl alpha-L-ribo hexopyranosyl) oxy] 6-O-méthyl 3-oxo érythromycine.

On hydrogène (1,.5 bar) 369 mg de produit obtenu au stade D dans 10 cm**³** de méthanol en présence de 100 mg de palladium à 9,5% sur charbon actif. On agite le milieu réactionnel pendant 20 heures, filtre, lave au méthanol, évapore le solvant et chromatographie le résidu sur silice. On obtient 200 mg de produit attendu. Rf = 0,23.

### EXEMPLE 39 : (E) 9-O-[2-[(3-(diéthylamino) propyl] méthylamino] éthyl] oxime de 3-dé [(2,6-didéoxy 3C-méthyl 3-O-méthyl alpha-L-ribo hexopyranosyl) oxy] 6-O-méthyl 3-oxo érythromycine.

On ajoute 7,5 µl d'acide formique et 14,8 µl d'aldéhyde formique à 100 mg de (E) 9-O-[2-[(3-(diéthylamino) propyl] amino] éthyl] oxime de 3-dé [(2,6-didéoxy 3C-méthyl 3-O-méthyl alpha-L-ribo hexopyranosyl) oxy] 6-O-méthyl 3-oxo érythromycine préparé comme indiqué à l'exemple 34 en solution dans 6 cm**³** de chloroforme. On agite 1 heure à température ambiante puis chauffe au reflux pendant 3 heures. On laisse revenir à température ambiante, ajoute 10 cm**³** d'eau, alcalinise le milieu réactionnel jusqu'à pH = 9 à l'aide de soude, extrait au chlorure de méthylène aqueux, sèche la phase organique et élimine le solvant sous pression réduite. On recueille 96 mg de produit brut que l'on chromatographie sur silice (éluant : acétate d'éthyle-triéthylamine 95-5). On reprend le résidu dans le chlorure de méthylène, filtre et évapore le solvant. On obtient 69 mg de produit attendu.

| Spectre RMN (ppm) | |
|---|---|
| 1,02 (t) 2,52 (q) | les NCH₂CH₃ |
| de 2,35 à 2,75 | les NCH₂CH₂ (6H)M |

En opérant comme à l'exemple 39, on a préparé les produits des exemples 40 à 43 en utilisant au départ, respectivement les produits obtenus aux exemples 23, 24, 26, 13.

**EXEMPLE 40 : (E) 9-O-[2-(méthylpropylamino) éthyl] oxime de 3-dé [(2,6-diaéomy 3C-méthyl 3-O-méthyl alpha-L-ribo hexopyranosyl) oxy] 6-O-méthyl 3-oxo érythromycine.**
Rf = 0,39 (AcOEt-TEA 96-4)

**EXEMPLE 41 : (E) 9-O-[2-[[2-(diméthylamino) éthyl] méthylamino] éthyl] oxine de 3-de [(2,6-didéoxy 3C-méthyl 3-O-méthyl alpha-L-ribo hexopyranosyl) oxy] 6-O-méthyl 3-oxo érythromycine.**
Rf = 0,25 (Ether iso-MEOH-TEA 80-10-10)

**EXEMPLE 42 : (E) 9-O-[2-[[3-(diméthylamino) propyl] méthylamino] éthyl] oxime de 3-dé [(2,6-didéoxy 3C-méthyl 3-O-méthyl alpha-L-ribo hexopyranosyl) oxy] 6-O-méthyl 3-oxo érythromycine.**
Rf = 0,30 (CH**₂**Cl**₂**-MEOH-TEA 94-3-3)

**EXEMPLE 43 : (E) 9-O-[2-[[2-(1-pyrrolidinyl) éthyl] méthylamino] éthyl] oxime de 3-dé [(2,6-didéomy 3C-méthyl 3-O-méthyl alpha-L-ribo hexopyranonyl) oxy] 6-O-méthyl 3-oxo érythromycine.**
Rf = 0,2 (AcOEt-MEOH-TEA 92-5-3)

**EXEMPLE 44 : 9-O-((2-méthoxyéthoxy) méthyl) oxime de 3-déoxy-2,3 didéhydro-3-O-dé(2,6didéoxy-3-C-méthyl-3-O-méthyl-alpha-L-ribohéxopyranosyl)-6-O-méthyl-érythromycine**
[alpha]_{**D**} = -32° ( C = 1% CHCl**₃**)

**EXEMPLE 45 : 9-O-((2-méthyl-4-thiazolyl)méthyl)oxime de** **-3-dé(2,6-didéoxy-3-C-méthyl-3-O-méthyl-alpha-L -ribohéxopyranosyl)-6-O-méthyl-3-oxo-érythromycine**
M.P. = 116-118°C

**EXEMPLE 46 : 9-O-((2-méthoxyéthoxy)méthyl)oxime de-3-O-dé-(2,6-didéoxy-3 -C-méthylalpha-L-ribohéxopyranosyl)-3-O-phénylaminocarbonyl)-érythromycine**
[alpha]_{**D**} : -18,5° ( C = 1% CHCl**₃**)

### EXEMPLE 47 : 3-dé(2,6-didéoxy-3-C-méthyl-3-O-méthyl-alpha-L-ribohéxopyranosyl)oxy-6-O-methyl-3-oxo-érythromycine

**EXEMPLE 48 : (E)9-O-(2-(((tétrahydro-2-furanyl)méthyl)éthyl) oxime de 3-dé(2,6-didéoxy-3-C-méthyl-3-O-méthyl-alpha-L-ribohéxopyranosyl)oxy)-6-O-méthyl-3-oxo-érythromycine**
M.P. = 129-131°C

**EXEMPLE 49 : (E)9-O-(2-((2-propényl)amino)de-3-dé(2,6-didéoxy-3-C-méthyl-3-O-méthyl-alpha-L-ribohéxopyranosyl)ozy)-6-O-méthyl-3-oxo-érythromysine**
M.P. = 174-176°C

**EXEMPLE 50 : (Z)9-oxime de 3-dé(2,6-didéoxy-3-C-méthyl-3-O-méthyl-alpha-L-ribohéxopyranosyl)oxy)-6-O-méthyl-3-oxoérythromycine**
M.P. = 228-230°C

**EXEMPLE 51 : (2R)(E)-(2-amino-3-méthoxy-3-oxo-propyl) oxime de 3-dé(2,6-didéoxy-3-C-méthyl-3-O-méthyl-alpha-L-ribohéxopyranosyl)oxy)-6-O-méthyl-3-oxo-érythromycine**
Rf : 0,33 (ETIso-MeOH-TEA 85/10/5)

**EXEMPLE 52 : (E)9-O-(2-((3-1H-imidasol-1-yl)propyl)-amino)-éthyl) oxime de-3-dé(2,6-didéoxy-3-C-méthyl-3-O-méthyl-alpha-L-ribohéxopyranosyl)oxy)-6-O-méthyl-3-oxo-érythromycine**
Rf : 0,4 (CHCl**₃**-MeoH-NH**₄**,OH 90/10/1)

**EXEMPLE 53 : (E) 9-O-((2-pipéridinyl)méthyl)oxime de-3-dé(2,6-didéoxy-3-C-méthyl-3-O-méthyl-alpha-L-ribohéxopyranosyl)oxy)-6-O-méthyl-3-oxo-érythromycine**
Rf : 0,5 (AcOEt-MeOH- TEA 10/5/5 )

**EXEMPLE 54 : (E) 9-O-((3-pipéridinyl)oxime de-3-dé(2,6-didéoxy-3-C-méthyl-3-O-méthyl-alpha-L-ribohéxopyranosyl)oxy)-6-O-méthyl-3-oxo-érythromycine**
Isomère A

### Rf : 0,35 (CHCl₃-MeOH-NH₄OH 92/8/0,5)

**EXEMPLE 55 : (E)9-O-((3-pipéridinyl)oxime de-3-dé(2,6-didéoxy-3-C-méthyl-3-O-méthyl-alpha-L-ribohéxopyranosyl)oxy)-6-O-méthyl-3-oxo-érythromycine**

### Isomère B

### Rf : 0,32 (CHCl₃-MeOH-NH₄OH 92/8/0,5)

**EXEMPLE 56 : (E)9-O-(2-(méthyl(2-propényl)amino)éthyl)oxime de 3-dé(2,6-didéoxy-3-C-méthyl-3-O-méthyl-alpha-L-ribohéxopyranosyl)oxy)-6-O-méthyl-3-oxo-érythromycine**
M.P. = 154-166°C

**EXEMPLE 57 : (E) 9-O-(2-(méthylamino)éthyl)oxime de 3-dé(2,6-didéoxy-3-C-méthyl-3-O-méthyl-alpha-L-ribohéxopyranosyl)oxy)-6-O-méthyl-3-oxo-érythromycine**
M.P. = 163-165°C

**EXEMPLE 58 : (E)9-O-(3-(1(diphénylméthyl)azétidinyl)oxime de 3-dé(2,6-didéoxy- 3-C-méthyl-3-O-méthyl-alpha-L-ribohéxopyranosyl)oxy)-6-O-méthyl-3-oxo-érythromycine**
Rf : 0,2 (ACOEt-TEA 98/2)

**EXEMPLE 59 : (E)9-O-((1-méthyl-2-pipéridinyl)méthyl) oxime de 3-dé(2,6-didéoxy-3-C-méthyl-3-O-méthyl-alpha-L-ribohéxopyranosyl)oxy)-6-O-méthyl-3-oxo-érythromycine**
Rf : 0,38 (ACOEt-TEA 95/5 )

**EXEMPLE 60 : (E)9-O-((1-azabicyclo(2.2.2)octan-3-yl)oxime de 3-dé(2,6-didéoxy-3-C-méthyl-3-O-méthyl-alpha-L-ribohéxopyranosyl)oxy)-6-O-méthyl-3-oxo-érythromycine (dioxalate)**
M.P. = 182-184°C

Comme exemples des produits de formule (I), on peut citer les produits suivants, correspondant aux produits des exemples précédents, dans lesquels X et X' ont la signification suivante : =NOH 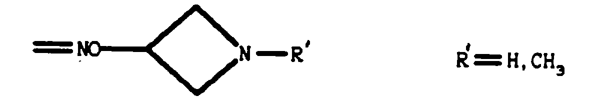 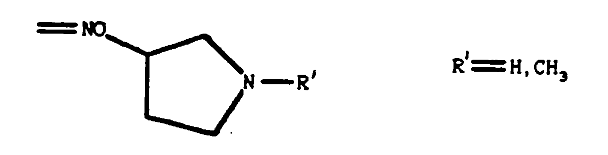 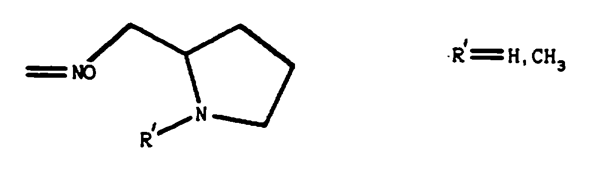 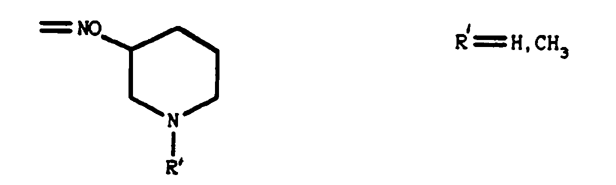 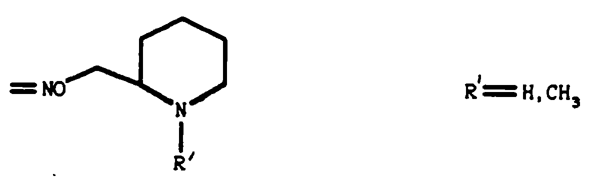 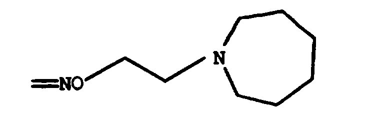 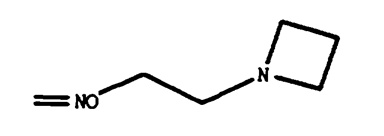 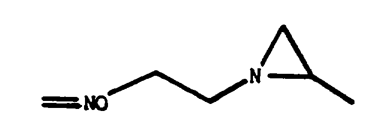 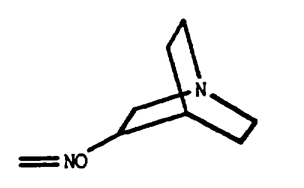 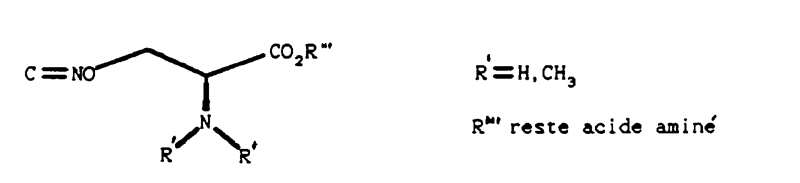 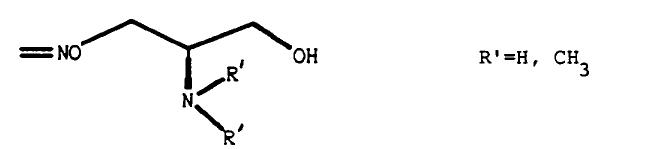 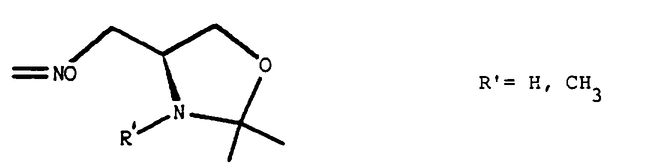 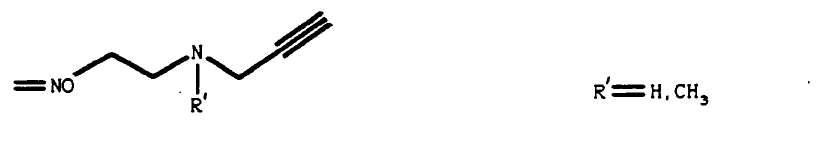 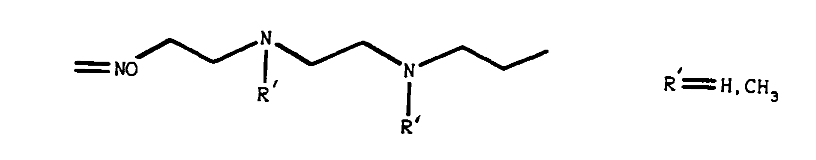 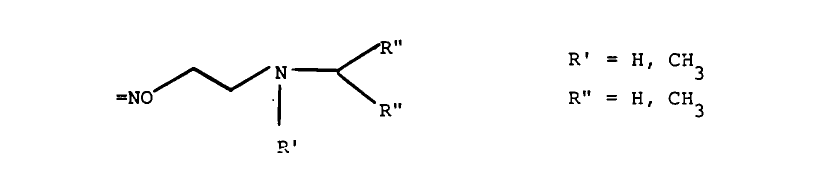 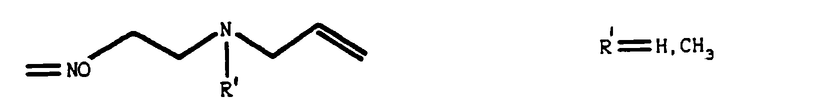 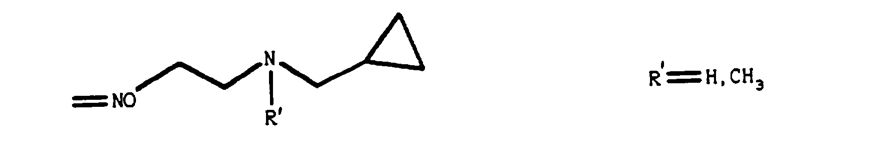 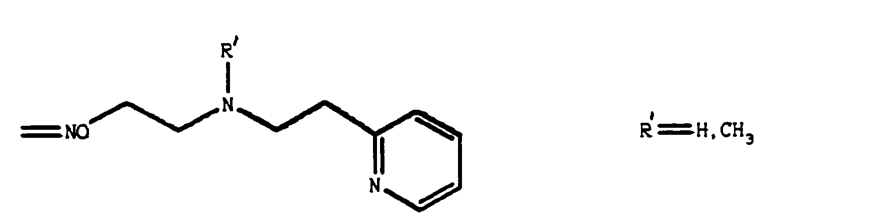 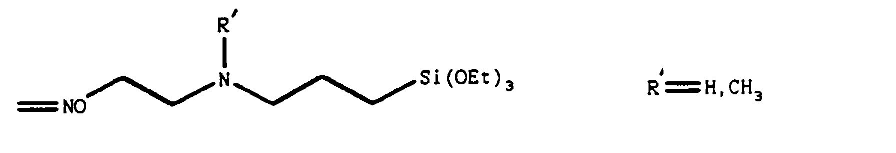 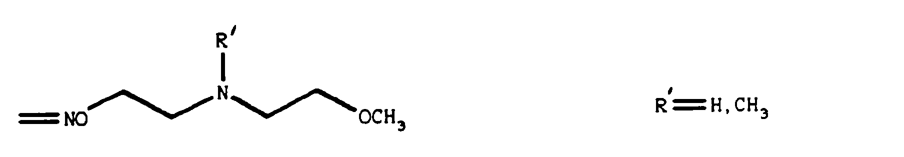 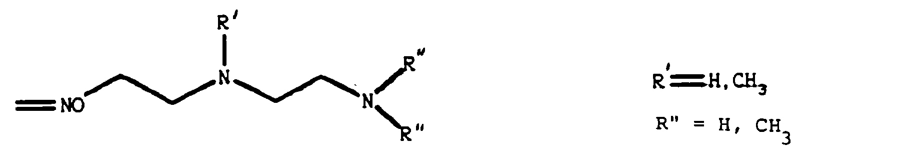 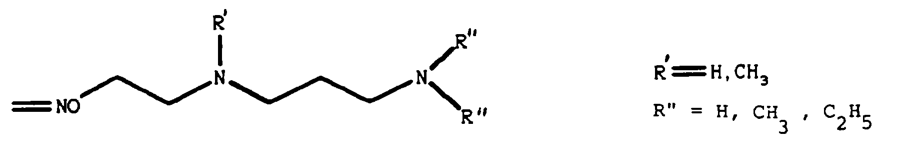 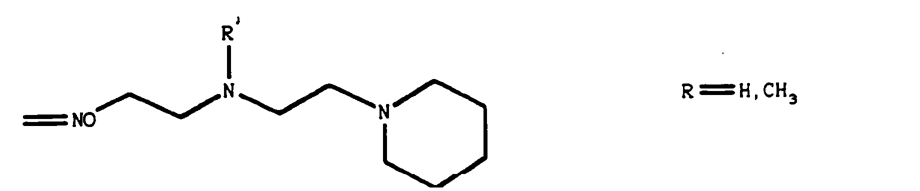 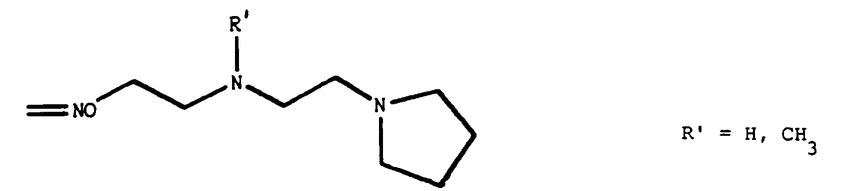 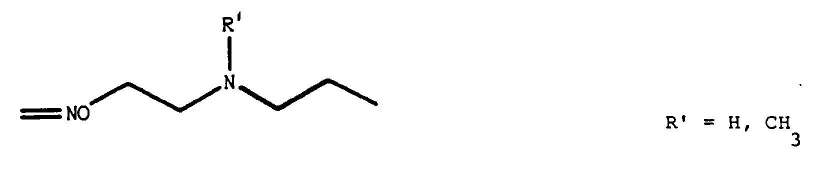 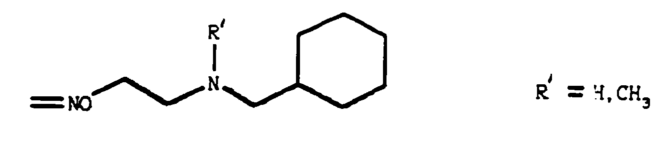 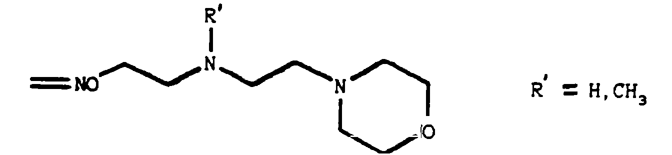 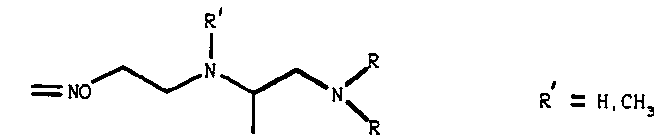 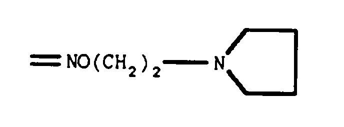 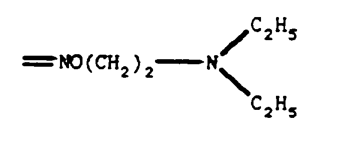 = NO(CH**₂**)**₂**-NH**₂** 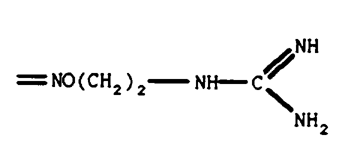 = NO(CH₂)₂Br

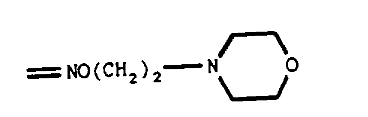

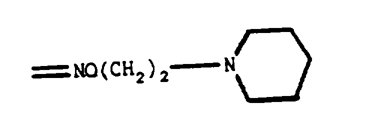

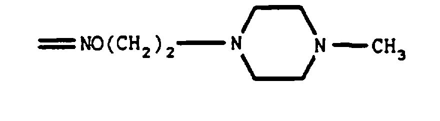

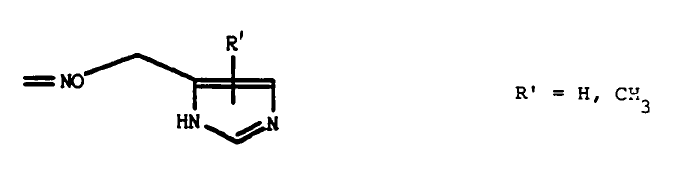

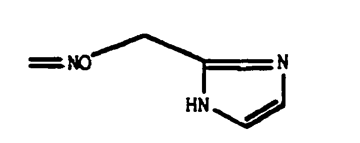

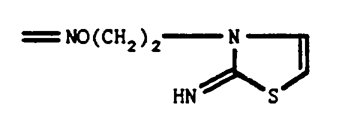

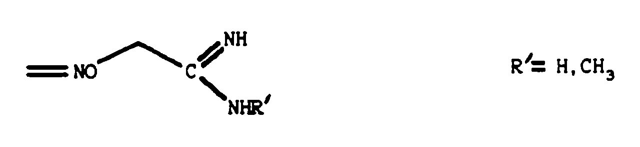

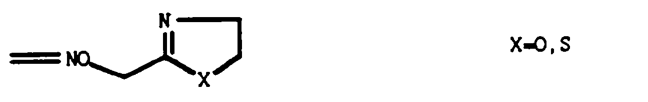

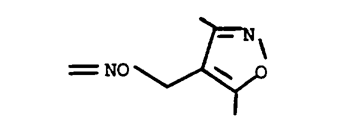

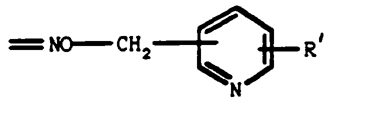

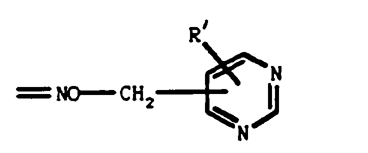

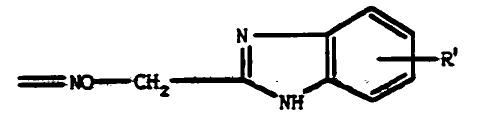

### EXEMPLES de compositions pharmacologiques

On a préparé des composés renfermant :

| | |
|---|---|
| Produit de l'exemple 1 | 150 mg |
| Excipient q.s.p. | 1 g |

Détail de l'excipient : amidon, talc, stéarate de magnésium.

| | |
|---|---|
| Produit de l'exemple 7 | 150 mg |
| Excipient q.s.p. | 1 g |

Détail de l'excipient : amidon, talc, stéarate de magnésium.

| | |
|---|---|
| Produit de l'exemple 10 | 150 mg |
| Excipient q.s.p. | 1 g |

Détail de l'excipient : amidon, talc, stéarate de magnésium.

### ETUDE PHARMACOLOGIQUE DES PRODUITS DE L'INVENTION.

### A) Activité in vitro.

### Méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm**³**.

Les résultats suivants ont été obtenus :
1) Produit de l'exemple 1 - Lecture après : 24 h. 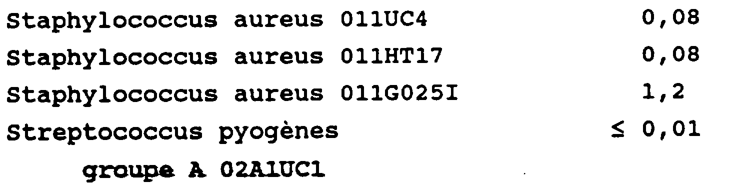 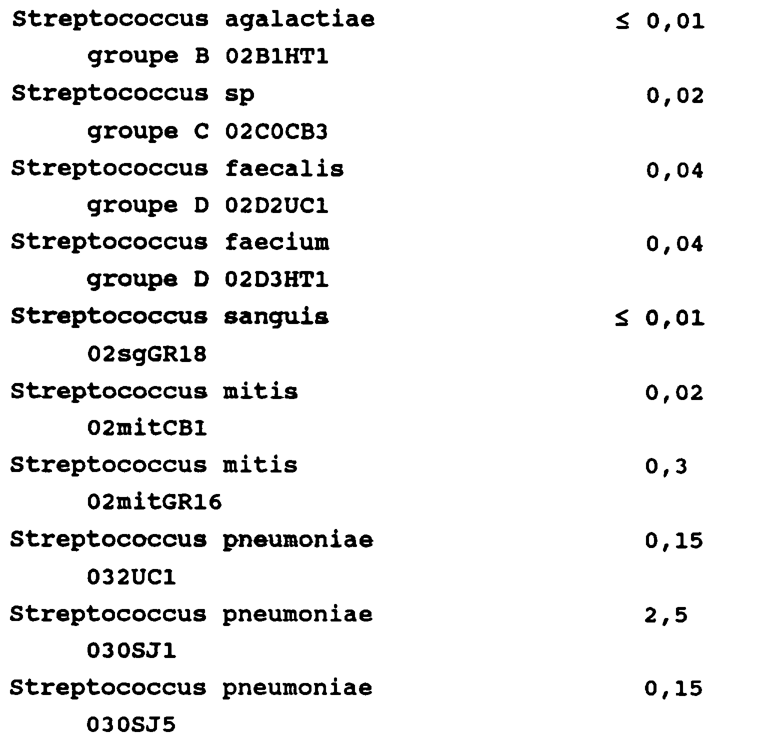
2) Produit de l'exemple 2 - Lecture après : 24 h. 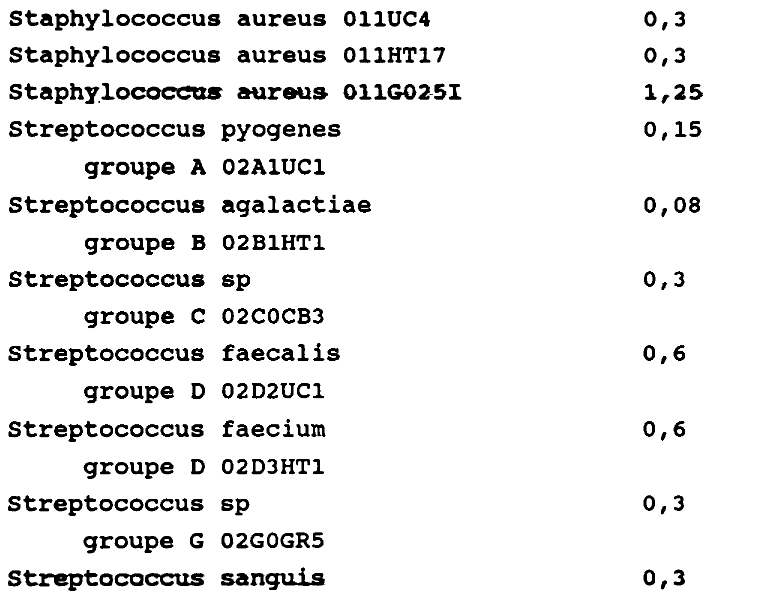 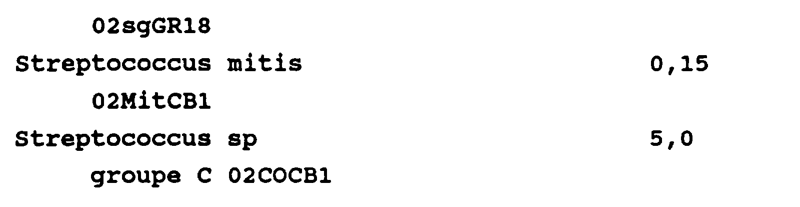
3) Produit de l'exemple 7 - Lecture après : 24 h.

| | |
|---|---|
| Staphylococcus aureus 011UC4 | 0,15 |
| Staphylococcus aureus 011HT17 | 0,08 |
| Staphylococcus aureus 011G025I | 0,3 |
| Streptococcus pyogenes | ≤ 0,02 |
| groupe A 02A1UC1 | |
| Streptococcus agalactiae | ≤ 0,02 |
| groupe B 02B1HT1 | |
| Streptococcus sp | 0,04 |
| groupe C 02C0CB3 | |
| Streptococcus faecalis | 0,04 |
| groupe D 02D2UC1 | |
| Streptococcus faecium | 0,04 |
| groupe D 02D3HT1 | |
| Streptococcus sanguis | 2,5 |
| 02sgGR18 | |
| Streptococcus mitis | ≤ 0,02 |
| 02 mitCB1 | |
| Streptococcus pneumoniae | ≤ 0,02 |
| 032UC1 | |
| Streptococcus pneumoniae | 0,3 |
| 030SJ5 | |

4) Produit de l'exemple 10 - Lecture après : 24 h. 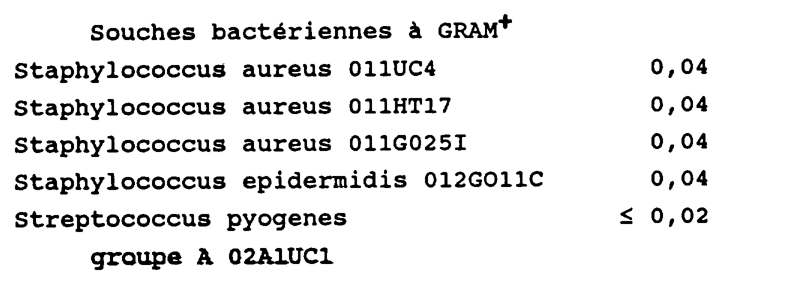 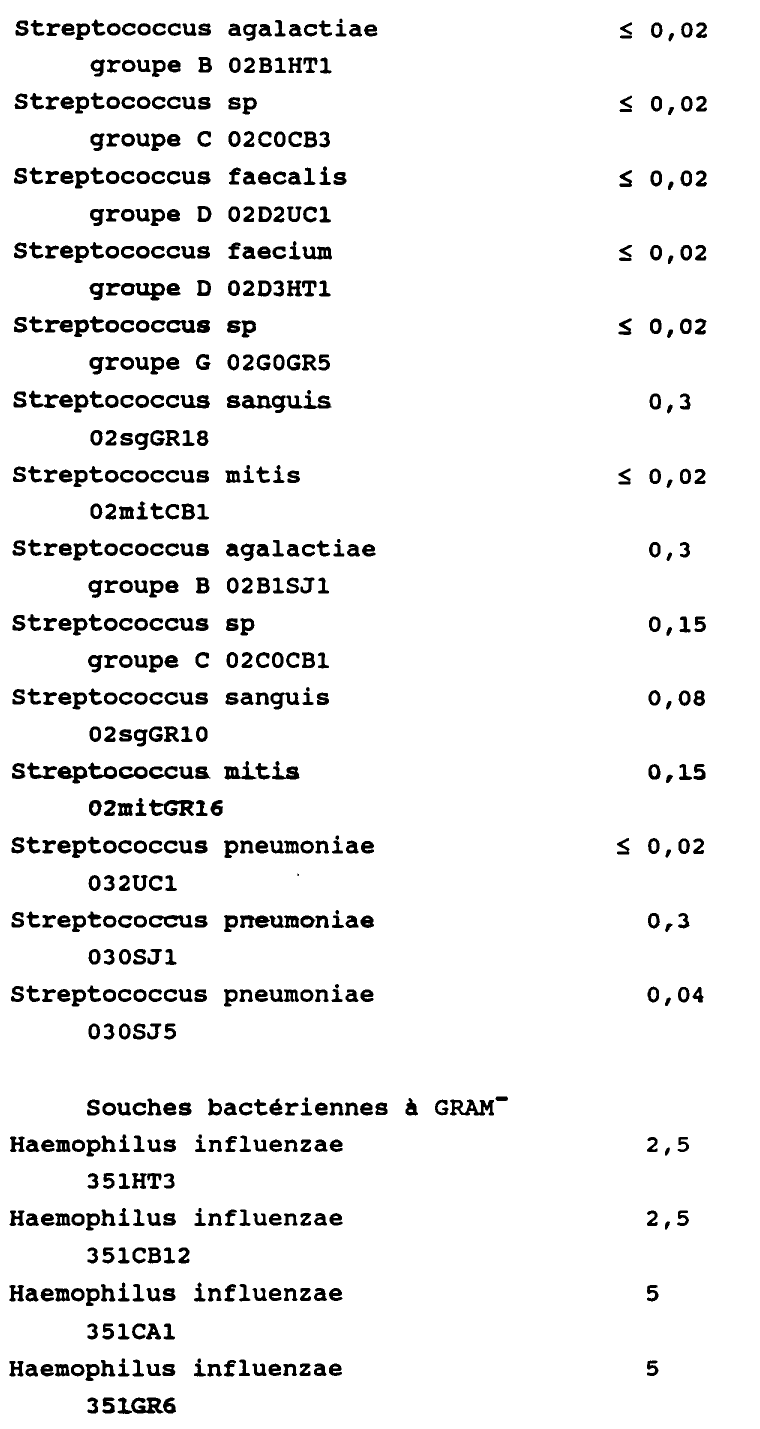 ≤≤≤≤
5) Produit de l'exemple 13 - Lecture après : 24 h.

| Souches bactériennes à GRAM⁺ | |
|---|---|
| Staphylococcus aureus 011UC4 | 0,3 |
| Staphylococcus aureus 011HT17 | 0,3 |
| Staphylococcus aureus 011G025I | 1,2 |
| Staphylococcus epidermidis 012GO11C | 0,6 |
| Streptococcus pyogenes | 0,04 |
| groupe A 02A1UC1 | |
| Streptococcus agalactiae | < 0,02 |
| groupe B 02B1HT1 | |
| Streptococcus sp | 0,08 |
| groupe C 02C0CB3 | |
| Streptococcus faecalis | 0,15 |
| groupe D 02D2UC1 | |
| Streptococcus faecium | 0,15 |
| groupe D 02D3HT1 | |
| Streptococcus sp | 0,04 |
| groupe G 02G0GR5 | |
| Streptococcus sanguis | ≤ 0,02 |
| 02sgGR18 | |
| Streptococcus mitis | 0,04 |
| 02mitCB1 | |
| Streptococcus agalactiae | - |
| groupe B 02B1SJ1 | |
| Streptococcus sp | - |
| groupe C 02C0CB1 | |
| Streptococcus sanguis | 2,5 |
| 02sgGR10 | |
| Streptococcus mitis | - |
| 02mitGHR16 | |
| Streptococcus pneumoniae | - |
| 032UC1 | |
| Streptococcus pneumoniae | 0,6 |
| 030SJ1 | |
| Streptococcus pneumoniae | 1,2 |
| 030SJ5 | |

6) Produit de l'exemple 36 - Lecture après : 24 h.

| Souches bactériennes à GRAM⁺ | |
|---|---|
| Staphylococcus aureus 011UC4 | 1,2 |
| Staphylococcus aureus 011HT17 | 0,3 |
| Staphylococcus aureus 011G025I | - |
| Staphylococcus epidermidis 012GO11C | 2,5 |
| Streptococcus pyogenes | 0,15 |
| groupe A 02A1UC1 | |
| Streptococcus agalactiae | 0,04 |
| groupe B 02B1HT1 | |
| Streptococcus sp | 0,3 |
| groupe C 02C0CB3 | |
| Streptococcus faecalis | 0,3 |
| groupe D 02D2UC1 | |
| Streptococcus faecium | 0,15 |
| groupe D 02D3HT1 | |
| Streptococcus sp | 0,3 |
| groupe G 02G0GR5 | |
| Streptococcus sanguis | - |
| 02sgGR18 | |
| Streptococcus mitis | 0,08 |
| 02mitCB1 | |
| Streptococcus agalactiae | - |
| groupe B 02B1SJ1 | |
| Streptococcus sp | - |
| groupe C 02C0CB1 | |
| Streptococcus sanguis | - |
| 02sgGR10 | |
| Streptococcus mitis | - |
| 02mitGHR16 | |
| Streptococcus pneumoniae | 0,08 |
| 032UC1 | |
| Streptococcus pneumoniae | - |
| 030SJ1 | |
| Streptococcus pneumoniae | - |
| 030SJ5 | |

7) Produit de l'exemple 37 - Lecture après : 24 h.

| Souches bactériennes à GRAM⁺ | |
|---|---|
| Staphylococcus aureus 011UC4 | 0,15 |
| Staphylococcus aureus 011HT17 | 0,08 |
| Staphylococcus aureus 011G025I | - |
| Staphylococcus epidermidis 012G011C | 0,15 |
| Streptococcus pyogenes | 0,04 |
| groupe A 02A1UC1 | |
| Streptococcus agalactiae | ≤ 0,02 |
| groupe B 02B1HT1 | |
| Streptococcus sp | 0,04 |
| groupe C 02C0CB3 | |
| Streptococcus faecalis | 0,08 |
| groupe D 02D2UC1 | |
| Streptococcus faecium | 0,08 |
| groupe D 02D3HT1 | |
| Streptococcus sp | 0,04 |
| groupe G 02G0GR5 | |
| Streptococcus sanguis | 1,2 |
| 02sgGR18 | |
| Streptococcus mitis | 0,04 |
| 02mitCB1 | |
| Streptococcus agalactiae | 1,2 |
| groupe B 02B1SJ1 | |
| Streptococcus sp | 1,2 |
| groupe C 02C0CB1 | |
| Streptococcus sanguis | 0,6 |
| 02sgGR10 | |
| Streptococcus mitis | 0,3 |
| 02mitGR16 | |
| Streptococcus pneumoniae | ≤ 0,02 |
| 032UC1 | |
| Streptococcus pneumoniae | 2,5 |
| 030SJ1 | |
| Streptococcus pneumoniae | 0,3 |
| 030SJ5 | |

**B) Activité in vivo.**

### Infection expérimentale à Staphylococcus aureus

On a étudié l'action du produit de l'exemple 2 sur une infection expérimentale à Staphylococcus aureus de la souris.

On a infecté des lots de dix souris mâles d'un poids de 18 à 20 g par injection intrapéritonéale de 0,5 cm**³** d'une culture de 22 heures en bouillon à pH 7 de la souche de Staphylococcus aureus n° 54 146, diluée au 1/6 par de l'eau physiologique.

On a administré per os au moment de l'infection et 4 heures après l'infection une quantité déterminée de produit.

Les résultats obtenus sont les suivants :

| MORTALITE ANIMAUX | | | | |
|---|---|---|---|---|
| POSOLOGIE en mg | J1 24 h | J2 48 h | J3 72 h | SURVIVANTS APRES 3 JOURS |
| Témoin | 9 | | | 1/10 |
| 0,1 | 7 | 3 | | 0/10 |
| 0,3 | 1 | 1 | 2 | 6/10 |
| 1 | 0 | | | 10/10 |
| 3 | 0 | | | 10/10 |

DP**₅₀** Administration totale : 12,49 mg/kg (Méthode de Reed et Muench).

Conclusion : les produits de l'invention présentent une bonne activité antibiotique in vivo.

## Revendications (Revendications pour l'(les) Etat(s) contractant(s) suivant(s): AT, BE, CH, DE, DK, FR, GB, IT, LI, LU, NL, SE)

1. Les composés de formule (I) : 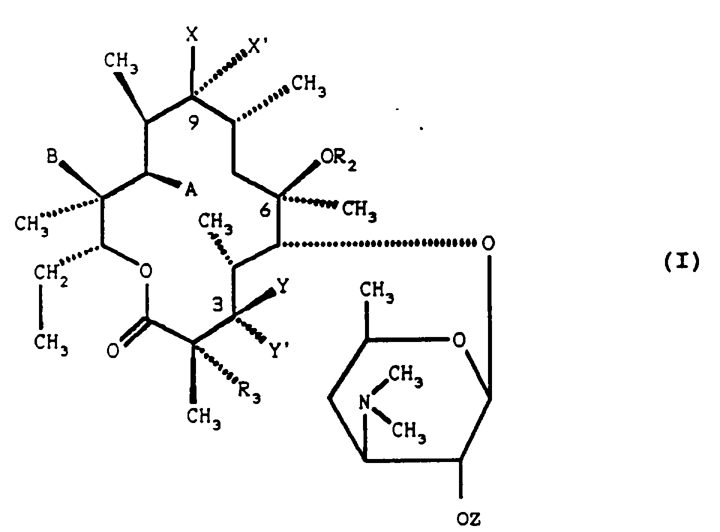
dans laquelle,
**ou bien** X et X' forment ensemble avec l'atome de carbone auquel ils sont liés un groupement C=O ou C=NOR, dans lequel R représente :
- un atome d'hydrogène,
- un radical hétérocyclique renfermant au moins un atome d'azote et éventuellement un autre hétéroatome, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- un radical alkyle, alkényle ou alkynyle linéaire, ramifié ou cyclique, renfermant jusqu'à 18 atomes de carbone éventuellement substitué par un ou plusieurs groupements :
. hydroxyle,
. halogène,
. cyano,
. nitro,
. amidinyle,
. guanidinyle,
. hétérocyclique, tel que défini précédemment,
. alkyloxy, alkényloxy ou alkynyloxy ayant au plus 6 atomes de carbone,
. alkylthio, alkénylthio ou alkynylthio ayant au plus 6 atomes de carbone, l'atome de soufre étant éventuellement oxydé en sulfoxyde ou en sulfone,
. aryle, aralkyle,
. aryloxy, aralkyloxy,
. arylthio, aralkylthio, l'atome de soufre étant éventuellement oxydé en sulfoxyde ou en sulfone, (chacun de ces radicaux alkyloxy, alkényloxy, alkynyloxy, alkylthio, alkénylthio ou alkynylthio, aryle, aralkyle, aryloxy, aralkyloxy, arylthio ou aralkylthio étant éventuellement substitué par un ou plusieurs des groupements suivants : hydroxy, alkyloxy, alkylthio ayant de 1 à 6 atomes de carbone, alkénylthio, alkynylthio ayant jusqu'à 6 atomes de carbone, amino, monoalkylamino ayant jusqu'à 6 atomes de carbone, dialkylamino ayant jusqu'à 12 atomes de carbone, un radical amidinyle, guanidinyle, un radical hétérocyclique tel que défini précédemment, les radicaux aryle, aryloxy, arylthio, aralkyle, aralkyloxy et aralkylthio étant de plus éventuellement substitués par les radicaux méthyle, éthyle, propyle, carbamoyle, aminométhyle, diméthylaminométhyle, aminoéthyle, diméthylaminoéthyle, carboxyle, méthyloxycarbonyle, éthyloxycarbonyle)
. 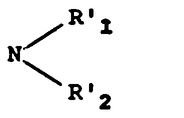 dans lequel :
**ou bien R'₁ et R'₂** identiques ou différents représentent un atome d'hydrogène, un radical alkyle, alkényle ou alkynyle, linéaire, ramifié ou cyclique renfermant jusqu'à 18 atomes de carbone, un radical aryle ou aralkyle, chacun de ces radicaux R'**₁** et R'**₂** étant éventuellement substitué par un ou plusieurs radicaux hydroxy, alkyloxy, alkényloxy, alkynyloxy, alkylthio, alkénylthio ou alkynylthio renfermant jusqu'à 8 atomes de carbone, amino, monoalkylamino renfermant jusqu'à 4 atomes de carbone, dialkylamino renfermant jusqu'à 8 atomes de carbone, cyano, carboxyle libre, estérifié ou salifié, acyle ou carbamoyle, renfermant jusqu'à 8 atomes de carbone, par un radical Si(alc)₃ ou Si(Oalc)**₃** dans lequel alc représente un radical alkyle renfermant jusqu'à 4 atomes de carbone, par un radical hétérocyclique tel que défini précédemment,
**ou bien R'₁ et R'₂** forment ensemble avec l'atome d'azote auquel ils sont liés un radical hétérocycle mono ou bicyclique, renfermant éventuellement un autre hétéroatome, saturé ou insaturé, aromatique ou non aromatique, comportant jusqu'à 12 chaînons ;
. un groupement ammonium quaternaire,
. 1,2-époxyéthyle ou 2,2-diméthyl 1,2-époxyéthyle ou un radical résultant de l'ouverture de ce groupement par un réactif nucléophile,
. 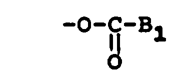 dans lequel B**₁** représente soit un radical alkyle ou alkyloxy ayant au plus 6 atomes de carbone, soit un radical aryle, aralkyle, aryloxy ou aralkyloxy,
. formyle libre ou protégé, carboxyle libre, estérifié ou salifilé, thiocyanate, acyle ou carbonyle,
. (CH**₂**)_{**n**}R', R' représentant le reste d'un acide aminé, et n représentant un nombre entier compris entre 0 et 6, **ou bien X** représente un radical 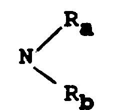
- R_{**a**} et R_{**b**} identiques ou différents l'un de l'autre, représentant un atome d'hydrogène ou un radical hydrocarboné renfermant jusqu'à 18 atomes de carbone, renfermant éventuellement un ou plusieurs hétéroatomes, éventuellement substitué par un ou plusieurs groupements fonctionnels, ou par un radical hétérocyclique renfermant au moins un atome d'azote et éventuellement un autre hétéroatome choisi parmi les atomes d'oxygène, de soufre ou d'azote, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- R_{**a**} et R_{**b**} pouvant éventuellement former avec l'atome d'azote auquel ils sont liés un radical hétérocycle, renfermant au moins un atome d'azote et éventuellement un autre hétéroatome choisi parmi les atomes d'oxygène, de soufre ou d'azote, mono ou bicyclique, saturé ou insaturé, aromatique ou non aromatique comportant jusqu'à 12 chaînons,
- R_{**a**} et R_{**b**} pouvant former avec le radical A un cycle 9-N, 11-O,
**et X'** représente un atome d'hydrogène,
**Y et Y'** identiques ou différents de X et X' ont la signification de X et X'
à représente un atome d'hydrogène ou un radical OR**₄**, R**₄** représentant un atome d'hydrogène ou forme avec A un radical carbonate ou carbamate,
**A** forme avec le carbone qui le porte et le carbone en 10, une double liaison,
**OU A** représente un radical OR'**₄**, R'**₄**, représentant un atome d'hydrogène ou forme avec B un radical carbonate,
**ou A** représente un radical 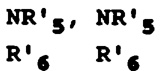 représentant un groupement C=O formant avec B un groupement carbamate, R'**₆** représentant un atome d'hydrogène ou un radical alkyle, aralkyle ou alkyloxy ayant jusqu'à 12 atomes de carbone ou un groupement 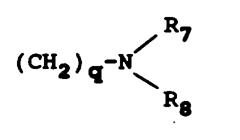
R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6,
**ou A** représente un radical 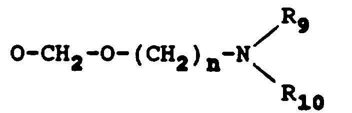
R₉ et R**₁₀** représentant un atome d'hydrogène ou un radical alkyle renfermant jusqu'à 8 atomes de carbone, ou formant avec l'atome d'azote un hétérocycle tel que défini précédemment et n représente un nombre entier compris entre 1 et 6,
**R₂** représente un radical alkyle renfermant jusqu'à 8 atomes de carbone, ou un radical CONH**₂** ou CONHCOR**₁₁** ou CONHSO**₂**R**₁₁** dans lesquels R**₁₁** représente un radical hydrocarboné renfermant jusqu'à 18 atomes de carbone comportant éventuellement un ou plusieurs hétéroatomes,
**R₃** en position alpha ou bêta représente un atome d'hydrogène ou un radical alkyle, renfermant jusqu'à 8 atomes de carbone ou un radical 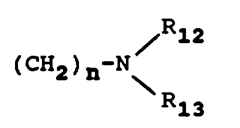 dans lequel R**₁₂** et R**₁₃** représente un atome d'hydrogène, un radical alkyle renfermant jusqu'à 8 atomes de carbone ou formant avec l'atome d'azote un hétérocycle tel que défini précédemment, n représentant un nombre entier compris entre 1 et 6, ou un radical 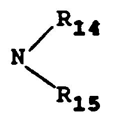
R**₁₄** et R**₁₅** identiques ou différents représentant un atome d'hydrogène ou un radical alkyle renfermant jusqu'à 8 atomes de carbone ou un hétéroatome ou un radical alkyle ou alkyloxy renfermant jusqu'à 8 atomes de carbone,
**Z** représente un atome d'hydrogène ou le reste d'un acide carboxylique renfermant jusqu'à 18 atomes de carbone les oximes que peuvent représenter X et X' ou Y et Y' peuvent être de configuration syn ou anti, ainsi que les sels d'addition avec les acides des composés de formule (I).

2. Les composés de formule (I) tels que définis à la revendication 1 dans lesquels X et X' forment ensemble avec l'atome de carbone auquel ils sont liés un groupement C=NOR, R conservant la même signification que dans la revendication 1.

3. Les composés de formule (I) tels que définis à la revendication 2 dans lesquels R représente un radical alkyle renfermant jusqu'à 6 atomes de carbone substitué par un radical 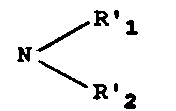 R'**₁** et R'**₂** conservant la même signification que dans la revendication 1.

4. Les composés de formule (I) tels que définis à la revendication 3 dans lesquels le radical R est un radical 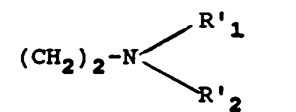 R'**₁** et R'**₂** représentant un radical alkyle renfermant jusqu'à 4 atomes de carbone.

5. Les composés de formule (I) tels que définis à la revendication 4 dans lesquels R représente un radical (CH**₂**)**₂**-N(CH**₃**)**₂**.

6. Les composés de formule (I) tels que définis à la revendication 3 dans lesquels le radical R est un radical 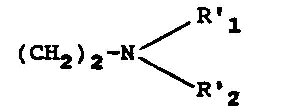 R'₁ représentant un atome d'hydrogène et R'**₂** représentant un radical alkyle renfermant jusqu'à 4 atomes de carbone substitué par un radical hétérocycle renfermant au moins un atome d'azote.

7. Les composés de formule (I) tels que définis à la revendication 6 dans lesquels R représente un radical

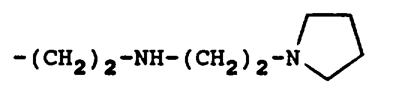

8. Les composés de formule (I) tels que définis à la revendication 2 dans lesquels R représente un radical alkyle renfermant jusqu'à 6 atomes de carbone, substitué par un radical alkyloxy renfermant jusqu'à 6 atomes de carbone éventuellement substitué par un radical méthoxy.

9. Les composés de formule (I) tels que définis à la revendication 6 dans lesquels R représente un radical :

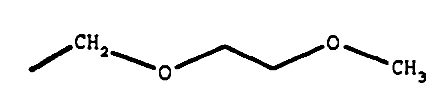

10. Les composés de formule (I) tels que définis à la revendication 2 dans lesquels R représente un hétérocycle renfermant au moins un atome d'azote.

11. Les composés de formule (I) tels que définis à la revendication 10 dans lesquels R représente un radical 3-pipéridinyle.

12. Les composés de formule (I) tels que définis à la revendication 1 dans lesquels X et X' forment ensemble avec l'atome de carbone auquel ils sont liés un groupement C=O.

13. Les composés de formule (I) tels que définis à la revendication 1 dans lesquels X et X' et Y et Y' forment ensemble un groupement C=O.

14. Les composés de formule (I) tels que définis à l'une quelconque des revendications 2 à 11 dans lesquels Y et Y' forment ensemble un groupement C=NOR, R conservant sa signification précédente et notamment un radical benzyle.

15. Les composés de formule (I) tels que définis à l'une quelconque.des revendications 1 à 14 dans lesquels R**₂** représente un radical alkyle renfermant de 1 à 4 atomes de carbone.

16. Les composés de formule (I) tels que définis à la revendication 15, dans lesquels R**₂** représente un radical méthyle.

17. Les composés de formule (I) tels que définis à l'une quelconque des revendications 1 à 16 dans lesquels R**₃** représente un atome d'hydrogène (alpha ou bêta).

18. Les composés de formule (I) tels que définis à l'une quelconque des revendications 1 à 17 dans lesquels A représente un radical OH.

19. Les composés de formule (I) tels que définis à l'une quelconque des revendications 1 à 18 dans lesquels B représente un radical OH.

20. Les composés de formule (I) tels que définis à l'une quelconque des revendications 1 à 17, dans lesquels A et B forment ensemble un groupement 11,12 carbonate cyclique.

21. Les composés de formule (I) tels que définis à l'une quelconque des revendications 1 à 17 dans lesquels A et B forment ensemble un radical divalent 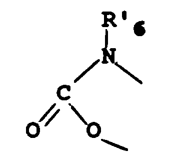 R'**₆** représentant un atome d'hydrogène ou un radical alkyle, aralkyle ou alkyloxy ayant jusqu'à 12 atomes de carbone ou un groupement : 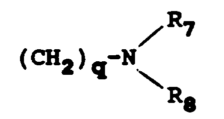 R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6,

22. Les composés de formule (I) tels que définis à la revendication 21 dans lesquels R'₆ représente un radical arakyle comportant jusqu'à 12 atomes de carbone.

23. Les composés de formule (I) tels que définis à la revendication 21 dans lesquels R'**₆** représente un radical (CH**₂**),C**₄**C**₆**H₅.

24. Les composés de formule (I) tels que définis à l'une quelconque des revendications 1 à 23 dans lesquels Z représente un atome d'hydrogène.

25. Le composé de formule (I) tel que défini à la revendication 1 dont le nom suit :
- la 9-[O-[2-(diméthylamino) éthyl] oxime] de 3-dé[(2,6-dideoxy 3-C-méthyl 3-O-méthyl alpha-L-ribo-hexopyranosyl) oxy] 6-O-méthyl 3-oxo érythromycine.

26. Le composé de formule (I) tels que défini à la revendication 1 dont le nom suit :
- la 11,12-dideoxy-3-de[(2,6-dideoxy 3-C-méthyl 3-O-méthyl alpha-L-ribohexopyranosyl) oxy] 6-O-méthyl 3-oxo12,11-[oxycarbonyl[(4-phénylbutyl) imino]] érythromycine.

27. Les composés de formule (I) tels que définis à la revendication 1 dont les noms suivent :
- la 9-[O-[(2-méthoxy éthoxy) méthyl] oxime] de 3-de[(2,6-dideoxy 3-C-méthyl 3-O-méthyl alpha-L-ribo-hexopyranosyl) oxy] 6-O-méthyl 3-oxo érythromycine,
- la 3-dé[(2,6-dideoxy 3-C-méthyl 3-O-méthyl alpha-L-ribo-hexopyranosyl) oxy] 6-O-méthyl 3-oxo érythromycine,
- le 11,12-carbonate cyclique de 3-de[(2,6-dideoxy 3-C-méthyl 3-O-méthyl alpha-L-ribohexopyranosyl) oxy] 6-O-méthyl 3-oxoérythromycine.

28. Les composés de formule (I) tels que définis à la revendication 1, dont les noms suivent :
- (E) 9-O-[2-[[2-(1-pyrrolidinyl éthyl] amino] éthyl] oxime de 3-dé [(2,6-didéoxy 3C-méthyl 3-O-méthyl alpha-L-ribo hexopyranosyl) oxy] 6-O-méthyl 3-oxo érythromycine,
- (E) 9-O-(3-pipéridinyl) oxime de 3-dé [(2,6-didéoxy 3C-méthyl 3-O-méthyl alpha-L-ribo hexopyranosyl) oxy] 6-O-méthyl 3-oxo érythromycine,
- (E) 9-O-[2-(diméthylamino) éthyl] oxime de 3-dé [(2,6-didéoxy 3C-méthyl 3-O-méthyl alpha-L-ribo hexopyranosyl) oxy] 11-déoxy 10,11-didéhydro 6-O-méthyl 3-oxo érythromycine.

29. A titre de médicaments, les composés de formule (I) tels que définis à l'une quelconque des revendications 1 à 25 ainsi que leurs sels d'addition avec les acides pharmaceutiquement acceptables.

30. A titre de médicaments, les composés de formule (I) tels que définis à l'une quelconque des revendications 25 à 28, ainsi que leurs sels d'addition avec les acides pharmaceutiquement acceptables.

31. Les compositions pharmaceutiques renfermant comme principe actif au moins un médicament défini à la revendication 29 ou 30.

32. Procédé de préparation des composés de formule (I) tels que définis à l'une quelconque des revendications 1 à 28, caractérisé en ce que l'on soumet un composé de formule (II) : 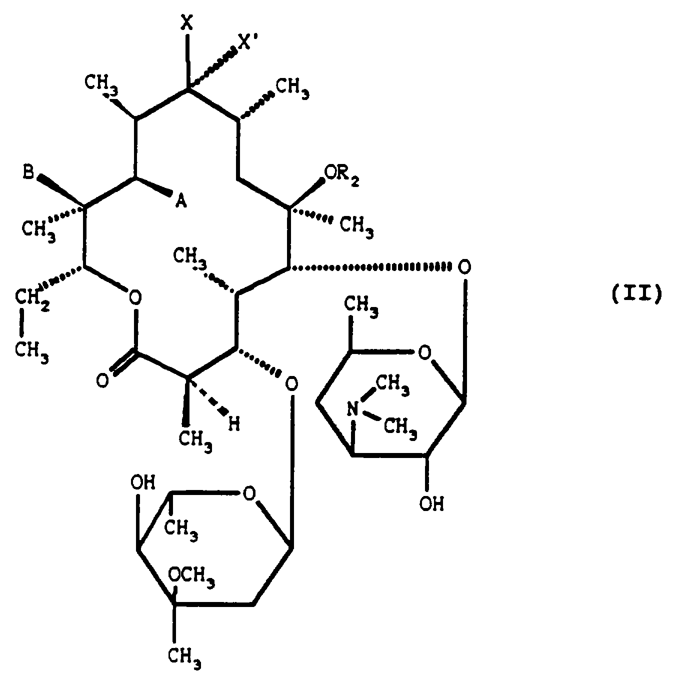 dans laquelle X, X', R**₂**, B et A conservent leur signification précédente, à l'action d'un acide en milieu aqueux pour obtenir le composé de formule (III) : 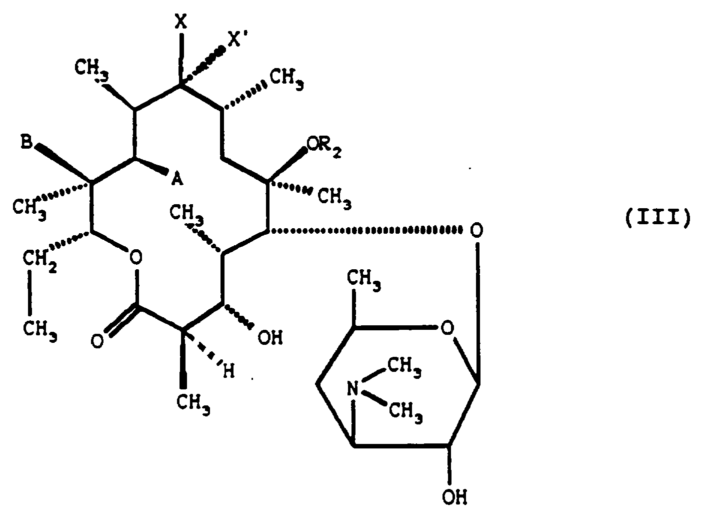 que l'on soumet à l'action d'un agent de blocage de la fonction hydroxyle en 2', pour obtenir un composé de formule (IV) : 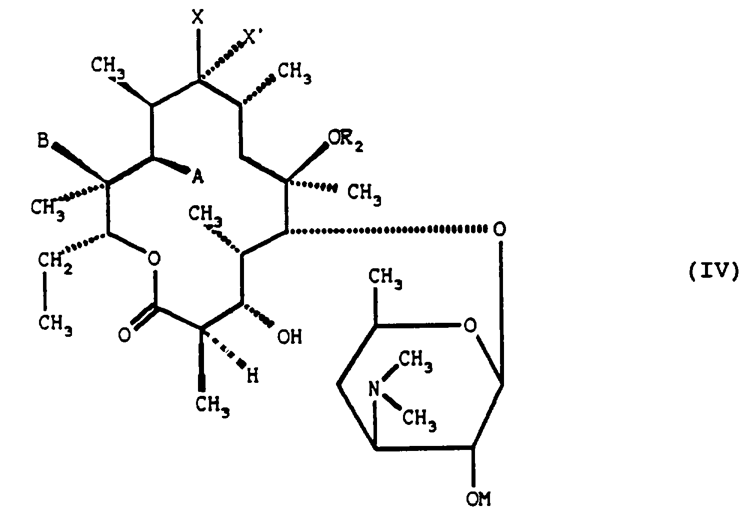 dans laquelle OM représente un groupement hydroxyle bloqué, et les autres substituants conservent leur signification précédente, que l'on soumet à l'action d'un agent d'oxydation de la fonction hydroxyle en 3, pour obtenir le composé de formule (V) : 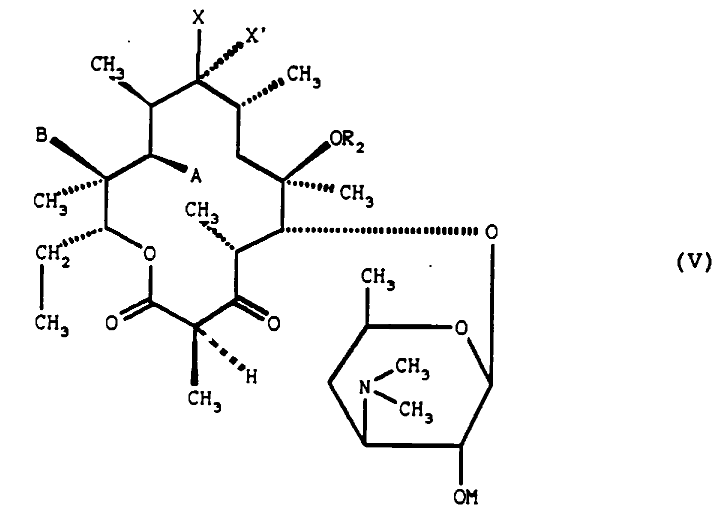 que l'on soumet, si désiré, à l'action d'un réactif susceptible d'introduire le radical R'**₃**, R'**₃** ayant la même valeur que R**₃** à l'exception de l'hydrogène, puis ou bien le cas échéant à l'action d'un agent de libération de la fonction hydroxyle en 2' pour obtenir le composé de formule (I_{**A**}) : 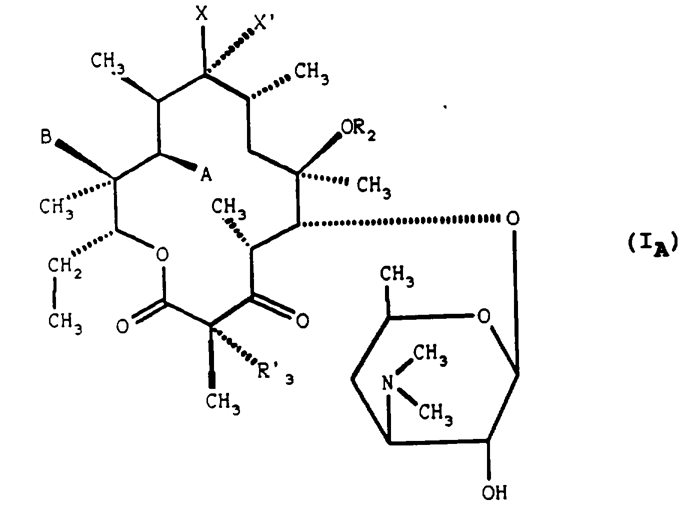 c'est-à-dire un composé de formule (I) dans laquelle Y et Y' forment ensemble avec l'atome de carbone auquel ils sont liés une fonction cétone, puis soumet si désiré, ce composé de formule (I_{**A**}) à l'action d'un agent d'oximation de la cétone ou béta-céto ester pour obtenir le composé de formule (I) recherché, puis si désiré soumet le composé obtenu à l'action d'un agent d'éstérification de l'hydroxyle en 2', ou bien d'abord à l'action d'un agent d'oximation de la fonction cétone ou bêta-céto ester, et ensuite le cas échéant à l'action d'un agent de libération de la fonction hydroxyle en 2' pour obtenir le composé de formule (I) recherché puis si désiré soumet le composé de formule (I) ainsi obtenu à l'action d'un acide pour en former le sel.

33. A titre de produits industriels nouveaux, les composés de formules (IV) et (V) définis à la revendication 32.

34. Variante du procédé de la revendication 32 pour préparer les produits de formule (I) dans lesquels X et X' forment ensemble un groupement C=NOR, caractérisé en ce que le produit de formule (IV_{**A**}) utilisé dans lequel X et X' représentent le groupement C=N-OR est préparé à partir de la cétone de formule (II) correspondante par action de NH**₂**OR en milieu acide, pour obtenir selon le pH de la réaction le produit de formule (IV_{**A**}) correspondant saturé ou insaturé en 10(11) : 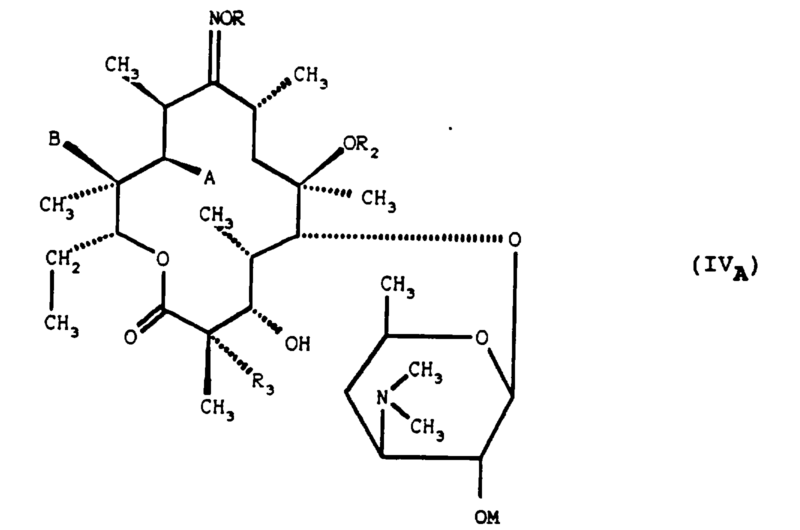 A représentant un radical OH s'il n'y a pas d'insaturation en 10(11) ou représentant un atome d'hydrogène s'il y a une insaturation en 10(11), R, R**₂** et Z conservant la même signification que précédemment.

35. Variante du procédé selon la revendication 32, pour préparer les composés de formule (I) dans lesquels X et X' forment un groupement C=NOR, R étant défini comme précédemment, caractérisé en ce que l'on soumet un composé de formule (I_{**A**}) dans lequel X et X' forment ensemble un groupement céto à l'action du composé de formule NH**₂**OR pour obtenir le composé de formule (I) correspondant, dans lequel X et X' forment un groupement C=NOR ET Z représente un atome d'hydrogène puis, le cas échéant, estérifie ou salifie.

36. Procédé selon la revendication 32, caractérisé en ce que le produit de formule (II) utilisé au départ ne porte pas d'insaturation en 10(11) .

## Revendications (Revendications pour l'(les) Etat(s) contractant(s) suivant(s): ES)

1. Procédé pour préparer des composés de formule (I) : 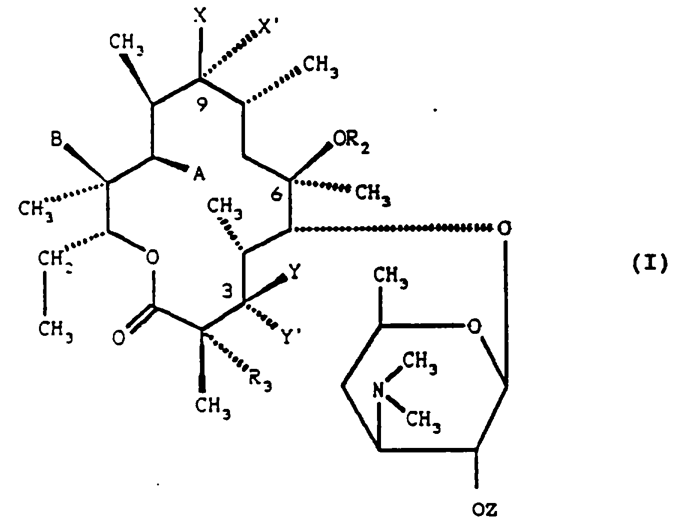
dans laquelle,
**ou bien** X et X' forment ensemble avec l'atome de carbone auquel ils sont liés un groupement C=O ou C-NOR, dans lequel R représente :
- un atome d'hydrogène,
- un radical hétérocyclique renfermant au moins un atome d'azote et éventuellement un autre hétéroatome, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- un radical alkyle, alkényle ou alkynyle linéaire, ramifié ou cyclique, renfermant jusqu'à 18 atomes de carbone éventuellement substitué par un ou plusieurs groupements
. hydroxyle,
. halogène,
. cyano,
. nitro,
. amidinyle,
. guanidinyle,
. hétérocyclique, tel que défini précédemment,
. alkyloxy, alkényloxy ou alkynyloxy ayant au plus 6 atomes de carbone,
. alkylthio, alkénylthio ou alkynylthio ayant au plus 6 atomes de carbone, l'atome de soufre étant éventuellement oxydé en sulfoxyde ou en sulfone,
. aryle, aralkyle,
. aryloxy, aralkyloxy,
. arylthio, aralkylthio, l'atome de soufre étant éventuellement oxydé en sulfoxyde ou en sulfone, (chacun de ces radicaux alkyloxy, alkényloxy, alkynyloxy, alkylthio, alkénylthio ou alkynylthio, aryle, aralkyle, aryloxy, aralkyloxy, arylthio ou aralkylthio étant éventuellement substitué par un ou plusieurs des groupements suivants : hydroxy, alkyloxy, alkylthio ayant de 1 à 6 atomes de carbone, alkénylthio, alkynylthio ayant jusqu'à 6 atomes de carbone, amino, monoalkylamino ayant jusqu'à 6 atomes de carbone, dialkylamino ayant jusqu'à 12 atomes de carbone, un radical amidinyle, guanidinyle, un radical hétérocyclique tel que défini précédemment, les radicaux aryle, aryloxy, arylthio, aralkyle, aralkyloxy et aralkylthio étant de plus éventuellement substitués par les radicaux méthyle, éthyle, propyle, carbamoyle, aminométhyle, diméthylaminométhyle, aminothyle, diméthylaminoéthyle, carboxyle, méthyloxycarbonyle, éthyloxycarbonyle)
. 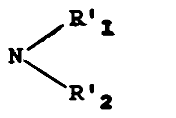 dans lequel
**ou bien R'₁ et R'₂** identiques ou différents représentent un atome d'hydrogène, un radical alkyle, alkényle ou alkynyle, linéaire, ramifié ou cyclique renfermant jusqu'à 18 atomes de carbone, un radical aryle ou aralkyle, chacun de ces radicaux R'**₁** et R'**₂** étant éventuellement substitué par un ou plusieurs radicaux hydroxy, alkyloxy, alkényloxy, alkynyloxy, alkylthio, alkénylthio ou alkynylthio renfermant jusqu'à 8 atomes de carbone, amino, monoalkylamino renfermant jusqu'à 4 atomes de carbone, dialkylamino renfermant jusqu'à 8 atomes de carbone, cyano, carboxyle libre, estérifié ou salifié, acyle ou carbamoyle, renfermant jusqu'à 8 atomes de carbone, par un radical si (alc)**₃** ou si (Oalc)**₃** dans lequel alc représente un radical alkyle renfermant jusqu'à 4 atomes de carbone, par un radical hétérocyclique tel que défini précédemment,
**ou bien R'₁ et R'₂** forment ensemble avec l'atome d'azote auquel ils sont liés un radical hétérocycle mono ou bicyclique, renfermant éventuellement un autre hétéroatome, saturé ou insaturé, aromatique ou non aromatique, comportant jusqu'à 12 chaînons ;
. un groupement ammonium quaternaire, . 1,2-époxyéthyle ou 2,2-diméthyl 1,2-époxyéthyle ou un radical résultant de l'ouverture de ce groupement par un réactif nucléophile,
. 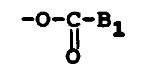 dans lequel B**₁** représente soit un radical alkyle ou alkyloxy ayant au plus 6 atomes de carbone, soit un radical aryle, aralkyle, aryloxy ou aralkyloxy,
. formyle libre ou protégé, carboxyle libre, estérifié ou salifié, thiocyanate, acyle ou carbamoyle,
. (CH**₂**)_{**n**}R', R' représentant le reste d'un acide aminé, et n représentant un nombre entier compris entre 0 et 6,
**ou bien X** représente un radical 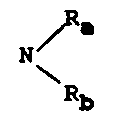
- Rₐ et R_{**b**} identiques ou différents l'un de l'autre, représentant un atome d'hydrogène ou un radical hydrocarboné renfermant jusqu'à 18 atomes de carbone, renfermant éventuellement un ou plusieurs hétéroatomes, éventuellement substitué par un ou plusieurs groupements fonctionnels, ou par un radical hétérocyclique renfermant au moins un atome d'azote et éventuellement un autre hétéroatome choisi parmi les atomes d'oxygène, de soufre ou d'azote, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- R_{**a**} et R_{**b**} pouvant éventuellement former avec l'atome d'azote auquel ils sont liés un radical hétérocycle, renfermant au moins un atome d'azote et éventuellement un autre hétéroatome choisi parmi les atomes d'oxygène, de soufre ou d'azote, mono ou bicyclique, saturé ou insaturé, aromatique ou non aromatique comportant jusqu'à 12 chaînons,
- R_{**a**} et R_{**b**} pouvant former avec le radical A un cycle 9-N, 11-O,
**et X'** représente un atome d'hydrogène,
**Y et Y'** identiques ou différents de X et X' ont la signification de X et X'
**B** représente un atome d'hydrogène ou un radical OR**₄**, R**₄** représentant un atome d'hydrogène ou forme avec A un radical carbonate ou carbamate,
**A** forme avec le carbone qui le porte et le carbone en 10, une double liaison,
**ou A** représente un radical OR'₄, R'**₄** représentant un atome d'hydrogène ou forme avec B un radical carbonate,
**ou A** représente un radical 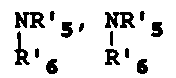 représentant un groupement C=O formant avec B un groupement carbamate, R'**₆** représentant un atome d'hydrogène ou un radical alkyle, aralkyle ou alkyloxy ayant jusqu'à 12 atomes de carbone ou un groupement 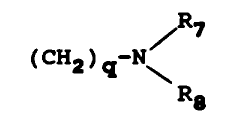
R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6,
**ou A** représente un radical 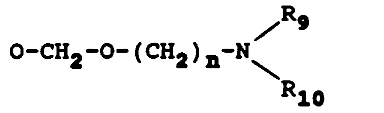
R**₉** et R**₁₀** représentant un atome d'hydrogène ou un radical alkyle renfermant jusqu'à 8 atomes de carbone, ou formant avec l'atome d'azote un hétérocycle tel que défini précédemment et n représente un nombre entier compris entre 1 et 6,
**R₂** représente un radical alkyle renfermant jusqu'à 8 atomes de carbone, ou un radical CONH**₂** ou CONHCOR**₁₁** ou CONHSO**₂**R**₁₁** dans lesquels R**₁₁** représente un radical hydrocarboné renfermant jusqu'à 18 atomes de carbone comportant éventuellement un ou plusieurs hétéroatomes,
**R₃** en position alpha ou béta représente un atome d'hydrogène ou un radical alkyle, renfermant jusqu'à 8 atomes de carbone ou un radical 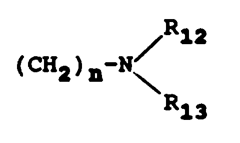 dans lequel R**₁₂** et R**₁**, représente un atome d'hydrogène, un radical alkyle renfermant jusqu'à 8 atomes de carbone ou formant avec l'atome d'azote un hétérocycle tel que défini précédemment, n représentant un nombre entier compris entre 1 et 6, ou un radical 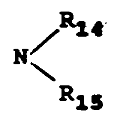
R**₁₄** et R**₁₅** identiques ou différents représentant un atome d'hydrogène ou un radical alkyle renfermant jusqu'à 8 atomes de carbone ou un hétéroatome ou un radical alkyle ou alkyloxy renfermant jusqu'à 8 atomes de carbone,
représente un atome d'hydrogène ou le reste d'un acide carboxylique renfermant jusqu'à 18 atomes de carbone les oximes que peuvent représenter X et X' ou Y et Y' peuvent être de configuration syn ou anti, ainsi que les sels d'addition avec les acides des composés de formule (I), caractérisé en ce que l'on soumet un composé de formule (II) : 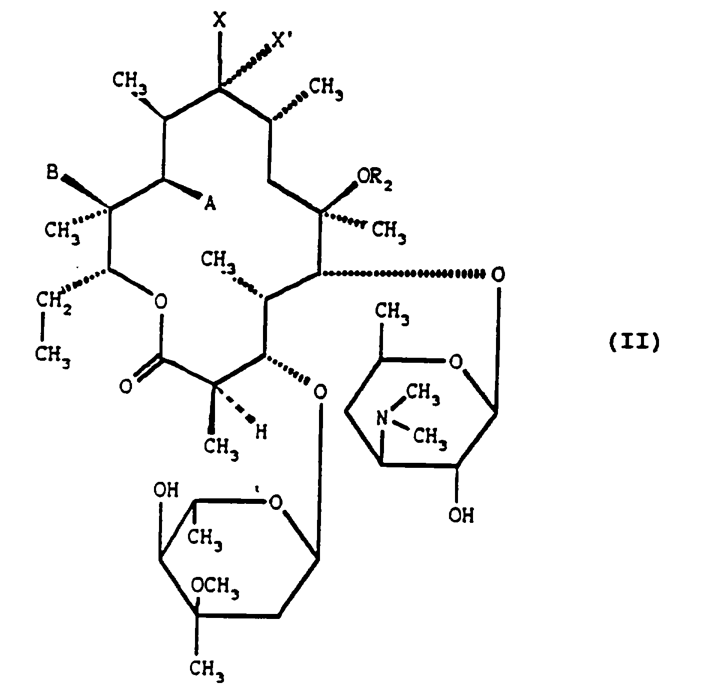 dans laquelle X, X', R**₂**, B. et A conservent leur signification précédente, à l'action d'un acide en milieu aqueux pour obtenir le composé de formule (III) : 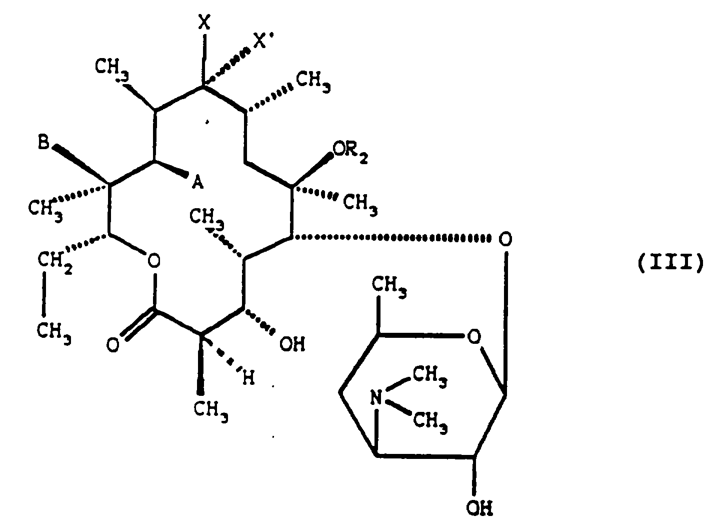 que l'on soumet à l'action d'un agent de blocage de la fonction hydroxyle en 2', pour obtenir un composé de formule (IV) : 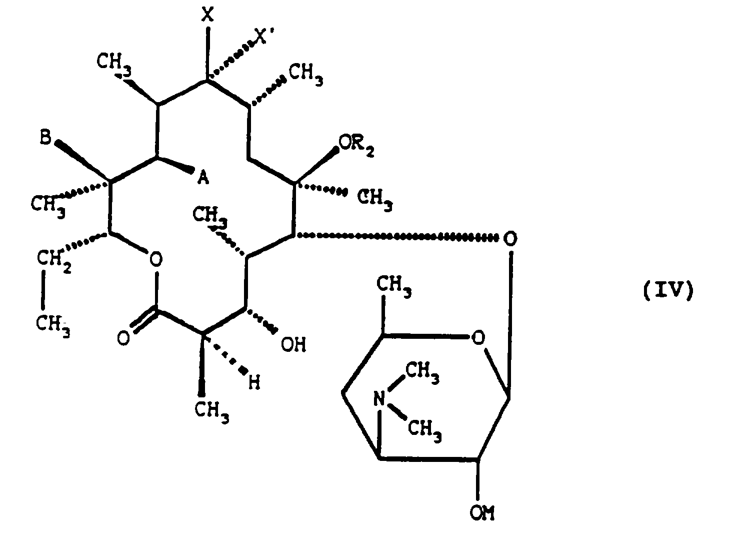 dans laquelle OM représente un groupement hydroxyle bloqué, et les autres substituants conservent leur signification précédente, que l'on soumet à l'action d'un agent d'oxydation de la fonction hydroxyle en 3, pour obtenir le composé de formule (V) : 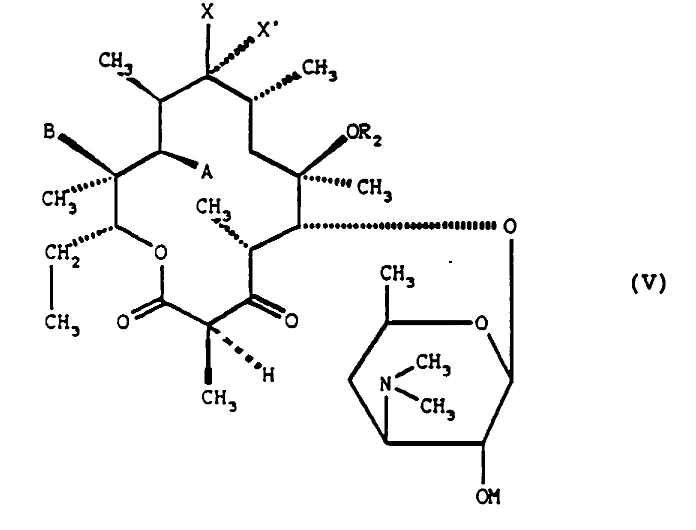 que l'on soumet, si désiré, à l'action d'un réactif susceptible d'introduire le radical R'**₃**, R'**₃** ayant la même valeur que R**₃** à l'exception de l'hydrogène, puis ou bien le cas échéant à l'action d'un agent de libération de la fonction hydroxyle en 2' pour obtenir le composé de formule (I_{**A**}) : 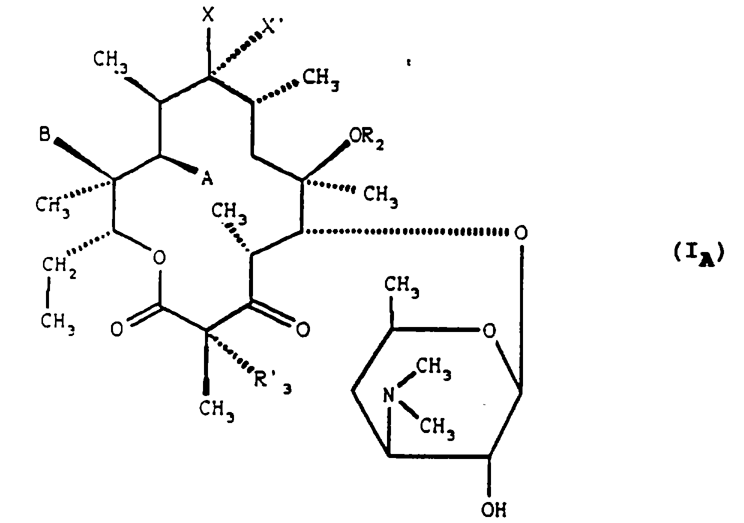
c'est-à-dire un composé de formule (I) dans laquelle Y et Y' forment ensemble avec l'atome de carbone auquel ils sont liés une fonction cétone, puis soumet si désiré, ce composé de formule (I_{**A**}) à l'action d'un agent d'oximation de la cétone ou béta-céto ester pour obtenir le composé de formule (I) recherché, puis si désiré soumet le composé obtenu à l'action d'un agent d'estérification de l'hydroxyle en 2', ou bien d'abord à l'action d'un agent d'oximation de la fonction cétone ou béta-céto ester, et ensuite le cas échéant à l'action d'un agent de libération de la fonction hydroxyle en 2' pour obtenir le composé de formule (I) recherché puis si désiré soumet le composé de formule (I) ainsi obtenu à l'action d'un acide pour en former le sel.

2. Procédé selon la revendication 1 pour préparer les produits de formule (I) dans lesquels X et X' forment ensemble un groupement C=NOR, caractérisé en ce que le produit de formule (IV_{**A**}) utilisé dans lequel X et X' représentent le groupement C=N-OR est préparé à partir de la cétone de formule (II) correspondante par action de NH**₂**OR en milieu acide, pour obtenir selon le pH de la réaction le produit de formule (IV_{**A**}) correspondant saturé ou insaturé en 10(11) : 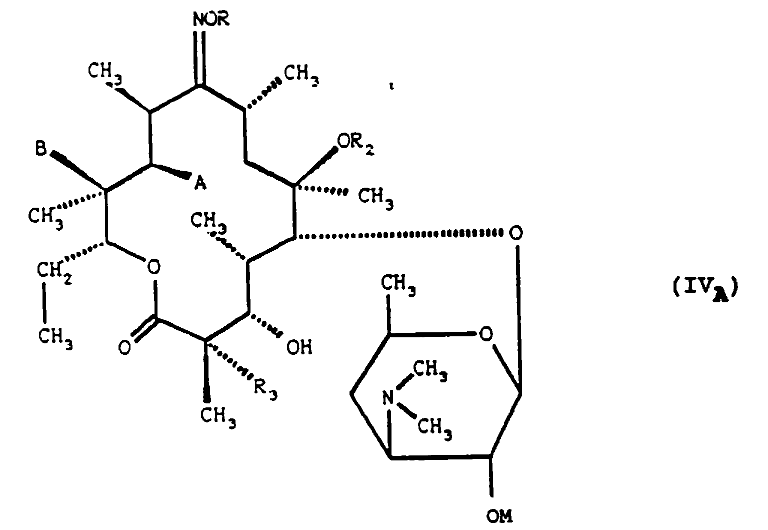 A représentant un radical OH s'il n'y a pas d'insaturation en 10(11) ou représentant un atome d'hydrogène s'il y a une insaturation en 10(11), R, R**₂** et Z conservant la même signification que dans la revendication 1.

3. Procédé selon la revendication 1, pour préparer les composés de formule (I) dans lesquels X et X' forment un groupement C=NOR, R étant défini comme précédemment, caractérisé en ce que l'on soumet un composé de formule (I_{**A**}) dans lequel X et X' forment ensemble un groupement céto à l'action du composé de formule NH**₂**OR pour obtenir le composé de formule (I) correspondant, dans lequel X et X' forment un groupement C=NOR ET Z représente un atome d'hydrogène puis, le cas échéant, estérifie ou salifie.

4. Procédé selon la revendication 1, caractérisé en ce que le produit de formule (II) utilisé au départ ne porte pas d'insaturation en 10(11).

5. Procédé selon l'une quelconque des revendications 1 à 4, caractérisé en ce que l'oximation de la fonction cétone peut être réalisée en une seule étape au moyen d'une hydroxylamime RONH**₂** portant le substituant R désiré soit au moyen d'une hydroxylamine H**₂**N-O-(CH**₂**)_{**n**}-Hal pour obtenir un composé de formule (I) dans laquelle 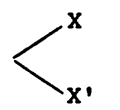 représente le groupement =N-O-(CH**₂**)ₙ-Hal, que l'on soumet si désiré à l'action d'une amine de formule 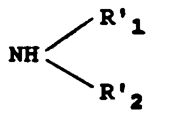 dans laquelle R'₁ et R'₂ ont la signification déjà indiquée, pour obtenir un composé de formule (I) dans laquelle 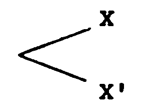 représente le groupement 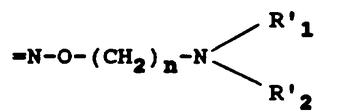 que le cas échéant l'on transforme, au moyen par exemple d'un agent d'alkylation, d'acylation, de réduction pour obtenir le composé de formule (I) désiré.

6. Procédé selon l'une quelconque des revendications 1 à 5, caractérisé en ce que pour préparer des produits de formule (I) dans laquelle X et X' représentent ensemble un groupement C=NOR, on utilise un agent d'oximation NH₂OR dans lequel R a la signification indiquée à la revendication 1.

7. Procédé selon l'une quelconque des revendications 1 à 6, caractérisé en ce que l'on utilise un agent d'oximation NH**₂**OR dans lequel R représente un radical alkyle renfermant jusqu'à 6 atomes de carbone substitué par un radical 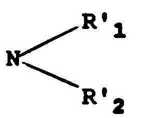 R'**₁** et R'**₂** conservant la même signification que dans la revendication 1.

8. Procédé selon l'une quelconque des revendications 1 à 6, caractérisé en ce que l'on utilise un agent d'oximation NH**₂**OR dans lequel le radical R est un radical 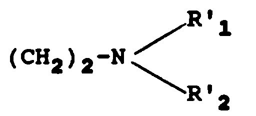 R'**₁** et R'**₂** représentant un radical alkyle renfermant jusqu'à 4 atomes de carbone.

9. Procédé selon la revendication 8 caractérisé en ce que l'on utilise un agent d'oximation NH**₂**OR dans lequel R représente un radical (CH₂)**₂**-N(CH₃)₂.

10. Procédé selon l'une quelconque des revendications 1 à 6, caractérisé en ce que l'on utilise un agent d'oximation NH₂OR dans lequel le radical R est un radical 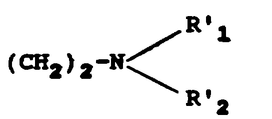 R'**₁** représentant un atome d'hydrogène et R'**₂** représentant un radical alkyle renfermant jusqu'à 4 atomes de carbone substitué par un radical hétérocycle renfermant au moins un atome d'azote.

11. Procédé selon la revendication 10, caractérisé en ce que l'on utilise un agent d'oximation NH**₂**OR dans lequel R représente un radical

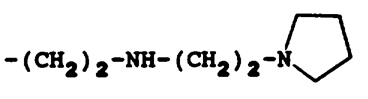

12. Procédé selon l'une quelconque des revendications 1 à 6, caractérisé en ce que l'on utilise un agent d'oximation NH**₂**OR dans lequel R représente un radical alkyle renfermant jusqu'à 6 atomes de carbone, substitué par un radical alkyloxy renfermant jusqu'à 6 atomes de carbone éventuellement substitué par un radical méthoxy.

13. Procédé selon la revendication 12, caractérisé en ce que l'on utilise un agent d'oximation NH**₂**OR dans lequel R représente un radical

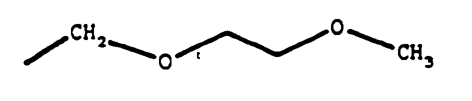

14. Procédé selon l'une quelconque des revendications 1 à 6, caractérisé en ce que l'on utilise un agent d'oximation NH**₂**OR dans lequel R représente un hétérocycle renfermant au moins un atome d'azote.

15. Procédé selon la revendication 14, caractérisé en ce l'on utilise un agent d'oximation NH**₂**OR dans lequel R représente un radical 3-pipéridinyle.

16. Procédé selon la revendication 1, caractérisé en ce que pour préparer un produit de formule (I_{**A**}) dans laquelle X et X' représentent ensemble une fonction cétone, on utilise au départ un produit de formule (II) dans laquelle X et X' ont la signification indiquée précédemment.

17. Procédé selon l'une quelconque des revendications 2 à 14, caractérisé en ce que pour préparer un produit de formule (I) dans laquelle Y et Y' forment ensemble un groupement C=NOR, on utilise un agent d'oximation NH**₂**OR dans lequel R a la signification indiquée à la revendication 1.

18. Procédé selon la revendication 17, caractérisé en ce que l'on utilise un agent d'oximation NH**₂**OR dans lequel R représente un radical benzyle.

19. Procédé selon l'une quelconque des revendications 1 à 18, caractérisé en ce que l'on utilise au départ un produit de formule (II) dans laquelle R**₂** représente un radical alkyle renfermant de 1 à 4 atomes de carbone.

20. Procédé selon la revendication 19, caractérisé en ce que l'on utilise au départ un produit de formule (II) dans laquelle R**₂** représente un radical méthyle.

21. Procédé selon l'une quelconque des revendications 1 à 20, caractérisé en ce que l'on soumet le produit de formule (V) ou bien le cas échéant à l'action d'un agent de libération de la fonction hydroxyle en 2' pour obtenir le composé de formule (I_{**A**}) dans laquelle R'**₃** représente un atome d'hydrogène, que l'on soumet si désiré à l'action d'un agent d'oximation de la cétone ou bêta-céto ester, puis si désiré à l'action d'un agent d'estérification de l'hydroxyle en 2' ou bien d'abord à l'action d'un agent d'oximation puis le cas échéant à l'action d'un agent de libération de la fonction hydroxyle en 2' pour obtenir un composé de formule (I) dans laquelle R**₃** représente un atome d'hydrogène, puis si désiré, soumet le composé de formule (I) ainsi obtenu à l'action d'un acide pour en former le sel.

22. Procédé selon l'une quelconque des revendications 1 à 21 caractérisé en ce que l'on utilise au départ un produit de formule (II) dans laquelle A représente un radical OH.

23. Procédé selon l'une quelconque des revendications 1 à 22, caractérisé en ce que l'on utilise au départ un produit de formule (II) dans laquelle B représente un radical OH.

24. Procédé selon l'une quelconque des revendications 1 à 21, caractérisé en ce que l'on utilise au départ un produit de formule (II) dans laquelle A et B forment ensemble un groupement 11,12 carbonate cyclique.

25. Procédé l'une quelconque des revendications 1 à 21, caractérisé en ce que l'on utilise au départ un produit de formule (II) dans laquelle A et B forment ensemble un radical divalent 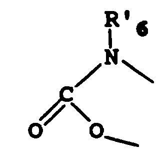 R'**₆** représentant un atome d'hydrogène ou un radical alkyle, aralkyle ou alkyloxy ayant jusqu'à 12 atomes de carbone ou un groupement 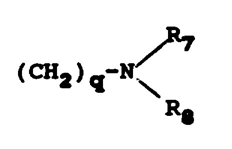 R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6.

26. Procédé selon la revendication 25, caractérisé en ce que l'on utilise au départ un produit de formule (II) dans laquelle A et B forment ensemble un radical divalent 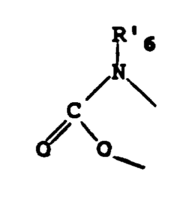 dans lequel R'**₆** représente un radical arakyle comportant jusqu'à 12 atomes de carbone.

27. Procédé selon la revendication 26 caractérisé en ce que l'on utilise au départ un produit de formule (II) dans laquelle A et B forment ensemble un radical divalent 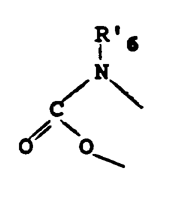 dans lequel R'**₆** représente un radical (CH**₂**)**₄**C**₆**H₅.

28. Procédé selon l'une quelconque des revendications 1 à 27, caractérisé en ce que l'on soumet le composé de formule (V) ou (I_{**A**}) à l'action d'un agent de libération de la fonction hydroxyle en 2' pour obtenir un produit de formule (I) dans laquelle Z représente un atome d'hydrogène.

## Revendications (Revendications pour l'(les) Etat(s) contractant(s) suivant(s): GR)

1. Procédé pour préparer des composés de formule (I) 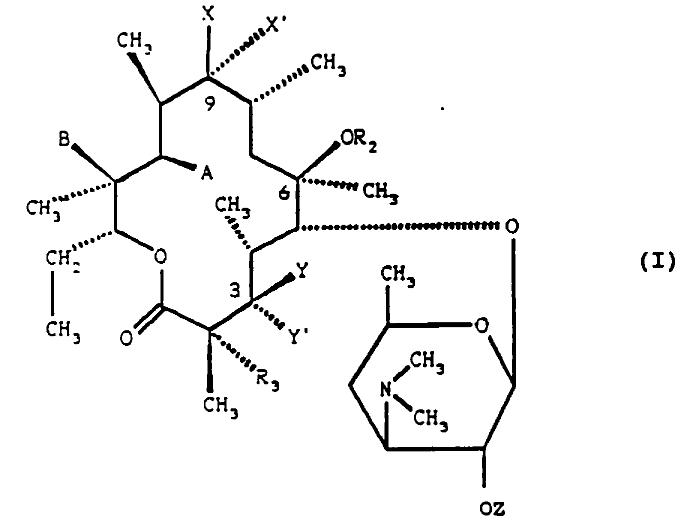 dans laquelle,
**ou bien** X et X' forment ensemble avec l'atome de carbone auquel ils sont liés un groupement C=O ou C=NOR, dans lequel R représente :
- un atome d'hydrogène,
- un radical hétérocyclique renfermant au moins un atome d'azote et éventuellement un autre hétéroatome, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- un radical alkyle, alkényle ou alkynyle linéaire, ramifié ou cyclique, renfermant jusqu'à 18 atomes de carbone éventuellement substitué par un ou plusieurs groupements
. hydroxyle,
. halogène,
. cyano,
. nitro,
. amidinyle,
. guanidinyle,
. hétérocyclique, tel que précédemment,
. alkyloxy, alkényloxy ou alkynyloxy ayant au plus 6 atomes de carbone,
. alkylthio, alkénylthio ou alkynylthio ayant au plus 6 atomes de carbone, l'atome de soufre étant éventuellement oxydé en sulfoxyde ou en sulfone,
. aryle, aralkyle,
. aryloxy, aralkyloxy,
. arylthio, aralkylthio, l'atome de soufre étant- éventuellement oxydé en sulfoxyde ou en sulfone, (chacun de ces radicaux alkyloxy, alkényloxy, alkynyloxy, alkylthio, alkénylthio ou alkynylthio, aryle, aralkyle, aryloxy, aralkyloxy, arylthio ou aralkylthio étant éventuellement substitué par un ou plusieurs des groupements suivants hydroxy, alkyloxy, alkylthio ayant de 1 à 6 atomes de carbone, alkénylthio, alkynylthio ayant jusqu'à 6 atomes de carbone, amino, monoalkylamino ayant jusqu'à 6 atomes de carbone, dialkylamino ayant jusqu'à 12 atomes de carbone, un radical amidinyle, guanidinyle, un radical hétérocyclique tel que défini précédemment, les radicaux aryle, aryloxy, arylthio, aralkyle, aralkyloxy et aralkylthio étant de plus éventuellement substitués, par les radicaux méthyle, éthyle, propyle, carbamoyle, aminométhyle, diméthylaminométhyle, aminoéthyle, diméthylaminoéthyle, carboxyle, méthyloxycarbonyle, éthyloxycarbonyle)
. 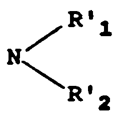 dans lequel :
**ou bien R'₁ et R'₂** identiques ou différents représentent un atome d'hydrogène, un radical alkyle, alkényle ou alkynyle, linéaire, ramifié ou cyclique renfermant jusqu'à 18 atomes de carbone, un radical aryle ou aralkyle, chacun de ces radicaux R'₁ et R'**₂** étant éventuellement substitué par un ou plusieurs radicaux hydroxy, alkyloxy, alkényloxy, alkynyloxy, alkylthio, alkénylthio ou alkynylthio renfermant jusqu'à 8 atomes de carbone, amino, monoalkylamino renfermant jusqu'à 4 atomes de carbone, dialkylamino renfermant jusqu'à 8 atomes de carbone, cyano, carboxyle libre, estérifié ou salifié, acyle ou carbamoyle, renfermant jusqu'à 8 atomes de carbone, par un radical Si(alc)₃ ou Si(Oalc)**₃** dans lequel alc représente un radical alkyle renfermant jusqu'à 4 atomes de carbone, par un radical hétérocyclique tel que défini précédemment,
**ou bien R'₁ et R'₂** forment ensemble avec l'atome d'azote auquel ils sont liés un radical hétérocycle mono ou bicyclique, renfermant éventuellement un autre hétéroatome, saturé ou insaturé, aromatique ou non aromatique, comportant jusqu' à 12 chaînons ;
. un groupement ammonium quaternaire,
. 1,2-époxyéthyle ou 2,2-diméthyl l,2-époxyéthyle ou un radical résultant de l'ouverture de ce groupement par un réactif nucléophile,
. 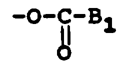 dans lequel B**₁** représente soit un radical alkyle ou alkyloxy ayant au plus 6 atomes de carbone, soit un radical aryle, aralkyle, aryloxy ou aralkyloxy,
. formyle libre ou protégé, carboxyle libre, estérifié ou salifié, thiocyanate, acyle ou carbamoyle,
. (CH**₂**)_{**n**}R', R' représentant le reste d'un acide aminé, et n représentant un nombre entier compris entre 0 et 6,
**ou bien X** représente un radical 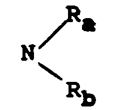
- R_{**a**} et R_{**b**} identiques ou différents l'un de l'autre, représentant un atome d'hydrogène ou un radical hydrocarboné renfermant jusqu'à 18 atomes de carbone, renfermant éventuellement un ou plusieurs hétéroatomes, éventuellement substitué par un ou plusieurs groupements fonctionnels, ou par un radical hétérocyclique renfermant au moins un atome d'azote et éventuellement un autre hétéroatome choisi parmi les atomes d'oxygène, de soufre ou d'azote, mono ou bicyclique, saturé ou insaturé, aromatique ou non aromatique comportant jusqu'à 12 chaînons, éventuellement substitué sur l'atome d'azote par un radical alkyle renfermant jusqu'à 4 atomes de carbone,
- R_{**a**} et R_{**b**} pouvant éventuellement former avec l'atome d'azote auquel ils sont liés un radical hétérocycle, renfermant au moins un atome d'azote et éventuellement un autre hétéroatome choisi parmi les atomes d'oxygène, de soufre ou d'azote, mono ou bicyclique, saturé ou insaturé, aromatique ou non aromatique comportant jusqu'à 12 chaînons,
- Rₐ et R_{**b**} pouvant former avec le radical A un cycle 9-N, 11-O,
**et X'** représente un atome d'hydrogène,
**Y et Y'** identiques ou différents de X et X' ont la signification de X et X'
**B** représente un atome d'hydrogène ou un radical OR**₄**, R**₄** représentant un atome d'hydrogène ou forme avec A un radical carbonate ou carbamate,
**A** forme avec le carbone qui le porte et le carbone en 10, une double liaison,
**ou A** représente un radical OR'**₄**, R'₄ représentant un atome d'hydrogène ou forme avec B un radical carbonate,
**ou A** représente un radical 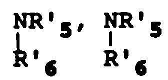 représentant un groupement C=O formant avec B un groupement carbamate, R'**₆** représentant un atome d'hydrogène ou un radical alkyle, aralkyle ou alkyloxy ayant jusqu'à 12 atomes de carbone ou un groupement 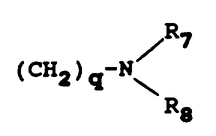 R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6,
**ou A** représente un radical 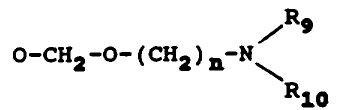 R**₉** et R**₁₀** représentant un atome d'hydrogène ou un radical alkyle renfermant jusqu'à 8 atomes de carbone, ou formant avec l'atome d'azote un hétérocycle tel que défini précédemment et n représente un nombre entier compris entre 1 et 6,
**R₂** représente un radical alkyle renfermant jusqu'à 8 atomes de carbone, ou un radical CONH**₂** ou CONHCOR**₁₁** ou CONHSO**₂**R**₁₁** dans lesquels R**₁₁** représente un radical hydrocarboné renfermant jusqu'à 18 atomes de carbone comportant éventuellement un ou plusieurs hétéroatomes,
**R₃** en position alpha ou béta représente un atome d'hydrogène ou un radical alkyle, renfermant jusqu'à 8 atomes de carbone ou un radical 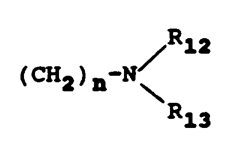 dans lequel R**₁₂** et R**₁₃** représente un atome d'hydrogène, un radical alkyle renfermant jusqu'à 8 atomes de carbone ou formant avec l'atome d'azote un hétérocycle tel que défini précédemment, n représentant un nombre entier compris entre 1 et 6, ou un radical 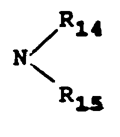 R**₁₄** et R**₁₅**, identiques ou différents représentant un atome d'hydrogène ou un radical alkyle renfermant jusqu'à 8 atomes de carbone ou un hétéroatome ou un radical alkyle ou alkyloxy renfermant jusqu'à 8 atomes de carbone,
**Z** représente un atome d'hydrogène ou le reste d'un acide carboxylique renfermant jusqu'à 18 atomes de carbone les oximes que peuvent représenter X et X' ou Y et Y' peuvent être de configuration syn ou anti, ainsi que les sels d'addition avec les acides des composés de formule (I), caractérisé en ce que l'on soumet un composé de formule (II) 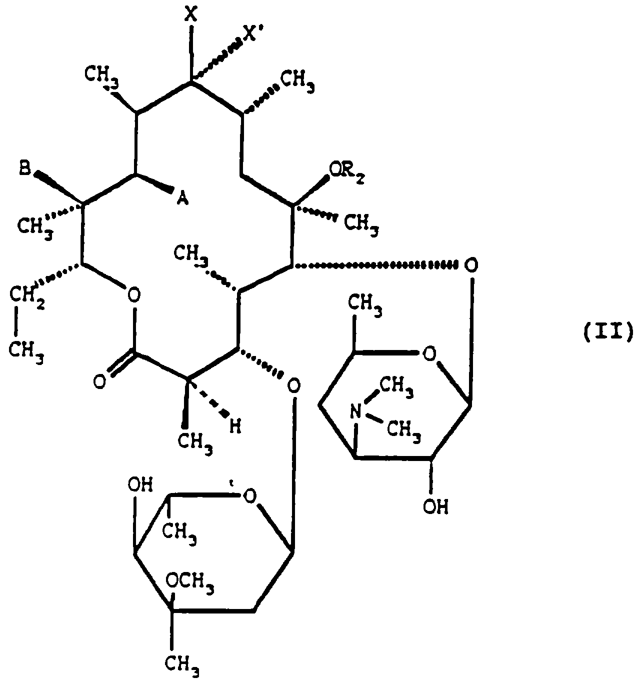 dans laquelle X, X′, R**₂**, B et A conservent leur signification précédente, à l'action d'un acide en milieu aqueux pour obtenir le composé de formule (III) 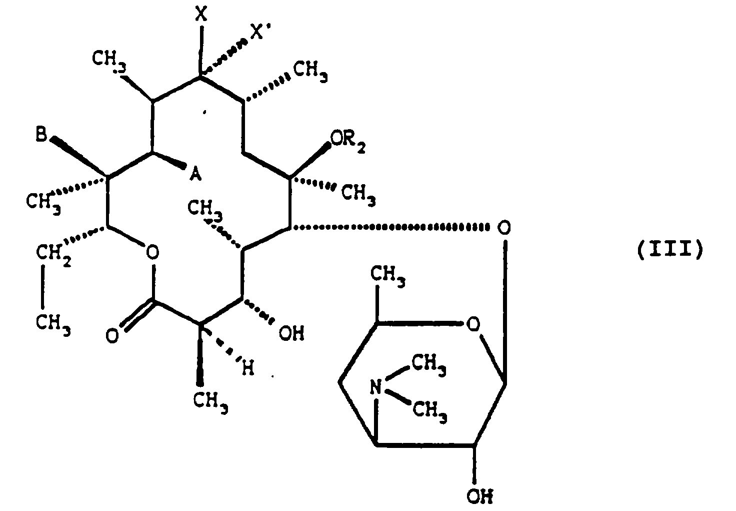 que l'on soumet à l'action d'un agent de blocage de la fonction hydroxyle en 2', pour obtenir un composé de formule (IV) : 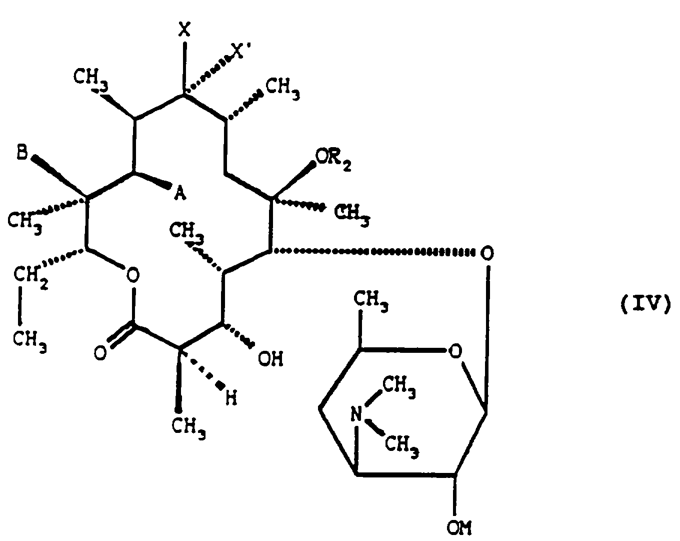 dans laquelle OM représente un groupement hydroxyle bloqué, et les autres substituants conservent leur signification précédente, que l'on soumet à l'action d'un agent d'oxydation de la fonction hydroxyle en 3, pour obtenir le composé de formule (V) : 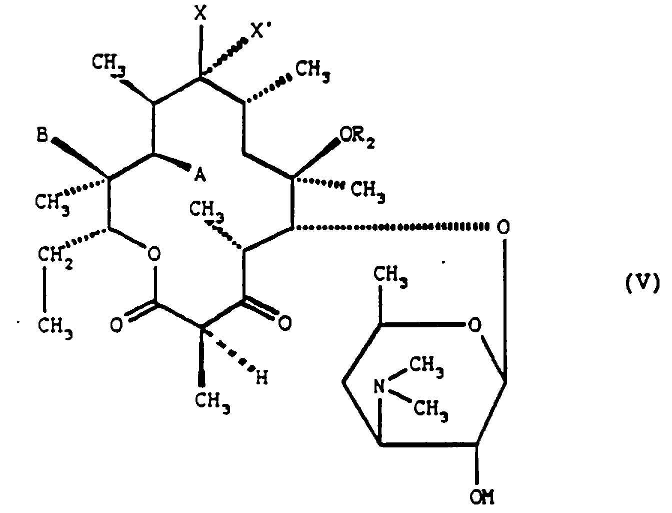 que l'on soumet, si désiré, à l'action d'un réactif susceptible d'introduire le radical R'**₃**, R'**₃** ayant la même valeur que R**₃** à l'exception de l'hydrogène, puis ou bien le cas échéant à l'action d'un agent de libération de la fonction hydroxyle en 2' pour obtenir le composé de formule (I_{**A**}) : 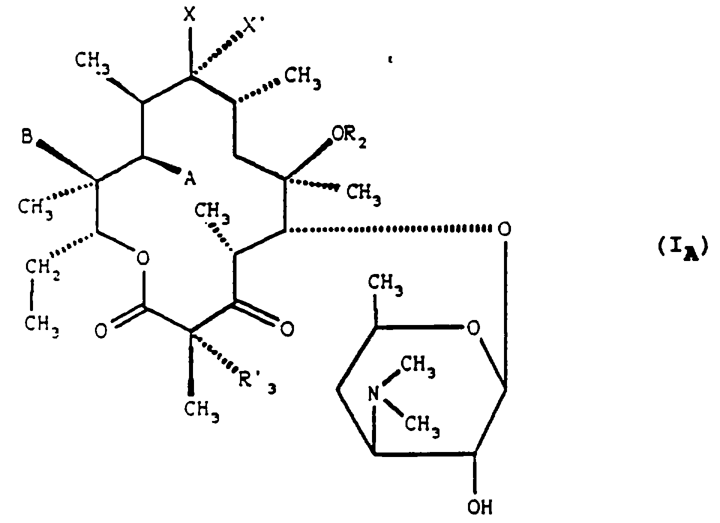
c'est-à-dire un composé de formule (I) dans laquelle Y et Y' forment ensemble avec l'atome de carbone auquel ils sont liés une fonction cétone, puis soumet si désiré, ce composé de formule (I_{**A**}) à l'action d'un agent d'oximation de la cétone ou béta-céto ester pour obtenir le composé de formule (I) recherché, puis si désiré soumet le composé obtenu à l'action d'un agent d'estérification de l'hydroxyle en 2', ou bien d'abord à l'action d'un agent d'oximation de la fonction cétone ou béta-céto ester, et ensuite le cas échéant à l'action d'un agent de libération de la fonction hydroxyle en 2' pour obtenir le composé de formule (I) recherché puis si désiré soumet le composé de formule (I) ainsi obtenu à l'action d'un acide pour en former le sel.

2. Procédé selon la revendication 1 pour préparer les produits de formule (I) dans lesquels X et X' forment ensemble un groupement C-NOR, caractérisé en ce que le produit de formule (IV_{**A**}) utilisé dans lequel X et X′ représentent le groupement C=N-OR est préparé à partir de la cétone de formule (II) correspondante par action de NH**₂**OR en milieu acide, pour obtenir selon le pH de la réaction le produit de formule (IV_{**A**}) correspondant saturé ou insaturé en 10(11) 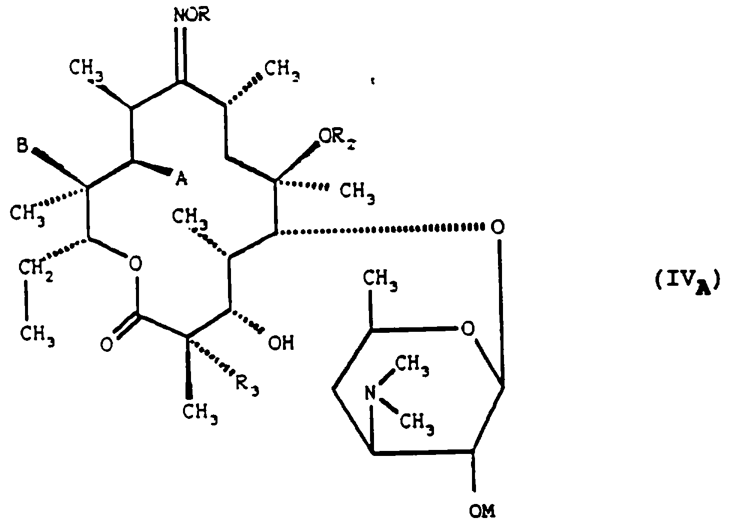 A représentant un radical OH s'il n'y a pas d'insaturation en 10(11) ou représentant un atome d'hydrogène s'il y a une insaturation en 10(11), R, R**₂** et Z conservant la même signification que dans la revendication 1.

3. Procédé selon la revendication 1, pour préparer les composés de formule (I) dans lesquels X et X' forment un groupement C=NOR, R étant défini comme précédemment, caractérisé en ce que l'on soumet un composé de formule (I_{**A**}) dans lequel X et X' forment ensemble un groupement céto à l'action du composé de formule NH**₂**OR pour obtenir le composé de formule (I) correspondant, dans lequel X et X' forment un groupement C=NOR ET Z représente un atome d'hydrogène puis, le cas échéant, estérifie ou salifie.

4. Procédé selon la revendication 1, caractérisé en ce que le produit de formule (II) utilisé au départ ne porte pas d'insaturation en 10(11).

5. Procédé selon l'une quelconque des revendications 1 à 4, caractérisé en ce que l'oximation de la fonction cétone peut être réalisé en une seule étape au moyen d'une hydroxylamine RONH**₂** portant le substituant R désiré soit au moyen d'une hydroxylamine H**₂**N-O-(CH**₂**)_{**n**}-Hal pour obtenir un composé de formule (I) dans laquelle 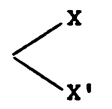 représente le groupement =N-O-(CH₂)_{**n**}-Hal, que l'on soumet si désiré à l'action d'une amine de formule 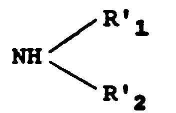 dans laquelle R'**₁** et R'**₂** ont la signification déjà indiquée, pour obtenir un composé de formule (I) dans laquelle 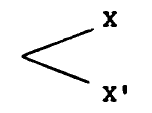 représente le groupement 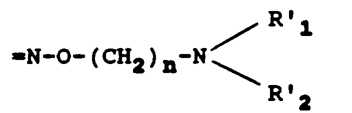 que le cas échéant l'on transforme, au moyen par exemple d'un agent d'alkylation, d'acylation, de réduction pour obtenir le composé de formule (I) désiré.

6. Procédé selon l'une quelconque des revendications 1 à 5, caractérisé en ce que pour préparer des produits de formule (I) dans laquelle X et X' représentent ensemble un groupement C=NOR, on utilise un agent d'oximation N**₂**OR dans lequel R a la signification indiquée à la revendication 1.

7. Procédé selon l'une quelconque des revendications 1 à 6, caractérisé en ce que l'on utilise un agent d'oximation NH**₂**OR dans lequel R représente un radical alkyle renfermant jusqu'à 6 atomes de carbone substitué par un radical 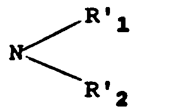 R'**₁** et R'**₂** conservant la même signification que dans la revendication 1.

8. Procédé selon l'une quelconque des revendications 1 à 6, caractérisé en ce que l'on utilise un agent d'oximation NH**₂**OR dans lequel le radical R est un radical 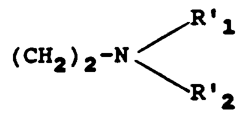 R'₁ et R'₂ représentant un radical alkyle renfermant jusqu'à 4 atomes de carbone.

9. Procédé selon la revendication 8 caractérisé en ce que l'on utilise un agent d'oximation NH₂OR dans lequel R représente un radical (CH**₂**)**₂**-N(CH**₃**)**₂**.

10. Procédé selon l'une quelconque des revendications 1 à 6, caractérisé en ce que l'on utilise un agent d'oximation NH**₂**OR dans lequel le radical R est un radical 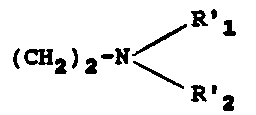 R'**₁** représentant un atome d'hydrogène et R'**₂** représentant un radical alkyle renfermant jusqu'à 4 atomes de carbone substitué par un radical hétérocycle renfermant au moins un atome d'azote.

11. Procédé selon la revendication 10, caractérisé en ce que l'on utilise un agent d'oximation NH**₂**OR dans lequel R représente un radical

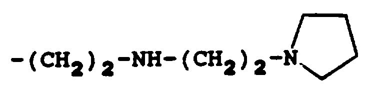

12. Procédé selon l'une quelconque des revendications 1 à 6, caractérisé en ce que l'on utilise un agent d'oximation NH**₂**OR dans lequel R représente un radical alkyle renfermant jusqu'à 6 atomes de carbone, substitué par un radical alkyloxy renfermant jusqu'à 6 atomes de carbone éventuellement substitué par un radical méthoxy.

13. Procédé selon la revendication 12, caractérisé en ce que l'on utilise un agent d'oximation NH**₂**OR dans lequel R représente un radical

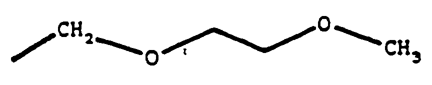

14. Procédé selon l'une quelconque des revendications 1 à 6, caractérisé en ce que l'on utilise un agent d'oximation NH**₂**OR dans lequel R représente un hétérocycle renfermant au moins un atome d'azote.

15. Procédé selon la revendication 14, caractérisé en ce que l'on utilise un agent d'oximation NH**₂**OR dans lequel R représente un radical 3-pipéridinyle.

16. Procédé selon la revendication 1, caractérisé en ce que pour préparer un produit de formule (I_{**A**}) dans laquelle X et X' représentent ensemble une fonction cétone, on utilise au départ un produit de formule (II) dans laquelle X et X' ont la signification indiquée précédemment.

17. Procédé selon l'une quelconque des revendications 2 à 14, caractérisé en ce que pour préparer un produit de formule (I) dans laquelle Y et Y' forment ensemble un groupement C=NOR, on utilise un agent d'oximation NH**₂**OR dans lequel R a la signification indiquée à la revendication 1.

18. Procédé selon la revendication 17, caractérisé en ce que l'on utilise un agent d'oximation NH**₂**OR dans lequel R représente un radical benzyle.

19. Procédé selon l'une quelconque des revendications 1 à 18, caractérisé en ce que l'on utilise au départ un produit de formule (II) dans laquelle R₂ représente un radical alkyle renfermant de 1 à 4 atomes de carbone.

20. Procédé selon la revendication 19, caractérisé en ce que l'on utilise au départ un produit de formule (II) dans laquelle R₂ représente un radical méthyle.

21. Procédé selon l'une quelconque des revendications 1 à 20, caractérisé en ce que l'on soumet le produit de formule (V) ou bien le cas échéant à l'action d'un agent de libération de la fonction hydroxyle en 2' pour obtenir le composé de formule (I_{**A**}) dans laquelle R'₃ représente un atome d'hydrogène, que l'on soumet si désiré à l'action d'un agent d'oximation de la cétone ou bêta-céto ester, puis si désiré à l'action d'un agent d'estérification de l'hydroxyle en 2' ou bien d'abord à l'action d'un agent d'oximation puis le cas échéant à l'action d'un agent de libération de la fonction hydroxyle en 2' pour obtenir un composé de formule (I) dans laquelle R**₃** représente un atome d'hydrogène, puis si désiré, soumet le composé de formule (I) ainsi obtenu à l'action d'un acide pour en former le sel.

22. Procédé selon l'une quelconque des revendications 1 à 21 caractérisé en ce que l'on utilise au départ un produit de formule (II) dans laquelle A représente un radical OH.

23. Procédé selon l'une quelconque des revendications 1 à 22, caractérisé en ce que l'on utilise au départ un produit de formule (II) dans laquelle B représente un radical OH.

24. Procédé selon l'une quelconque des revendications 1 à 21, caractérisé en ce que l'on utilise au départ un produit de formule (II) dans laquelle A et B forment ensemble un groupement 11,12 carbonate cyclique.

25. Procédé selon l'une quelconque des revendications 1 à 21, caractérisé en ce que l'on utilise au départ un produit de formule (II) dans laquelle A et B forment ensemble un radical divalent 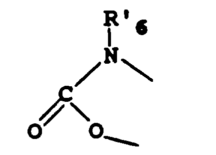 R'**₆** représentant un atome d'hydrogène ou un radical alkyle, aralkyle ou alkyloxy ayant jusqu'à 12 atomes de carbone ou un groupement : 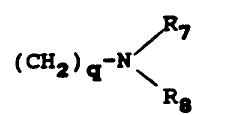 R**₇** et R**₈** identiques ou différents représentant un atome d'hydrogène ou un radical alkyle ou aralkyle, renfermant jusqu'à 18 atomes de carbone, ou formant avec l'atome d'azote un hétérocycle tel que défini précédemment, et q représente un nombre entier compris entre 1 et 6.

26. Procédé selon la revendication 25, caractérisé en ce que l'on utilise au départ un produit de formule (II) dans laquelle A et B forment ensemble un radical divalent 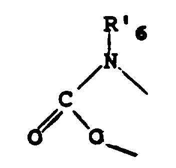 dans lequel R'**₆** représente un radical arakyle comportant jusqu'à 12 atomes de carbone.

27. Procédé selon la revendication 26 caractérisé en ce que l'on utilise au départ un produit de formule (II) dans laquelle A et B forment ensemble un radical divalent 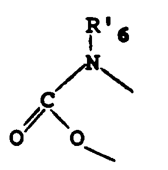 dans lequel R'₆ représente un radical (CH₂)**₄**C**₆**H**₅**.

28. Procédé selon l'une quelconque des revendications 1 à 27, caractérisé en ce que l'on soumet le composé de formule (V) ou (I_{**A**}) à l'action d'un agent de libération de la fonction hydroxyle en 2' pour obtenir un produit de formule (I) dans laquelle Z représente un atome d'hydrogène.

29. Procédé selon la revendication 1, caractérisé en ce que l'on choisit le produit de formule (II) et les réactifs mis en oeuvre de manière telle que l'on prépare la 9-[O-[2-(diméthylamino) éthyl] oxime] de 3-dé[(2,6-dideoxy 3-C-méthyl 3-O-méthyl alpha-L-ribo-hexopyranosyl) oxy] 6-O-méthyl 3-oxo érythromycine.

30. Procédé selon la revendication 1, caractérisé en ce que l'on choisit le produit de formule (II) et les réactifs mis en oeuvre de manière telle que l'on prépare la 11,12-dideoxy-3-de[(2,6-dideoxy 3-C-méthyl 3-0-méthyl alpha-L-ribohexopyranosyl) oxy] 6-O-méthyl 3-oxo12,11-[oxycarbonyl[(4-phénylbutyl) imino]] érythromycine.

31. Procédé selon la revendication 1, caractérisé en ce que l'on choisit le produit de formule (II) et les réactifs mis en oeuvre de manière telle que l'on prépare l'un quelconque des composés de formule (I) dont les noms suivent :
- la 9-[O-[(2-méthoxy éthoxy) méthyl] oxime] de 3-de((2,6-dideoxy 3-C-méthyl 3-O-méthyl alpha-L-ribo-hexopyranosyl) oxy] 6-O-méthyl 3-oxo érythromycine,
- la 3-dé[(2,6-dideoxy 3-C-méthyl 3-O-méthyl alpha-L-ribo-hexopyranosyl) oxy] 6-O-méthyl 3-oxo érythromycine,
- le 11,12-carbonate cyclique de 3-de[(2,6-dideoxy 3-C-méthyl 3-O-méthyl alpha-L-ribohexopyranosyl) oxy] 6-O-méthyl 3-oxoérythromycine.

32. Procédé selon la revendication 1, caractérisé en ce que l'on choisit le produit de formule (II) et les réactifs mis en oeuvre de manière telle que l'on prépare l'un quelconque des composés de formule (I) dont les noms suivent
- (E) 9-O-[2-[[2-(1-pyrrolidinyl éthyl] amino] éthyl] oxime de 3-dé [(2,6-didéoxy 3C-méthyl 3-O-méthyl alpha-L-ribo hexopyranosyl) oxy] 6-O-méthyl 3-oxo érythromycine,
- (E) 9-O-(3-pipéridinyl) oxime de 3-dé [(2,6-didéoxy 3C-méthyI 3-O-méthyl alpha-L-ribo hexopyranosyl) oxy] 6-O-méthyl 3-oxo érythromycine,
- (E) 9-O-[2-9-O-[2-(diméthylamino) éthyl] oxime de 3-dé [(2,6-didéoxy 3C-méthyl 3-O-méthyl alpha-L-ribo hexopyranosyl) oxy] 11-déoxy 10,11-didéhydro 6-O-méthyl 3-oxo érythromycine.

33. Procédé de préparation de compositions pharmaceutiques, caractérisé en ce que l'on met à titre de principe actif l'un au moins des dérivés de formule (I) telle que définie à la revendication 1 ou l'un au moins de leurs sels d'addition avec les acides pharmaceutiquement acceptables, sous une forme destinée à cet usage.

34. Procédé de préparation de compositions pharmaceutiques, caractérisé en ce que l'on met à titre de principe actif l'un au moins des dérivés de formule (I) telle que définie à l'une quelconque des revendications 2 à 28 ou l'un au moins de leurs sels d'addition avec les acides pharmaceutiquement acceptables, sous une forme destinée à cet usage.

35. Procédé de préparation de compositions pharmaceutiques, caractérisé en ce que l'on met à titre de principe actif l'un au moins des dérivés de formule (I) telle que définie à l'une quelconque des revendications 29 à 32 ou l'un au moins de leurs sels d'addition avec les acides pharmaceutiquement acceptables, sous une forme destinée à cet usage.

36. A titre de produits industriels nouveaux, les composés de formule (IV) et (V) définis à la revendication 1.

## Patentansprüche (Patentansprüche für folgende(n) Vertragsstaat(en): AT, BE, CH, DE, DK, FR, GB, IT, LI, LU, NL, SE)

1. Verbindungen der Formel (I): 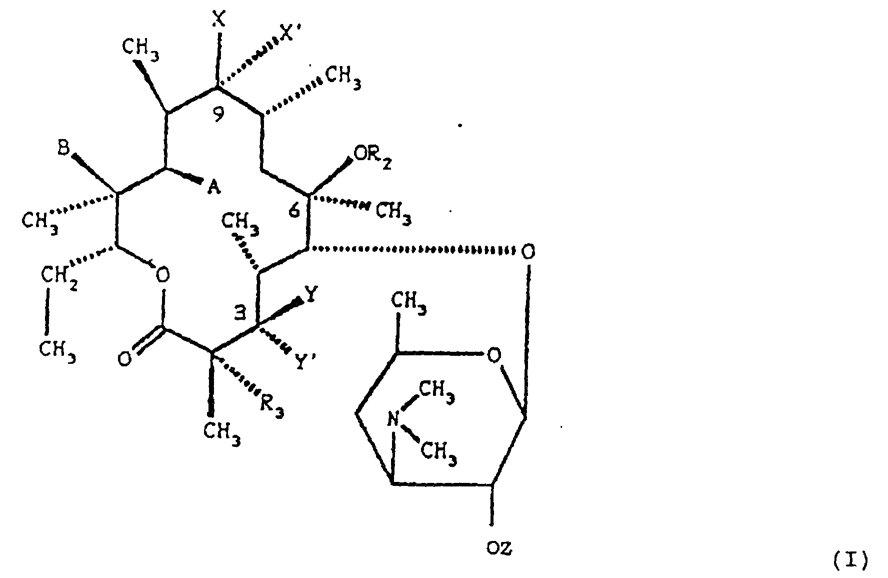 worin:
**entweder** X und X' zusammen mit dem Kohlenstoffatom, an das sie gebunden sind, eine Gruppierung C=O oder C=NOR bilden, worin R für
- ein Wasserstoffatom,
- einen mono- oder bicyclischen, gesättigten oder ungesättigten, aromatischen oder nicht-aromatischen, heterocyclischen Rest, umfassend mindestens ein Stickstoffatom und ggf. ein anderes Heteroatom, umfassend bis zu 12 Kettenglieder, gegebenenfalls am Stickstoffatom durch einen Alkylrest mit bis zu 4 Kohlenstoffatomen substituiert,
- einen linearen, verzweigten oder cyclischen Alkyl-, Alkenyl- oder Alkinylrest mit bis zu 18 Kohlenstoffatomen, gegebenenfalls substituiert durch eine oder mehrere Gruppierungen:
. Hydroxyl,
. Halogen,
. Cyano,
. Nitro,
. Amidinyl,
. Guanidinyl,
. Heterozyklus, wie vorstehend definiert,
. Alkyloxy, Alkenyloxy oder Alkinyloxy mit höchstens 6 Kohlenstoffatomen,
. Alkylthio, Alkenylthio oder Alkinylthio mit höchstens 6 Kohlenstoffatomen, wobei das Schwefelatom gegebenenfalls zu einem Sulfoxid oder Sulfon oxidiert ist,
. Aryl, Aralkyl,
. Aryloxy, Aralkyloxy,
. Arylthio, Aralkylthio, wobei das Schwefelatom gegebenenfalls zu einem Sulfoxid oder Sulfon oxidiert ist,
(jeder der Alkyloxy-, Alkenyloxy-, Alkinyloxy-, Alkylthio-, Alkenylthio- oder Alkinylthio-, Aryl-, Aralkyl-, Aryloxy-, Aralkyloxy-, Arylthio- oder Aralkylthioreste ist gegebenenfalls durch ein oder mehrere der folgenden Gruppierungen substituiert: Hydroxy, Alkyloxy, Alkylthio mit 1 bis 6 Kohlenstoffatomen, Alkenylthio, Alkinylthio mit bis zu 6 Kohlenstoffatomen, Amino, Monoalkylamino mit bis zu 6 Kohlenstoffatomen, Dialkylamino mit bis zu 12 Kohlenstoffatomen, ein Amidinyl-, Guanidinylrest, ein heterocyclischer Rest, wie vorstehend definiert, Aryl-, Aryloxy-, Arylthio-, Aralkyl-, Aralkyloxy- und Aralkylthioreste, die gegebenenfalls außerdem durch Methyl-, Ethyl-, Propyl-, Carbamoyl-, Aminomethyl-, Dimethylaminomethyl-, Aminoethyl-, Dimethylaminoethyl-, Carboxyl-, Methyloxycarbonyl-, Ethyloxycarbonylreste substituiert sind)
. 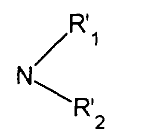 worin:
**entweder R'₁ und R'₂** gleich oder verschieden sind und für ein Wasserstoffatom, eine linearen, verzweigten oder cyclischen Alkyl-, Alkenyl- oder Alkinylrest mit bis zu 18 Kohlenstoffatomen, einen Aryl- oder Aralkylrest stehen, wobei jeder der Reste R'₁ und R'₂ gegebenenfalls substituiert durch ein oder mehrere Hydroxyl-, Alkyloxy-, Alkenyloxy-, Alkinyloxy-, Alkylthio-, Alkenylthio- oder Alkinylthioreste mit bis zu 8 Kohlenstoffatomen, Aminorest, Monoalkylaminorest mit bis zu 4 Kohlenstoffatomen, Dialkylaminorest mit bis zu 8 Kohlenstoffatomen, Cyanogruppe, freie, veresterte oder in ein Salz überführte carboxylgruppe, Acylrest oder Carbamoylrest umfassend bis zu 8 Kohlenstoffatome, durch einen Rest si (alc)₃ oder Si(Oalc)₃, worin alc für einen Alkylrest mit bis zu 4 Kohlenstoffatomen steht, einen heterocyclischen Rest, wie vorstehend definiert,
**oder R'₁ und R'₂** zusammen mit dem Stickstoffatom, an das sie gebunden sind, einen mono- oder bicyclischen, gesättigten oder ungesättigten, aromatischen oder nicht-aromatischen, heterocyclischen Rest, umfassend gegebenenfalls ein weiteres Heteroatom und umfassend bis zu 12 Kettenglieder, bilden;
. eine quaternäre Ammoniumgruppe,
. 1,2-Epoxyethyl oder 2,2-Dimethyl-1,2-epoxyethyl oder einen Rest als Ergebnis der Öffnung dieser Gruppierung durch ein nucleophiles Reagens,
. 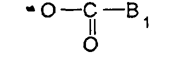 worin B₁ entweder einen Alkyl- oder Alkyloxyrest mit höchstens 6 Kohlenstoffatomen oder einen Aryl-, Aralkyl-, Aryloxy- oder Aralkyloxyrest bedeutet,
. ein freies oder geschütztes Formyl, freies, verestertes oder in ein Salz überführtes Carboxyl, Thiocyanat, Acyl oder Carbamoyl,
. (CH₂)ₙR', wobei R' den Rest einer Aminosäure bedeutet,
und n eine ganze Zahl zwischen 0 und 6 bedeutet, steht,
**oder X** einen Rest 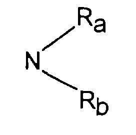 bedeutet,worin
- Rₐ und R_{b,} die gleich oder voneinander verschieden sind, für ein Wasserstoffatom oder einen Kohlenwasserstoffrest mit bis zu 18 Kohlenstoffatomen, umfassend gegebenenfalls ein oder mehrere Heteroatome, gegebenenfalls substituiert durch ein oder mehrere funktionelle Gruppierungen oder einen monooder bicyclischen, gesättigten oder ungesättigten, aromatischen oder nicht-aromatischen, heterocyclischen Rest, umfassend mindestens ein Stickstoffatom, und gegebenenfalls ein anderes Heteroatom, ausgewählt aus den Atomen Sauerstoff, Schwefel oder Stickstoff, umfassend bis zu 12 Kettenglieder, gegebenenfalls substituiert am Stickstoffatom durch einen Alkylrest, umfassend bis zu 4 Kohlenstoffatome, bedeuten,
- Rₐ und R_{b} gegebenenfalls mit dem Stickstoffatom, an das sie gebunden sind, einen mono- oder bicyclischen, gesättigten oder ungesättigten, aromatischen oder nichtaromatischen, heterocyclischen Rest, umfassend mindestens ein Stickstoffatom und gegebenenfalls ein anderes Heteroatom, ausgewählt aus den Atomen Sauerstoff, Schwefel oder Stickstoff, umfassend bis zu 12 Kettenglieder, bilden können,
- Rₐ und R_{b} mit dem Rest A einen Zyklus 9-N, 11-0, bilden können,
**und X'** ein Wasserstoffatom bedeutet,
**Y und Y'**, die gleich oder verschieden von X und X' sind, wie X und X' definiert sind,
**B** ein Wasserstoffatom oder einen Rest OR₄ bedeutet, wobei R₄ ein Wasserstoffatom bedeutet oder mit A einen Carbonat- oder Carbamatrest bildet,
**A** mit dem Kohlenstoff, der diesen Rest trägt und dem Kohlenstoff in Position 10 eine Doppelbindung bildet,
**oder A** einen Rest OR'₄ bedeutet, wobei R'₄ ein Wasserstoffatom bedeutet oder mit B einen Carbonatrest bildet,
**oder A** einen Rest 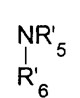 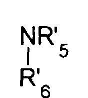 bedeutet, wobei eine Gruppierung C=O, die mit B eine Carbamatgruppierung bildet, bedeutet, wobei R'₆ für ein Wasserstoffatom oder einen Alkyl-, Aralkyl- oder Alkyloxyrest mit bis zu 12 Kohlenstoffatomen oder eine Gruppierung 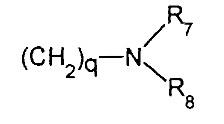 steht, wobei R₇ und R₈, die gleich oder verschieden sind, ein Wasserstoffatom oder einen Alkyl- oder Aralkylrest, umfassend bis zu 18 Kohlenstoffatome, bedeuten, oder mit dem Stickstoffatom einen, wie vorstehend definierten, Heterozyklus bilden, und q eine ganze Zahl zwischen 1 und 6 bedeutet, **oder A** einen Rest 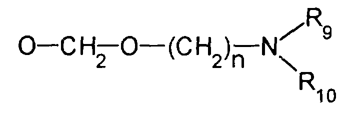 bedeutet, wobei R₉ und R₁₀ ein Wasserstoffatom oder einen Alkylrest, umfassend bis zu 8 Kohlenstoffatome, bedeuten, oder mit dem Stickstoffatom einen, wie vorstehend definierten Heterozyklus bilden, und n eine ganze Zahl zwischen 1 und 6 bedeutet,
**R₂** einen Alkylrest, umfassend bis zu 8 Kohlenstoffatome oder einen Rest CONH₂ oder CONHCOR₁₁ oder CONHSO₂R₁₁ bedeutet, worin R₁₁ einen Kohlenwasserstoffrest, umfassend bis zu 18 Kohlenstoffatome, umfassend gegebenenfalls ein oder mehrere Heteroatome, bedeutet,
**R₃** in Position α oder β ein Wasserstoffatom oder einen Alkylrest, umfassend bis zu 8 Kohlenstoffatome, oder einen Rest 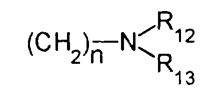 bedeutet, worin R₁₂ und R₁₃ ein Wasserstoffatom, einen Alkylrest, umfassend bis zu 8 Kohlenstoffatome, bedeuten oder mit dem Stickstoffatom einen, wie vorstehend definierten, Heterozyklus bilden, n eine ganze Zahl zwischen 1 und 6 bedeutet, oder einen Rest 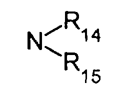 bedeutet, worin R₁₄ und R₁₅, die gleich oder verschieden sind, ein Wasserstoffatom oder einen Alkylrest, umfassend bis zu 8 Kohlenstoffatome, oder ein Heteroatom oder einen Alkyl- oder Alkyloxyrest, umfassend bis zu 8 Kohlenstoffatome, bedeuten,
**Z** ein Wasserstoffatom oder den Rest einer Carbonsäure, umfassend bis zu 18 Kohlenstoffatome, bedeutet, wobei die Oxime, die X und X' oder Y und Y' bedeuten können, in der Syn- oder Anti-Konfiguration vorliegen können, sowie die Säureadditionssalze der Verbindungen der Formel (I).

2. Verbindungen der Formel (I) nach Anspruch 1, worin X und X' zusammen mit dem Kohlenstoffatom, an das sie gebunden sind, eine Gruppierung C=NOR bilden, wobei R wie in Anspruch 1 definiert ist.

3. Verbindungen der Formel (I) nach Anspruch 2, worin R einen Alkylrest, umfassend bis zu 6 Kohlenstoffatome, und substituiert durch einen Rest 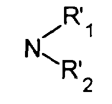 bedeutet, wobei R'₁ und R'₂ wie in Anspruch 1 definiert sind.

4. Verbindungen der Formel (I) nach Anspruch 3, worin der Rest R ein Rest 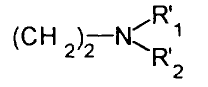 ist, wobei R'₁ und R'₂ einen Alkylrest mit bis zu 4 Kohlenstoffatomen bedeuten.

5. Verbindungen der Formel (I) nach Anspruch 4, worin R einen Rest (CH₂)₂-N(CH₃)₂ bedeutet.

6. Verbindungen der Formel (I) nach Anspruch 3, worin der Rest R ein Rest 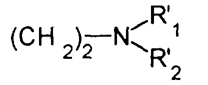 ist, wobei R'₁ ein Wasserstoffatom bedeutet, und R'₂ einen Alkylrest, umfassend bis zu 4 Kohlenstoffatome, substituiert durch einen heterocyclischen Rest, umfassend mindestens ein Stickstoffatom, bedeutet.

7. Verbindungen der Formel (I) nach Anspruch 6, worin R einen Rest 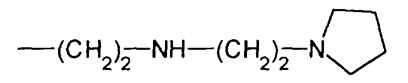 bedeutet.

8. Verbindungen der Formel (I) nach Anspruch 2, worin R einen Alkylrest, umfassend bis zu 6 Kohlenstoffatome, substituiert durch einen Alkyloxyrest, umfassend bis zu 6 Kohlenstoffatome, der gegebenenfalls durch eine Methoxygruppe substituiert ist, bedeutet.

9. Verbindungen der Formel (I) nach Anspruch 6, worin R einen Rest 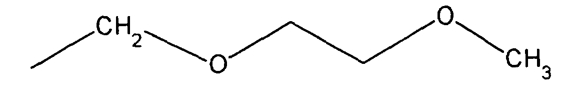 bedeutet.

10. Verbindungen der Formel (I) nach Anspruch 2, worin R einen Heterozyklus, umfassend mindestens ein Stickstoffatom bedeutet.

11. Verbindungen der Formel (I) nach Anspruch 10, worin R eine 3-Piperidinylgruppe bedeutet.

12. Verbindungen der Formel (I) nach Anspruch 1, worin X und X' zusammen mit dem Kohlenstoffatom, an das sie gebunden sind, eine Gruppierung C=O bilden.

13. Verbindung der Formel (I) nach Anspruch 1, worin X und X' und Y und Y' zusammen eine Gruppierung C=O bilden.

14. Verbindungen der Formel (I) nach einem der Ansprüche 2 bis 11, worin Y und Y' zusammen eine Gruppierung C=NOR bilden, wobei R wie vorstehend definiert und insbesondere ein Benzylrest ist.

15. Verbindungen der Formel (I) nach einem der Ansprüche 1 bis 14, worin R₂ einen Alkylrest, umfassend 1 bis 4 Kohlenstoffatome, bedeutet.

16. Verbindungen der Formel (I) nach Anspruch 15, worin R₂ einen Methylrest bedeutet.

17. Verbindungen der Formel (I) nach einem der Ansprüche 1 bis 16, worin R₃ ein Wasserstoffatom (α oder β) bedeutet.

18. Verbindungen der Formel (I) nach einem der Ansprüche 1 bis 17, worin A eine OH-Gruppe bedeutet.

19. Verbindungen der Formel (I) nach einem der Ansprüche 1 bis 18, worin B eine OH-Gruppe bedeutet.

20. Verbindungen der Formel (I) nach einem der Ansprüche 1 bis 17, worin A und B zusammen eine cyclische 11,12-Carbonatgruppierung bilden.

21. Verbindungen der Formel (I) nach einem der Ansprüche 1 bis 17, worin A und B zusammen einen zweiwertigen Rest 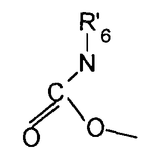 bilden, worin R'₆ ein Wasserstoffatom oder einen Alkyl-, Aralkyl- oder Alkyloxyrest mit bis zu 12 Kohlenstoffatomen oder eine Gruppierung: 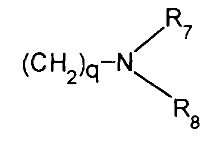 bedeutet, worin R₇ und R₈, die gleich oder verschieden sind, ein Wasserstoffatom oder einen Alkyl- oder Aralkylrest, umfassend bis zu 18 Kohlenstoffatome, bedeuten oder mit dem Stickstoffatom einen Heterozyklus, wie vorstehend definiert, bilden, und q eine ganze Zahl zwischen 1 und 6 bedeutet.

22. Verbindungen der Formel (I) nach Anspruch 21, worin R'₆ einen Aralkylrest, umfassend bis zu 12 Kohlenstoffatome, bedeutet.

23. Verbindungen der Formel (I) nach Anspruch 21, worin R'₆ eine Gruppe (CH₂)₄C₆H₅ bedeutet.

24. Verbindungen der Formel (I) nach einem der Ansprüche 1 bis 23, worin Z ein Wasserstoffatom bedeutet.

25. Verbindung der Formel (I) nach Anspruch 1, nämlich: 3-Des-[(2,6-didesoxy-3-C-methyl-3-O-methyl-α-L-ribohexopyranosyl)oxy]-6-O-methyl-3-oxoerythromycin-9-[O-[2-(dimethyl-amino)ethyl]oxim.

26. Verbindung der Formel (I) nach Anspruch 1, nämlich: 11,12-Didexoxy-3-des-[(2,6-didesoxy-3-C-methyl-3-O-methyl-α-L-ribohexopyranosyl)oxy]6-O-methyl-3-oxo-12,11-[oxycarbonyl-[(4-phenylbutyl)imino]]erythromycin.

27. Verbindungen der Formel (I) nach Anspruch 1, nämlich:
- 3-Des-[(2,6-didesoxy-3-C-methyl-3-O-methyl-α-L-ribohexopyranosyl)oxy]-6-O-methyl-3-oxoerythromycin-9-[O-[(2-methoxyethoxy)methyl]oxim],
- 3-Des-[(2,6-didesoxy-3-C-methyl-3-O-methyl-α-L-ribohexopyranosyl)oxy]6-O-methyl-3-oxoerythromycin,
- cyclisches 3-Des-[(2,6-didesoxy-3-C-methyl-3-O-methyl-α-L-ribohexopyranosyl)oxy]-6-O-methyl-3-oxoerythromycin-11,12-carbonat.

28. Verbindungen der Formel (I) nach Anspruch 1, nämlich:
- 3-Des-[(2,6-didesoxy-3-C-methyl-3-O-methyl-α-L-ribohexopyranoxyl)oxy]-6-O-methyl-3-oxoerythromycin-(E)-9-O-[2-[[2-(1-pyrrolidinylethyl]amino]ethyl]oxim,
- 3-Des-[(2,6-didesoxy-3-C-methyl-3-O-methyl-α-L-ribohexopyranosyl)oxy]-6-O-methyl-3-oxoerythromycin-(E)-9-O-(3-piperidinyl)oxim,
- 3-Des-[(2,6-didesoxy-3-C-methyl-3-O-methyl-α-L-ribohexopyranosyl)oxy]-11-desoxy-10,11-didehydro-6-O-methyl-3-oxoerythromycin-(E)-9-O-[2-(dimethylamino)ethyl]oxim.

29. Verbindungen der Formel (I) nach einem der Ansprüche 1 bis 25 sowie ihre Additionssalze mit pharmazeutisch verträglichen Säuren als Arzneistoffe.

30. Verbindungen der Formel (I) nach einem der Ansprüche 25 bis 28 sowie ihre Additionssalze mit pharmazeutisch verträglichen Säuren als Arzneistoffe.

31. Pharmazeutische Präparate, umfassend als Wirkstoff mindestens einen Arzneistoff nach Anspruch 29 oder 30.

32. Verfahren zur Herstellung einer Verbindung der Formel (I) nach einem der Ansprüche 1 bis 28, dadurch **gekennzeichnet**, daß man eine Verbindung der Formel (II): 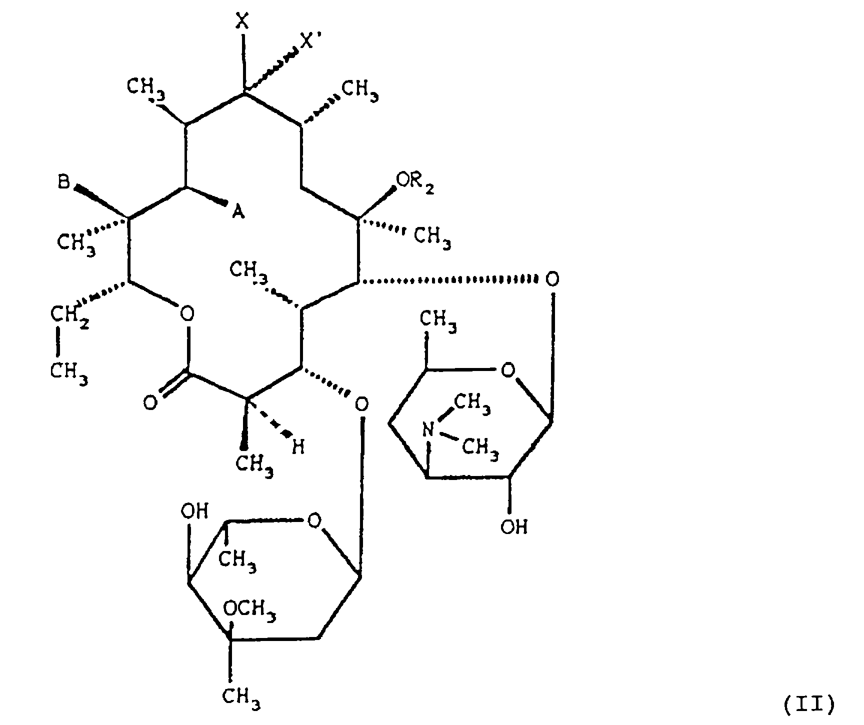 worin X, X', R₂, B und A wie vorstehend definiert sind, mit einer Säure in einem wäßrigen Medium umsetzt, um die Verbindung der Formel (III): 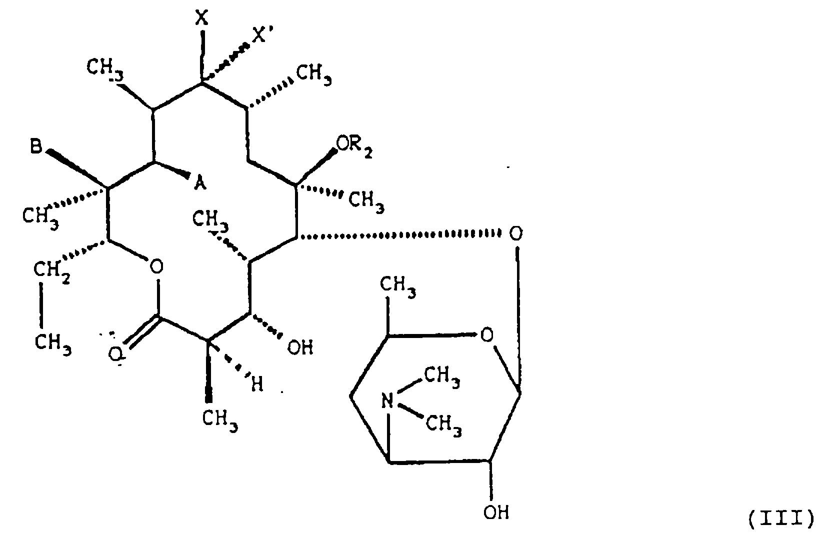 zu erhalten, die man mit einem Blockierungsmittel für die Hydroxylfunktion in 2' umsetzt, um eine Verbindung der Formel (IV): 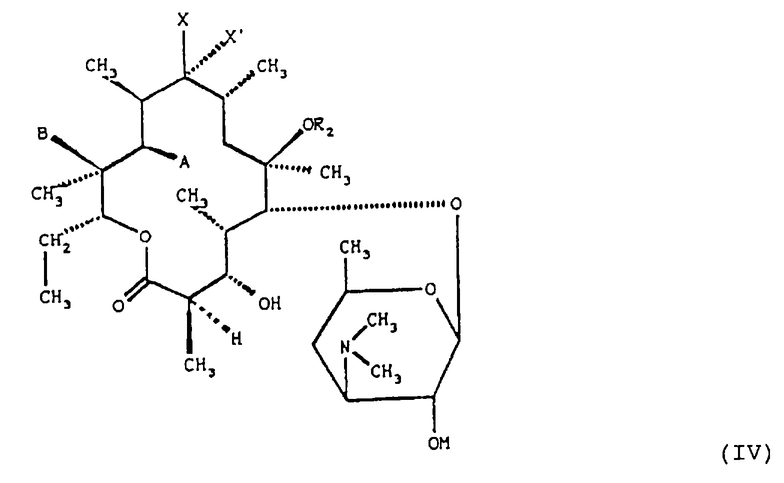 zu erhalten, worin OM eine blockierte Hydroxylgruppe bedeutet, und die anderen Substituenten wie vorstehend definiert sind, wobei man die Verbindung mit einem Oxidationsmittel für die Hydroxylfunktion in 3 umsetzt, um die Verbindung der Formel (V) : 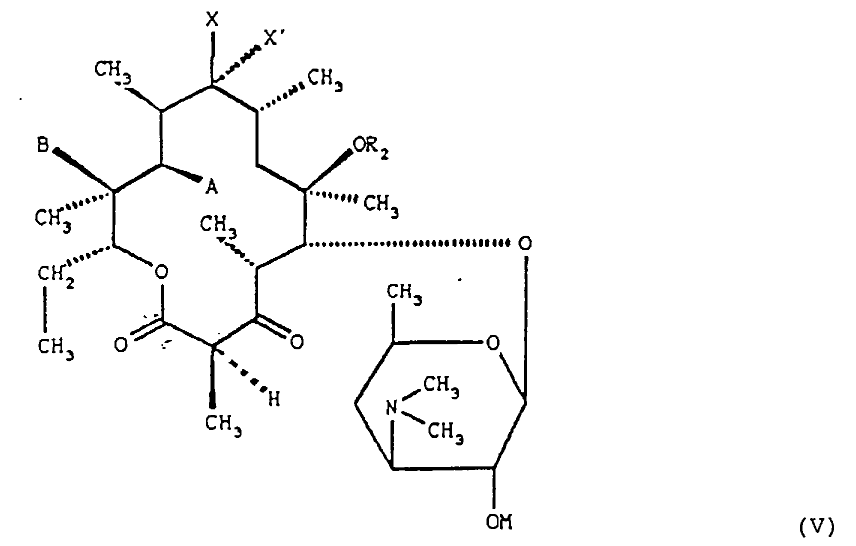 zu erhalten, die man gegebenenfalls mit einem Reagens, das den R'₃-Rest einführen kann, umsetzt, wobei R'₃ wie R₃, ausgenommen Wasserstoff, definiert ist, dann entweder gegebenenfalls mit einem Freisetzungsmittel für die Hydroxylfunktion in 2' umsetzt, um die Verbindung der Formel (I_{A}) : 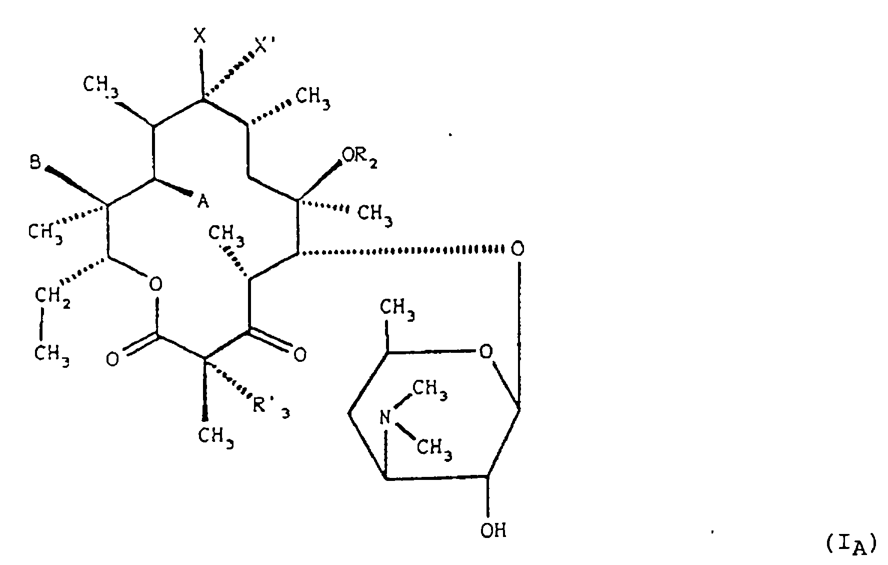 zu erhalten, d.h. eine Verbindung der Formel (I), worin Y und Y' zusammen mit dem Kohlenstoffatom, an das sie gebunden sind, eine Ketofunktion bilden, dann gegebenenfalls diese Verbindung der Formel (I_{A}) mit einem Oximierungsmittel des Ketons oder des β-Ketoesters umsetzt, um die gewünschte Verbindung der Formel (I) zu erhalten, dann gegebenenfalls die erhaltene Verbindung mit einem Veresterungsmittel für die Hydroxylgruppe in 2' umsetzt, oder zuerst mit einem Oximierungsmittel der Keto- oder β-Ketoesterfunktion umsetzt und anschließend gegebenenfalls mit einem Freisetzungsmittel für die Hydroxylfunktion in 2' umsetzt, um die gewünschte Verbindung der Formel (I) zu erhalten, dann gegebenenfalls die so erhaltene Verbindung der Formel (I) mit einer Säure zur Salzbildung umsetzt.

33. Verbindungen der Formeln (IV) und (V) nach Anspruch 32 als neue industrielle Produkte.

34. Ausführungsform des Verfahrens nach Anspruch 32 zur Herstellung der Verbindungen der Formel (I), worin X und X' zusammen eine Gruppierung C=NOR bilden, dadurch **gekennzeichnet**, daß man die verwendete Verbindung der Formel (IV_{A}), worin X und X' die Gruppierung C=N-OR bilden, aus dem entsprechenden Keton der Formel (II) durch Wirkung von NH₂OR in saurem Medium herstellt, um entsprechend dem pH-Wert der Reaktion die entsprechende an 10(11) gesättigte oder ungesättigte Verbindung der Formel (IV_{A}) : 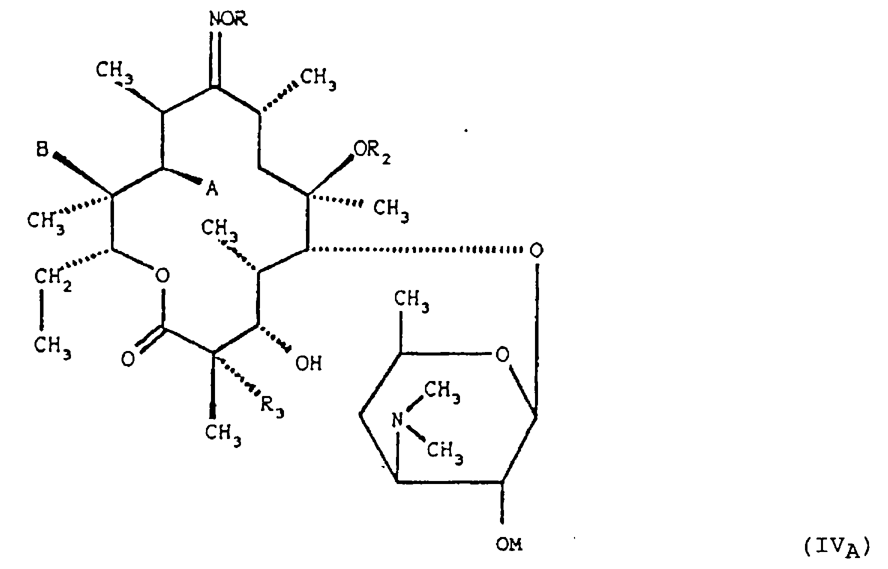 zu erhalten, worin A eine OH-Gruppe bedeutet, wenn keine Unsättigung in 10(11) vorhanden ist, oder ein Wasserstoffatom bedeutet, wenn eine Unsättigung in 10(11) vorhanden ist, R, R₂ und Z wie vorstehend definiert sind.

35. Ausführungsform des Verfahrens nach Anspruch 32 zur Herstellung der Verbindungen der Formel (I), worin X und X' eine Gruppierung C=NOR bilden, worin R wie vorstehend definiert ist, dadurch **gekennzeichnet**, daß man eine Verbindung der Formel (I_{A}), worin X und X' zusammen eine Ketogruppierung bilden, mit der Verbindung der Formel NH₂OR umsetzt, um die entsprechende Verbindung der Formel (I), worin X und X' eine Gruppierung C=NOR bilden und Z ein Wasserstoffatom bedeutet, zu erhalten und dann gegebenenfalls verestert oder in ein Salz überführt.

36. Verfahren nach Anspruch 32, dadurch **gekennzeichnet**, daß die als Ausgangsverbindung verwendete Verbindung der Formel (II) keine Unsättigung in 10(11) besitzt.

## Patentansprüche (Patentansprüche für folgende(n) Vertragsstaat(en): ES)

1. Verfahren zur Herstellung der Verbindungen der Formel (I) : 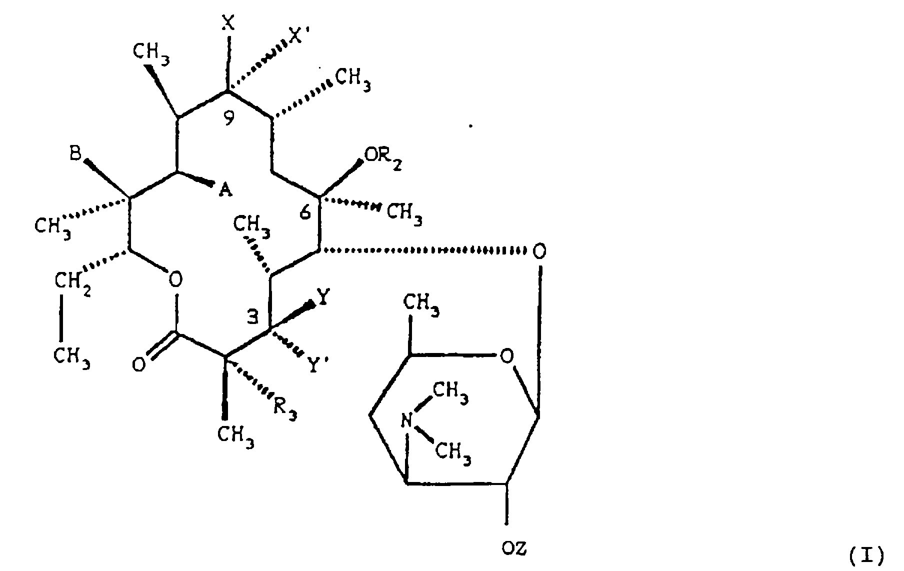
worin:
**entweder** X und X' zusammen mit dem Kohlenstoffatom, an das sie gebunden sind, eine Gruppierung C=O oder C=NOR bilden, worin R für
- ein Wasserstoffatom,
- einen mono- oder bicyclischen, gesättigten oder ungesättigten, aromatischen oder nicht-aromatischen, heterocyclischen Rest, umfassend mindestens ein Stickstoffatom und ggf. ein anderes Heteroatom, umfassend bis zu 12 Kettenglieder, gegebenenfalls am Stickstoffatom durch einen Alkylrest mit bis zu 4 Kohlenstoffatomen substituiert,
- einen linearen, verzweigten oder cyclischen Alkyl-, Alkenyl- oder Alkinylrest mit bis zu 18 Kohlenstoffatomen, gegebenenfalls substituiert durch eine oder mehrere Gruppierungen:
. Hydroxyl,
. Halogen,
. Cyano,
. Nitro,
. Amidinyl,
. Guanidinyl,
. Heterozyklus, wie vorstehend definiert,
. Alkyloxy, Alkenyloxy oder Alkinyloxy mit höchstens 6 Kohlenstoffatomen,
. Alkylthio, Alkenylthio oder Alkinylthio mit höchstens 6 Kohlenstoffatomen, wobei das Schwefelatom gegebenenfalls zu einem Sulfoxid oder Sulfon oxidiert ist,
. Aryl, Aralkyl,
. Aryloxy, Aralkyloxy,
. Arylthio, Aralkylthio, wobei das Schwefelatom gegebenenfalls zu einem Sulfoxid oder Sulfon oxidiert ist,
(jeder der Alkyloxy-, Alkenyloxy-, Alkinyloxy-, Alkylthio-, Alkenylthio- oder Alkinylthio-, Aryl-, Aralkyl-, Aryloxy-, Aralkyloxy-, Arylthio- oder Aralkylthioreste ist gegebenenfalls durch ein oder mehrere der folgenden Gruppierungen substituiert: Hydroxy, Alkyloxy, Alkylthio mit 1 bis 6 Kohlenstoffatomen, Alkenylthio, Alkinylthio mit bis zu 6 Kohlenstoffatomen, Amino, Monoalkylamino mit bis zu 6 Kohlenstoffatomen, Dialkylamino mit bis zu 12 Kohlenstoffatomen, ein Amidinyl-, Guanidinylrest, ein heterocyclischer Rest, wie vorstehend definiert, Aryl-, Aryloxy-, Arylthio-, Aralkyl-, Aralkyloxy- und Aralkylthioreste, die gegebenenfalls außerdem durch Methyl-, Ethyl-, Propyl-, Carbamoyl-, Aminomethyl-, Dimethylaminomethyl-, Aminoethyl-, Dimethylaminoethyl-, Carboxyl-, Methyloxycarbonyl-, Ethyloxycarbonylreste substituiert sind)
. 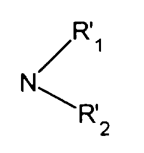 worin:
**entweder R'₁ und R'₂** gleich oder verschieden sind und für ein Wasserstoffatom, eine linearen, verzweigten oder cyclischen Alkyl-, Alkenyl- oder Alkinylrest mit bis zu 18 Kohlenstoffatomen, einen Aryl- oder Aralkylrest stehen, wobei jeder der Reste R'₁ und R'₂ gegebenenfalls substituiert durch ein oder mehrere Hydroxyl-, Alkyloxy-, Alkenyloxy-, Alkinyloxy-, Alkylthio-, Alkenylthio- oder Alkinylthioreste mit bis zu 8 Kohlenstoffatomen, Aminorest, Monoalkylaminorest mit bis zu 4 Kohlenstoffatomen, Dialkylaminorest mit bis zu 8 Kohlenstoffatomen, Cyanogruppe, freie, veresterte oder in ein Salz überführte Carboxylgruppe, Acylrest oder Carbamoylrest umfassend bis zu 8 Kohlenstoffatome, durch einen Rest Si(alc)₃ oder Si(Oalc)₃, worin alc für einen Alkylrest mit bis zu 4 Kohlenstoffatomen steht, einen heterocyclischen Rest, wie vorstehend definiert,
**oder R'₁ und R'₂** zusammen mit dem Stickstoffatom, an das sie gebunden sind, einen mono- oder bicyclischen, gesättigten oder ungesättigten, aromatischen oder nicht-aromatischen, heterocyclischen Rest, umfassend gegebenenfalls ein weiteres Heteroatom und umfassend bis zu 12 Kettenglieder, bilden;
. eine quaternäre Ammoniumgruppe,
. 1,2-Epoxyethyl oder 2,2-Dimethyl-1,2-epoxyethyl oder einen Rest als Ergebnis der Öffnung dieser Gruppierung durch ein nucleophiles Reagens,
. 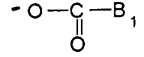 worin B₁ entweder einen Alkyl- oder Alkyloxyrest mit höchstens 6 Kohlenstoffatomen oder einen Aryl-, Aralkyl-, Aryloxy- oder Aralkyloxyrest bedeutet,
. ein freies oder geschütztes Formyl, freies, verestertes oder in ein Salz überführtes Carboxyl, Thiocyanat, Acyl oder Carbamoyl,
. (CH₂)ₙR', wobei R' den Rest einer Aminosäure bedeutet,
und n eine ganze Zahl zwischen 0 und 6 bedeutet, steht, **oder X** einen Rest 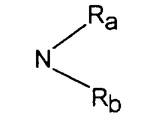 bedeutet,worin
- Rₐ und R_{b,} die gleich oder voneinander verschieden sind, für ein Wasserstoffatom oder einen Kohlenwasserstoffrest mit bis zu 18 Kohlenstoffatomen, umfassend gegebenenfalls ein oder mehrere Heteroatome, gegebenenfalls substituiert durch ein oder mehrere funktionelle Gruppierungen oder einen monooder bicyclischen, gesättigten oder ungesättigten, aromatischen oder nicht-aromatischen, heterocyclischen Rest, umfassend mindestens ein Stickstoffatom, und gegebenenfalls ein anderes Heteroatom, ausgewählt aus den Atomen Sauerstoff, Schwefel oder Stickstoff, umfassend bis zu 12 Kettenglieder, gegebenenfalls substituiert am Stickstoffatom durch einen Alkylrest, umfassend bis zu 4 Kohlenstoffatome, bedeuten,
- Rₐ und R_{b} gegebenenfalls mit dem Stickstoffatom, an das sie gebunden sind, einen mono- oder bicyclischen, gesättigten oder ungesättigten, aromatischen oder nichtaromatischen, heterocyclischen Rest, umfassend mindestens ein Stickstoffatom und gegebenenfalls ein anderes Heteroatom, ausgewählt aus den Atomen Sauerstoff, Schwefel oder Stickstoff, umfassend bis zu 12 Kettenglieder, bilden können,
- Rₐ und R_{b} mit dem Rest A einen Zyklus 9-N, 11-O, bilden können,
**und X'** ein Wasserstoffatom bedeutet,
**Y und Y'**, die gleich oder verschieden von X und X' sind, wie X und X' definiert sind,
**B** ein Wasserstoffatom oder einen Rest OR₄ bedeutet, wobei R₄ ein Wasserstoffatom bedeutet oder mit A einen Carbonat- oder Carbamatrest bildet,
**A** mit dem Kohlenstoff, der diesen Rest trägt und dem Kohlenstoff in Position 10 eine Doppelbindung bildet,
**oder A** einen Rest OR'₄ bedeutet, wobei R'₄ ein Wasserstoffatom bedeutet oder mit B einen Carbonatrest bildet,
**oder A** einen Rest 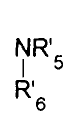 bedeutet, wobei 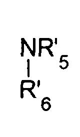 eine Gruppierung C=O, die mit B eine Carbamatgruppierung bildet, bedeutet, wobei R'₆ für ein Wasserstoffatom oder einen Alkyl-, Aralkyl- oder Alkyloxyrest mit bis zu 12 Kohlenstoffatomen oder eine Gruppierung 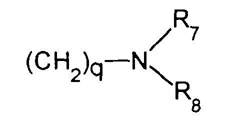 steht, wobei R₇ und R₈, die gleich oder verschieden sind, ein Wasserstoffatom oder einen Alkyl- oder Aralkylrest, umfassend bis zu 18 Kohlenstoffatome, bedeuten, oder mit dem Stickstoffatom einen, wie vorstehend definierten, Heterozyklus bilden, und q eine ganze Zahl zwischen 1 und 6 bedeutet, **oder A** einen Rest 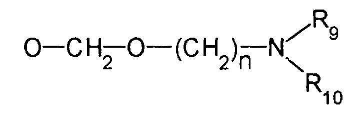 bedeutet, wobei R₉ und R₁₀ ein Wasserstoffatom oder einen Alkylrest, umfassend bis zu 8 Kohlenstoffatome, bedeuten, oder mit dem Stickstoffatom einen, wie vorstehend definierten Heterozyklus bilden, und n eine ganze Zahl zwischen 1 und 6 bedeutet,
**R₂** einen Alkylrest, umfassend bis zu 8 Kohlenstoffatome oder einen Rest CONH₂ oder CONHCOR₁₁ oder CONHSO₂R₁₁ bedeutet, worin R₁₁ einen Kohlenwasserstoffrest, umfassend bis zu 18 Kohlenstoffatome, umfassend gegebenenfalls ein oder mehrere Heteroatome, bedeutet,
**R₃** in Position α oder β ein Wasserstoffatom oder einen Alkylrest, umfassend bis zu 8 Kohlenstoffatome, oder einen Rest 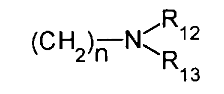 bedeutet, worin R₁₂ und R₁₃ ein Wasserstoffatom, einen Alkylrest, umfassend bis zu 8 Kohlenstoffatome, bedeuten oder mit dem Stickstoffatom einen, wie vorstehend definierten, Heterozyklus bilden, n eine ganze Zahl zwischen 1 und 6 bedeutet, oder einen Rest 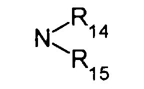 bedeutet, worin R₁₄ und R₁₅, die gleich oder verschieden sind, ein Wasserstoffatom oder einen Alkylrest, umfassend bis zu 8 Kohlenstoffatome, oder ein Heteroatom oder einen Alkyl- oder Alkyloxyrest, umfassend bis zu 8 Kohlenstoffatome, bedeuten,
**Z** ein Wasserstoffatom oder den Rest einer Carbonsäure, umfassend bis zu 18 Kohlenstoffatome, bedeutet, wobei die Oxime, die X und X' oder Y und Y' bedeuten können, in der Syn- oder Anti-Konfiguration vorliegen können, sowie der Säureadditionssalze der Verbindungen der Formel (I), dadurch **gekennzeichnet**, daß man eine Verbindung der Formel (II) : 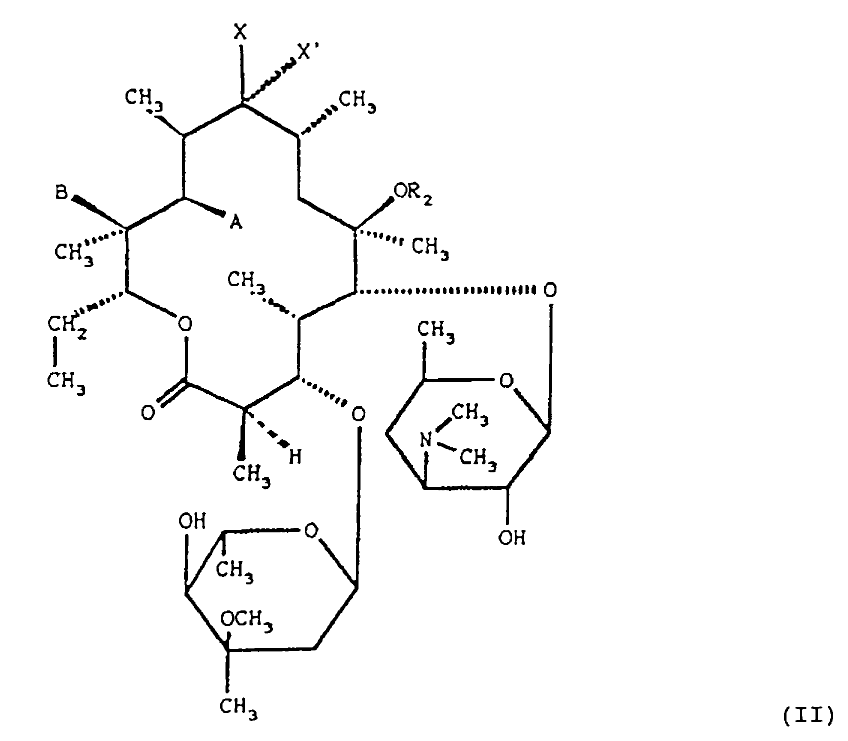 worin X, X', R₂, B und A wie vorstehend definiert sind, mit einer Säure in einem wäßrigen Medium umsetzt, um die Verbindung der Formel (III): 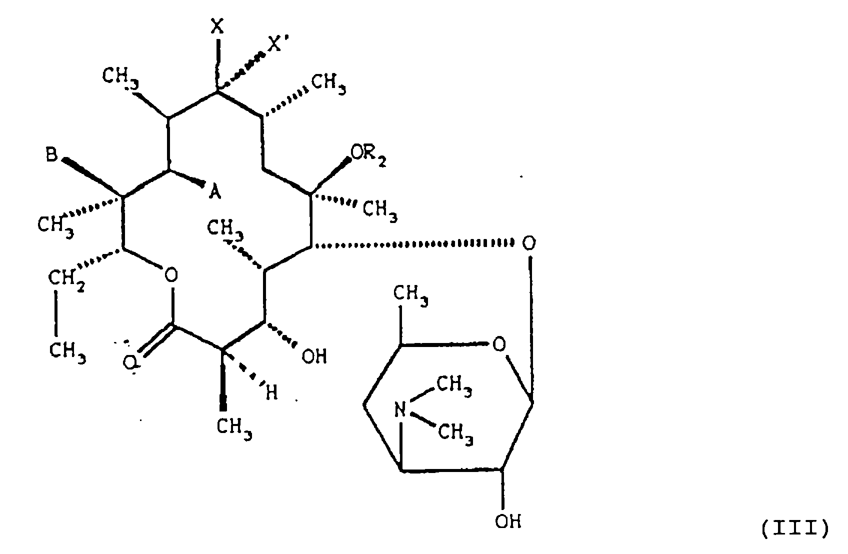 zu erhalten, die man mit einem Blockierungsmittel für die Hydroxylfunktion in 2' umsetzt, um eine Verbindung der Formel (IV): 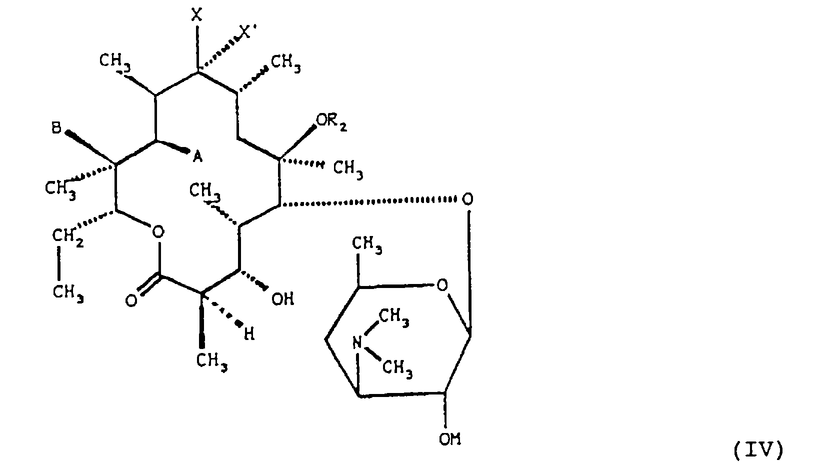 zu erhalten, worin OM eine blockierte Hydroxylgruppe bedeutet, und die anderen Substituenten wie vorstehend definiert sind, wobei man die Verbindung mit einem Oxidationsmittel für die Hydroxylfunktion in 3 umsetzt, um die Verbindung der Formel (V): 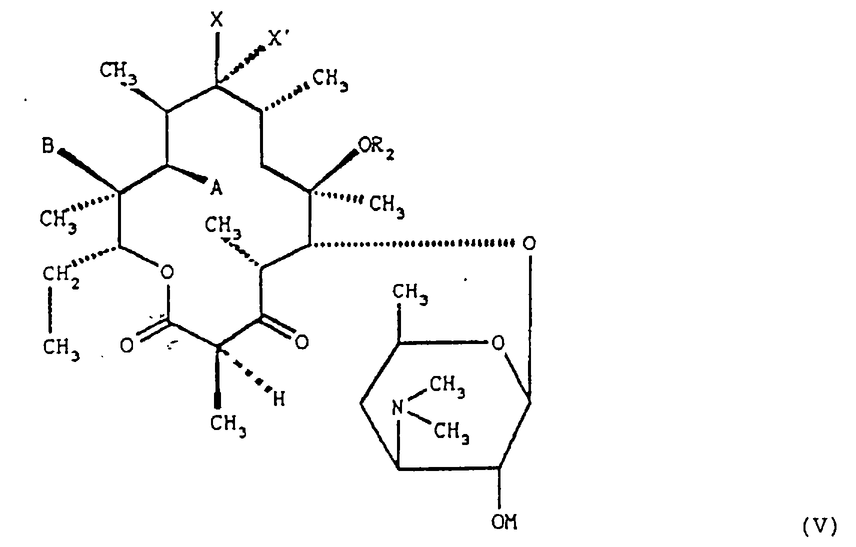 zu erhalten, die man gegebenenfalls mit einem Reagens, das den R'₃-Rest einführen kann, umsetzt, wobei R'₃ wie R₃, ausgenommen Wasserstoff, definiert ist, dann entweder gegebenenfalls mit einem Freisetzungsmittel für die Hydroxylfunktion in 2' umsetzt, um die Verbindung der Formel (I_{A}) : 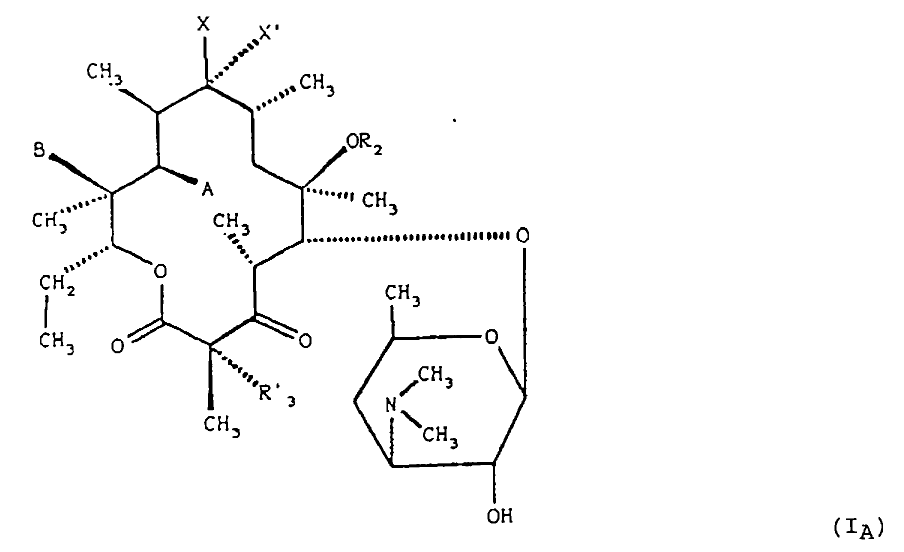 zu erhalten, d.h. eine Verbindung der Formel (I), worin Y und Y' zusammen mit dem Kohlenstoffatom, an das sie gebunden sind, eine Ketofunktion bilden, dann gegebenenfalls diese Verbindung der Formel (I_{A}) mit einem Oximierungsmittel des Ketons oder des β-Ketoesters umsetzt, um die gewünschte Verbindung der Formel (I) zu erhalten, dann gegebenenfalls die erhaltene Verbindung mit einem Veresterungsmittel für die Hydroxylgruppe in 2' umsetzt, oder zuerst mit einem Oximierungsmittel der Keto- oder β-Ketoesterfunktion umsetzt und anschließend gegebenenfalls mit einem Freisetzungsmittel für die Hydroxylfunktion in 2' umsetzt, um die gewünschte Verbindung der Formel (I) zu erhalten, dann gegebenenfalls die so erhaltene Verbindung der Formel (I) mit einer Säure zur Salzbildung umsetzt.

2. Verfahren nach Anspruch 1, zur Herstellung von Verbindungen der Formel (I), worin X und X' zusammen eine Gruppierung C=NOR bilden, dadurch **gekennzeichnet**, daß man die verwendete Verbindung der Formel (IV_{A}), worin X und X' die Gruppierung C=N-OR bilden, aus dem entsprechenden Keton der Formel (II) durch Wirkung von NH₂OR in saurem Medium herstellt, um entsprechend dem pH-Wert der Reaktion die entsprechende an 10(11) gesättigte oder ungesättigte Verbindung der Formel (IV_{A}) : 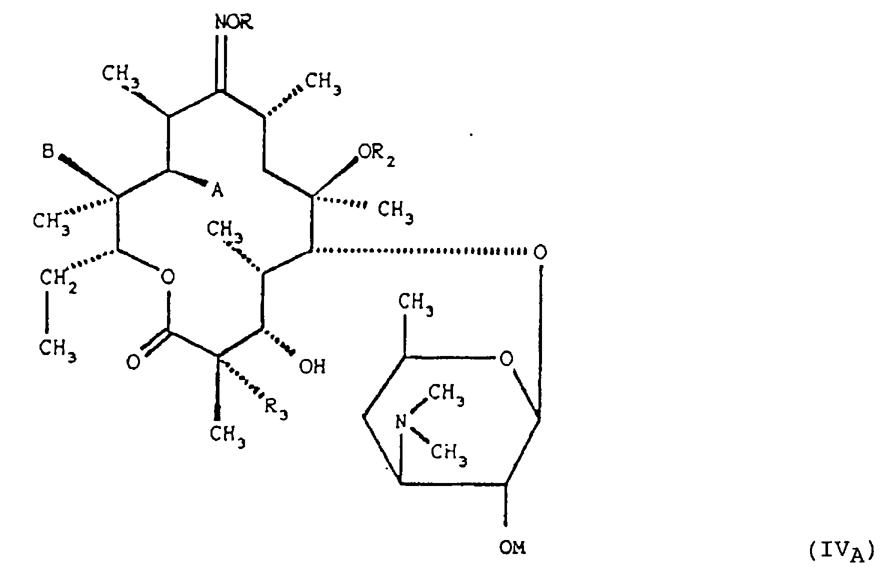 zu erhalten, worin A eine OH-Gruppe bedeutet, wenn keine Unsättigung in 10(11) vorhanden ist, oder ein Wasserstoffatom bedeutet, wenn eine Unsättigung in 10(11) vorhanden ist, R, R₂ und Z wie in Anspruch 1 definiert sind.

3. Verfahren nach Anspruch 1 zur Herstellung der Verbindungen der Formel (I), worin X und X' eine Gruppierung C=NOR bilden, worin R wie vorstehend definiert ist, dadurch **gekennzeichnet**, daß man eine Verbindung der Formel (I_{A}), worin X und X' zusammen eine Ketogruppierung bilden, mit der Verbindung der Formel NH₂OR umsetzt, um die entsprechende Verbindung der Formel (I), worin X und X' eine Gruppierung C=NOR bilden und Z ein Wasserstoffatom bedeutet, zu erhalten, und dann gegebenenfalls verestert oder in ein Salz überführt.

4. Verfahren nach Anspruch 1, dadurch **gekennzeichnet**, daß die als Ausgangsverbindung verwendete Verbindung der Formel (II) keine Unsättigung in 10(11) besitzt.

5. Verfahren nach einem der Ansprüche 1 bis 4, dadurch **gekennzeichnet**, daß man die Oximierung der Ketofunktion in einer einzigen Stufe mit einem Hydroxylamin RONH₂, das den gewünschten Substituenten R trägt, durchführt nämlich mit einem Hydroxylamin H₂N-O-(CH₂)ₙ-Hal, um eine Verbindung der Formel (I) zu erhalten, worin 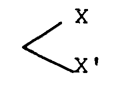 die Gruppierung =N-O-(CH₂)ₙ-Hal bedeutet, die man gegebenenfalls mit einem Amin der Formel 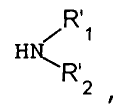 worin R'₁ und R'₂ wie vorstehend definiert sind, umsetzt, um eine Verbindung der Formel (I) zu erhalten, worin 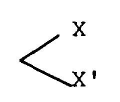 die Gruppierung 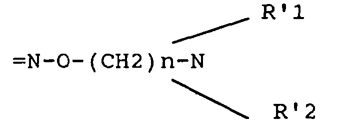 bedeutet, die man gegebenenfalls beispielsweise mit einem Alkylierungs-, Acylierungs-, Reduktionsmittel umsetzt, um die gewünschte Verbindung der Formel (I) zu erhalten.

6. Verfahren nach einem der Ansprüche 1 bis 5, dadurch **gekennzeichnet**, daß man zur Herstellung der Verbindungen der Formel (I), worin X und X' zusammen eine Gruppierung C=NOR bilden, ein Oximierungsmittel NH₂OR verwendet, worin R wie in Anspruch 1 definiert ist.

7. Verfahren nach einem der Ansprüche 1 bis 6, dadurch **gekennzeichnet**, daß man ein Oximierungsmittel NH₂OR verwendet, worin R einen Alkylrest, umfassend bis zu 6 Kohlenstoffatome, und substituiert durch einen Rest 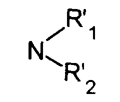 bedeutet, wobei R'₁ und R'₂ wie in Anspruch 1 definiert sind.

8. Verfahren nach einem der Ansprüche 1 bis 6, dadurch **gekennzeichnet**, daß man ein Oximierungsmittel NH₂OR verwendet, worin der Rest R ein Rest 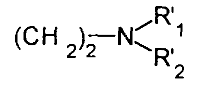 ist, wobei R'₁ und R'₂ einen Alkylrest mit bis zu 4 Kohlenstoffatomen bedeuten.

9. Verfahren nach Anspruch 8, dadurch **gekennzeichnet**, daß man ein Oximierungsmittel NH₂OR verwendet, worin R einen Rest (CH₂)₂-N(CH₃)₂ bedeutet.

10. Verfahren nach einem der Ansprüche 1 bis 6, dadurch **gekennzeichnet**, daß man ein Oximierungsmittel NH₂OR verwendet, worin der Rest R ein Rest 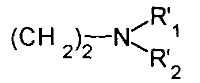 ist, wobei R'₁ ein Wasserstoffatom bedeutet, und R'₂ einen Alkylrest, umfassend bis zu 4 Kohlenstoffatome, substituiert durch einen heterocyclischen Rest, umfassend mindestens ein Stickstoffatom, bedeutet.

11. Verfahren nach Anspruch 10, dadurch **gekennzeichnet**, daß man ein Oximierungsmittel NH₂OR verwendet, worin R einen Rest 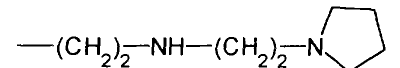 bedeutet.

12. Verfahren nach einem der Ansprüche 1 bis 6, dadurch **gekennzeichnet**, daß man ein Oximierungsmittel NH₂OR verwendet, worin R einen Alkylrest, umfassend bis zu 6 Kohlenstoffatome, substituiert durch einen Alkyloxyrest, umfassend bis zu 6 Kohlenstoffatome, der gegebenenfalls durch eine Methoxygruppe substituiert ist, bedeutet.

13. Verfahren nach Anspruch 12, dadurch **gekennzeichnet**, daß man ein Oximierungsmittel NH₂OR verwendet, worin R einen Rest 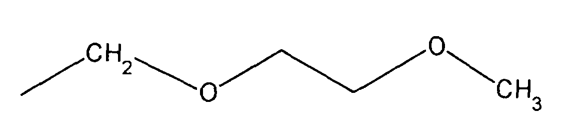 bedeutet.

14. Verfahren nach einem der Ansprüche 1 bis 6, dadurch **gekennzeichnet**, daß man ein Oximierungsmittel NH₂OR verwendet, worin R einen Heterozyklus, umfassend mindestens ein Stickstoffatom, bedeutet.

15. Verfahren nach Anspruch 14, dadurch **gekennzeichnet**, daß man ein Oximierungsmittel NH₂OR verwendet, worin R eine 3-Piperidinylgruppe bedeutet.

16. Verfahren nach Anspruch 1, dadurch **gekennzeichnet**, daß man zur Herstellung einer Verbindung der Formel (I_{A}) , worin X und X' zusammen eine Ketofunktion bilden, als Ausgangsverbindung eine Verbindung der Formel (II) verwendet, worin X und X' wie vorstehend definiert sind.

17. Verfahren nach einem der Ansprüche 2 bis 14, dadurch **gekennzeichnet**, daß man zur Herstellung einer Verbindung der Formel (I) , worin Y und Y' zusammen eine Gruppierung C=NOR bilden, ein Oximierungsmittel NH₂OR verwendet, worin R wie in Anspruch 1 definiert ist.

18. Verfahren nach Anspruch 17, dadurch **gekennzeichnet**, daß man ein Oximierungsmittel NH₂OR verwendet, worin R ein Benzylrest ist.

19. Verfahren nach einem der Ansprüche 1 bis 18, dadurch **gekennzeichnet**, daß man als Ausgangsverbindung eine Verbindung der Formel (II) verwendet, worin R₂ einen Alkylrest, umfassend 1 bis 4 Kohlenstoffatome, bedeutet.

20. Verfahren nach Anspruch 19, dadurch **gekennzeichnet**, daß man als Ausgangsverbindung eine Verbindung der Formel (II) verwendet, worin R₂ einen Methylrest bedeutet.

21. Verfahren nach einem der Ansprüche 1 bis 20, dadurch **gekennzeichnet**, daß man die Verbindungen der Formel (V) entweder gegebenenfalls mit einem Mittel zur Freisetzung der Hydroxylfunktion in 2' umsetzt, um die Verbindung der Formel (I_{A}) zu erhalten, worin R'₃ ein Wasserstoffatom bedeutet, die man gegebenenfalls mit einem Oximierungsmittel der Keto- oder β-Ketoesterfunktion umsetzt, anschließend gegebenenfalls mit einem Mittel zur Verseterung der Hydroxylgruppe in 2' umsetzt, oder zuerst mit einem Oximierungsmittel und dann gegebenenfalls mit einem Mittel zur Freisetzung der Hydroxylfunktion in 2' umsetzt, um eine Verbindung der Formel (I) zu erhalten, worin R₃ ein Wasserstoffatom bedeutet, dann gegebenenfalls die so erhaltene Verbindung der Formel (I) mit einer Säure umsetzt, um daraus ein Salz zu bilden.

22. Verfahren nach einem der Ansprüche 1 bis 21, dadurch **gekennzeichnet**, daß man als Ausgangsverbindung eine Verbindung der Formel (II) verwendet, worin A eine OH-Gruppe bedeutet.

23. Verfahren nach einem der Ansprüche 1 bis 22, dadurch **gekennzeichnet**, daß man als Ausgangsverbindung eine Verbindung der Formel (II) verwendet, worin B eine OH-Gruppe bedeutet.

24. Verfahren nach einem der Ansprüche 1 bis 21, dadurch **gekennzeichnet**, daß man als Ausgangsverbindung eine Verbindung der Formel (II) verwendet, worin A und B zusammen eine cyclische 11,12-Carbonatgruppierung bilden.

25. Verfahren nach einem der Ansprüche 1 bis 21, dadurch **gekennzeichnet**, daß man als Ausgangsverbindung eine Verbindung der Formel (II) verwendet, worin A und B zusammen einen zweiwertigen Rest 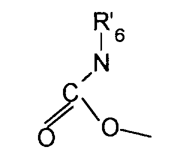 bilden, worin R'₆ ein Wasserstoffatom oder einen Alkyl-, Aralkyl- oder Alkyloxyrest mit bis zu 12 Kohlenstoffatomen oder eine Gruppierung: 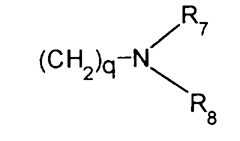 bedeutet, worin R₇ und R₈, die gleich oder verschieden sind, ein Wasserstoffatom oder einen Alkyl- oder Aralkylrest, umfassend bis zu 18 Kohlenstoffatome, bedeuten oder mit dem Stickstoffatom einen Heterozyklus, wie vorstehend definiert, bilden, und q eine ganze Zahl zwischen 1 und 6 bedeutet.

26. Verfahren nach Anspruche 25, dadurch **gekennzeichnet**, daß man als Ausgangsverbindung eine Verbindung der Formel (II) verwendet, worin A und B zusammen einen zweiwertigen Rest 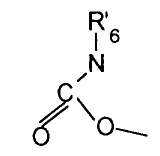 bilden, worin R'₆ einen Aralkylrest mit bis zu 12 Kohlenstoffatomen bedeutet.

27. Verfahren nach Anspruch 26, dadurch **gekennzeichnet**, daß man als Ausgangsverbindung eine Verbindung der Formel (II) verwendet, worin A und B zusammen einen zweiwertigen Rest 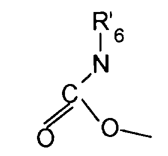 bilden, worin R'₆ einen Rest (CH₂)₄C₆H₅ bedeutet.

28. Verfahren nach einem der Ansprüche 1 bis 27, dadurch **gekennzeichnet**, daß man die Verbindung der Formel (V) oder (I_{A}) mit einem Mittel zur Freisetzung der Hydroxylgruppe in 2' umsetzt, um eine Verbindung der Formel (I) zu erhalten, worin Z ein Wasserstoffatom bedeutet.

## Patentansprüche (Patentansprüche für folgende(n) Vertragsstaat(en): GR)

1. Verfahren zur Herstellung der Verbindungen der Formel (I): 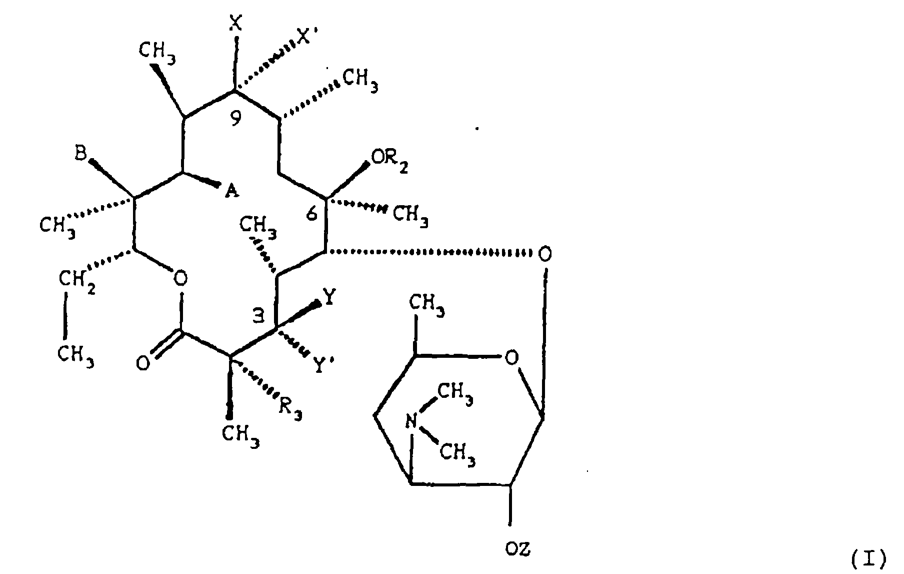 worin:
**entweder** X und X' zusammen mit dem Kohlenstoffatom, an das sie gebunden sind, eine Gruppierung C=O oder C=NOR bilden, worin R für
- ein Wasserstoffatom,
- einen mono- oder bicyclischen, gesättigten oder ungesättigten, aromatischen oder nicht-aromatischen, heterocyclischen Rest, umfassend mindestens ein Stickstoffatom und ggf. ein anderes Heteroatom, umfassend bis zu 12 Kettenglieder, gegebenenfalls am Stickstoffatom durch einen Alkylrest mit bis zu 4 Kohlenstoffatomen substituiert,
- einen linearen, verzweigten oder cyclischen Alkyl-, Alkenyl- oder Alkinylrest mit bis zu 18 Kohlenstoffatomen, gegebenenfalls substituiert durch eine oder mehrere Gruppierungen:
. Hydroxyl,
. Halogen,
. Cyano,
. Nitro,
. Amidinyl,
. Guanidinyl,
. Heterozyklus, wie vorstehend definiert,
. Alkyloxy, Alkenyloxy oder Alkinyloxy mit höchstens 6 Kohlenstoffatomen,
. Alkylthio, Alkenylthio oder Alkinylthio mit höchstens 6 Kohlenstoffatomen, wobei das Schwefelatom gegebenenfalls zu einem Sulfoxid oder Sulfon oxidiert ist,
. Aryl, Aralkyl,
. Aryloxy, Aralkyloxy,
. Arylthio, Aralkylthio, wobei das Schwefelatom gegebenenfalls zu einem Sulfoxid oder Sulfon oxidiert ist,
(jeder der Alkyloxy-, Alkenyloxy-, Alkinyloxy-, Alkylthio-, Alkenylthio- oder Alkinylthio-, Aryl-, Aralkyl-, Aryloxy-, Aralkyloxy-, Arylthio- oder Aralkylthioreste ist gegebenenfalls durch ein oder mehrere der folgenden Gruppierungen substituiert: Hydroxy, Alkyloxy, Alkylthio mit 1 bis 6 Kohlenstoffatomen, Alkenylthio, Alkinylthio mit bis zu 6 Kohlenstoffatomen, Amino, Monoalkylamino mit bis zu 6 Kohlenstoffatomen, Dialkylamino mit bis zu 12 Kohlenstoffatomen, ein Amidinyl-, Guanidinylrest, ein heterocyclischer Rest, wie vorstehend definiert-, Aryl-, Aryloxy-, Arylthio-, Aralkyl-, Aralkyloxy- und Aralkylthioreste, die gegebenenfalls außerdem durch Methyl-, Ethyl-, Propyl-, Carbamoyl-, Aminomethyl-, Dimethylaminomethyl-, Aminoethyl-, Dimethylaminoethyl-, Carboxyl-, Methyloxycarbonyl-, Ethyloxycarbonylreste substituiert sind)
. 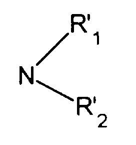
worin:
**entweder R'₁ und R'₂** gleich oder verschieden sind und für ein Wasserstoffatom, eine linearen, verzweigten oder cyclischen Alkyl-, Alkenyl- oder Alkinylrest mit bis zu 18 Kohlenstoffatomen, einen Aryl- oder Aralkylrest stehen, wobei jeder der Reste R'₁ und R'₂ gegebenenfalls substituiert durch ein oder mehrere Hydroxyl-, Alkyloxy-, Alkenyloxy-, Alkinyloxy-, Alkylthio-, Alkenylthio- oder Alkinylthioreste mit bis zu 8 Kohlenstoffatomen, Aminorest, Monoalkylaminorest mit bis zu 4 Kohlenstoffatomen, Dialkylaminorest mit bis zu 8 Kohlenstoffatomen, Cyanogruppe, freie, veresterte oder in ein Salz überführte Carboxylgruppe, Acylrest oder Carbamoylrest umfassend bis zu 8 Kohlenstoffatome, durch einen Rest si(alc) ₃ oder Si(Oalc)₃, worin alc für einen Alkylrest mit bis zu 4 Kohlenstoffatomen steht, einen heterocyclischen Rest, wie vorstehend definiert,
**oder R'₁ und R'₂** zusammen mit dem Stickstoffatom, an das sie gebunden sind, einen mono- oder bicyclischen, gesättigten oder ungesättigten, aromatischen oder nicht-aromatischen, heterocyclischen Rest, umfassend gegebenenfalls ein weiteres Heteroatom und umfassend bis zu 12 Kettenglieder, bilden;
. eine quaternäre Ammoniumgruppe,
. 1,2-Epoxyethyl oder 2,2-Dimethyl-1,2-epoxyethyl oder einen Rest als Ergebnis der Öffnung dieser Gruppierung durch ein nucleophiles Reagens,
. 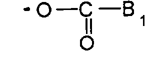 worin B₁ entweder einen Alkyl- oder Alkyloxyrest mit höchstens 6 Kohlenstoffatomen oder einen Aryl-, Aralkyl-, Aryloxy- oder Aralkyloxyrest bedeutet,
. ein freies oder geschütztes Formyl, freies, verestertes oder in ein Salz überführtes Carboxyl, Thiocyanat, Acyl oder Carbamoyl,
. (CH₂)ₙR', wobei R' den Rest einer Aminosäure bedeutet,
und n eine ganze Zahl zwischen 0 und 6 bedeutet, steht, **oder X** einen Rest 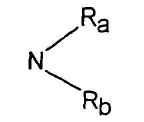 bedeutet,worin
- Rₐ und R_{b,} die gleich oder voneinander verschieden sind, für ein Wasserstoffatom oder einen Kohlenwasserstoffrest mit bis zu 18 Kohlenstoffatomen, umfassend gegebenenfalls ein oder mehrere Heteroatome, gegebenenfalls substituiert durch ein oder mehrere funktionelle Gruppierungen oder einen monooder bicyclischen, gesättigten oder ungesättigten, aromatischen oder nicht-aromatischen, heterocyclischen Rest, umfassend mindestens ein stickstoffatom, und gegebenenfalls ein anderes Heteroatom, ausgewählt aus den Atomen Sauerstoff, Schwefel oder Stickstoff, umfassend bis zu 12 Kettenglieder, gegebenenfalls substituiert am Stickstoffatom durch einen Alkylrest, umfassend bis zu 4 Kohlenstoffatome, bedeuten,
- Rₐ und R_{b} gegebenenfalls mit dem Stickstoffatom, an das sie gebunden sind, einen mono- oder bicyclischen, gesättigten oder ungesättigten, aromatischen oder nichtaromatischen, heterocyclischen Rest, umfassend mindestens ein Stickstoffatom und gegebenenfalls ein anderes Heteroatom, ausgewählt aus den Atomen Sauerstoff, Schwefel oder Stickstoff, umfassend bis zu 12 Kettenglieder, bilden können,
- Rₐ und R_{b} mit dem Rest A einen Zyklus 9-N, 11-O, bilden können,
**und X'** ein Wasserstoffatom bedeutet,
**Y und Y'**, die gleich oder verschieden von X und X' sind, wie X und X' definiert sind,
**B** ein Wasserstoffatom oder einen Rest OR₄ bedeutet, wobei R₄ ein Wasserstoffatom bedeutet oder mit A einen Car.bonat- oder Carbamatrest bildet,
**A** mit dem Kohlenstoff, der diesen Rest trägt und dem Kohlenstoff in Position 10 eine Doppelbindung bildet,
**oder A** einen Rest OR'₄ bedeutet, wobei R'₄ ein Wasserstoffatom bedeutet oder mit B einen Carbonatrest bildet,
**oder A** einen Rest 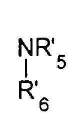 bedeutet, wobei 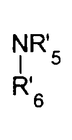 eine Gruppierung C=O, die mit B eine Carbamatgruppierung bildet, bedeutet, wobei R'₆ für ein Wasserstoffatom oder einen Alkyl-, Aralkyl- oder Alkyloxyrest mit bis zu 12 Kohlenstoffatomen oder eine Gruppierung 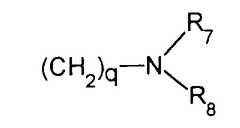 steht, wobei R₇ und R₈, die gleich oder verschieden sind, ein Wasserstoffatom oder einen Alkyl- oder Aralkylrest, umfassend bis zu 18 Kohlenstoffatome, bedeuten, oder mit dem Stickstoffatom einen, wie vorstehend definierten, Heterozyklus bilden, und q eine ganze Zahl zwischen 1 und 6 bedeutet, **oder A** einen Rest 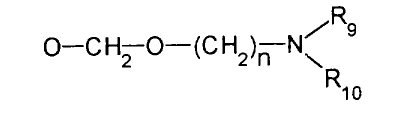 bedeutet, wobei R₉ und R₁₀ ein Wasserstoffatom oder einen Alkylrest, umfassend bis zu 8 Kohlenstoffatome, bedeuten, oder mit dem Stickstoffatom einen, wie vorstehend definierten Heterozyklus bilden, und n eine ganze Zahl zwischen 1 und 6 bedeutet,
**R₂** einen Alkylrest, umfassend bis zu 8 Kohlenstoffatome oder einen Rest CONH₂ oder CONHCOR₁₁ oder CONHSO₂R₁₁ bedeutet, worin R₁₁ einen Kohlenwasserstoffrest, umfassend bis zu 18 Kohlenstoffatome, umfassend gegebenenfalls ein oder mehrere Heteroatome, bedeutet,
**R₃** in Position α oder β ein Wasserstoffatom oder einen Alkylrest, umfassend bis zu 8 Kohlenstoffatome, oder einen Rest 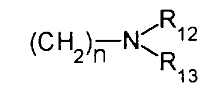 bedeutet, worin R₁₂ und R₁₃ ein Wasserstoffatom, einen Alkylrest, umfassend bis zu 8 Kohlenstoffatome, bedeuten oder mit dem Stickstoffatom einen, wie vorstehend definierten, Heterozyklus bilden, n eine ganze Zahl zwischen 1 und 6 bedeutet, oder einen Rest 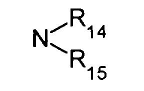 bedeutet, worin R₁₄ und R₁₅, die gleich oder verschieden sind, ein Wasserstoffatom oder einen Alkylrest, umfassend bis zu 8 Kohlenstoffatome, oder ein Heteroatom oder einen Alkyl- oder Alkyloxyrest, umfassend bis zu 8 Kohlenstoffatome, bedeuten,
**Z** ein Wasserstoffatom oder den Rest einer Carbonsäure, umfassend bis zu 18 Kohlenstoffatome, bedeutet, wobei die Oxime, die X und X' oder Y und Y' bedeuten können, in der Syn- oder Anti-Konfiguration vorliegen können, sowie der Säureadditionssalze der Verbindungen der Formel (I), dadurch **gekennzeichnet**, daß man eine Verbindung der Formel (II): 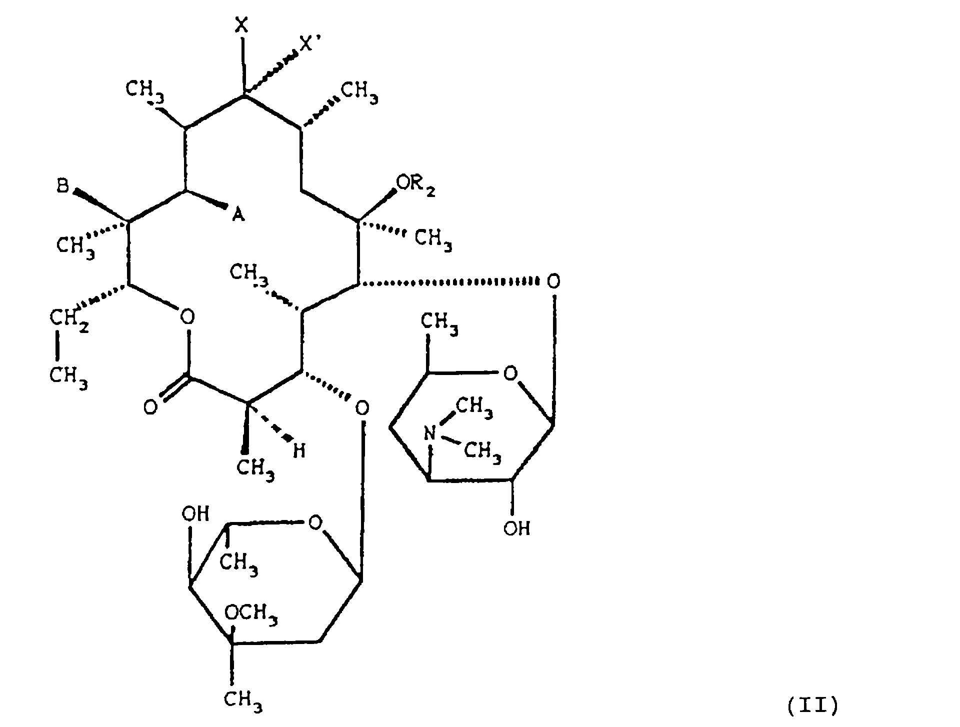 worin X, X', R₂, B und A wie vorstehend definiert sind, mit einer Säure in einem wäßrigen Medium umsetzt, um die Verbindung der Formel (III): 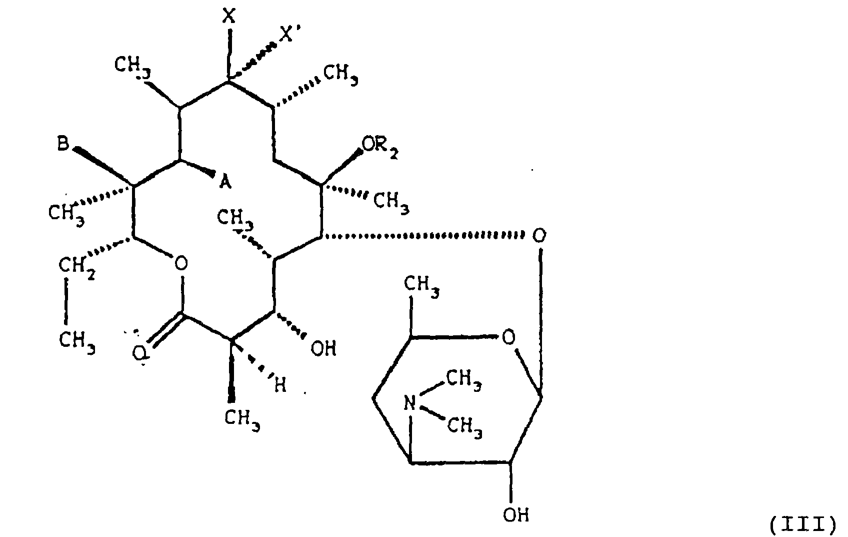 zu erhalten, die man mit einem Blockierungsmittel für die Hydroxylfunktion in 2' umsetzt, um eine Verbindung der Formel (IV) : 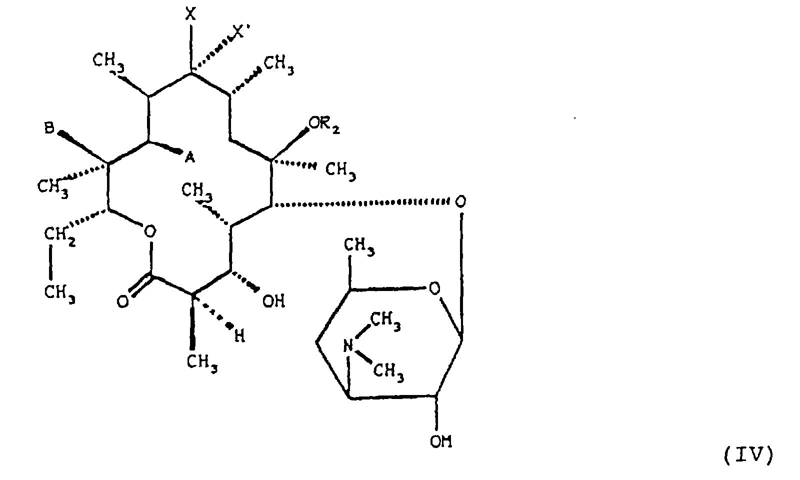 zu erhalten, worin OM eine blockierte Hydroxylgruppe bedeutet, und die anderen Substituenten wie vorstehend definiert sind, wobei man die Verbindung mit einem Oxidationsmittel für die Hydroxylfunktion in 3 umsetzt, um die Verbindung der Formel (V): 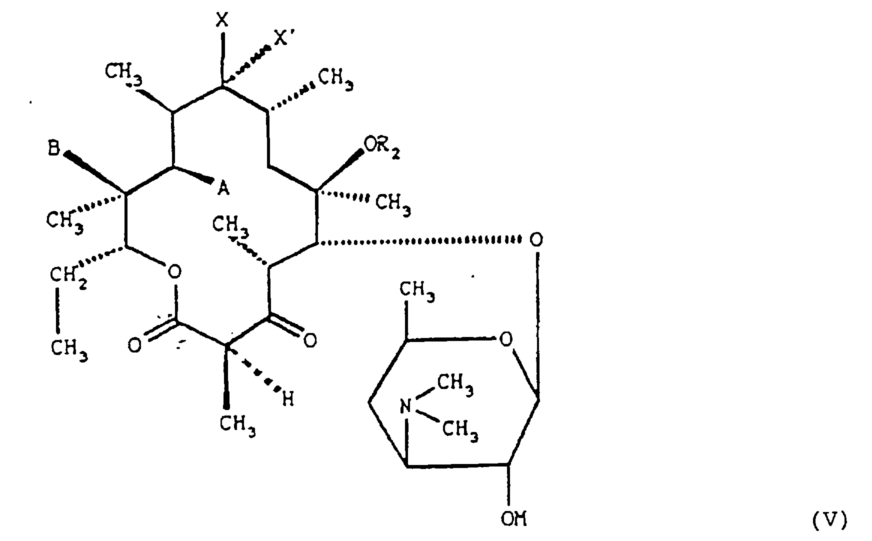 zu erhalten, die man gegebenenfalls mit einem Reagens, das den R'₃-Rest einführen kann, umsetzt, wobei R'₃ wie R₃, ausgenommen Wasserstoff, definiert ist, dann entweder gegebenenfalls mit einem Freisetzungsmittel für die Hydroxylfunktion in 2' umsetzt, um die Verbindung der Formel (I_{A}): 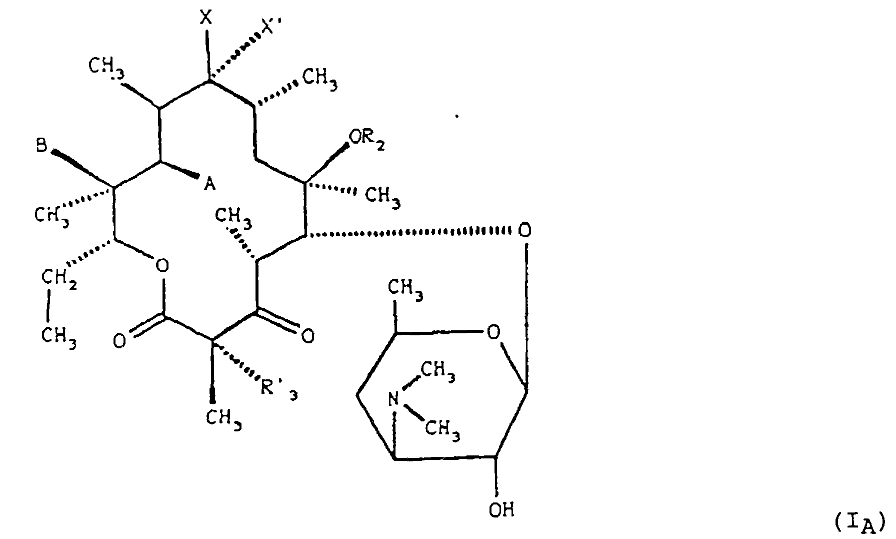 zu erhalten, d.h. eine Verbindung der Formel (I), worin Y und Y' zusammen mit dem Kohlenstoffatom, an das sie gebunden sind, eine Ketofunktion bilden, dann gegebenenfalls diese Verbindung der Formel (I_{A}) mit einem Oximierungsmittel des Ketons oder des β-Ketoesters umsetzt, um die gewünschte Verbindung der Formel (I) zu erhalten, dann gegebenenfalls die erhaltene Verbindung mit einem Veresterungsmittel für die Hydroxylgruppe in 2' umsetzt, oder zuerst mit einem Oximierungsmittel der Keto- oder β-Ketoesterfunktion umsetzt und anschließend gegebenenfalls mit einem Freisetzungsmittel für die Hydroxylfunktion in 2' umsetzt, um die gewünschte Verbindung der Formel (I) zu erhalten, dann gegebenenfalls die so erhaltene Verbindung der Formel (I) mit einer Säure zur Salzbildung umsetzt.

2. Verfahren nach Anspruch 1, zur Herstellung von Verbindungen der Formel (I), worin X und X' zusammen eine Gruppierung C=NOR bilden, dadurch **gekennzeichnet**, daß man die verwendete Verbindung der Formel 1 (IV_{A}), worin X und X' die Gruppierung C=N-OR bilden, aus dem entsprechenden Keton der Formel (II) durch Wirkung von NH₂OR in saurem Medium herstellt, um entsprechend dem pH-Wert der Reaktion die entsprechende an 10(11) gesättigte oder ungesättigte Verbindung der Formel (IV_{A}): 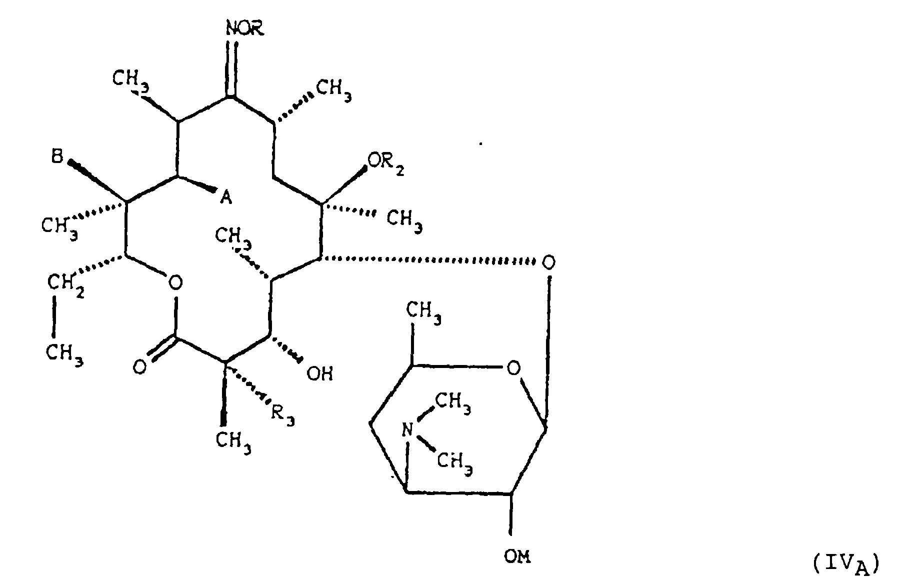 zu erhalten, worin A eine OH-Gruppe bedeutet, wenn keine Unsättigung in 10(11) vorhanden ist, oder ein Wasserstoffatom bedeutet, wenn eine Unsättigung in 10(11) vorhanden ist, R, R₂ und Z wie in Anspruch 1 definiert sind.

3. Verfahren nach Anspruch 1 zur Herstellung der Verbindungen der Formel (I), worin X und X' eine Gruppierung C=NOR bilden, worin R wie vorstehend definiert ist, dadurch **gekennzeichnet**, daß man eine Verbindung der Formel (I_{A}), worin X und X' zusammen eine Ketogruppierung bilden, mit der Verbindung der Formel NH₂OR umsetzt, um die entsprechende Verbindung der Formel (I), worin X und X' eine Gruppierung C=NOR bilden und Z ein Wasserstoffatom bedeutet, zu erhalten, und dann gegebenenfalls verestert oder in ein Salz überführt.

4. Verfahren nach Anspruch 1, dadurch **gekennzeichnet**, daß die als Ausgangsverbindung verwendete Verbindung der Formel (II) keine Unsättigung in 10(11) besitzt.

5. Verfahren nach einem der Ansprüche 1 bis 4, dadurch **gekennzeichnet**, daß man die Oximierung der Ketofunktion in einer einzigen Stufe mit einem Hydroxylamin RONH₂, das den gewünschten Substituenten R trägt, durchführt nämlich mit einem Hydroxylamin H₂N-O-(CH₂)ₙ-Hal, um eine Verbindung der Formel (I) zu erhalten, worin 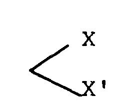 die Gruppierung =N-O-(CH₂)ₙ-Hal bedeutet, die man gegebenenfalls mit einem Amin der Formel 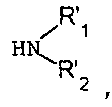 worin R'₁ und R'₂ wie vorstehend definiert sind, umsetzt, um eine Verbindung der Formel (I) zu erhalten, worin 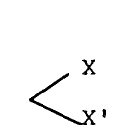 die Gruppierung 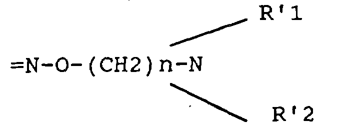 bedeutet, die man gegebenenfalls beispielsweise mit einem Alkylierungs-, Acylierungs-, Reduktionsmittel umsetzt, um die gewünschte Verbindung der Formel (I) zu erhalten.

6. Verfahren nach einem der Ansprüche 1 bis 5, dadurch **gekennzeichnet**, daß man zur Herstellung der Verbindungen der Formel (I), worin X und X' zusammen eine Gruppierung C=NOR bilden, ein Oximierungsmittel NH₂OR verwendet, worin R wie in Anspruch 1 definiert ist.

7. Verfahren nach einem der Ansprüche 1 bis 6, dadurch **gekennzeichnet**, daß man ein Oximierungsmittel NH₂OR verwendet, worin R einen Alkylrest, umfassend bis zu 6 Kohlenstoffatome, und substituiert durch einen Rest 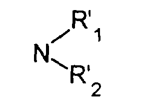 bedeutet, wobei R'₁ und R'₂ wie in Anspruch 1 definiert sind.

8. Verfahren nach einem der Ansprüche 1 bis 6, dadurch **gekennzeichnet**, daß man ein Oximierungsmittel NH₂OR verwendet, worin der Rest R ein Rest 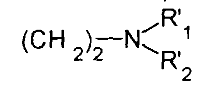 ist, wobei R'₁ und R'₂ einen Alkylrest mit bis zu 4 Kohlenstoffatomen bedeuten.

9. Verfahren nach Anspruch 8, dadurch **gekennzeichnet**, daß man ein Oximierungsmittel NH₂OR verwendet, worin R einen Rest (CH₂)₂-N(CH₃)₂ bedeutet.

10. Verfahren nach einem der Ansprüche 1 bis 6, dadurch **gekennzeichnet**, daß man ein Oximierungsmittel NH₂OR verwendet, worin der Rest R ein Rest 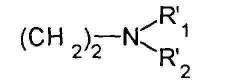 ist, wobei R'₁ ein Wasserstoffatom bedeutet, und R'₂ einen Alkylrest, umfassend bis zu 4 Kohlenstoffatome, substituiert durch einen heterocyclischen Rest, umfassend mindestens ein Stickstoffatom, bedeutet.

11. Verfahren nach Anspruch 10, dadurch **gekennzeichnet**, daß man ein Oximierungsmittel NH₂OR verwendet, worin R einen Rest 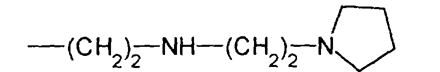 bedeutet.

12. Verfahren nach einem der Ansprüche 1 bis 6, dadurch **gekennzeichnet**, daß man ein Oximierungsmittel NH₂OR verwendet, worin R einen Alkylrest, umfassend bis zu 6 Kohlenstoffatome, substituiert durch einen Alkyloxyrest, umfassend bis zu 6 Kohlenstoffatome, der gegebenenfalls durch eine Methoxygruppe substituiert ist, bedeutet.

13. Verfahren nach Anspruch 12, dadurch **gekennzeichnet**, daß man ein Oximierungsmittel NH₂OR verwendet, worin R einen Rest 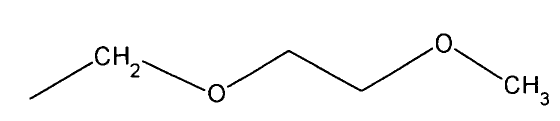 bedeutet.

14. Verfahren nach einem der Ansprüche 1 bis 6, dadurch **gekennzeichnet**, daß man ein Oximierungsmittel NH₂OR verwendet, worin R einen Heterozyklus, umfassend mindestens ein Stickstoffatom, bedeutet.

15. Verfahren nach Anspruch 14, dadurch **gekennzeichnet**, daß man ein Oximierungsmittel NH₂OR verwendet, worin R eine 3-Piperidinylgruppe bedeutet.

16. Verfahren nach Anspruch 1, dadurch **gekennzeichnet**, daß man zur Herstellung einer Verbindung der Formel (I_{A}) , worin X und X' zusammen eine Ketofunktion bilden, als Ausgangsverbindung eine Verbindung der Formel (II) verwendet, worin X und X' wie vorstehend definiert sind.

17. Verfahren nach einem der Ansprüche 2 bis 14, dadurch **gekennzeichnet**, daß man zur Herstellung einer Verbindung der Formel (I) , worin Y und Y' zusammen eine Gruppierung C=NOR bilden, ein Oximierungsmittel NH₂OR verwendet, worin R wie in Anspruch 1 definiert ist.

18. Verfahren nach Anspruch 17, dadurch **gekennzeichnet**, daß man ein Oximierungsmittel NH₂OR verwendet, worin R ein Benzylrest ist.

19. Verfahren nach einem der Ansprüche 1 bis 18, dadurch **gekennzeichnet**, daß man als Ausgangsverbindung eine Verbindung der Formel (II) verwendet, worin R₂ einen Alkylrest, umfassend 1 bis 4 Kohlenstoffatome, bedeutet.

20. Verfahren nach Anspruch 19, dadurch **gekennzeichnet**, daß man als Ausgangsverbindung eine Verbindung der Formel (II) verwendet, worin R₂ einen Methylrest bedeutet.

21. Verfahren nach einem der Ansprüche 1 bis 20, dadurch **gekennzeichnet**, daß man die Verbindungen der Formel (V) entweder gegebenenfalls mit einem Mittel zur Freisetzung der Hydroxylfunktion in 2' umsetzt, um die Verbindung der Formel (I_{A}) zu erhalten, worin R'₃ ein Wasserstoffatom bedeutet, die man gegebenenfalls mit einem Oximierungsmittel der Keto- oder β-Ketoesterfunktion umsetzt, anschließend gegebenenfalls mit einem Mittel zur Verseterung der Hydroxylgruppe in 2' umsetzt, oder zuerst mit einem Oximierungsmittel und dann gegebenenfalls mit einem Mittel zur Freisetzung der Hydroxylfunktion in 2' umsetzt, um eine Verbindung der Formel (I) zu erhalten, worin R₃ ein Wasserstoffatom bedeutet, dann gegebenenfalls die so erhaltene Verbindung der Formel (I) mit einer Säure umsetzt, um daraus ein Salz zu bilden.

22. Verfahren nach einem der Ansprüche 1 bis 21, dadurch **gekennzeichnet**, daß man als Ausgangsverbindung eine Verbindung der Formel (II) verwendet, worin A eine OH-Gruppe bedeutet.

23. Verfahren nach einem der Ansprüche 1 bis 22, dadurch **gekennzeichnet**, daß man als Ausgangsverbindung eine Verbindung der Formel (II) verwendet, worin B eine OH-Gruppe bedeutet.

24. Verfahren nach einem der Ansprüche 1 bis 21, dadurch **gekennzeichnet**, daß man als Ausgangsverbindung eine Verbindung der Formel (II) verwendet, worin A und B zusammen eine cyclische 11,12-Carbonatgruppierung bilden.

25. Verfahren nach einem der Ansprüche 1 bis 21, dadurch **gekennzeichnet**, daß man als Ausgangsverbindung eine Verbindung der Formel (II) verwendet, worin A und B zusammen einen zweiwertigen Rest 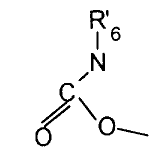 bilden, worin R'₆ ein Wasserstoffatom oder einen Alkyl-, Aralkyl- oder Alkyloxyrest mit bis zu 12 Kohlenstoffatomen oder eine Gruppierung: 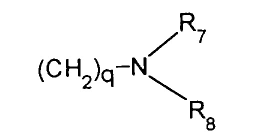 bedeutet, worin R₇ und R₈, die gleich oder verschieden sind, ein Wasserstoffatom oder einen Alkyl- oder Aralkylrest, umfassend bis zu 18 Kohlenstoffatome, bedeuten oder mit dem Stickstoffatom einen Heterozyklus, wie vorstehend definiert, bilden, und q eine ganze Zahl zwischen 1 und 6 bedeutet.

26. Verfahren nach Anspruche 25, dadurch **gekennzeichnet**, daß man als Ausgangsverbindung eine Verbindung der Formel (II) verwendet, worin A und B zusammen einen zweiwertigen Rest 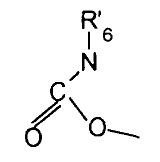 bilden, worin R'₆ einen Aralkylrest mit bis zu 12 Kohlenstoffatomen bedeutet.

27. Verfahren nach Anspruch 26, dadurch **gekennzeichnet** , daß man als Ausgangsverbindung eine Verbindung der Formel (II) verwendet, worin A und B zusammen einen zweiwertigen Rest 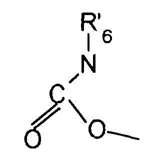 bilden, worin R'₆ einen Rest (CH₂)₄C₆H₅ bedeutet.

28. Verfahren nach einem der Ansprüche 1 bis 27, dadurch **gekennzeichnet**, daß man die Verbindung der Formel (V) oder (I_{A}) mit einem Mittel zur Freisetzung der Hydroxylgruppe in 2' umsetzt, um eine Verbindung der Formel (I) zu erhalten, worin Z ein Wasserstoffatom bedeutet.

29. Verfahren nach Anspruch 1, dadurch **gekennzeichnet** , daß man die Verbindung der Formel (II) und die verwendeten Reagentien so auswählt, daß man 3-Des-[(2,6-didesoxy-3-C-methyl-3-O-methyl-α-L-ribohexopyranosyl)oxy]-6-O-methyl-3-oxoerythromycin-9-[O-[2-(dimethylamino)ethyl]-oxim herstellt.

30. Verfahren nach Anspruch 1, dadurch **gekennzeichnet** daß man die Verbindung der Formel (II) und die verwendeten Reagentien so auswählt, daß man 11,12- Didexoxy-3-des-[(2,6-didesoxy-3-C-methyl-3-O-methyl-α-L-ribohexopyranosyl)oxy]6-O-methyl-3-oxo-12,11-[oxycarbonyl-[(4-phenylbutyl)imino]]erythromycin herstellt.

31. Verfahren nach Anspruch 1, dadurch **gekennzeichnet** daß man die Verbindung der Formel (II) und die verwendeten Reagentien so auswählt, daß man die folgenden Verbindungen der Formel (I):
- 3-Des-[(2,6-didesoxy-3-C-methyl-3-O-methyl-α-L-ribohexopyranosyl)oxy]-6-O-methyl-3-oxoerythromycin-9-[O-[(2-methoxyethoxy)methyl]oxim],
- 3-Des-[(2,6-didesoxy-3-C-methyl-3-O-methyl-α-L-ribohexopyranosyl)oxy]6-O-methyl-3-oxoerythromycin,
- cyclisches 3-Des-[(2,6-didesoxy-3-C-methyl-3-O-methyl-α-L-ribohexopyranosyl)oxy]-6-O-methyl-3-oxoerythromycin-11,12-carbonat herstellt.

32. Verfahren nach Anspruch 1, dadurch **gekennzeichnet** daß man die Verbindung der Formel (II) und die verwendeten Reagentien so auswählt, daß man die folgenden Verbindungen der Formel (I):
- 3-Des-[(2,6-didesoxy-3-C-methyl-3-O-methyl-α-L-ribohexopyranoxyl)oxy]-6-O-methyl-3-oxoerythromycin-(E)-9-O-[2-[[2-(1-pyrrolidinylethyl]amino]ethyl]oxim,
- 3-Des-[(2,6-didesoxy-3-C-methyl-3-O-methyl-α-L-ribohexopyranosyl)oxy]-6-O-methyl-3-oxoerythromycin-(E)-9-O-(3-piperidinyl)oxim,
- 3-Des-[(2,6-didesoxy-3-C-methyl-3-O-methyl-α-L-ribohexopyranosyl)oxy]-11-desoxy-10,11-didehydro-6-O-methyl-3-oxoerythromycin-(E)-9-O-[2-(dimethylamino)ethyl]oxim
herstellt.

33. Verfahren zur Herstellung pharmazeutischer Präparate, dadurch **gekennzeichnet**, daß man als Wirkstoff mindestens eines der Derivate der Formel (I) nach Anspruch 1 oder mindestens eines ihrer Additionssalze mit pharmazeutisch verträglichen Säuren in einer für diese Verwendung geeigneten Form einsetzt.

34. Verfahren zur Herstellung pharmazeutischer Präparate, dadurch **gekennzeichnet**, daß man als Wirkstoff mindestens eines der Derivate der Formel (I) nach einem der Ansprüche 2 bis 28 oder mindestens eines ihrer Additionssalze mit pharmazeutisch verträglichen Säuren in einer für diese Verwendung geeigneten Form einsetzt.

35. Verfahren zur Herstellung pharmazeutischer Präparate, dadurch **gekennzeichnet**, daß man als Wirkstoff mindestens eines der Derivate der Formel (I) nach einem der Ansprüche 29 bis 32 oder mindestens eines ihrer Additionssalze mit pharmazeutisch verträglichen Säuren in einer für diese Verwendung geeigneten Form einsetzt.

36. Verbindungen der Formeln (IV) und (V) nach Anspruch 1 als neue industrielle Produkte.

## Claims (Claims for the following Contracting State(s): AT, BE, CH, DE, DK, FR, GB, IT, LI, LU, NL, SE)

1. The compounds of formula (I): 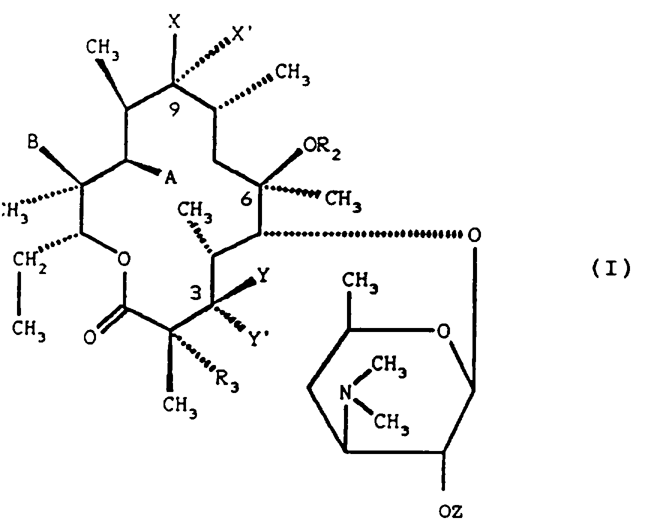 in which,
**either** X and X' form with the carbon atom to which they are linked a C=O or C=NOR group, in which R represents:
- a hydrogen atom,
- a heterocyclic radical containing at least one nitrogen atom and optionally another heteroatom, mono or bicyclic, saturated or unsaturated, aromatic or non-aromatic containing up to 12 links, optionally substituted on the nitrogen atom by an alkyl radical containing up to 4 carbon atoms,
- a linear, branched or cyclic alkyl, alkenyl or alkynyl radical containing up to 18 carbon atoms optionally substituted by one or more groups:
. hydroxyl,
. halogen,
. cyano,
. nitro,
. amidinyl,
. guanidinyl,
. heterocyclic, as defined previously,
. alkyloxy, alkenyloxy or alkynyloxy having at most 6 carbon atoms,
. alkylthio, alkenylthio or alkynylthio having at most 6 carbon atoms, the sulphur atom being optionally oxidized into sulphoxide or sulphone,
. aryl, aralkyl, . aryloxy, aralkyloxy,
. arylthio, aralkylthio, the sulphur atom being
optionally oxidized into sulphoxide or sulphone, (each of these alkyloxy, alkenyloxy, alkynyloxy, alkylthio, alkenylthio or alkynylthio, aryl, aralkyl, aryloxy, aralkyloxy, arylthio or aralkylthio radicals being optionally substituted by one or more of the following groups: hydroxy, alkyloxy, alkylthio having 1 to 6 carbon atoms, alkenylthio, alkynylthio having up to 6 carbon atoms, amino, monoalkylamino having up to 6 carbon atoms, dialkylamino having up to 12 carbon atoms, an amidinyl, guanidinyl radical, a heterocyclic radical as defined previously, aryl, aryloxy, arylthio, aralkyl, aralkyloxy and aralkylthio radicals also being optionally substituted by the following radicals: methyl, ethyl, propyl, carbamoyl, aminomethyl, dimethylaminomethyl, aminoethyl, dimethylaminoethyl, carboxyl, methyloxycarbonyl, ethyloxycarbonyl)
. 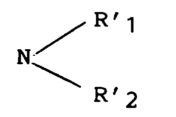
in which:
**either R'₁ and R'₂**, identical or different, represent a hydrogen atom, a linear, branched or cyclic alkyl, alkenyl or alkynyl radical, containing up to 18 carbon atoms, an aryl or aralkyl radical, each of these R'₁ and R'₂ radicals being optionally substituted by one or more of the following radicals: hydroxy, alkyloxy, alkenyloxy, alkynyloxy, alkylthio, alkenylthio or alkynylthio containing up to 8 carbon atoms, amino, monoalkylamino containing up to 4 carbon atoms, dialkylamino containing up to 8 carbon atoms, cyano, free, esterified or salified carboxyl, acyl or carbamoyl, containing up to 8 carbon atoms, by an Si(alk)₃ or Si(Oalk)₃ radical in which alk represents an alkyl radical containing up to 4 carbon atoms, by a heterocyclic radical as defined previously,
**or R'₁ and R'₂** form with the nitrogen atom to which they are linked a mono or bicyclic heterocycle radical optionally containing another heteroatom, saturated or unsaturated, aromatic or non-aromatic, containing up to 12 links;
. a quaternary ammonium group,
. 1, 2-epoxyethyl or 2,2-dimethyl-1,2-epoxyethyl or a radical resulting from the opening of this group by a nucleophile reagent,
. 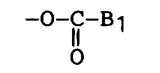 in which B₁ represents either an alkyl or alkyloxy radical having at most 6 carbon atoms, or an aryl, aralkyl, aryloxy or aralkyloxy radical,
. free or protected formyl, free, esterified or salified carboxyl, thiocyanate, acyl or carbamoyl,
. (CH₂)ₙR', R' representing the remainder of an amino acid, and n representing an integer comprised between O and 6, **or X** represents an 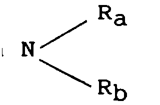 radical
- Rₐ and R_{b}, identical or different from each other, representing a hydrogen atom or a hydrocarbonated radical containing up to 18 carbon atoms, optionally containing one or more heteroatoms, optionally substituted by one or more functional groups, or by a heterocyclic radical containing at least one nitrogen atom and optionally another heteroatom chosen from oxygen, sulphur and nitrogen, mono or bicyclic, saturated or unsaturated, aromatic or non-aromatic containing up to 12 links, optionally substituted on the nitrogen atom by an alkyl radical containing up to 4 carbon atoms,
- Rₐ and R_{b} optionally being able to form with the nitrogen atom to which they are linked a heterocycle radical, containing at least one nitrogen atom and optionally another heteroatom chosen from oxygen, sulphur and nitrogen, mono or bicyclic, saturated or unsaturated, aromatic or non-aromatic containing up to 12 links,
- Rₐ and R_{b} being able to form with radical A a 9-N 11-0 ring,
**and X'** represents a hydrogen atom,
**Y and Y'**, identical or different from X and X', have the meaning of X and X'
**B** represents a hydrogen atom or an OR₄ radical, R₄ representing a hydrogen atom, or forms with A a carbonate or carbamate radical,
**A** forms with the carbon which carries it and the carbon in position 10, a bond double,
**or A** represents an OR'₄ radical, R'₄ representing a hydrogen atom, or forms with B a carbonate radical,
**or A** represents an 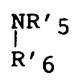 radical, 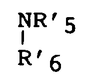 representing a C=O group forming with B a carbamate group, R'₆ representing a hydrogen atom or an alkyl, aralkyl or alkyloxy radical having up to 12 carbon atoms or a group: 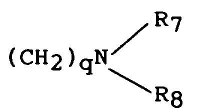 R₇ and R₈, identical or different, representing a hydrogen atom or an alkyl or aralkyl radical, containing up to 18 carbon atoms, or forming with the nitrogen atom a heterocycle as defined previously, q representing an integer comprised between 1 and 6,
**or A** represents an 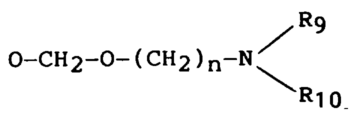 R₉ and R₁₀ representing a hydrogen atom or an alkyl radical containing up to 8 carbon atoms, or forming with the nitrogen atom a heterocycle as defined previously, n representing an integer comprised between 1 and 6,
**R₂** represents an alkyl radical containing up to 8 carbon atoms, or a CONH₂ or CONHCOR₁₁ or CONHSO₂R₁₁ in which R₁₁ represents a hydrocarbonated radical containing up to 18 carbon atoms comprising optionally one or more heteroatoms,
**R₃** in alpha or beta position represents a hydrogen atom or an alkyl radical, containing up to 8 carbon atoms or a 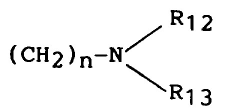 radical in which R₁₂ and R₁₃ represent a hydrogen atom, an alkyl radical containing up to 8 carbon atoms or form with the nitrogen atom a heterocycle as defined previously, n representing an integer comprised between 1 and 6 or an 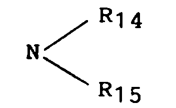 radical, R₁₄ and R₁₅, identical or different, representing a hydrogen atom or an alkyl radical containing up to 8 carbon atoms or a heteroatom or an alkyl or alkyloxy radical containing up to 8 carbon atoms,
**Z** represents a hydrogen atom or the remainder of a carboxylic acid containing up to 18 carbon atoms, the oximes that can be represented by X and X' or Y and Y' can be of syn or anti configuration, as well as the addition salts with acids of the compounds of formula (I).

2. The compounds of formula (I) as defined in claim 1 in which X and X' form with the carbon atom to which they are linked a C=NOR group, R keeping the same meaning as in claim 1.

3. The compounds of formula (I) as defined in claim 2 in which R represents an alkyl radical containing up to 6 carbon atoms substituted by an 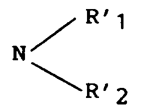 radical R'₁ and R'₂ keeping the same meaning as in claim 1.

4. The compounds of formula (I) as defined in claim 3 in which the R radical is an 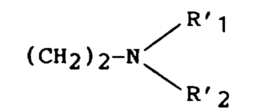 radical, R'₁ and R'₂ representing an alkyl radical containing up to 4 carbon atoms.

5. The compounds of formula (I) as defined in claim 4 in which R represents a (CH₂)₂-N(CH₃)₂ radical.

6. The compounds of formula (I) as defined in claim 3 in which the R radical is a 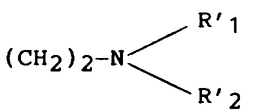 radical, R'₁ representing a hydrogen atom and R'₂ representing an alkyl radical containing up to 4 carbon atoms substituted by a heterocycle radical containing at least one nitrogen atom.

7. The compounds of formula (I) as defined in claim 6 in which R represents a radical

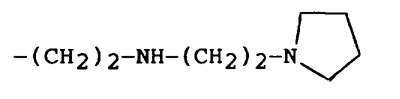

8. The compounds of formula (I) as defined in claim 2 in which R represents an alkyl radical containing up to 6 carbon atoms, substituted by an alkyloxy radical containing up to 6 carbon atoms optionally substituted by a methoxy radical.

9. The compounds of formula (I) as defined in claim 6 in which R represents a radical:

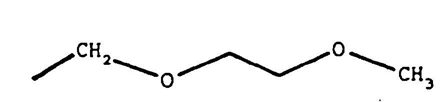

10. The compounds of formula (I) as defined in claim 2 in which R represents a heterocycle containing at least one nitrogen atom.

11. The compounds of formula (I) as defined in claim 10 in which R represents a 3-piperidinyl radical.

12. The compounds of formula (I) as defined in claim 1 in which X and X' form with the carbon atom to which they are linked a C=O group.

13. The compounds of formula (I) as defined in claim 1 in which X and X' and Y and Y' form a C=O group

14. The compounds of formula (I) as defined in any one of claims 2 to 11 in which Y and Y' form a C=NOR group, R keeping its previous meaning and notably a benzyl radical.

15. The compounds of formula (I) as defined in any one of claims 1 to 14 in which R₂ represents an alkyl radical containing 1 to 4 carbon atoms.

16. The compounds of formula (I) as defined in claim 15, in which R₂ represents a methyl radical.

17. The compounds of formula (I) as defined in any one of claims 1 to 16 in which R₃ represents a hydrogen atom (alpha or beta).

18. The compounds of formula (I) as defined in any one of claims 1 to 17 in which A represents an OH radical.

19. The compounds of formula (I) as defined in any one of claims 1 to 18 in which B represents an OH radical.

20. The compounds of formula (I) as defined in any one of claims 1 to 17, in which A and B form a cyclic 11,12-carbonate group.

21. The compounds of formula (I) as defined in any one of claims 1 to 17 in which A and B form a divalent radical 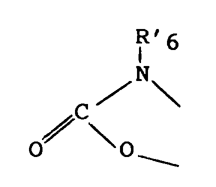 R'₆ representing a hydrogen atom or an alkyl, aralkyl or alkyloxy radical having up to 12 carbon atoms or a group: 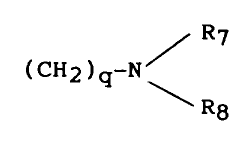 R₇ and R₈, identical or different, representing a hydrogen atom or an alkyl or aralkyl radical, containing up to 18 carbon atoms, or forming with the nitrogen atom a heterocycle as defined previously, and q represents an integer comprised between 1 and 6.

22. The compounds of formula (I) as defined in claim 21 in which R'₆ represents an aralkyl radical containing up to 12 carbon atoms.

23. The compounds of formula (I) as defined in claim 21 in which R'₆ represents a (CH₂)₄C₆H₅ radical.

24. The compounds of formula (I) as defined in any one of claims 1 to 23 in which Z represents a hydrogen atom.

25. The compound of formula (I) as defined in claim 1 the name of which follows:
- 9-[O-[2-(dimethylamino)-ethyl]-oxime] of 3-de[(2,6-dideoxy-3-C-methyl 3-O-methyl alpha-L-ribo-hexopyranosyl)-oxy]-6-O-methyl-3-oxo erythromycin.

26. The compound of formula (I) as defined in claim 1 the name of which follows:
- 11,12-dideoxy-3-de[(2,6-dideoxy-3-C-methyl 3-O-methyl alpha-L-ribo-hexopyranosyl)-oxy]-6-O-methyl-3-oxo-12,11-[oxycarbonyl-[(4-phenylbutyl)-imino]] erythromycin.

27. The compounds of formula (I) as defined in claim 1 the names of which follow:
- 9-[O-[(2-methoxy-ethoxy)-methyl]-oxime] of 3-de[(2,6-dideoxy-3-C-methyl 3-O-methyl alpha-L-ribo-hexopyranosyl)oxy]-6-O-methyl 3-oxo erythromycin,
- 3-de[(2,6-dideoxy-3-C-methyl 3-O-methyl alpha-L-ribohexopyranosyl)-oxy]-6-O-methyl 3-oxo erythromycin,
- cyclic 11,12-carbonate of 3-de[(2,6-dideoxy-3-C-methyl 3-O-methyl alpha-L-ribo-hexopyranosyl)-oxy]-6-O-methyl 3-oxo erythromycin.

28. The compounds of formula (I) as defined in claim 1, the names of which follow:
- (E) 9-O-[2-[[2-(1-pyrrolidinyl-ethyl]-amino]-ethyll-oxime of 3-de[(2,6-dideoxy-3-C-methyl 3-O-methyl alpha-L-ribohexopyranosyl)-oxy]-6-O-methyl 3-oxo erythromycin,
- (E) 9-O-(3-piperidinyl)-oxime of 3-de[(2,6-dideoxy-3-C-methyl 3-O-methyl alpha-L-ribo-hexopyranosyl)-oxy]-6-O-methyl-3-oxo erythromycin,
- (E) 9-O-12-(dimethylamino)-ethyl]-oxime of 3-del(2,6-dideoxy-3-C-methyl 3-O-methyl alpha-L-ribo-hexopyranosyl)-oxy]-11-deoxy-10,11-didehydro-6-O-methyl 3-oxo erythromycin.

29. As medicaments, the compounds of formula (I) as defined in any one of claims 1 to 25 as well as their addition salts with pharmaceutically acceptable acids.

30. As medicaments, the compounds of formula (I) as defined in any one of claims 25 to 28, as well as their addition salts with pharmaceutically acceptable acids.

31. The pharmaceutical compositions containing as active ingredient at least one medicament defined in claim 29 or 30.

32. Preparation process for compounds of formula (I) as defined in one any of claims 1 to 28, characterized in that a compound of formula (II): 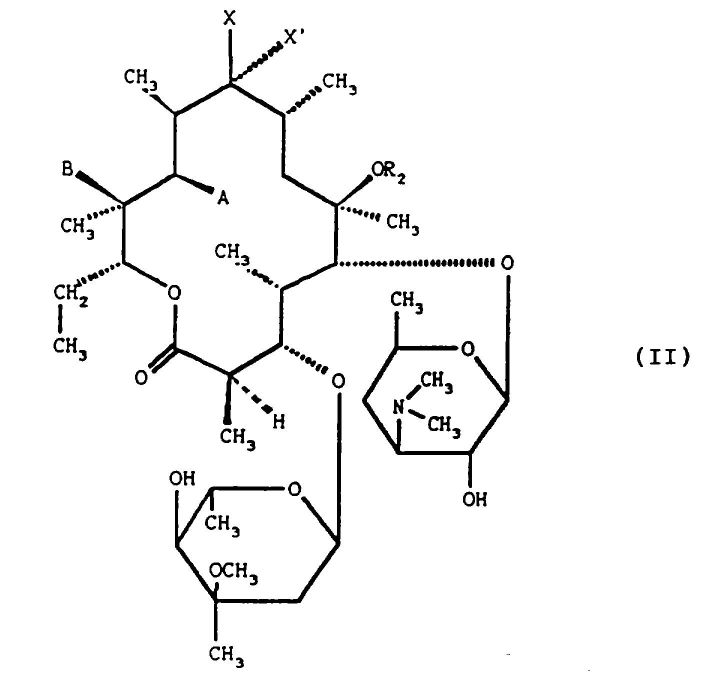 in which X, X', R₂, B and A keep their previous meaning, is subjected to the action of an acid in an aqueous medium in order to obtain the compound of formula (III): 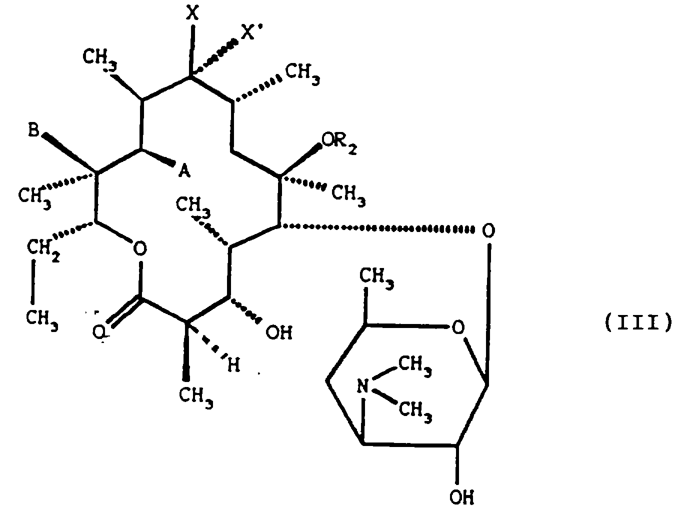 which is subjected to the action of a blocking agent of the hydroxyl function in position 2', in order to obtain a compound of formula (IV): 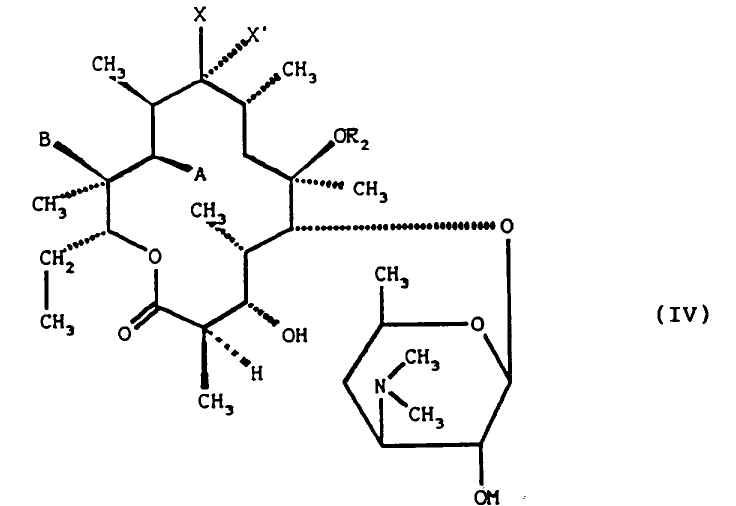 in which OM represents a blocked hydroxyl group, and the other substituents keep their previous meaning, which is subjected to the action of an oxidizing agent of the hydroxyl function in position 3, in order to obtain the compound of formula (V): 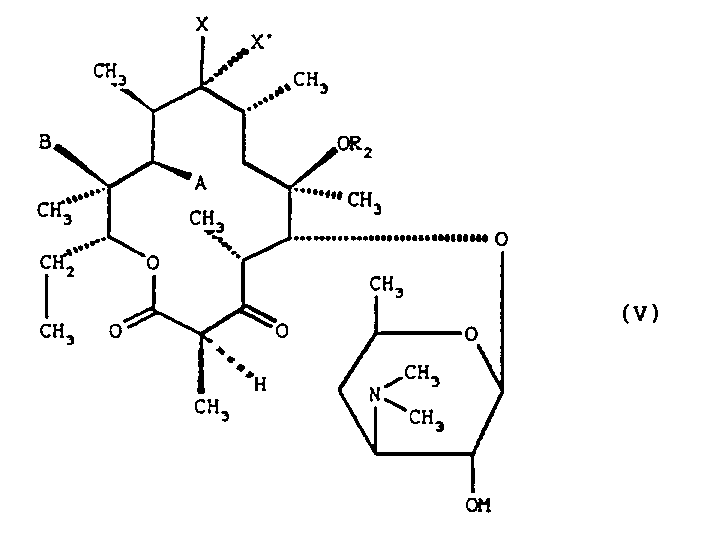 which is subjected, if desired, to the action of a reagent capable of introducing the R'₃ radical, R'₃ having the same value as R₃ with the exception of hydrogen, then either if appropriate to the action of a releasing agent of the hydroxyl function in position 2' in order to obtain the compound of formula (I_{A}): 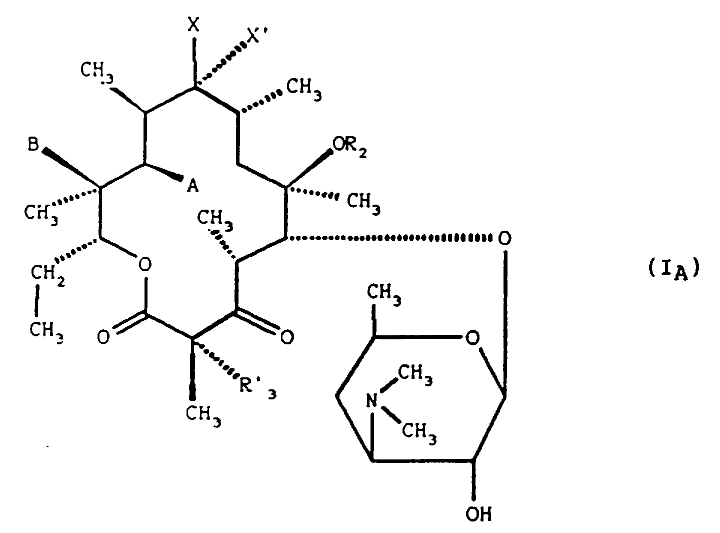 that is a compound of formula (I) in which Y and Y' form with the carbon atom to which they are linked a ketone function, then if desired, this compound of formula (I_{A}) is subjected to the action of an oximation agent of the ketone or beta-keto ester in order to obtain the desired compound of formula (I), then if desired the compound obtained is subjected to the action of an esterification agent of the hydroxyl in position 2', or first to the action of an oximation agent of the ketone or beta-keto ester function, and then if appropriate to the action of a releasing agent of the hydroxyl function in position 2' in order to obtain the desired compound of formula (I) then if desired the compound of formula (I) thus obtained is subjected to the action of an acid in order to form the salt.

33. As new industrial products, the compounds of formulae (II), (III), (IV) and (V) defined in claim 32.

34. Variant of the process of claim 32 for preparing the products of formula (I) in which X and X' form a C=NOR group, characterized in that the product of formula (IV_{A}) used in which X and X' represent the C=N-OR group is prepared from the corresponding ketone of formula (II) by the action of NH₂OR in an acid medium, in order to obtain according to the pH of the reaction the corresponding product of formula (IVA) saturated or unsaturated in position 10(11): 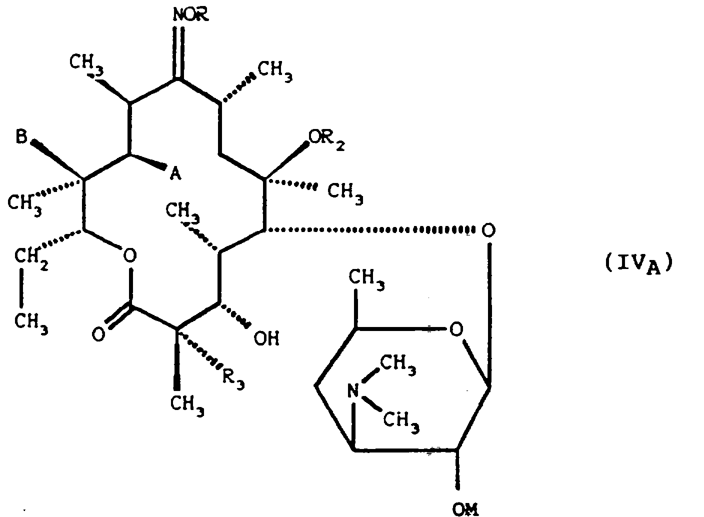 A representing an OH radical if there is no unsaturation in position 10(11) or representing a hydrogen atom if there is an unsaturation in position 10(11), R, R₂ and Z keeping the same meaning as previously.

35. Variant of the process according to claim 32, for preparing the compounds of formula (I) in which X and X' form a C=NOR group, R being defined as previously, characterized in that a compound of formula (I_{A}) in which X and X' form a keto group is subjected to the action of the compound of formula NH₂OR in order to obtain the corresponding compound of formula (I), in which X and X' form a C=NOR group and Z represents a hydrogen atom then, if appropriate, the compound is esterified or salified.

36. Process according to claim 32, characterized in that the product of formula (II) used at the start does not carry an unsaturation in position 10(11).

## Claims (Claims for the following Contracting State(s): ES)

1. Process for preparing the compounds of formula (I): 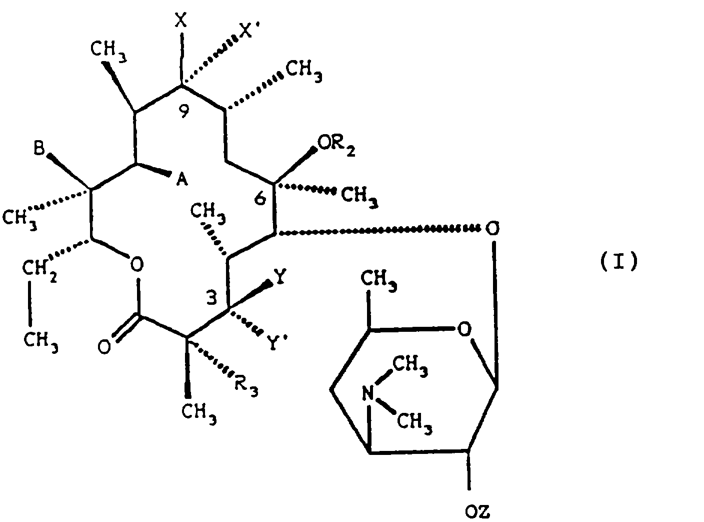 in which,
**either** X and X' form with the carbon atom to which they are linked a C=O or C=NOR group, in which R represents:
- a hydrogen atom,
- a heterocyclic radical containing at least one nitrogen atom and optionally another heteroatom, mono or bicyclic, saturated or unsaturated, aromatic or non-aromatic containing up to 12 links, optionally substituted on the nitrogen atom by an alkyl radical containing up to 4 carbon atoms,
- a linear, branched or cyclic alkyl, alkenyl or alkynyl radical containing up to 18 carbon atoms optionally substituted by one or more groups:
. hydroxyl,
. halogen,
. cyano,
. nitro,
. amidinyl,
. guanidinyl,
. heterocyclic, as defined previously,
. alkyloxy, alkenyloxy or alkynyloxy having at most 6 carbon atoms,
. alkylthio, alkenylthio or alkynylthio having at most 6 carbon atoms, the sulphur atom being optionally oxidized into sulphoxide or sulphone,
. aryl, aralkyl,
. aryloxy, aralkyloxy,
. arylthio, aralkylthio, the sulphur atom being
optionally oxidized into sulphoxide or sulphone, (each of these alkyloxy, alkenyloxy, alkynyloxy, alkylthio, alkenylthio or alkynylthio, aryl, aralkyl, aryloxy, aralkyloxy, arylthio or aralkylthio radicals being optionally substituted by one or more of the following groups: hydroxy, alkyloxy, alkylthio having 1 to 6 carbon atoms, alkenylthio, alkynylthio having up to 6 carbon atoms, amino, monoalkylamino having up to 6 carbon atoms, dialkylamino having up to 12 carbon atoms, an amidinyl, guanidinyl radical, a heterocyclic radical as defined previously, aryl, aryloxy, arylthio, aralkyl, aralkyloxy and aralkylthio radicals also being optionally substituted by the following radicals: methyl, ethyl, propyl, carbamoyl, aminomethyl, dimethylaminomethyl, aminoethyl, dimethylaminoethyl, carboxyl, methyloxycarbonyl, ethyloxycarbonyl)
. 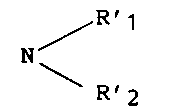 in which:
**either R'₁ and R'₂**, identical or different, represent a hydrogen atom, a linear, branched or cyclic alkyl, alkenyl or alkynyl radical, containing up to 18 carbon atoms, an aryl or aralkyl radical, each of these R'₁ and R'₂ radicals being optionally substituted by one or more of the following radicals: hydroxy, alkyloxy, alkenyloxy, alkynyloxy, alkylthio, alkenylthio or alkynylthio containing up to 8 carbon atoms, amino, monoalkylamino containing up to 4 carbon atoms, dialkylamino containing up to 8 carbon atoms, cyano, free, esterified or salified carboxyl, acyl or carbamoyl, containing up to 8 carbon atoms, by an Si(alk)₃ or Si(Oalk)₃ radical in which alk represents an alkyl radical containing up to 4 carbon atoms, by a heterocyclic radical as defined previously,
**or R'₁ and R'₂** form with the nitrogen atom to which they are linked a mono or bicyclic heterocycle radical optionally containing another heteroatom, saturated or unsaturated, aromatic or non-aromatic, containing up to 12 links;
. a quaternary ammonium group,
. 1,2-epoxyethyl or 2,2-dimethyl-1,2-epoxyethyl or a radical resulting from the opening of this group by a nucleophile reagent,
. 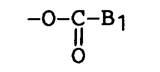 in which B₁ represents either an alkyl or alkyloxy radical having at most 6 carbon atoms, or an aryl, aralkyl, aryloxy or aralkyloxy radical,
. free or protected formyl, free, esterified or salified carboxyl, thiocyanate, acyl or carbamoyl,
. (CH₂)ₙR', R' representing the remainder of an amino acid, and n representing an integer comprised between 0 and 6, **or X** represents an 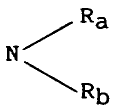 radical
- Rₐ and R_{b}, identical or different from each other, representing a hydrogen atom or a hydrocarbonated radical containing up to 18 carbon atoms, optionally containing one or more heteroatoms, optionally substituted by one or more functional groups, or by a heterocyclic radical containing at least one nitrogen atom and optionally another heteroatom chosen from oxygen, sulphur and nitrogen, mono or bicyclic, saturated or unsaturated, aromatic or non-aromatic containing up to 12 links, optionally substituted on the nitrogen atom by an alkyl radical containing up to 4 carbon atoms,
- Rₐ and R_{b} optionally being able to form with the nitrogen atom to which they are linked a heterocycle radical, containing at least one nitrogen atom and optionally another heteroatom chosen from oxygen, sulphur and nitrogen, mono or bicyclic, saturated or unsaturated, aromatic or non-aromatic, containing up to 12 links,
- Rₐ and R_{b} being able to form with radical A a 9-N 11-0 ring,
**and X'** represents a hydrogen atom,
**Y and Y'**, identical or different from X and X', have the meaning of X and X'
**B** represents a hydrogen atom or an OR₄ radical, R₄ representing a hydrogen atom, or forms with A a carbonate or carbamate radical,
**A** forms with the carbon which carries it and the carbon in position 10, a bond double,
**or A** represents an OR'4 radical, R'₄ representing a hydrogen atom, or forms with B a carbonate radical,
**or A** represents an 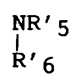 radical, 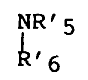 representing a C=O group forming with B a carbamate group, R'₆ representing a hydrogen atom or an alkyl, aralkyl or alkyloxy radical having up to 12 carbon atoms or a group: 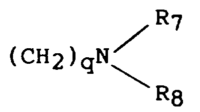 R₇ and R₈, identical or different, representing a hydrogen atom or an alkyl or aralkyl radical, containing up to 18 carbon atoms, or forming with the nitrogen atom a heterocycle as defined previously, q representing an integer comprised between 1 and 6,
**or A** represents an 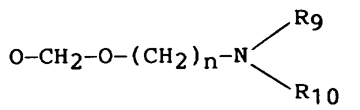 R₉ and R₁₀ representing a hydrogen atom or an alkyl radical containing up to 8 carbon atoms, or forming with the nitrogen atom a heterocycle as defined previously, n representing an integer comprised between 1 and 6,
**R₂** represents an alkyl radical containing up to 8 carbon atoms, or a CONH₂ or CONHCOR₁₁ or CONHSO₂R₁₁ in which R11 represents a hydrocarbonated radical containing up to 18 carbon atoms comprising optionally one or more heteroatoms,
**R₃** in alpha or beta position represents a hydrogen atom or an alkyl radical, containing up to 8 carbon atoms or a 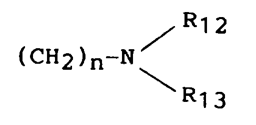 radical in which R₁₂ and R₁₃ represent a hydrogen atom, an alkyl radical containing up to 8 carbon atoms or form with the nitrogen atom a heterocycle as defined previously, n representing an integer comprised between 1 and 6 or an 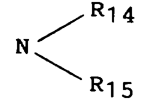 radical, R₁₄ and R₁₅, identical or different, representing a hydrogen atom or an alkyl radical containing up to 8 carbon atoms or a heteroatom or an alkyl or alkyloxy radical containing up to 8 carbon atoms,
**Z** represents a hydrogen atom or the remainder of a carboxylic acid containing up to 18 carbon atoms, the oximes that can be represented by X and X' or Y and Y' can be of syn or anti configuration, as well as the addition salts with acids of the compounds of formula (I), characterized in that a compound of formula (II): 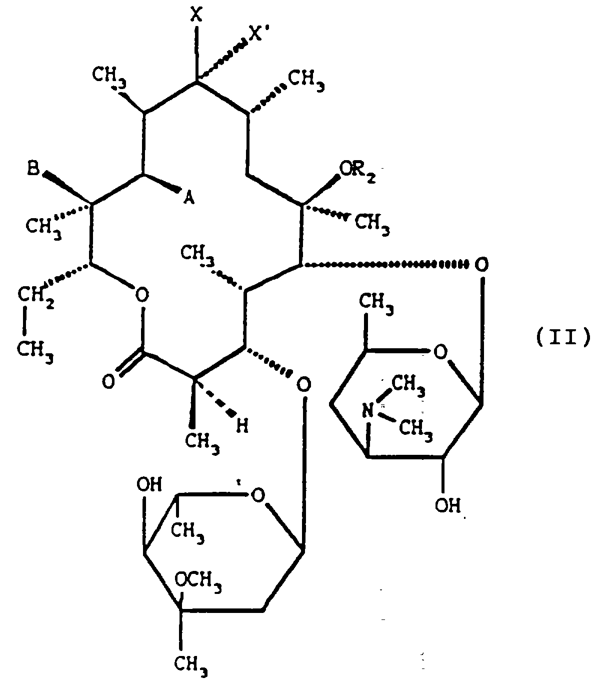 in which X, X', R₂, B and A keep their previous meaning, is subjected to the action of an acid in an aqueous medium in order to obtain the compound of formula (III): 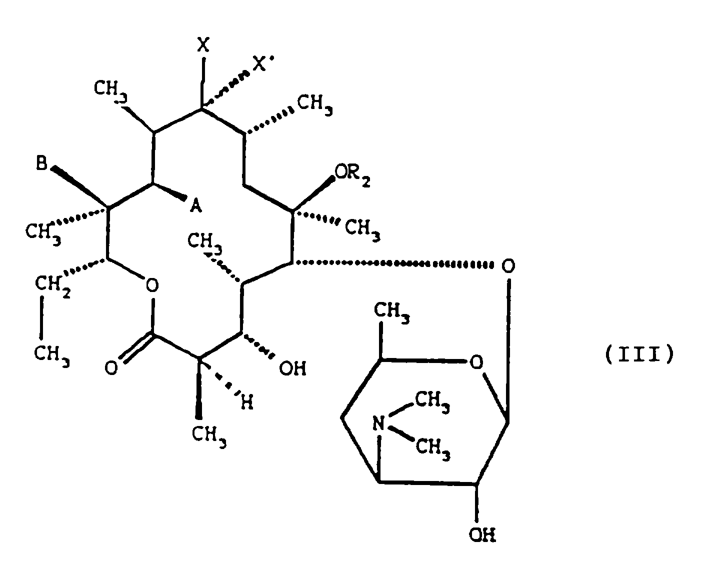 which is subjected to the action of a blocking agent of the hydroxyl function in position 2', in order to obtain a compound of formula (IV): 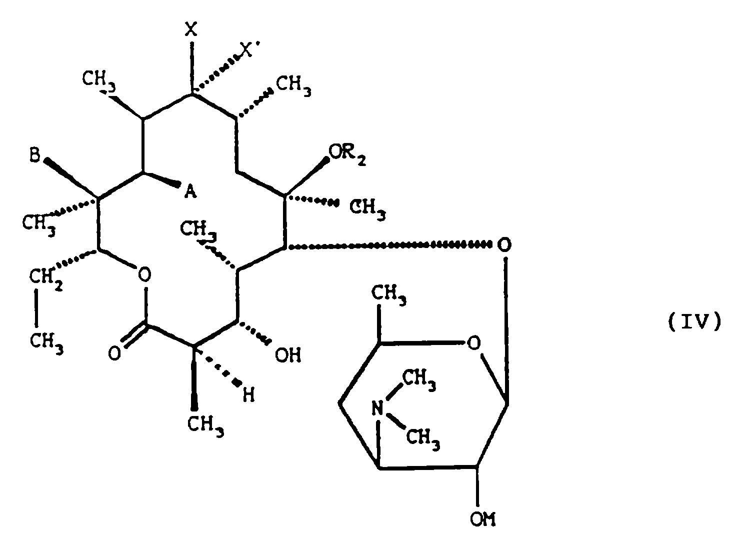 in which OM represents a blocked hydroxyl group, and the other substituents keep their previous meaning, which is subjected to the action of an oxidizing agent of the hydroxyl function in position 3, in order to obtain the compound of formula (V): 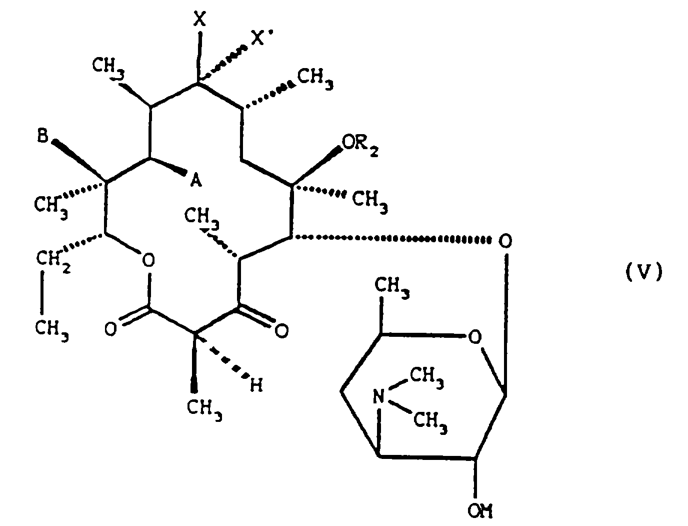 which is subjected, if desired, to the action of a reagent capable of introducing the R'₃ radical, R'₃ having the same value as R₃ with the exception of hydrogen, then either if appropriate to the action of a releasing agent of the hydroxyl function in position 2' in order to obtain the compound of formula (I_{A}): 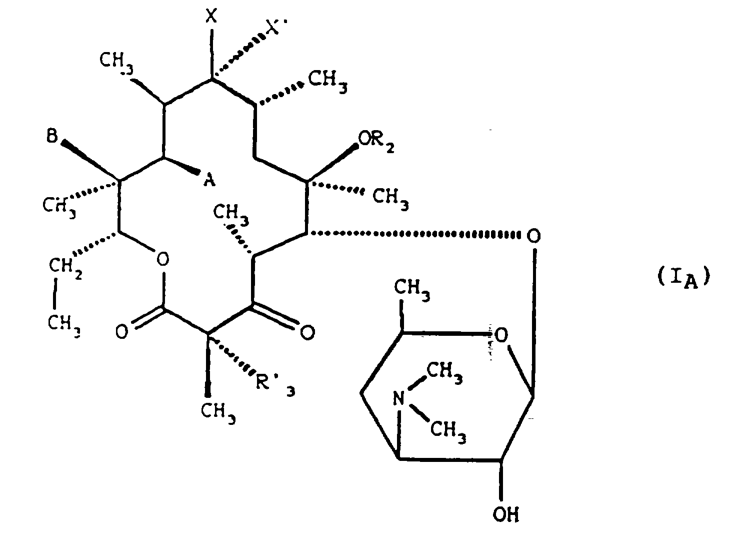 that is a compound of formula (I) in which Y and Y' form with the carbon atom to which they are linked a ketone function, then if desired, this compound of formula (I_{A}) is subjected to the action of an oximation agent of the ketone or beta-keto ester in order to obtain the desired compound of formula (I), then if desired the compound obtained is subjected to the action of an esterification agent of the hydroxyl in position 2', or first to the action of an oximation agent of the ketone or beta-keto ester function, and then if appropriate to the action of a releasing agent of the hydroxyl function in position 2' in order to obtain the desired compound of formula (I) then if desired the compound of formula (I) thus obtained is subjected to the action of an acid in order to form the salt.

2. Process according to claim 1 for preparing the products of formula (I) in which X and X' together form a C=NOR group, characterized in that the product of formula (IV_{A}) used in which X and X' represent the C=N-OR group is prepared from the corresponding ketone of formula (II) by the action of NH₂OR in an acid medium, in order to obtain according to the pH of the reaction the corresponding product of formula (IV_{A}) saturated or unsaturated in position 10(11): 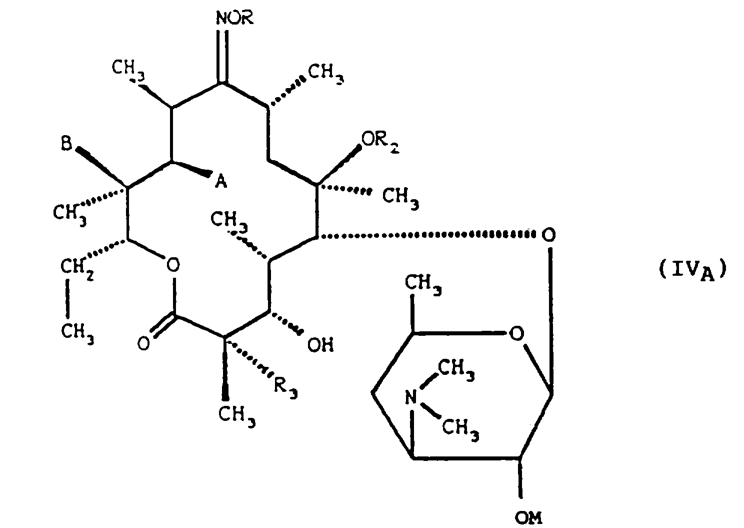 A representing an OH radical if there is no unsaturation in position 10(11) or representing a hydrogen atom if there is an unsaturation in position 10(11), R, R₂ and Z keeping the same meaning as in claim 1.

3. Process according to claim 1, for preparing the compounds of formula (I) in which X and X' form a C=NOR group, R being defined as previously, characterized in that a compound of formula (I_{A}) in which X and X' together form a keto group is subjected to the action of the compound of formula NH₂OR in order to obtain the corresponding compound of formula (I), in which X and X' form a C=NOR group and Z represents a hydrogen atom then, if appropriate, the compound is esterified or salified.

4. Process according to claim 1, characterized in that the product of formula (II) used at the start does not carry an unsaturation in position 10(11).

5. Process according to any one of claims 1 to 4, characterized in that the oximation of the ketone function can be carried out in a single stage by means of an RONH₂ hydroxylamine carrying the desired R substituent or by means of an H₂N-O-(CH₂)ₙ-Hal hydroxylamine in order to obtain a compound of formula (I) in which 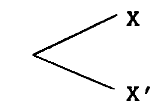 represents the =N-O-(CH₂)ₙ-Hal group, which is subjected if desired to the action of an amine of formula 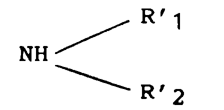 in which R'₁ and R'₂ have the meaning already indicated, in order to obtain a compound of formula (I) in which 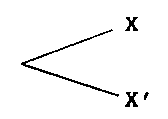 represents the 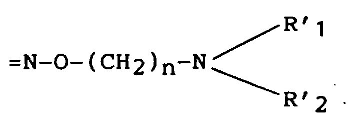 group which if appropriate is converted, by means for example of an alkylation, acylation, reduction agent in order to obtain the desired compound of formula (I).

6. Process according to any one of claims 1 to 5, characterized in that to prepare the products of formula (I) in which X and X' together represent a C=NOR group, an NH₂OR oximation agent is used in which R has the meaning indicated in claim 1.

7. Process according to any one of claims 1 to 6, characterized in that an NH₂OR oximation agent is used in which R represents an alkyl radical containing up to 6 carbon atoms substituted by an 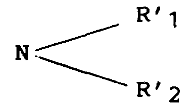 radical, R'₁ and R'₂ keeping the same meaning as in claim 1.

8. Process according to any one of claims 1 to 6, characterized in that an NH₂OR oximation agent is used in which the R radical is a 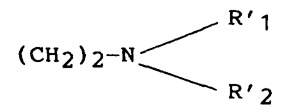 radical, R'₁ and R'₂ representing an alkyl radical containing up to 4 carbon atoms.

9. Process according to claim 8 characterized in that an NH₂OR oximation agent is used in which R represents a (CH₂)₂-N(CH₃)₂ radical.

10. Process according to any one of claims 1 to 6, characterized in that an NH_{R}OR oximation agent is used in which the R radical is a 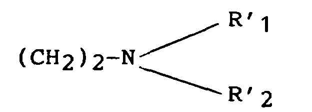 radical, R'₁ representing a hydrogen atom and R'₂ representing an alkyl radical containing up to 4 carbon atoms substituted by a heterocycle radical containing at least one nitrogen atom.

11. Process according to claim 10, characterized in that an NH₂OR oximation agent is used in which R represents a 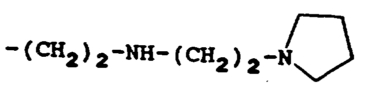 radical.

12. Process according to any one of claims 1 to 6, characterized in that an NH₂OR oximation agent is used in which R represents an alkyl radical containing up to 6 carbon atoms, substituted by an alkyloxy radical containing up to 6 carbon atoms optionally substituted by a methoxy radical.

13. Process according to claim 12, characterized in that an NH₂OR oximation agent is used in which R represents a radical:

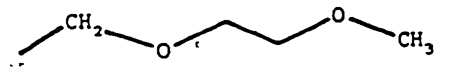

14. Process according to any one of claims 1 to 6, characterized in that an NH₂OR oximation agent is used in which R represents a heterocycle containing at least one nitrogen atom.

15. Process according to claim 14, characterized in that an NH₂OR oximation agent is used in which R represents a 3-piperidinyl radical.

16. Process according to claim 1, characterized in that to prepare a product of formula (I_{A}) in which X and X' together represent a ketone function, a product of formula (II) is used at the start in which X and X' have the meaning indicated previously.

17. Process according to any one of claims 2 to 14, characterized in that to prepare a product of formula (I) in which Y and Y' together form a C=NOR group, an NH₂OR oximation agent is used in which R has the meaning indicated in claim 1.

18. Process according to claim 17, characterized in that an NH₂OR oximation agent is used in which R represents a benzyl radical.

19. Process according to any one of claims 1 to 18, characterized in that a product of formula (II) is used at the start in which R₂ represents an alkyl radical containing 1 to 4 carbon atoms.

20. Process according to claim 19, characterized in that a product of formula (II) is used at the start in which R₂ represents a methyl radical.

21. Process according to any one of claims 1 to 20, characterized in that the product of formula (V) is subjected either if appropriate to the action of a releasing agent of the hydroxyl function in position 2' in order to obtain the compound of formula (I_{A}) in which R'₃ represents a hydrogen atom, which if desired is subjected to the action of an oximation agent of the ketone or beta-keto ester, then if desired to the action of an esterification agent of the hydroxyl in position 2', or firstly to the action of an oximation agent then if appropriate to the action of a releasing agent of the hydroxyl function in position 2' in order to obtain a compound of formula (I) in which R₃ represents a hydrogen atom, then if desired, the compound of formula (I) thus obtained is subjected to the action of an acid in order to form the salt.

22. Process according to any one of claims 1 to 21 characterized in that a product of formula (II) is used at the start in which A represents an OH radical.

23. Process according to any one of claims 1 to 22, characterized in that a product of formula (II) is used at the start in which B represents an OH radical.

24. Process according to any one of claims 1 to 21, characterized in that a product of formula (II) is used at the start in which A and B together form a cyclic 11,12 carbonate group.

25. Process according to any one of claims 1 to 21, characterized in that a product of formula (I) is used at the start in which A and B together form a divalent radical 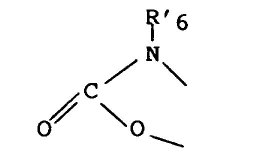 R'₆ representing a hydrogen atom or an alkyl, aralkyl or alkyloxy radical having up to 12 carbon atoms or a group: 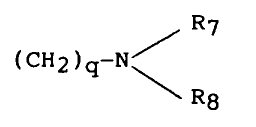 R₇ and R₈, identical or different, representing a hydrogen atom or an alkyl or aralkyl radical, containing up to 18 carbon atoms, or forming with the nitrogen atom a heterocycle as defined previously, and q represents an integer comprised between 1 and 6.

26. Process according to claim 25, characterized in that a product of formula (II) is used at the start in which A and B together form a divalent radical 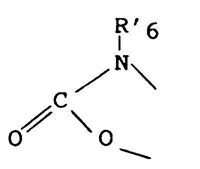 in which R'₆ represents an aralkyl radical containing up to 12 carbon atoms.

27. Process according to claim 26, characterized in that a product of formula (II) is used at the start in which A and B together form a divalent radical 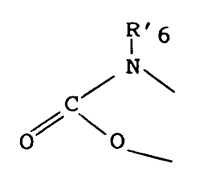 in which R'₆ represents a (CH₂)₄C₆H₅ radical.

28. Process according to any one of claims 1 to 27, characterized in that the compound of formula (V) or (I_{A}) is subjected to the action of a releasing agent of the hydroxyl function in position 2' in order to obtain a product of formula (I) in which Z represents a hydrogen atom.

## Claims (Claims for the following Contracting State(s): GR)

1. Process for preparing the compounds of formula (I): 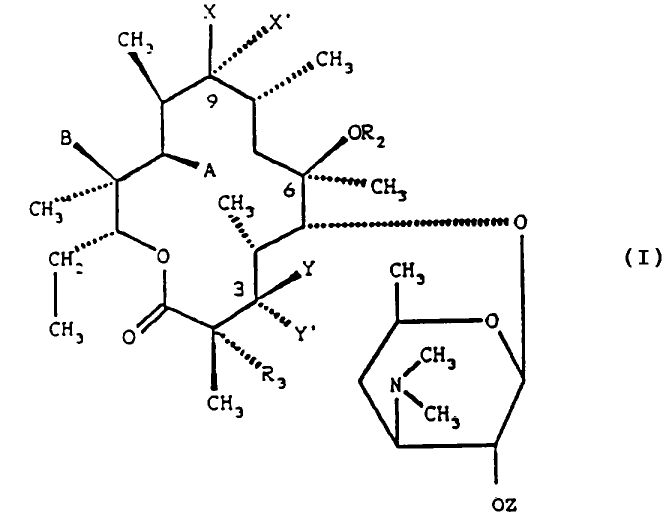 in which,
**either** X and X' form with the carbon atom to which they are linked a C=O or C=NOR group, in which R represents:
- a hydrogen atom,
- a heterocyclic radical containing at least one nitrogen atom and optionally another heteroatom, mono or bicyclic, saturated or unsaturated, aromatic or non-aromatic containing up to 12 links, optionally substituted on the nitrogen atom by an alkyl radical containing up to 4 carbon atoms,
- a linear, branched or cyclic alkyl, alkenyl or alkynyl radical containing up to 18 carbon atoms Optionally substituted by one or more groups:
. hydroxyl,
. halogen,
. cyano,
. nitro,
. amidinyl,
. guanidinyl,
. heterocyclic, as defined previously,
. alkyloxy, alkenyloxy or alkynyloxy having at most 6 carbon atoms,
. alkylthio, alkenylthio or alkynylthio having at most 6 carbon atoms, the sulphur atom being optionally oxidized into sulphoxide or sulphone,
. aryl, aralkyl,
. aryloxy, aralkyloxy,
. arylthio, aralkylthio, the sulphur atom being optionally oxidized into sulphoxide or sulphone,
(each of these alkyloxy, alkenyloxy, alkynyloxy, alkylthio, alkenylthio or alkynylthio, aryl, aralkyl, aryloxy, aralkyloxy, arylthio or aralkylthio radicals being optionally substituted by one or more of the following groups: hydroxy, alkyloxy, alkylthio having 1 to 6 carbon atoms, alkenylthio, alkynylthio having up to 6 carbon atoms, amino, monoalkylamino having up to 6 carbon atoms, dialkylamino having up to 12 carbon atoms, an amidinyl, guanidinyl radical, a heterocyclic radical as defined previously, aryl, aryloxy, arylthio, aralkyl, aralkyloxy and aralkylthio radicals also being optionally substituted by the following radicals: methyl, ethyl, propyl, carbamoyl, aminomethyl, dimethylaminomethyl, aminoethyl, dimethylaminoethyl, carboxyl, methyloxycarbonyl, ethyloxycarbonyl)
. 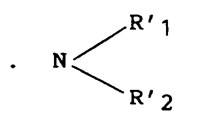 in which:
**either R'₁ and R'₂**, identical or different, represent a hydrogen atom, a linear, branched or cyclic alkyl, alkenyl or alkynyl radical, containing up to 18 carbon atoms, an aryl or aralkyl radical, each of these R'₁ and R'₂ radicals being optionally substituted by one or more of the following radicals: hydroxy, alkyloxy, alkenyloxy, alkynyloxy, alkylthio, alkenylthio or alkynylthio containing up to 8 carbon atoms, amino, monoalkylamino containing up to 4 carbon atoms, dialkylamino containing up to 8 carbon atoms, cyano, free, esterified or salified carboxyl, acyl or carbamoyl, containing up to 8 carbon atoms, by an Si(alk)₃ or Si(Oalk)₃ radical in which alk represents an alkyl radical containing up to 4 carbon atoms, by a heterocyclic radical as defined previously,
**or R'₁ and R'₂** form with the nitrogen atom to which they are linked a mono or bicyclic heterocycle radical optionally containing another heteroatom, saturated or unsaturated, aromatic or non-aromatic, containing up to 12 links;
. a quaternary ammonium group,
. 1,2-epoxyethyl or 2,2-dimethyl-1,2-epoxyethyl or a radical resulting from the opening of this group by a nucleophile reagent,
. 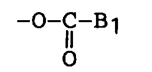 in which B₁ represents either an alkyl or alkyloxy radical having at most 6 carbon atoms, or an aryl, aralkyl, aryloxy or aralkyloxy radical,
. free or protected formyl, free, esterified or salified carboxyl, thiocyanate, acyl or carbamoyl,
. (CH₂)ₙR', R' representing the remainder of an amino acid, and n representing an integer comprised between 0 and 6, **or X** represents an 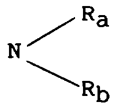 radical
- Rₐ and R_{b}, identical or different from each other, representing a hydrogen atom or a hydrocarbonated radical containing up to 18 carbon atoms, optionally containing one or more heteroatoms, optionally substituted by one or more functional groups, or by a heterocyclic radical containing at least one nitrogen atom and optionally another heteroatom chosen from oxygen, sulphur and nitrogen, mono or bicyclic, saturated or unsaturated, aromatic or non-aromatic containing up to 12 links, optionally substituted on the nitrogen atom by an alkyl radical containing up to 4 carbon atoms,
- Rₐ and R_{b} optionally being able to form with the nitrogen atom to which they are linked a heterocycle radical, containing at least one nitrogen atom and optionally another heteroatom chosen from oxygen, sulphur and nitrogen, mono or bicyclic, saturated or unsaturated, aromatic or non-aromatic, containing up to 12 links,
- Rₐ and R_{b} being able to form with radical A a 9-N 11-0 ring,
**and X'** represents a hydrogen atom,
**Y and Y'**, identical or different from X and X', have the meaning of X and X'
**B** represents a hydrogen atom or an OR₄ radical, R₄ representing a hydrogen atom, or forms with A a carbonate or carbamate radical,
**A** forms with the carbon which carries it and the carbon in position 10, a bond double,
**or A** represents an OR'₄ radical, R'₄ representing a hydrogen atom, or forms with B a carbonate radical,
**or A** represents an 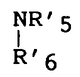 radical, 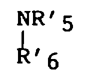 representing a C=O group forming with B a carbamate group, R'₆ representing a hydrogen atom or an alkyl, aralkyl or alkyloxy radical having up to 12 carbon atoms or a group: 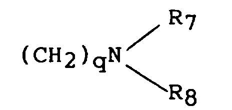 R₇ and R₈, identical or different, representing a hydrogen atom or an alkyl or aralkyl radical, containing up to 18 carbon atoms, or forming with the nitrogen atom a heterocycle as defined previously, q representing an integer comprised between 1 and 6,
**or A** represents an 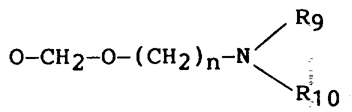 R₉ and R₁₀ representing a hydrogen atom or an alkyl radical containing up to 8 carbon atoms, or forming with the nitrogen atom a heterocycle as defined previously, n representing an integer comprised between 1 and 6,
**R₂** represents an alkyl radical containing up to 8 carbon atoms, or a CONH₂ or CONHCOR₁₁ or CONHSO₂R₁₁ in which R₁₁ represents a hydrocarbonated radical containing up to 18 carbon atoms comprising optionally one or more heteroatoms,
**R₃** in alpha or beta position represents a hydrogen atom or an alkyl radical, containing up to 8 carbon atoms or a 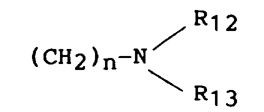 radical in which R₁₂ and R₁₃ represent a hydrogen atom, an alkyl radical containing up to 8 carbon atoms or form with the nitrogen atom a heterocycle as defined previously, n representing an integer comprised between 1 and 6 or an 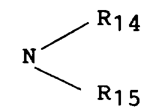 radical, R₁₄ and R₁₅, identical or different, representing a hydrogen atom or an alkyl radical containing up to 8 carbon atoms or a heteroatom or an alkyl or alkyloxy radical containing up to 8 carbon atoms,
**Z** represents a hydrogen atom or the remainder of a carboxylic acid containing up to 18 carbon atoms, the oximes that can be represented by X and X' or Y and Y' can be of syn or anti configuration, as well as the addition salts with acids of the compounds of formula (I), characterized in that a compound of formula (II): 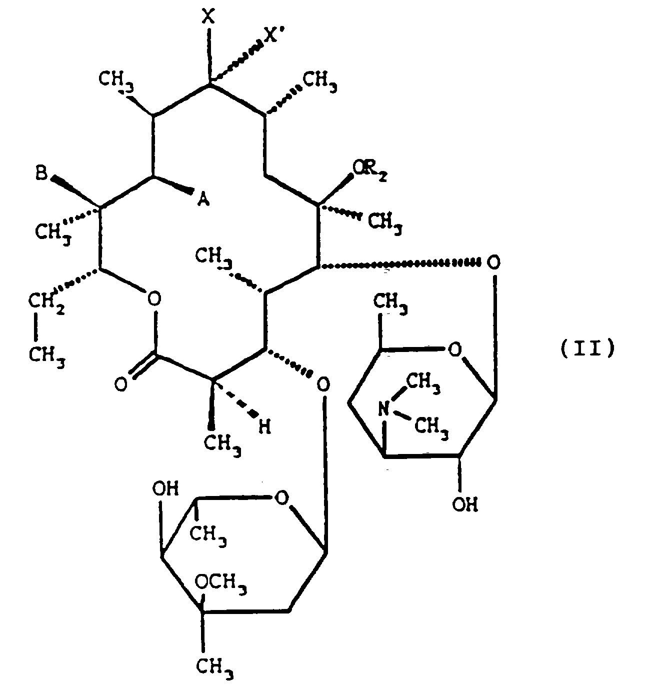 in which X, X', R₂, B and A keep their previous meaning, is subjected to the action of an acid in an aqueous medium in order to obtain the compound of formula (III): 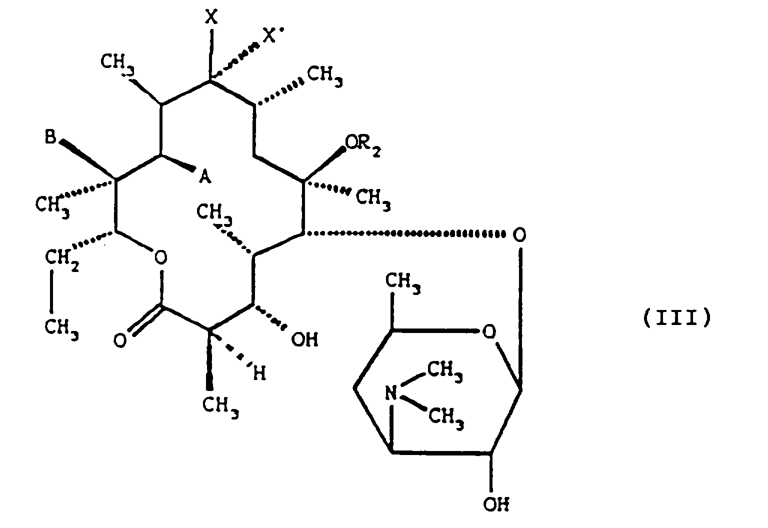 which is subjected to the action of a blocking agent of the hydroxyl function in position 2', in order to obtain a compound of formula (IV): 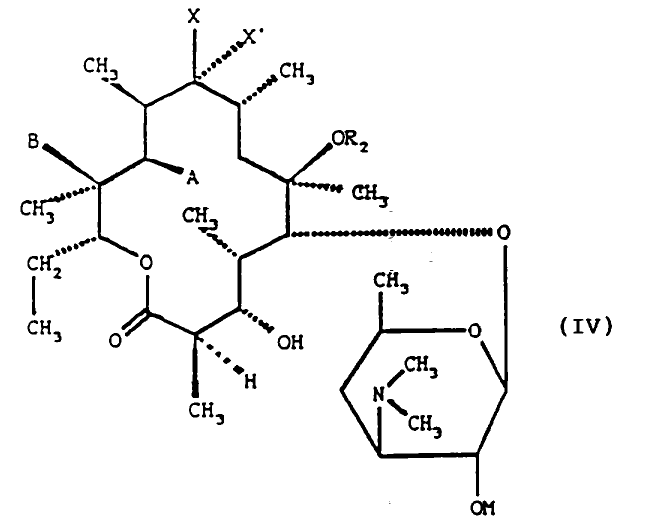 in which OM represents a blocked hydroxyl group, and the other substituents keep their previous meaning, which is subjected to the action of an oxidizing agent of the hydroxyl function in position 3, in order to obtain the compound of formula (V): 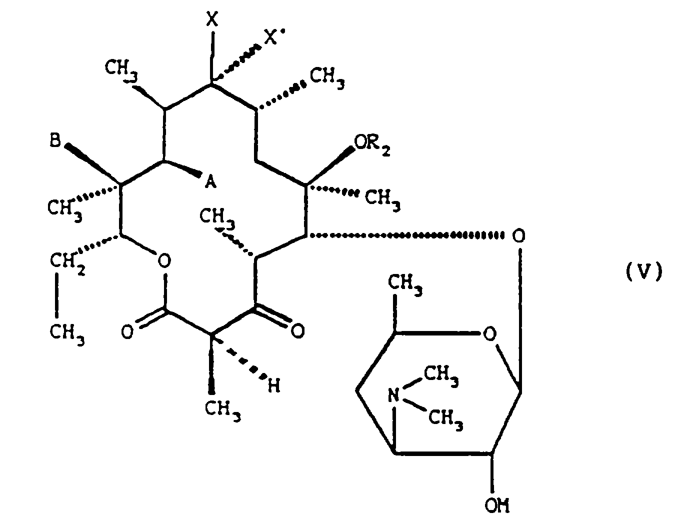 which is subjected, if desired, to the action of a reagent capable of introducing the R'₃ radical, R'₃ having the same value as R₃ with the exception of hydrogen, then either if appropriate to the action of a releasing agent of the hydroxyl function in position 2' in order to obtain the compound of formula (I_{A}): 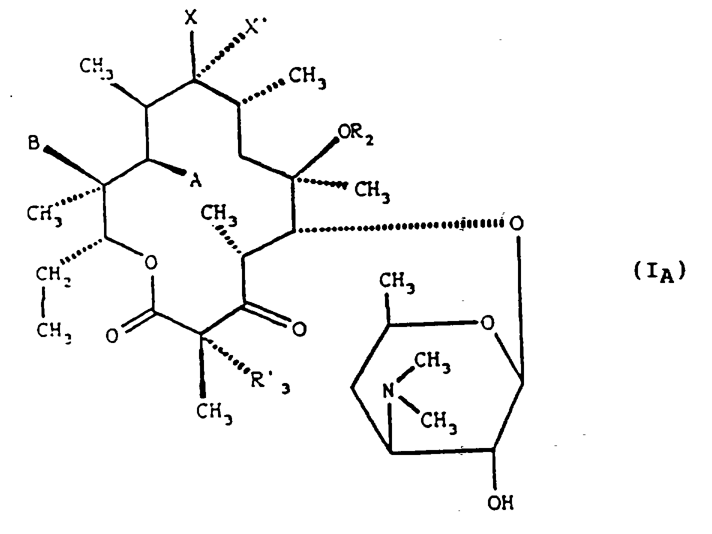 that is a compound of formula (I) in which Y and Y' form with the carbon atom to which they are linked a ketone function, then if desired, this compound of formula (I_{A}) is subjected to the action of an oximation agent of the ketone or beta-keto ester in order to obtain the desired compound of formula (I), then if desired the compound obtained is subjected to the action of an esterification agent of the hydroxyl in position 2', or first to the action of an oximation agent of the ketone or beta-keto ester function, and then if appropriate to the action of a releasing agent of the hydroxyl function in position 2' in order to obtain the desired compound of formula (I) then if desired the compound of formula (I) thus obtained is subjected to the action of an acid in order to form the salt.

2. Process according to claim 1 for preparing the products of formula (I) in which X and X' together form a C=NOR group, characterized in that the product of formula (IV_{A}) used in which X and X' represent the C=N-OR group is prepared from the corresponding ketone of formula (II) by the action of NH₂OR in an acid medium, in order to obtain according to the pH of the reaction the corresponding product of formula (IVA) saturated or unsaturated in position 10(11): 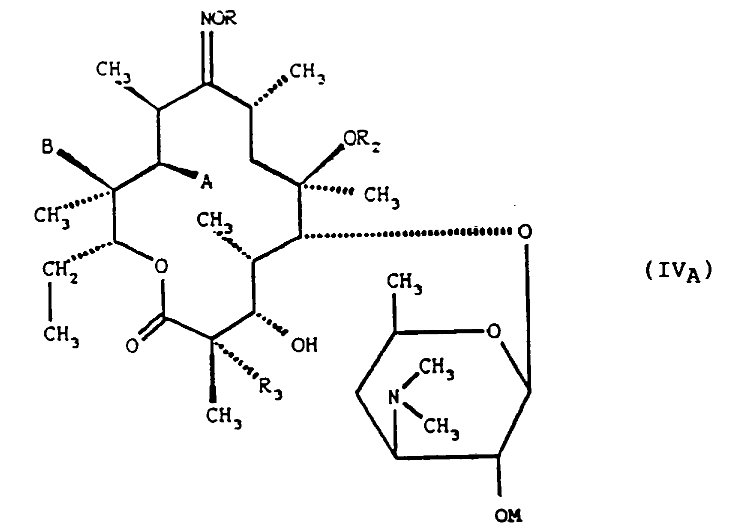 A representing an OH radical if there is no unsaturation in position 10(11) or representing a hydrogen atom if there is an unsaturation in position 10(11), R, R₂ and Z keeping the same meaning as in claim 1.

3. Process according to claim 1, for preparing the compounds of formula (I) in which X and X' form a C=NOR group, R being defined as previously, characterized in that a compound of formula (I_{A}) in which X and X' together form a keto group is subjected to the action of the compound of formula NH₂OR in order to obtain the corresponding compound of formula (I), in which X and X' form a C=NOR group and Z represents a hydrogen atom then, if appropriate, the compound is esterified or salified.

4. Process according to claim 1, characterized in that the product of formula (II) used at the start does not carry an unsaturation in position 10(11).

5. Process according to any one of claims 1 to 4, characterized in that the oximation of the ketone function can be carried out in a single stage by means of an RONH₂ hydroxylamine carrying the desired R substituent or by means of an H₂N-O-(CH₂)ₙ-Hal hydroxylamine in order to obtain a compound of formula (I) in which 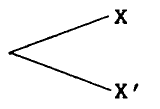 represents the =N-O-(CH₂)ₙ-Hal group, which is subjected if desired to the action of an amine of formula 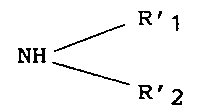 in which R'₁ and R'₂ have the meaning already indicated, in order to obtain a compound of formula (I) in which 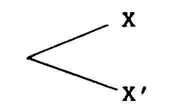 represents the 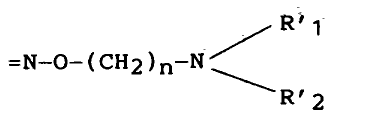 group which if appropriate is converted, by means for example of an alkylation, acylation, reduction agent in order to obtain the desired compound of formula (I).

6. Process according to any one of claims 1 to 5, characterized in that to prepare the products of formula (I) in which X and X' together represent a C=NOR group, an NH₂OR oximation agent is used in which R has the meaning indicated in claim 1.

7. Process according to any one of claims 1 to 6, characterized in that an NH₂OR oximation agent is used in which R represents an alkyl radical containing up to 6 carbon atoms substituted by an 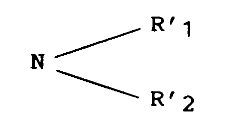 radical, R'₁ and R'₂ keeping the same meaning as in claim 1.

8. Process according to any one of claims 1 to 6, characterized in that an NH₂OR oximation agent is used in which the R radical is a 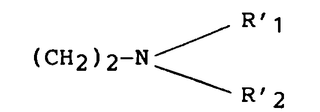 radical, R'₁ and R'₂ representing an alkyl radical containing up to 4 carbon atoms.

9. Process according to claim 8 characterized in that an NH₂OR oximation agent is used in which R represents a (CH₂)₂-N(CH₃)₂ radical.

10. Process according to any one of claims 1 to 6, characterized in that an NH_{R}OR oximation agent is used in which the R radical is a 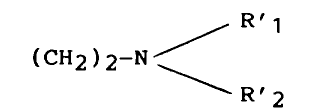 radical, R'₁ representing a hydrogen atom and R'₂ representing an alkyl radical containing up to 4 carbon atoms substituted by a heterocycle radical containing at least one nitrogen atom.

11. Process according to claim 10, characterized in that an NH₂OR oximation agent is used in which R represents a 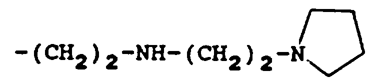 radical.

12. Process according to any one of claims 1 to 6, characterized in that an NH₂OR oximation agent is used in which R represents an alkyl radical containing up to 6 carbon atoms, substituted by an alkyloxy radical containing up to 6 carbon atoms optionally substituted by a methoxy radical.

13. Process according to claim 12, characterized in that an NH₂OR oximation agent is used in which R represents a radical:

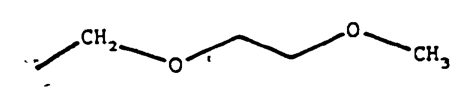

14. Process according to any one of claims 1 to 6, characterized in that an NH₂OR oximation agent is used in which R represents a heterocycle containing at least one nitrogen atom.

15. Process according to claim 14, characterized in that an NH₂OR oximation agent is used in which R represents a 3-piperidinyl radical.

16. Process according to claim 1, characterized in that to prepare a product of formula (I_{A}) in which X and X' together represent a ketone function, a product of formula (II) is used at the start in which X and X' have the meaning indicated previously.

17. Process according to any one of claims 2 to 14, characterized in that to prepare a product of formula (I) in which Y and Y' together form a C=NOR group, an NH₂OR oximation agent is used in which R has the meaning indicated in claim 1.

18. Process according to claim 17, characterized in that an NH₂OR oximation agent is used in which R represents a benzyl radical.

19. Process according to any one of claims 1 to 18, characterized in that a product of formula (II) is used at the start in which R₂ represents an alkyl radical containing 1 to 4 carbon atoms.

20. Process according to claim 19, characterized in that a product of formula (II) is used at the start in which R₂ represents a methyl radical.

21. Process according to any one of claims 1 to 20, characterized in that the product of formula (V) is subjected either if appropriate to the action of a releasing agent of the hydroxyl function in position 2' in order to obtain the compound of formula (I_{A}) in which R'₃ represents a hydrogen atom, which if desired is subjected to the action of an oximation agent of the ketone or beta-keto ester, then if desired to the action of an esterification agent of the hydroxyl in position 2', or firstly to the action of an oximation agent then if appropriate to the action of a releasing agent of the hydroxyl function in position 2' in order to obtain a compound of formula (I) in which R₃ represents a hydrogen atom, then if desired, the compound of formula (I) thus obtained is subjected to the action of an acid in order to form the salt.

22. Process according to any one of claims 1 to 21 characterized in that a product of formula (II) is used at the start in which A represents an OH radical.

23. Process according to any one of claims 1 to 22, characterized in that a product of formula (II) is used at the start in which B represents an OH radical.

24. Process according to any one of claims 1 to 21, characterized in that a product of formula (II) is used at the start in which A and B together form a cyclic 11,12 carbonate group.

25. Process according to any one of claims 1 to 21, characterized in that a product of formula (I) is used at the start in which A and B together form a divalent radical 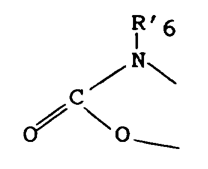 R'₆ representing a hydrogen atom or an alkyl, aralkyl or alkyloxy radical having up to 12 carbon atoms or a group: 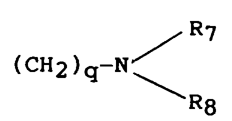 R₇ and R₈, identical or different, representing a hydrogen atom or an alkyl or aralkyl radical, containing up to 18 carbon atoms, or forming with the nitrogen atom a heterocycle as defined previously, and q represents an integer comprised between 1 and 6.

26. Process according to claim 25, characterized in that a product of formula (II) is used at the start in which A and B together form a divalent radical 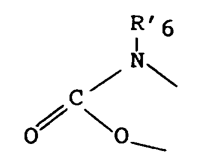 in which R'₆ represents an aralkyl radical containing up to 12 carbon atoms.

27. Process according to claim 26, characterized in that a product of formula (II) is used at the start in which A and B together form a divalent radical 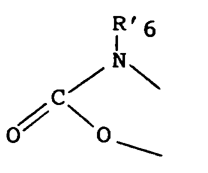 in which R'₆ represents a (CH₂)₄C₆H₅ radical.

28. Process according to any one of claims 1 to 27, characterized in that the compound of formula (V) or (I_{A}) is subjected to the action of a releasing agent of the hydroxyl function in position 2' in order to obtain a product of formula (I) in which Z represents a hydrogen atom.

29. Process according to claim 1, characterized in that the product of formula (II) and the reagents used are chosen in such a way as to prepare 9-[O-[2-(dimethylamino) ethyl] oxime] of 3-de[(2,6-dideoxy 3-C-methyl 3-O-methyl alpha-L-ribo-hexopyranosyl) oxy] 6-O-methyl 3-oxo erythromycin.

30. Process according to claim 1, characterized in that the product of formula (II) and the reagents used are chosen in such a way as to prepare 11,12-dideoxy-3-de[(2,6-dideoxy 3-C-methyl 3-O-methyl alpha-L-ribohexopyranosyl) oxy] 6-O-methyl 3-oxo 12,11-[oxycarbonyl[(4-phenylbutyl) imino]] erythromycin.

31. Process according to claim 1, characterized in that the product of formula (II) and the reagents used are chosen in such a way as to prepare any one of the compounds of formula (I) of which the names follow:
- 9-[0-[(2-methoxy-ethoxy)-methyl]-oxime] of 3-de[(2,6-dideoxy-3-C-methyl 3-O-methyl alpha-L-ribo-hexopyranosyl)oxy]-6-O-methyl 3-oxo erythromycin,
- 3-de[(2,6-dideoxy-3-C-methyl 3-O-methyl alpha-L-ribohexopyranosyl)-oxy]-6-O-methyl 3-oxo erythromycin,
- cyclic 11,12-carbonate of 3-de[(2,6-dideoxy-3-C-methyl 3-O-methyl alpha-L-ribo-hexopyranosyl)-oxy]-6-0-methyl 3-oxo erythromycin.

32. Process according to claim 1, characterized in that the product of formula (II) and the reagents used are chosen in such a way as to prepare any one of the compounds of formula (I) of which the names follow:
- (E) 9-O-[2-[[2-(1-pyrrolidinyl-ethyl]-amino)-ethyl]-oxime of 3-de[(2,6-dideoxy-3-C-methyl 3-O-methyl alpha-L-ribohexopyranosyl)-oxy]-6-O-methyl 3-oxo erythromycin,
- (E) 9-O-(3-piperidinyl)-oxime of 3-de[(2,6-dideoxy-3-C-methyl 3-O-methyl alpha-L-ribo-hexopyranosyl)-oxy]-6-O-methyl-3-oxo erythromycin,
- (E) 9-O-[2-(dimethylamino)-ethyl]-oxime of 3-de[(2,6-dideoxy-3-C-methyl 3-O-methyl alpha-L-ribo-hexopyranosyl)-oxy]-11-deoxy-10,11-didehydro-6-O-methyl 3-oxo erythromycin.

33. Preparation process for pharmaceutical compositions, characterized in that at least one of the derivatives of formula (I) as defined in claim 1 or at least one of their addition salts with pharmaceutically acceptable acids is used as active ingredient in a form intended for this use.

34. Preparation process for pharmaceutical compositions, characterized in that at least one of the derivatives of formula (I) as defined in claims 2 to 28 or at least one of their addition salts with pharmaceutically acceptable acids is used as active ingredient in a form intended for this use.

35. Preparation process for pharmaceutical compositions, characterized in that at least one of the derivatives of formula (I) as defined in claims 29 to 32 or at least one of their addition salts with pharmaceutically acceptable acids is used as active ingredient in a form intended for this use.

36. As new industrial products, the compounds of formulae (IV) and (V) defined in claim 1.
